# EUROPEAN PATENT APPLICATION

(11) **EP 3 827 844 A1**
(43) Date of publication of application: **02.06.2021**
(21) Application number: 20199101.5
(22) Date of filing: 14.05.2014
(51) Int. Cl.: A61K 39/395, A61K 47/68, A61K 31/277, C07K 16/30, A61P 35/00

(54) **COMBINATION THERAPIES WITH PSMA LIGAND CONJUGATES**

(30) Priority: 18.10.2013 US 201361893145 P; 13.11.2013 JP 2013235506; 13.11.2013 US 201361903589 P
(62) Divisional of application: 14734570.6
(71) Applicant: Psma Development Company, L.L.C., Tarrytown, New York 10591 (US)
(72) Inventor: OLSON, William C., Yorktown Heights, New York 10598 (US); DIPIPPO, Vincent, Mahopac, New York 10541 (US)
(74) Representative: Kehoe, Laura Ellen

(57) **Abstract**

Compositions and methods related to inhibiting the proliferation of or killing of prostate-specific membrane antigen (PSMA)-expressing cells are provided herein. In some embodiments, PSMA-expressing cells are contacted with (i) a compound that increases cell surface expression of PSMA and (ii) a PSMA ligand conjugate. In other embodiments, the PSMA-expressing cells are contacted with (i) prednisone and (ii) a PSMA ligand conjugate. In some of these embodiment ts, the PSMA-expressing cells are further contacted with (iii) a compound that increases cell surface expression of PSMA.

SEQUENCE LISTING

<110>  PSMA Development Company, LLC
       Olson, William C.

<120>  COMBINATION THERAPIES WITH PSMA LIGAND CONJUIGATES

<130>  P0741.70014WO00

<140>  TBD
<141>  2014-05-14

<150>  US 61/893,145
<151>  2013-10-18

<150>  US 61/903,589
<151>  2013-11-13

<150>  JP2013-235506
<151>  2013-11-13

<160>  7

<170>  PatentIn version 3.5
<210>  1
<211>  750
<212>  PRT
<213>  Homo sapiens

<400>  1

Met Trp Asn Leu Leu His Glu Thr Asp Ser Ala Val Ala Thr Ala Arg
1               5                   10                  15

Arg Pro Arg Trp Leu Cys Ala Gly Ala Leu Val Leu Ala Gly Gly Phe
            20                  25                  30

Phe Leu Leu Gly Phe Leu Phe Gly Trp Phe Ile Lys Ser Ser Asn Glu
        35                  40                  45

Ala Thr Asn Ile Thr Pro Lys His Asn Met Lys Ala Phe Leu Asp Glu
    50                  55                  60

Leu Lys Ala Glu Asn Ile Lys Lys Phe Leu Tyr Asn Phe Thr Gln Ile
65                  70                  75                  80

Pro His Leu Ala Gly Thr Glu Gln Asn Phe Gln Leu Ala Lys Gln Ile
                85                  90                  95

Gln Ser Gln Trp Lys Glu Phe Gly Leu Asp Ser Val Glu Leu Ala His
            100                 105                 110

Tyr Asp Val Leu Leu Ser Tyr Pro Asn Lys Thr His Pro Asn Tyr Ile
        115                 120                 125

Ser Ile Ile Asn Glu Asp Gly Asn Glu Ile Phe Asn Thr Ser Leu Phe
    130                 135                 140

Glu Pro Pro Pro Pro Gly Tyr Glu Asn Val Ser Asp Ile Val Pro Pro
145                 150                 155                 160

Phe Ser Ala Phe Ser Pro Gln Gly Met Pro Glu Gly Asp Leu Val Tyr
                165                 170                 175

Val Asn Tyr Ala Arg Thr Glu Asp Phe Phe Lys Leu Glu Arg Asp Met
            180                 185                 190

Lys Ile Asn Cys Ser Gly Lys Ile Val Ile Ala Arg Tyr Gly Lys Val
        195                 200                 205

Phe Arg Gly Asn Lys Val Lys Asn Ala Gln Leu Ala Gly Ala Lys Gly
    210                 215                 220

Val Ile Leu Tyr Ser Asp Pro Ala Asp Tyr Phe Ala Pro Gly Val Lys
225                 230                 235                 240

Ser Tyr Pro Asp Gly Trp Asn Leu Pro Gly Gly Gly Val Gln Arg Gly
                245                 250                 255

Asn Ile Leu Asn Leu Asn Gly Ala Gly Asp Pro Leu Thr Pro Gly Tyr
            260                 265                 270

Pro Ala Asn Glu Tyr Ala Tyr Arg Arg Gly Ile Ala Glu Ala Val Gly
        275                 280                 285

Leu Pro Ser Ile Pro Val His Pro Ile Gly Tyr Tyr Asp Ala Gln Lys
    290                 295                 300

Leu Leu Glu Lys Met Gly Gly Ser Ala Pro Pro Asp Ser Ser Trp Arg
305                 310                 315                 320

Gly Ser Leu Lys Val Pro Tyr Asn Val Gly Pro Gly Phe Thr Gly Asn
                325                 330                 335

Phe Ser Thr Gln Lys Val Lys Met His Ile His Ser Thr Asn Glu Val
            340                 345                 350

Thr Arg Ile Tyr Asn Val Ile Gly Thr Leu Arg Gly Ala Val Glu Pro
        355                 360                 365

Asp Arg Tyr Val Ile Leu Gly Gly His Arg Asp Ser Trp Val Phe Gly
    370                 375                 380

Gly Ile Asp Pro Gln Ser Gly Ala Ala Val Val His Glu Ile Val Arg
385                 390                 395                 400

Ser Phe Gly Thr Leu Lys Lys Glu Gly Trp Arg Pro Arg Arg Thr Ile
                405                 410                 415

Leu Phe Ala Ser Trp Asp Ala Glu Glu Phe Gly Leu Leu Gly Ser Thr
            420                 425                 430

Glu Trp Ala Glu Glu Asn Ser Arg Leu Leu Gln Glu Arg Gly Val Ala
        435                 440                 445

Tyr Ile Asn Ala Asp Ser Ser Ile Glu Gly Asn Tyr Thr Leu Arg Val
    450                 455                 460

Asp Cys Thr Pro Leu Met Tyr Ser Leu Val His Asn Leu Thr Lys Glu
465                 470                 475                 480

Leu Lys Ser Pro Asp Glu Gly Phe Glu Gly Lys Ser Leu Tyr Glu Ser
                485                 490                 495

Trp Thr Lys Lys Ser Pro Ser Pro Glu Phe Ser Gly Met Pro Arg Ile
            500                 505                 510

Ser Lys Leu Gly Ser Gly Asn Asp Phe Glu Val Phe Phe Gln Arg Leu
        515                 520                 525

Gly Ile Ala Ser Gly Arg Ala Arg Tyr Thr Lys Asn Trp Glu Thr Asn
    530                 535                 540

Lys Phe Ser Gly Tyr Pro Leu Tyr His Ser Val Tyr Glu Thr Tyr Glu
545                 550                 555                 560

Leu Val Glu Lys Phe Tyr Asp Pro Met Phe Lys Tyr His Leu Thr Val
                565                 570                 575

Ala Gln Val Arg Gly Gly Met Val Phe Glu Leu Ala Asn Ser Ile Val
            580                 585                 590

Leu Pro Phe Asp Cys Arg Asp Tyr Ala Val Val Leu Arg Lys Tyr Ala
        595                 600                 605

Asp Lys Ile Tyr Ser Ile Ser Met Lys His Pro Gln Glu Met Lys Thr
    610                 615                 620

Tyr Ser Val Ser Phe Asp Ser Leu Phe Ser Ala Val Lys Asn Phe Thr
625                 630                 635                 640

Glu Ile Ala Ser Lys Phe Ser Glu Arg Leu Gln Asp Phe Asp Lys Ser
                645                 650                 655

Asn Pro Ile Val Leu Arg Met Met Asn Asp Gln Leu Met Phe Leu Glu
            660                 665                 670

Arg Ala Phe Ile Asp Pro Leu Gly Leu Pro Asp Arg Pro Phe Tyr Arg
        675                 680                 685

His Val Ile Tyr Ala Pro Ser Ser His Asn Lys Tyr Ala Gly Glu Ser
    690                 695                 700

Phe Pro Gly Ile Tyr Asp Ala Leu Phe Asp Ile Glu Ser Lys Val Asp
705                 710                 715                 720

Pro Ser Lys Ala Trp Gly Glu Val Lys Arg Gln Ile Tyr Val Ala Ala
                725                 730                 735

Phe Thr Val Gln Ala Ala Ala Glu Thr Leu Ser Glu Val Ala
            740                 745                 750

<210>  2

<211>  7570

<212>  DNA

<213>  Artificial Sequence

<220>

<223>  Plasmid

<400>  2
gacggatcgg gagatctccc gatcccctat ggtcgactct cagtacaatc tgctctgatg      60

ccgcatagtt aagccagtat ctgctccctg cttgtgtgtt ggaggtcgct gagtagtgcg     120

cgagcaaaat ttaagctaca acaaggcaag gcttgaccga caattgcatg aagaatctgc     180

ttagggttag gcgttttgcg ctgcttcgcg atgtacgggc cagatatacg cgttgacatt     240

gattattgac tagttattaa tagtaatcaa ttacggggtc attagttcat agcccatata     300

tggagttccg cgttacataa cttacggtaa atggcccgcc tggctgaccg cccaacgacc     360

cccgcccatt gacgtcaata atgacgtatg ttcccatagt aacgccaata gggactttcc     420

attgacgtca atgggtggac tatttacggt aaactgccca cttggcagta catcaagtgt     480

atcatatgcc aagtacgccc cctattgacg tcaatgacgg taaatggccc gcctggcatt     540

atgcccagta catgacctta tgggactttc ctacttggca gtacatctac gtattagtca     600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctaga 900

ggtaccaagc ttggatctca ccatggagtt gggactgcgc tggggcttcc tcgttgctct 960

tttaagaggt gtccagtgtc aggtgcaatt ggtggagtct gggggaggcg tggtccagcc 1020

tgggaggtcc ctgagactct cctgtgcagc gtctggattc gccttcagta gatatggcat 1080

gcactgggtc cgccaggctc caggcaaggg gctggagtgg gtggcagtta tatggtatga 1140

tggaagtaat aaatactatg cagactccgt gaagggccga ttcaccatct ccagagacaa 1200

ttccaagaac acgcagtatc tgcaaatgaa cagcctgaga gccgaggaca cggctgtgta 1260

ttactgtgcg agaggcggtg acttcctcta ctactactat tacggtatgg acgtctgggg 1320

ccaagggacc acggtcaccg tctcctcagc ctccaccaag ggcccatcgg tcttccccct 1380

ggcaccctct agcaagagca cctctggggg cacagcggcc ctgggctgcc tggtcaagga 1440

ctacttcccc gaaccggtga cggtgtcgtg gaactcaggc gccctgacca gcggcgtgca 1500

caccttcccg gctgtcctac agtcctcagg actctactcc ctcagcagcg tggtgaccgt 1560

gccctccagc agcttgggca cccagaccta catctgcaac gtgaatcaca agcccagcaa 1620

caccaaggtg gacaagagag ttggtgagag gccagcacag ggagggaggg tgtctgctgg 1680

aagccaggct cagcgctcct gcctggacgc atcccggcta tgcagtccca gtccagggca 1740

gcaaggcagg ccccgtctgc ctcttcaccc ggaggcctct gcccgcccca ctcatgctca 1800

gggagagggt cttctggctt tttccccagg ctctgggcag gcacaggcta ggtgccccta 1860

acccaggccc tgcacacaaa ggggcaggtg ctgggctcag acctgccaag agccatatcc 1920

gggaggaccc tgcccctgac ctaagcccac cccaaaggcc aaactctcca ctccctcagc 1980

tcggacacct tctctcctcc cagattccag taactcccaa tcttctctct gcagagccca 2040

aatcttgtga caaaactcac acatgcccac cgtgcccagg taagccagcc caggcctcgc 2100

cctccagctc aaggcgggac aggtgcccta gagtagcctg catccaggga caggccccag 2160

ccgggtgctg acacgtccac ctccatctct tcctcagcac ctgaactcct ggggggaccg 2220

tcagtcttcc tcttcccccc aaaacccaag gacaccctca tgatctcccg gacccctgag 2280

gtcacatgcg tggtggtgga cgtgagccac gaagaccctg aggtcaagtt caactggtac 2340

gtggacggcg tggaggtgca taatgccaag acaaagccgc gggaggagca gtacaacagc 2400

acgtaccgtg tggtcagcgt cctcaccgtc ctgcaccagg actggctgaa tggcaaggag 2460

tacaagtgca aggtctccaa caaagccctc ccagccccca tcgagaaaac catctccaaa 2520

gccaaaggtg ggacccgtgg ggtgcgaggg ccacatggac agaggccggc tcggcccacc 2580

ctctgccctg agagtgaccg ctgtaccaac ctctgtccct acagggcagc cccgagaacc 2640

acaggtgtac accctgcccc catcccggga ggagatgacc aagaaccagg tcagcctgac 2700

ctgcctggtc aaaggcttct atcccagcga catcgccgtg gagtgggaga gcaatgggca 2760

gccggagaac aactacaaga ccacgcctcc cgtgctggac tccgacggct ccttcttcct 2820

ctatagcaag ctcaccgtgg acaagagcag gtggcagcag gggaacgtct tctcatgctc 2880

cgtgatgcat gaggctctgc acaaccacta cacgcagaag agcctctccc tgtctccggg 2940

taaatgagaa ttcctcgagt ctagagggcc cgtttaaacc cgctgatcag cctcgactgt 3000

gccttctagt tgccagccat ctgttgtttg cccctccccc gtgccttcct tgaccctgga 3060

aggtgccact cccactgtcc tttcctaata aaatgaggaa attgcatcgc attgtctgag 3120

taggtgtcat tctattctgg ggggtggggt ggggcaggac agcaaggggg aggattggga 3180

agacaatagc aggcatgctg gggatgcggt gggctctatg gcttctgagg cggaaagaac 3240

cagctggggc tctagggggt atccccacgc gccctgtagc ggcgcattaa gcgcggcggg 3300

tgtggtggtt acgcgcagcg tgaccgctac acttgccagc gccctagcgc ccgctccttt 3360

cgctttcttc ccttcctttc tcgccacgtt cgccggcttt ccccgtcaag ctctaaatcg 3420

gggcatccct ttagggttcc gatttagtgc tttacggcac ctcgacccca aaaaacttga 3480

ttagggtgat ggttcacgta gtgggccatc gccctgatag acggtttttc gccctttgac 3540

gttggagtcc acgttcttta atagtggact cttgttccaa actggaacaa cactcaaccc 3600

tatctcggtc tattcttttg atttataagg gattttgggg atttcggcct attggttaaa 3660

aaatgagctg atttaacaaa aatttaacgc gaattaattc tgtggaatgt gtgtcagtta 3720

gggtgtggaa agtccccagg ctccccaggc aggcagaagt atgcaaagca tgcatctcaa 3780

ttagtcagca accaggtgtg gaaagtcccc aggctcccca gcaggcagaa gtatgcaaag 3840

catgcatctc aattagtcag caaccatagt cccgccccta actccgccca tcccgcccct 3900

aactccgccc agttccgccc attctccgcc ccatggctga ctaatttttt ttatttatgc 3960

agaggccgag gccgcctctg cctctgagct attccagaag tagtgaggag gcttttttgg 4020

aggcctaggc ttttgcaaaa agctcccggg agcttgtata tccattttcg gatctgatca 4080

gcacgtgatg aaaaagcctg aactcaccgc gacgtctgtc gagaagtttc tgatcgaaaa 4140

gttcgacagc gtctccgacc tgatgcagct ctcggagggc gaagaatctc gtgctttcag 4200

cttcgatgta ggagggcgtg gatatgtcct gcgggtaaat agctgcgccg atggtttcta 4260

caaagatcgt tatgtttatc ggcactttgc atcggccgcg ctcccgattc cggaagtgct 4320

tgacattggg gaattcagcg agagcctgac ctattgcatc tcccgccgtg cacagggtgt 4380

cacgttgcaa gacctgcctg aaaccgaact gcccgctgtt ctgcagccgg tcgcggaggc 4440

catggatgcg atcgctgcgg ccgatcttag ccagacgagc gggttcggcc cattcggacc 4500

gcaaggaatc ggtcaataca ctacatggcg tgatttcata tgcgcgattg ctgatcccca 4560

tgtgtatcac tggcaaactg tgatggacga caccgtcagt gcgtccgtcg cgcaggctct 4620

cgatgagctg atgctttggg ccgaggactg ccccgaagtc cggcacctcg tgcacgcgga 4680

tttcggctcc aacaatgtcc tgacggacaa tggccgcata acagcggtca ttgactggag 4740

cgaggcgatg ttcggggatt cccaatacga ggtcgccaac atcttcttct ggaggccgtg 4800

gttggcttgt atggagcagc agacgcgcta cttcgagcgg aggcatccgg agcttgcagg 4860

atcgccgcgg ctccgggcgt atatgctccg cattggtctt gaccaactct atcagagctt 4920

ggttgacggc aatttcgatg atgcagcttg ggcgcagggt cgatgcgacg caatcgtccg 4980

atccggagcc gggactgtcg ggcgtacaca aatcgcccgc agaagcgcgg ccgtctggac 5040

cgatggctgt gtagaagtac tcgccgatag tggaaaccga cgccccagca ctcgtccgag 5100

ggcaaaggaa tagcacgtgc tacgagattt cgattccacc gccgccttct atgaaaggtt 5160

gggcttcgga atcgttttcc gggacgccgg ctggatgatc ctccagcgcg gggatctcat 5220

gctggagttc ttcgcccacc ccaacttgtt tattgcagct tataatggtt acaaataaag 5280

caatagcatc acaaatttca caaataaagc atttttttca ctgcattcta gttgtggttt 5340

gtccaaactc atcaatgtat cttatcatgt ctgtataccg tcgacctcta gctagagctt 5400

ggcgtaatca tggtcatagc tgtttcctgt gtgaaattgt tatccgctca caattccaca 5460

caacatacga gccggaagca taaagtgtaa agcctggggt gcctaatgag tgagctaact 5520

cacattaatt gcgttgcgct cactgcccgc tttccagtcg ggaaacctgt cgtgccagct 5580

gcattaatga atcggccaac gcgcggggag aggcggtttg cgtattgggc gctcttccgc 5640

ttcctcgctc actgactcgc tgcgctcggt cgttcggctg cggcgagcgg tatcagctca 5700

ctcaaaggcg gtaatacggt tatccacaga atcaggggat aacgcaggaa agaacatgtg 5760

agcaaaaggc cagcaaaagg ccaggaaccg taaaaaggcc gcgttgctgg cgtttttcca 5820

taggctccgc ccccctgacg agcatcacaa aaatcgacgc tcaagtcaga ggtggcgaaa 5880

cccgacagga ctataaagat accaggcgtt tccccctgga agctccctcg tgcgctctcc 5940

tgttccgacc ctgccgctta ccggatacct gtccgccttt ctcccttcgg gaagcgtggc 6000

gctttctcaa tgctcacgct gtaggtatct cagttcggtg taggtcgttc gctccaagct 6060

gggctgtgtg cacgaacccc ccgttcagcc cgaccgctgc gccttatccg gtaactatcg 6120

tcttgagtcc aacccggtaa gacacgactt atcgccactg gcagcagcca ctggtaacag 6180

gattagcaga gcgaggtatg taggcggtgc tacagagttc ttgaagtggt ggcctaacta 6240

cggctacact agaaggacag tatttggtat ctgcgctctg ctgaagccag ttaccttcgg 6300

aaaaagagtt ggtagctctt gatccggcaa acaaaccacc gctggtagcg gtggtttttt 6360

tgtttgcaag cagcagatta cgcgcagaaa aaaaggatct caagaagatc ctttgatctt 6420

ttctacgggg tctgacgctc agtggaacga aaactcacgt taagggattt tggtcatgag 6480

attatcaaaa aggatcttca cctagatcct tttaaattaa aaatgaagtt ttaaatcaat 6540

ctaaagtata tatgagtaaa cttggtctga cagttaccaa tgcttaatca gtgaggcacc 6600

tatctcagcg atctgtctat ttcgttcatc catagttgcc tgactccccg tcgtgtagat 6660

aactacgata cgggagggct taccatctgg ccccagtgct gcaatgatac cgcgagaccc 6720

acgctcaccg gctccagatt tatcagcaat aaaccagcca gccggaaggg ccgagcgcag 6780

aagtggtcct gcaactttat ccgcctccat ccagtctatt aattgttgcc gggaagctag 6840

agtaagtagt tcgccagtta atagtttgcg caacgttgtt gccattgcta caggcatcgt 6900

ggtgtcacgc tcgtcgtttg gtatggcttc attcagctcc ggttcccaac gatcaaggcg 6960

agttacatga tcccccatgt tgtgcaaaaa agcggttagc tccttcggtc ctccgatcgt 7020

tgtcagaagt aagttggccg cagtgttatc actcatggtt atggcagcac tgcataattc 7080

tcttactgtc atgccatccg taagatgctt ttctgtgact ggtgagtact caaccaagtc 7140

attctgagaa tagtgtatgc ggcgaccgag ttgctcttgc ccggcgtcaa tacgggataa 7200

taccgcgcca catagcagaa ctttaaaagt gctcatcatt ggaaaacgtt cttcggggcg 7260

aaaactctca aggatcttac cgctgttgag atccagttcg atgtaaccca ctcgtgcacc 7320

caactgatct tcagcatctt ttactttcac cagcgtttct gggtgagcaa aaacaggaag 7380

gcaaaatgcc gcaaaaaagg gaataagggc gacacggaaa tgttgaatac tcatactctt 7440

cctttttcaa tattattgaa gcatttatca gggttattgt ctcatgagcg gatacatatt 7500

tgaatgtatt tagaaaaata aacaaatagg ggttccgcgc acatttcccc gaaaagtgcc 7560

acctgacgtc 7570

<210> 3

<211> 7597

<212> DNA

<213> Artificial Sequence

<220>

<223> Plasmid

<400> 3
gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctaga 900

ggtaccaagc ttggatctca ccatggggtc aaccgccatc ctcaccatgg agttggggct 960

gcgctgggtt ctcctcgttg ctcttttaag aggtgtccag tgtcaggtgc agctggtgga 1020

gtctggggga ggcgtggtcc agcctgggag gtccctgaga ctctcctgtg cagcgtctgg 1080

attcaccttc agtaactatg tcatgcactg ggtccgccag gctccaggca aggggctgga 1140

gtgggtggca attatatggt atgatggaag taataaatac tatgcagact ccgtgaaggg 1200

ccgattcacc atctccagag acaattccaa gaacacgctg tatctgcaaa tgaacagcct 1260

gagagccgag gacacggctg tgtattactg tgcgggtgga tataactgga actacgagta 1320

ccactactac ggtatggacg tctggggcca agggaccacg gtcaccgtct cctcagcctc 1380

caccaagggc ccatcggtct tccccctggc accctctagc aagagcacct ctgggggcac 1440

agcggccctg ggctgcctgg tcaaggacta cttccccgaa ccggtgacgg tgtcgtggaa 1500

ctcaggcgcc ctgaccagcg gcgtgcacac cttcccggct gtcctacagt cctcaggact 1560

ctactccctc agcagcgtgg tgaccgtgcc ctccagcagc ttgggcaccc agacctacat 1620

ctgcaacgtg aatcacaagc ccagcaacac caaggtggac aagagagttg gtgagaggcc 1680

agcacaggga gggagggtgt ctgctggaag ccaggctcag cgctcctgcc tggacgcatc 1740

ccggctatgc agtcccagtc cagggcagca aggcaggccc cgtctgcctc ttcacccgga 1800

ggcctctgcc cgccccactc atgctcaggg agagggtctt ctggcttttt ccccaggctc 1860

tgggcaggca caggctaggt gcccctaacc caggccctgc acacaaaggg gcaggtgctg 1920

ggctcagacc tgccaagagc catatccggg aggaccctgc ccctgaccta agcccacccc 1980

aaaggccaaa ctctccactc cctcagctcg gacaccttct ctcctcccag attccagtaa 2040

ctcccaatct tctctctgca gagcccaaat cttgtgacaa aactcacaca tgcccaccgt 2100

gcccaggtaa gccagcccag gcctcgccct ccagctcaag gcgggacagg tgccctagag 2160

tagcctgcat ccagggacag gccccagccg ggtgctgaca cgtccacctc catctcttcc 2220

tcagcacctg aactcctggg gggaccgtca gtcttcctct tccccccaaa acccaaggac 2280

accctcatga tctcccggac ccctgaggtc acatgcgtgg tggtggacgt gagccacgaa 2340

gaccctgagg tcaagttcaa ctggtacgtg gacggcgtgg aggtgcataa tgccaagaca 2400

aagccgcggg aggagcagta caacagcacg taccgtgtgg tcagcgtcct caccgtcctg 2460

caccaggact ggctgaatgg caaggagtac aagtgcaagg tctccaacaa agccctccca 2520

gcccccatcg agaaaaccat ctccaaagcc aaaggtggga cccgtggggt gcgagggcca 2580

catggacaga ggccggctcg gcccaccctc tgccctgaga gtgaccgctg taccaacctc 2640

tgtccctaca gggcagcccc gagaaccaca ggtgtacacc ctgcccccat cccgggagga 2700

gatgaccaag aaccaggtca gcctgacctg cctggtcaaa ggcttctatc ccagcgacat 2760

cgccgtggag tgggagagca atgggcagcc ggagaacaac tacaagacca cgcctcccgt 2820

gctggactcc gacggctcct tcttcctcta tagcaagctc accgtggaca agagcaggtg 2880

gcagcagggg aacgtcttct catgctccgt gatgcatgag gctctgcaca accactacac 2940

gcagaagagc ctctccctgt ctccgggtaa atgagaattc ctcgagtcta gagggcccgt 3000

ttaaacccgc tgatcagcct cgactgtgcc ttctagttgc cagccatctg ttgtttgccc 3060

ctcccccgtg ccttccttga ccctggaagg tgccactccc actgtccttt cctaataaaa 3120

tgaggaaatt gcatcgcatt gtctgagtag gtgtcattct attctggggg gtggggtggg 3180

gcaggacagc aagggggagg attgggaaga caatagcagg catgctgggg atgcggtggg 3240

ctctatggct tctgaggcgg aaagaaccag ctggggctct agggggtatc cccacgcgcc 3300

ctgtagcggc gcattaagcg cggcgggtgt ggtggttacg cgcagcgtga ccgctacact 3360

tgccagcgcc ctagcgcccg ctcctttcgc tttcttccct tcctttctcg ccacgttcgc 3420

cggctttccc cgtcaagctc taaatcgggg catcccttta gggttccgat ttagtgcttt 3480

acggcacctc gaccccaaaa aacttgatta gggtgatggt tcacgtagtg ggccatcgcc 3540

ctgatagacg gtttttcgcc ctttgacgtt ggagtccacg ttctttaata gtggactctt 3600

gttccaaact ggaacaacac tcaaccctat ctcggtctat tcttttgatt tataagggat 3660

tttggggatt tcggcctatt ggttaaaaaa tgagctgatt taacaaaaat ttaacgcgaa 3720

ttaattctgt ggaatgtgtg tcagttaggg tgtggaaagt ccccaggctc cccaggcagg 3780

cagaagtatg caaagcatgc atctcaatta gtcagcaacc aggtgtggaa agtccccagg 3840

ctccccagca ggcagaagta tgcaaagcat gcatctcaat tagtcagcaa ccatagtccc 3900

gcccctaact ccgcccatcc cgcccctaac tccgcccagt tccgcccatt ctccgcccca 3960

tggctgacta atttttttta tttatgcaga ggccgaggcc gcctctgcct ctgagctatt 4020

ccagaagtag tgaggaggct tttttggagg cctaggcttt tgcaaaaagc tcccgggagc 4080

ttgtatatcc attttcggat ctgatcagca cgtgatgaaa aagcctgaac tcaccgcgac 4140

gtctgtcgag aagtttctga tcgaaaagtt cgacagcgtc tccgacctga tgcagctctc 4200

ggagggcgaa gaatctcgtg ctttcagctt cgatgtagga gggcgtggat atgtcctgcg 4260

ggtaaatagc tgcgccgatg gtttctacaa agatcgttat gtttatcggc actttgcatc 4320

ggccgcgctc ccgattccgg aagtgcttga cattggggaa ttcagcgaga gcctgaccta 4380

ttgcatctcc cgccgtgcac agggtgtcac gttgcaagac ctgcctgaaa ccgaactgcc 4440

cgctgttctg cagccggtcg cggaggccat ggatgcgatc gctgcggccg atcttagcca 4500

gacgagcggg ttcggcccat tcggaccgca aggaatcggt caatacacta catggcgtga 4560

tttcatatgc gcgattgctg atccccatgt gtatcactgg caaactgtga tggacgacac 4620

cgtcagtgcg tccgtcgcgc aggctctcga tgagctgatg ctttgggccg aggactgccc 4680

cgaagtccgg cacctcgtgc acgcggattt cggctccaac aatgtcctga cggacaatgg 4740

ccgcataaca gcggtcattg actggagcga ggcgatgttc ggggattccc aatacgaggt 4800

cgccaacatc ttcttctgga ggccgtggtt ggcttgtatg gagcagcaga cgcgctactt 4860

cgagcggagg catccggagc ttgcaggatc gccgcggctc cgggcgtata tgctccgcat 4920

tggtcttgac caactctatc agagcttggt tgacggcaat ttcgatgatg cagcttgggc 4980

gcagggtcga tgcgacgcaa tcgtccgatc cggagccggg actgtcgggc gtacacaaat 5040

cgcccgcaga agcgcggccg tctggaccga tggctgtgta gaagtactcg ccgatagtgg 5100

aaaccgacgc cccagcactc gtccgagggc aaaggaatag cacgtgctac gagatttcga 5160

ttccaccgcc gccttctatg aaaggttggg cttcggaatc gttttccggg acgccggctg 5220

gatgatcctc cagcgcgggg atctcatgct ggagttcttc gcccacccca acttgtttat 5280

tgcagcttat aatggttaca aataaagcaa tagcatcaca aatttcacaa ataaagcatt 5340

tttttcactg cattctagtt gtggtttgtc caaactcatc aatgtatctt atcatgtctg 5400

tataccgtcg acctctagct agagcttggc gtaatcatgg tcatagctgt ttcctgtgtg 5460

aaattgttat ccgctcacaa ttccacacaa catacgagcc ggaagcataa agtgtaaagc 5520

ctggggtgcc taatgagtga gctaactcac attaattgcg ttgcgctcac tgcccgcttt 5580

ccagtcggga aacctgtcgt gccagctgca ttaatgaatc ggccaacgcg cggggagagg 5640

cggtttgcgt attgggcgct cttccgcttc ctcgctcact gactcgctgc gctcggtcgt 5700

tcggctgcgg cgagcggtat cagctcactc aaaggcggta atacggttat ccacagaatc 5760

aggggataac gcaggaaaga acatgtgagc aaaaggccag caaaaggcca ggaaccgtaa 5820

aaaggccgcg ttgctggcgt ttttccatag gctccgcccc cctgacgagc atcacaaaaa 5880

tcgacgctca agtcagaggt ggcgaaaccc gacaggacta taaagatacc aggcgtttcc 5940

ccctggaagc tccctcgtgc gctctcctgt tccgaccctg ccgcttaccg gatacctgtc 6000

cgcctttctc ccttcgggaa gcgtggcgct ttctcaatgc tcacgctgta ggtatctcag 6060

ttcggtgtag gtcgttcgct ccaagctggg ctgtgtgcac gaaccccccg ttcagcccga 6120

ccgctgcgcc ttatccggta actatcgtct tgagtccaac ccggtaagac acgacttatc 6180

gccactggca gcagccactg gtaacaggat tagcagagcg aggtatgtag gcggtgctac 6240

agagttcttg aagtggtggc ctaactacgg ctacactaga aggacagtat ttggtatctg 6300

cgctctgctg aagccagtta ccttcggaaa aagagttggt agctcttgat ccggcaaaca 6360

aaccaccgct ggtagcggtg gtttttttgt ttgcaagcag cagattacgc gcagaaaaaa 6420

aggatctcaa gaagatcctt tgatcttttc tacggggtct gacgctcagt ggaacgaaaa 6480

ctcacgttaa gggattttgg tcatgagatt atcaaaaagg atcttcacct agatcctttt 6540

aaattaaaaa tgaagtttta aatcaatcta aagtatatat gagtaaactt ggtctgacag 6600

ttaccaatgc ttaatcagtg aggcacctat ctcagcgatc tgtctatttc gttcatccat 6660

agttgcctga ctccccgtcg tgtagataac tacgatacgg gagggcttac catctggccc 6720

cagtgctgca atgataccgc gagacccacg ctcaccggct ccagatttat cagcaataaa 6780

ccagccagcc ggaagggccg agcgcagaag tggtcctgca actttatccg cctccatcca 6840

gtctattaat tgttgccggg aagctagagt aagtagttcg ccagttaata gtttgcgcaa 6900

cgttgttgcc attgctacag gcatcgtggt gtcacgctcg tcgtttggta tggcttcatt 6960

cagctccggt tcccaacgat caaggcgagt tacatgatcc cccatgttgt gcaaaaaagc 7020

ggttagctcc ttcggtcctc cgatcgttgt cagaagtaag ttggccgcag tgttatcact 7080

catggttatg gcagcactgc ataattctct tactgtcatg ccatccgtaa gatgcttttc 7140

tgtgactggt gagtactcaa ccaagtcatt ctgagaatag tgtatgcggc gaccgagttg 7200

ctcttgcccg gcgtcaatac gggataatac cgcgccacat agcagaactt taaaagtgct 7260

catcattgga aaacgttctt cggggcgaaa actctcaagg atcttaccgc tgttgagatc 7320

cagttcgatg taacccactc gtgcacccaa ctgatcttca gcatctttta ctttcaccag 7380

cgtttctggg tgagcaaaaa caggaaggca aaatgccgca aaaaagggaa taagggcgac 7440

acggaaatgt tgaatactca tactcttcct ttttcaatat tattgaagca tttatcaggg 7500

ttattgtctc atgagcggat acatatttga atgtatttag aaaaataaac aaataggggt 7560

tccgcgcaca tttccccgaa aagtgccacc tgacgtc 7597

<210> 4

<211>  7579

<212>  DNA

<213>  Artificial Sequence

<220>

<223>  Plasmid

<400>  4
gacggatcgg gagatctccc gatcccctat ggtcgactct cagtacaatc tgctctgatg      60

ccgcatagtt aagccagtat ctgctccctg cttgtgtgtt ggaggtcgct gagtagtgcg     120

cgagcaaaat ttaagctaca acaaggcaag gcttgaccga caattgcatg aagaatctgc     180

ttagggttag gcgttttgcg ctgcttcgcg atgtacgggc cagatatacg cgttgacatt     240

gattattgac tagttattaa tagtaatcaa ttacggggtc attagttcat agcccatata     300

tggagttccg cgttacataa cttacggtaa atggcccgcc tggctgaccg cccaacgacc     360

cccgcccatt gacgtcaata atgacgtatg ttcccatagt aacgccaata gggactttcc     420

attgacgtca atgggtggac tatttacggt aaactgccca cttggcagta catcaagtgt     480

atcatatgcc aagtacgccc cctattgacg tcaatgacgg taaatggccc gcctggcatt     540

atgcccagta catgacctta tgggactttc ctacttggca gtacatctac gtattagtca     600

tcgctattac catggtgatg cggttttggc agtacatcaa tgggcgtgga tagcggtttg     660

actcacgggg atttccaagt ctccacccca ttgacgtcaa tgggagtttg ttttggcacc     720

aaaatcaacg ggactttcca aaatgtcgta acaactccgc cccattgacg caaatgggcg     780

gtaggcgtgt acggtgggag gtctatataa gcagagctct ctggctaact agagaaccca     840

ctgcttactg gcttatcgaa attaatacga ctcactatag ggagacccaa gctggctaga     900

ggtaccaagc ttggatctca ccatggagtt gggacttagc tgggttttcc tcgttgctct     960

tttaagaggt gtccagtgtc aggtccagct ggtggagtct gggggaggcg tggtccagcc    1020

tgggaggtcc ctgagactct cctgtgcagc gtctggattc accttcagta gctatggcat    1080

gcactgggtc cgccaggctc caggcaaggg gctggactgg gtggcaatta tttggcatga    1140

tggaagtaat aaatactatg cagactccgt gaagggccga ttcaccatct ccagagacaa    1200

ttccaagaag acgctgtacc tgcaaatgaa cagtttgaga gccgaggaca cggctgtgta    1260

ttactgtgcg agagcttggg cctatgacta cggtgactat gaatactact tcggtatgga    1320

cgtctggggc caagggacca cggtcaccgt ctcctcagcc tccaccaagg gcccatcggt    1380

cttccccctg gcaccctcta gcaagagcac ctctgggggc acagcggccc tgggctgcct    1440

ggtcaaggac tacttccccg aaccggtgac ggtgtcgtgg aactcaggcg ccctgaccag    1500

cggcgtgcac accttcccgg ctgtcctaca gtcctcagga ctctactccc tcagcagcgt    1560

ggtgaccgtg ccctccagca gcttgggcac ccagacctac atctgcaacg tgaatcacaa 1620

gcccagcaac accaaggtgg acaagagagt tggtgagagg ccagcacagg gagggagggt 1680

gtctgctgga agccaggctc agcgctcctg cctggacgca tcccggctat gcagtcccag 1740

tccagggcag caaggcaggc cccgtctgcc tcttcacccg gaggcctctg cccgccccac 1800

tcatgctcag ggagagggtc ttctggcttt ttccccaggc tctgggcagg cacaggctag 1860

gtgcccctaa cccaggccct gcacacaaag gggcaggtgc tgggctcaga cctgccaaga 1920

gccatatccg ggaggaccct gcccctgacc taagcccacc ccaaaggcca aactctccac 1980

tccctcagct cggacacctt ctctcctccc agattccagt aactcccaat cttctctctg 2040

cagagcccaa atcttgtgac aaaactcaca catgcccacc gtgcccaggt aagccagccc 2100

aggcctcgcc ctccagctca aggcgggaca ggtgccctag agtagcctgc atccagggac 2160

aggccccagc cgggtgctga cacgtccacc tccatctctt cctcagcacc tgaactcctg 2220

gggggaccgt cagtcttcct cttcccccca aaacccaagg acaccctcat gatctcccgg 2280

acccctgagg tcacatgcgt ggtggtggac gtgagccacg aagaccctga ggtcaagttc 2340

aactggtacg tggacggcgt ggaggtgcat aatgccaaga caaagccgcg ggaggagcag 2400

tacaacagca cgtaccgtgt ggtcagcgtc ctcaccgtcc tgcaccagga ctggctgaat 2460

ggcaaggagt acaagtgcaa ggtctccaac aaagccctcc cagcccccat cgagaaaacc 2520

atctccaaag ccaaaggtgg gacccgtggg gtgcgagggc cacatggaca gaggccggct 2580

cggcccaccc tctgccctga gagtgaccgc tgtaccaacc tctgtcccta cagggcagcc 2640

ccgagaacca caggtgtaca ccctgccccc atcccgggag gagatgacca agaaccaggt 2700

cagcctgacc tgcctggtca aaggcttcta tcccagcgac atcgccgtgg agtgggagag 2760

caatgggcag ccggagaaca actacaagac cacgcctccc gtgctggact ccgacggctc 2820

cttcttcctc tatagcaagc tcaccgtgga caagagcagg tggcagcagg ggaacgtctt 2880

ctcatgctcc gtgatgcatg aggctctgca caaccactac acgcagaaga gcctctccct 2940

gtctccgggt aaatgagaat tcctcgagtc tagagggccc gtttaaaccc gctgatcagc 3000

ctcgactgtg ccttctagtt gccagccatc tgttgtttgc ccctcccccg tgccttcctt 3060

gaccctggaa ggtgccactc ccactgtcct ttcctaataa aatgaggaaa ttgcatcgca 3120

ttgtctgagt aggtgtcatt ctattctggg gggtggggtg gggcaggaca gcaaggggga 3180

ggattgggaa gacaatagca ggcatgctgg ggatgcggtg ggctctatgg cttctgaggc 3240

ggaaagaacc agctggggct ctagggggta tccccacgcg ccctgtagcg gcgcattaag 3300

cgcggcgggt gtggtggtta cgcgcagcgt gaccgctaca cttgccagcg ccctagcgcc 3360

cgctcctttc gctttcttcc cttcctttct cgccacgttc gccggctttc cccgtcaagc 3420

tctaaatcgg ggcatccctt tagggttccg atttagtgct ttacggcacc tcgaccccaa 3480

aaaacttgat tagggtgatg gttcacgtag tgggccatcg ccctgataga cggtttttcg 3540

ccctttgacg ttggagtcca cgttctttaa tagtggactc ttgttccaaa ctggaacaac 3600

actcaaccct atctcggtct attcttttga tttataaggg attttgggga tttcggccta 3660

ttggttaaaa aatgagctga tttaacaaaa atttaacgcg aattaattct gtggaatgtg 3720

tgtcagttag ggtgtggaaa gtccccaggc tccccaggca ggcagaagta tgcaaagcat 3780

gcatctcaat tagtcagcaa ccaggtgtgg aaagtcccca ggctccccag caggcagaag 3840

tatgcaaagc atgcatctca attagtcagc aaccatagtc ccgcccctaa ctccgcccat 3900

cccgccccta actccgccca gttccgccca ttctccgccc catggctgac taattttttt 3960

tatttatgca gaggccgagg ccgcctctgc ctctgagcta ttccagaagt agtgaggagg 4020

cttttttgga ggcctaggct tttgcaaaaa gctcccggga gcttgtatat ccattttcgg 4080

atctgatcag cacgtgatga aaaagcctga actcaccgcg acgtctgtcg agaagtttct 4140

gatcgaaaag ttcgacagcg tctccgacct gatgcagctc tcggagggcg aagaatctcg 4200

tgctttcagc ttcgatgtag gagggcgtgg atatgtcctg cgggtaaata gctgcgccga 4260

tggtttctac aaagatcgtt atgtttatcg gcactttgca tcggccgcgc tcccgattcc 4320

ggaagtgctt gacattgggg aattcagcga gagcctgacc tattgcatct cccgccgtgc 4380

acagggtgtc acgttgcaag acctgcctga aaccgaactg cccgctgttc tgcagccggt 4440

cgcggaggcc atggatgcga tcgctgcggc cgatcttagc cagacgagcg ggttcggccc 4500

attcggaccg caaggaatcg gtcaatacac tacatggcgt gatttcatat gcgcgattgc 4560

tgatccccat gtgtatcact ggcaaactgt gatggacgac accgtcagtg cgtccgtcgc 4620

gcaggctctc gatgagctga tgctttgggc cgaggactgc cccgaagtcc ggcacctcgt 4680

gcacgcggat ttcggctcca acaatgtcct gacggacaat ggccgcataa cagcggtcat 4740

tgactggagc gaggcgatgt tcggggattc ccaatacgag gtcgccaaca tcttcttctg 4800

gaggccgtgg ttggcttgta tggagcagca gacgcgctac ttcgagcgga ggcatccgga 4860

gcttgcagga tcgccgcggc tccgggcgta tatgctccgc attggtcttg accaactcta 4920

tcagagcttg gttgacggca atttcgatga tgcagcttgg gcgcagggtc gatgcgacgc 4980

aatcgtccga tccggagccg ggactgtcgg gcgtacacaa atcgcccgca gaagcgcggc 5040

cgtctggacc gatggctgtg tagaagtact cgccgatagt ggaaaccgac gccccagcac 5100

tcgtccgagg gcaaaggaat agcacgtgct acgagatttc gattccaccg ccgccttcta 5160

tgaaaggttg ggcttcggaa tcgttttccg ggacgccggc tggatgatcc tccagcgcgg 5220

ggatctcatg ctggagttct tcgcccaccc caacttgttt attgcagctt ataatggtta 5280

caaataaagc aatagcatca caaatttcac aaataaagca tttttttcac tgcattctag 5340

ttgtggtttg tccaaactca tcaatgtatc ttatcatgtc tgtataccgt cgacctctag 5400

ctagagcttg gcgtaatcat ggtcatagct gtttcctgtg tgaaattgtt atccgctcac 5460

aattccacac aacatacgag ccggaagcat aaagtgtaaa gcctggggtg cctaatgagt 5520

gagctaactc acattaattg cgttgcgctc actgcccgct ttccagtcgg gaaacctgtc 5580

gtgccagctg cattaatgaa tcggccaacg cgcggggaga ggcggtttgc gtattgggcg 5640

ctcttccgct tcctcgctca ctgactcgct gcgctcggtc gttcggctgc ggcgagcggt 5700

atcagctcac tcaaaggcgg taatacggtt atccacagaa tcaggggata acgcaggaaa 5760

gaacatgtga gcaaaaggcc agcaaaaggc caggaaccgt aaaaaggccg cgttgctggc 5820

gtttttccat aggctccgcc cccctgacga gcatcacaaa aatcgacgct caagtcagag 5880

gtggcgaaac ccgacaggac tataaagata ccaggcgttt ccccctggaa gctccctcgt 5940

gcgctctcct gttccgaccc tgccgcttac cggatacctg tccgcctttc tcccttcggg 6000

aagcgtggcg ctttctcaat gctcacgctg taggtatctc agttcggtgt aggtcgttcg 6060

ctccaagctg ggctgtgtgc acgaaccccc cgttcagccc gaccgctgcg ccttatccgg 6120

taactatcgt cttgagtcca acccggtaag acacgactta tcgccactgg cagcagccac 6180

tggtaacagg attagcagag cgaggtatgt aggcggtgct acagagttct tgaagtggtg 6240

gcctaactac ggctacacta gaaggacagt atttggtatc tgcgctctgc tgaagccagt 6300

taccttcgga aaaagagttg gtagctcttg atccggcaaa caaaccaccg ctggtagcgg 6360

tggttttttt gtttgcaagc agcagattac gcgcagaaaa aaaggatctc aagaagatcc 6420

tttgatcttt tctacggggt ctgacgctca gtggaacgaa aactcacgtt aagggatttt 6480

ggtcatgaga ttatcaaaaa ggatcttcac ctagatcctt ttaaattaaa aatgaagttt 6540

taaatcaatc taaagtatat atgagtaaac ttggtctgac agttaccaat gcttaatcag 6600

tgaggcacct atctcagcga tctgtctatt tcgttcatcc atagttgcct gactccccgt 6660

cgtgtagata actacgatac gggagggctt accatctggc cccagtgctg caatgatacc 6720

gcgagaccca cgctcaccgg ctccagattt atcagcaata aaccagccag ccggaagggc 6780

cgagcgcaga agtggtcctg caactttatc cgcctccatc cagtctatta attgttgccg 6840

ggaagctaga gtaagtagtt cgccagttaa tagtttgcgc aacgttgttg ccattgctac 6900

aggcatcgtg gtgtcacgct cgtcgtttgg tatggcttca ttcagctccg gttcccaacg 6960

atcaaggcga gttacatgat cccccatgtt gtgcaaaaaa gcggttagct ccttcggtcc 7020

tccgatcgtt gtcagaagta agttggccgc agtgttatca ctcatggtta tggcagcact 7080

gcataattct cttactgtca tgccatccgt aagatgcttt tctgtgactg gtgagtactc 7140

aaccaagtca ttctgagaat agtgtatgcg gcgaccgagt tgctcttgcc cggcgtcaat 7200

acgggataat accgcgccac atagcagaac tttaaaagtg ctcatcattg gaaaacgttc 7260

ttcggggcga aaactctcaa ggatcttacc gctgttgaga tccagttcga tgtaacccac 7320

tcgtgcaccc aactgatctt cagcatcttt tactttcacc agcgtttctg ggtgagcaaa 7380

aacaggaagg caaaatgccg caaaaaaggg aataagggcg acacggaaat gttgaatact 7440

catactcttc ctttttcaat attattgaag catttatcag ggttattgtc tcatgagcgg 7500

atacatattt gaatgtattt agaaaaataa acaaataggg gttccgcgca catttccccg 7560

aaaagtgcca cctgacgtc 7579

<210> 5

<211> 7558

<212> DNA

<213> Artificial Sequence

<220>

<223> Plasmid

<400> 5
gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctaga 900

ggtaccaagc ttggatccca ccatggggtc aaccgtcatc ctcgccctcc tcctggctgt 960

tctccaagga gtctgtgccg aggtgcagct ggtgcagtct ggagcagagg tgaaaaagcc 1020

cggggagtct ctgaagatct cctgtaaggg ttctggatac agctttacca gttactggat 1080

cggctgggtg cgccagatgc ccgggaaagg cctggagtgg atggggatca tctatcctgg 1140

tgactctgat accagataca gcccgtcctt ccaaggccag gtcaccatct cagccgacaa 1200

gtccatcagc accgcctacc tgcagtggag cagcctgaag gcctcggaca ccgccatgta 1260

ttactgtgcg agacggatgg cagcagctgg cccctttgac tactggggcc agggaaccct 1320

ggtcaccgtc tcctcagcct ccaccaaggg cccatcggtc ttccccctgg caccctctag 1380

caagagcacc tctgggggca cagcggccct gggctgcctg gtcaaggact acttccccga 1440

accggtgacg gtgtcgtgga actcaggcgc cctgaccagc ggcgtgcaca ccttcccggc 1500

tgtcctacag tcctcaggac tctactccct cagcagcgtg gtgaccgtgc cctccagcag 1560

cttgggcacc cagacctaca tctgcaacgt gaatcacaag cccagcaaca ccaaggtgga 1620

caagagagtt ggtgagaggc cagcacaggg agggagggtg tctgctggaa gccaggctca 1680

gcgctcctgc ctggacgcat cccggctatg cagtcccagt ccagggcagc aaggcaggcc 1740

ccgtctgcct cttcacccgg aggcctctgc ccgccccact catgctcagg gagagggtct 1800

tctggctttt tccccaggct ctgggcaggc acaggctagg tgcccctaac ccaggccctg 1860

cacacaaagg ggcaggtgct gggctcagac ctgccaagag ccatatccgg gaggaccctg 1920

cccctgacct aagcccaccc caaaggccaa actctccact ccctcagctc ggacaccttc 1980

tctcctccca gattccagta actcccaatc ttctctctgc agagcccaaa tcttgtgaca 2040

aaactcacac atgcccaccg tgcccaggta agccagccca ggcctcgccc tccagctcaa 2100

ggcgggacag gtgccctaga gtagcctgca tccagggaca ggccccagcc gggtgctgac 2160

acgtccacct ccatctcttc ctcagcacct gaactcctgg ggggaccgtc agtcttcctc 2220

ttccccccaa aacccaagga caccctcatg atctcccgga cccctgaggt cacatgcgtg 2280

gtggtggacg tgagccacga agaccctgag gtcaagttca actggtacgt ggacggcgtg 2340

gaggtgcata atgccaagac aaagccgcgg gaggagcagt acaacagcac gtaccgtgtg 2400

gtcagcgtcc tcaccgtcct gcaccaggac tggctgaatg gcaaggagta caagtgcaag 2460

gtctccaaca aagccctccc agcccccatc gagaaaacca tctccaaagc caaaggtggg 2520

acccgtgggg tgcgagggcc acatggacag aggccggctc ggcccaccct ctgccctgag 2580

agtgaccgct gtaccaacct ctgtccctac agggcagccc cgagaaccac aggtgtacac 2640

cctgccccca tcccgggagg agatgaccaa gaaccaggtc agcctgacct gcctggtcaa 2700

aggcttctat cccagcgaca tcgccgtgga gtgggagagc aatgggcagc cggagaacaa 2760

ctacaagacc acgcctcccg tgctggactc cgacggctcc ttcttcctct atagcaagct 2820

caccgtggac aagagcaggt ggcagcaggg gaacgtcttc tcatgctccg tgatgcatga 2880

ggctctgcac aaccactaca cgcagaagag cctctccctg tctccgggta aatgagaatt 2940

cctcgagtct agagggcccg tttaaacccg ctgatcagcc tcgactgtgc cttctagttg 3000

ccagccatct gttgtttgcc cctcccccgt gccttccttg accctggaag gtgccactcc 3060

cactgtcctt tcctaataaa atgaggaaat tgcatcgcat tgtctgagta ggtgtcattc 3120

tattctgggg ggtggggtgg ggcaggacag caagggggag gattgggaag acaatagcag 3180

gcatgctggg gatgcggtgg gctctatggc ttctgaggcg gaaagaacca gctggggctc 3240

tagggggtat ccccacgcgc cctgtagcgg cgcattaagc gcggcgggtg tggtggttac 3300

gcgcagcgtg accgctacac ttgccagcgc cctagcgccc gctcctttcg ctttcttccc 3360

ttcctttctc gccacgttcg ccggctttcc ccgtcaagct ctaaatcggg gcatcccttt 3420

agggttccga tttagtgctt tacggcacct cgaccccaaa aaacttgatt agggtgatgg 3480

ttcacgtagt gggccatcgc cctgatagac ggtttttcgc cctttgacgt tggagtccac 3540

gttctttaat agtggactct tgttccaaac tggaacaaca ctcaacccta tctcggtcta 3600

ttcttttgat ttataaggga ttttggggat ttcggcctat tggttaaaaa atgagctgat 3660

ttaacaaaaa tttaacgcga attaattctg tggaatgtgt gtcagttagg gtgtggaaag 3720

tccccaggct ccccaggcag gcagaagtat gcaaagcatg catctcaatt agtcagcaac 3780

caggtgtgga aagtccccag gctccccagc aggcagaagt atgcaaagca tgcatctcaa 3840

ttagtcagca accatagtcc cgcccctaac tccgcccatc ccgcccctaa ctccgcccag 3900

ttccgcccat tctccgcccc atggctgact aatttttttt atttatgcag aggccgaggc 3960

cgcctctgcc tctgagctat tccagaagta gtgaggaggc ttttttggag gcctaggctt 4020

ttgcaaaaag ctcccgggag cttgtatatc cattttcgga tctgatcagc acgtgatgaa 4080

aaagcctgaa ctcaccgcga cgtctgtcga gaagtttctg atcgaaaagt tcgacagcgt 4140

ctccgacctg atgcagctct cggagggcga agaatctcgt gctttcagct tcgatgtagg 4200

agggcgtgga tatgtcctgc gggtaaatag ctgcgccgat ggtttctaca aagatcgtta 4260

tgtttatcgg cactttgcat cggccgcgct cccgattccg gaagtgcttg acattgggga 4320

attcagcgag agcctgacct attgcatctc ccgccgtgca cagggtgtca cgttgcaaga 4380

cctgcctgaa accgaactgc ccgctgttct gcagccggtc gcggaggcca tggatgcgat 4440

cgctgcggcc gatcttagcc agacgagcgg gttcggccca ttcggaccgc aaggaatcgg 4500

tcaatacact acatggcgtg atttcatatg cgcgattgct gatccccatg tgtatcactg 4560

gcaaactgtg atggacgaca ccgtcagtgc gtccgtcgcg caggctctcg atgagctgat 4620

gctttgggcc gaggactgcc ccgaagtccg gcacctcgtg cacgcggatt tcggctccaa 4680

caatgtcctg acggacaatg gccgcataac agcggtcatt gactggagcg aggcgatgtt 4740

cggggattcc caatacgagg tcgccaacat cttcttctgg aggccgtggt tggcttgtat 4800

ggagcagcag acgcgctact tcgagcggag gcatccggag cttgcaggat cgccgcggct 4860

ccgggcgtat atgctccgca ttggtcttga ccaactctat cagagcttgg ttgacggcaa 4920

tttcgatgat gcagcttggg cgcagggtcg atgcgacgca atcgtccgat ccggagccgg 4980

gactgtcggg cgtacacaaa tcgcccgcag aagcgcggcc gtctggaccg atggctgtgt 5040

agaagtactc gccgatagtg gaaaccgacg ccccagcact cgtccgaggg caaaggaata 5100

gcacgtgcta cgagatttcg attccaccgc cgccttctat gaaaggttgg gcttcggaat 5160

cgttttccgg gacgccggct ggatgatcct ccagcgcggg gatctcatgc tggagttctt 5220

cgcccacccc aacttgttta ttgcagctta taatggttac aaataaagca atagcatcac 5280

aaatttcaca aataaagcat ttttttcact gcattctagt tgtggtttgt ccaaactcat 5340

caatgtatct tatcatgtct gtataccgtc gacctctagc tagagcttgg cgtaatcatg 5400

gtcatagctg tttcctgtgt gaaattgtta tccgctcaca attccacaca acatacgagc 5460

cggaagcata aagtgtaaag cctggggtgc ctaatgagtg agctaactca cattaattgc 5520

gttgcgctca ctgcccgctt tccagtcggg aaacctgtcg tgccagctgc attaatgaat 5580

cggccaacgc gcggggagag gcggtttgcg tattgggcgc tcttccgctt cctcgctcac 5640

tgactcgctg cgctcggtcg ttcggctgcg gcgagcggta tcagctcact caaaggcggt 5700

aatacggtta tccacagaat caggggataa cgcaggaaag aacatgtgag caaaaggcca 5760

gcaaaaggcc aggaaccgta aaaaggccgc gttgctggcg tttttccata ggctccgccc 5820

ccctgacgag catcacaaaa atcgacgctc aagtcagagg tggcgaaacc cgacaggact 5880

ataaagatac caggcgtttc cccctggaag ctccctcgtg cgctctcctg ttccgaccct 5940

gccgcttacc ggatacctgt ccgcctttct cccttcggga agcgtggcgc tttctcaatg 6000

ctcacgctgt aggtatctca gttcggtgta ggtcgttcgc tccaagctgg gctgtgtgca 6060

cgaacccccc gttcagcccg accgctgcgc cttatccggt aactatcgtc ttgagtccaa 6120

cccggtaaga cacgacttat cgccactggc agcagccact ggtaacagga ttagcagagc 6180

gaggtatgta ggcggtgcta cagagttctt gaagtggtgg cctaactacg gctacactag 6240

aaggacagta tttggtatct gcgctctgct gaagccagtt accttcggaa aaagagttgg 6300

tagctcttga tccggcaaac aaaccaccgc tggtagcggt ggtttttttg tttgcaagca 6360

gcagattacg cgcagaaaaa aaggatctca agaagatcct ttgatctttt ctacggggtc 6420

tgacgctcag tggaacgaaa actcacgtta agggattttg gtcatgagat tatcaaaaag 6480

gatcttcacc tagatccttt taaattaaaa atgaagtttt aaatcaatct aaagtatata 6540

tgagtaaact tggtctgaca gttaccaatg cttaatcagt gaggcaccta tctcagcgat 6600

ctgtctattt cgttcatcca tagttgcctg actccccgtc gtgtagataa ctacgatacg 6660

ggagggctta ccatctggcc ccagtgctgc aatgataccg cgagacccac gctcaccggc 6720

tccagattta tcagcaataa accagccagc cggaagggcc gagcgcagaa gtggtcctgc 6780

aactttatcc gcctccatcc agtctattaa ttgttgccgg gaagctagag taagtagttc 6840

gccagttaat agtttgcgca acgttgttgc cattgctaca ggcatcgtgg tgtcacgctc 6900

gtcgtttggt atggcttcat tcagctccgg ttcccaacga tcaaggcgag ttacatgatc 6960

ccccatgttg tgcaaaaaag cggttagctc cttcggtcct ccgatcgttg tcagaagtaa 7020

gttggccgca gtgttatcac tcatggttat ggcagcactg cataattctc ttactgtcat 7080

gccatccgta agatgctttt ctgtgactgg tgagtactca accaagtcat tctgagaata 7140

gtgtatgcgg cgaccgagtt gctcttgccc ggcgtcaata cgggataata ccgcgccaca 7200

tagcagaact ttaaaagtgc tcatcattgg aaaacgttct tcggggcgaa aactctcaag 7260

gatcttaccg ctgttgagat ccagttcgat gtaacccact cgtgcaccca actgatcttc 7320

agcatctttt actttcacca gcgtttctgg gtgagcaaaa acaggaaggc aaaatgccgc 7380

aaaaaaggga ataagggcga cacggaaatg ttgaatactc atactcttcc tttttcaata 7440

ttattgaagc atttatcagg gttattgtct catgagcgga tacatatttg aatgtattta 7500

gaaaaataaa caaatagggg ttccgcgcac atttccccga aaagtgccac ctgacgtc 7558

<210> 6

<211> 7576

<212> DNA

<213> Artificial Sequence

<220>

<223> Plasmid

<400> 6
gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctaga 900

ggtaccaagc ttggatctca ccatggagtt tgggctgtgc tggattttcc tcgttgctct 960

tttaagaggt gtccagtgtc aggtgcagct ggtggagtct gggggaggcg tggtccagcc 1020

tgggaggtcc ctgagactct cctgtgcagc ctctggattc accttcatta gctatggcat 1080

gcactgggtc cgccaggctc caggcaaggg gctggagtgg gtggcagtta tatcatatga 1140

tggaagtaat aaatactatg cagactccgt gaagggccga ttcaccatct ccagagacaa 1200

ttccaagaac acgctgtatc tgcaaatgaa cagcctgaga gctgaggaca cggctgtgta 1260

ttactgtgcg agagtattag tgggagcttt atattattat aactactacg ggatggacgt 1320

ctggggccaa gggaccacgg tcaccgtctc ctcagcctcc accaagggcc catcggtctt 1380

ccccctggca ccctctagca agagcacctc tgggggcaca gcggccctgg gctgcctggt 1440

caaggactac ttccccgaac cggtgacggt gtcgtggaac tcaggcgccc tgaccagcgg 1500

cgtgcacacc ttcccggctg tcctacagtc ctcaggactc tactccctca gcagcgtggt 1560

gaccgtgccc tccagcagct tgggcaccca gacctacatc tgcaacgtga atcacaagcc 1620

cagcaacacc aaggtggaca agagagttgg tgagaggcca gcacagggag ggagggtgtc 1680

tgctggaagc caggctcagc gctcctgcct ggacgcatcc cggctatgca gtcccagtcc 1740

agggcagcaa ggcaggcccc gtctgcctct tcacccggag gcctctgccc gccccactca 1800

tgctcaggga gagggtcttc tggctttttc cccaggctct gggcaggcac aggctaggtg 1860

cccctaaccc aggccctgca cacaaagggg caggtgctgg gctcagacct gccaagagcc 1920

atatccggga ggaccctgcc cctgacctaa gcccacccca aaggccaaac tctccactcc 1980

ctcagctcgg acaccttctc tcctcccaga ttccagtaac tcccaatctt ctctctgcag 2040

agcccaaatc ttgtgacaaa actcacacat gcccaccgtg cccaggtaag ccagcccagg 2100

cctcgccctc cagctcaagg cgggacaggt gccctagagt agcctgcatc cagggacagg 2160

ccccagccgg gtgctgacac gtccacctcc atctcttcct cagcacctga actcctgggg 2220

ggaccgtcag tcttcctctt ccccccaaaa cccaaggaca ccctcatgat ctcccggacc 2280

cctgaggtca catgcgtggt ggtggacgtg agccacgaag accctgaggt caagttcaac 2340

tggtacgtgg acggcgtgga ggtgcataat gccaagacaa agccgcggga ggagcagtac 2400

aacagcacgt accgtgtggt cagcgtcctc accgtcctgc accaggactg gctgaatggc 2460

aaggagtaca agtgcaaggt ctccaacaaa gccctcccag cccccatcga gaaaaccatc 2520

tccaaagcca aaggtgggac ccgtggggtg cgagggccac atggacagag gccggctcgg 2580

cccaccctct gccctgagag tgaccgctgt accaacctct gtccctacag ggcagccccg 2640

agaaccacag gtgtacaccc tgcccccatc ccgggaggag atgaccaaga accaggtcag 2700

cctgacctgc ctggtcaaag gcttctatcc cagcgacatc gccgtggagt gggagagcaa 2760

tgggcagccg gagaacaact acaagaccac gcctcccgtg ctggactccg acggctcctt 2820

cttcctctat agcaagctca ccgtggacaa gagcaggtgg cagcagggga acgtcttctc 2880

atgctccgtg atgcatgagg ctctgcacaa ccactacacg cagaagagcc tctccctgtc 2940

tccgggtaaa tgagaattcc tcgagtctag agggcccgtt taaacccgct gatcagcctc 3000

gactgtgcct tctagttgcc agccatctgt tgtttgcccc tcccccgtgc cttccttgac 3060

cctggaaggt gccactccca ctgtcctttc ctaataaaat gaggaaattg catcgcattg 3120

tctgagtagg tgtcattcta ttctgggggg tggggtgggg caggacagca agggggagga 3180

ttgggaagac aatagcaggc atgctgggga tgcggtgggc tctatggctt ctgaggcgga 3240

aagaaccagc tggggctcta gggggtatcc ccacgcgccc tgtagcggcg cattaagcgc 3300

ggcgggtgtg gtggttacgc gcagcgtgac cgctacactt gccagcgccc tagcgcccgc 3360

tcctttcgct ttcttccctt cctttctcgc cacgttcgcc ggctttcccc gtcaagctct 3420

aaatcggggc atccctttag ggttccgatt tagtgcttta cggcacctcg accccaaaaa 3480

acttgattag ggtgatggtt cacgtagtgg gccatcgccc tgatagacgg tttttcgccc 3540

tttgacgttg gagtccacgt tctttaatag tggactcttg ttccaaactg gaacaacact 3600

caaccctatc tcggtctatt cttttgattt ataagggatt ttggggattt cggcctattg 3660

gttaaaaaat gagctgattt aacaaaaatt taacgcgaat taattctgtg gaatgtgtgt 3720

cagttagggt gtggaaagtc cccaggctcc ccaggcaggc agaagtatgc aaagcatgca 3780

tctcaattag tcagcaacca ggtgtggaaa gtccccaggc tccccagcag gcagaagtat 3840

gcaaagcatg catctcaatt agtcagcaac catagtcccg cccctaactc cgcccatccc 3900

gcccctaact ccgcccagtt ccgcccattc tccgccccat ggctgactaa ttttttttat 3960

ttatgcagag gccgaggccg cctctgcctc tgagctattc cagaagtagt gaggaggctt 4020

ttttggaggc ctaggctttt gcaaaaagct cccgggagct tgtatatcca ttttcggatc 4080

tgatcagcac gtgatgaaaa agcctgaact caccgcgacg tctgtcgaga agtttctgat 4140

cgaaaagttc gacagcgtct ccgacctgat gcagctctcg gagggcgaag aatctcgtgc 4200

tttcagcttc gatgtaggag ggcgtggata tgtcctgcgg gtaaatagct gcgccgatgg 4260

tttctacaaa gatcgttatg tttatcggca ctttgcatcg gccgcgctcc cgattccgga 4320

agtgcttgac attggggaat tcagcgagag cctgacctat tgcatctccc gccgtgcaca 4380

gggtgtcacg ttgcaagacc tgcctgaaac cgaactgccc gctgttctgc agccggtcgc 4440

ggaggccatg gatgcgatcg ctgcggccga tcttagccag acgagcgggt tcggcccatt 4500

cggaccgcaa ggaatcggtc aatacactac atggcgtgat ttcatatgcg cgattgctga 4560

tccccatgtg tatcactggc aaactgtgat ggacgacacc gtcagtgcgt ccgtcgcgca 4620

ggctctcgat gagctgatgc tttgggccga ggactgcccc gaagtccggc acctcgtgca 4680

cgcggatttc ggctccaaca atgtcctgac ggacaatggc cgcataacag cggtcattga 4740

ctggagcgag gcgatgttcg gggattccca atacgaggtc gccaacatct tcttctggag 4800

gccgtggttg gcttgtatgg agcagcagac gcgctacttc gagcggaggc atccggagct 4860

tgcaggatcg ccgcggctcc gggcgtatat gctccgcatt ggtcttgacc aactctatca 4920

gagcttggtt gacggcaatt tcgatgatgc agcttgggcg cagggtcgat gcgacgcaat 4980

cgtccgatcc ggagccggga ctgtcgggcg tacacaaatc gcccgcagaa gcgcggccgt 5040

ctggaccgat ggctgtgtag aagtactcgc cgatagtgga aaccgacgcc ccagcactcg 5100

tccgagggca aaggaatagc acgtgctacg agatttcgat tccaccgccg ccttctatga 5160

aaggttgggc ttcggaatcg ttttccggga cgccggctgg atgatcctcc agcgcgggga 5220

tctcatgctg gagttcttcg cccaccccaa cttgtttatt gcagcttata atggttacaa 5280

ataaagcaat agcatcacaa atttcacaaa taaagcattt ttttcactgc attctagttg 5340

tggtttgtcc aaactcatca atgtatctta tcatgtctgt ataccgtcga cctctagcta 5400

gagcttggcg taatcatggt catagctgtt tcctgtgtga aattgttatc cgctcacaat 5460

tccacacaac atacgagccg gaagcataaa gtgtaaagcc tggggtgcct aatgagtgag 5520

ctaactcaca ttaattgcgt tgcgctcact gcccgctttc cagtcgggaa acctgtcgtg 5580

ccagctgcat taatgaatcg gccaacgcgc ggggagaggc ggtttgcgta ttgggcgctc 5640

ttccgcttcc tcgctcactg actcgctgcg ctcggtcgtt cggctgcggc gagcggtatc 5700

agctcactca aaggcggtaa tacggttatc cacagaatca ggggataacg caggaaagaa 5760

catgtgagca aaaggccagc aaaaggccag gaaccgtaaa aaggccgcgt tgctggcgtt 5820

tttccatagg ctccgccccc ctgacgagca tcacaaaaat cgacgctcaa gtcagaggtg 5880

gcgaaacccg acaggactat aaagatacca ggcgtttccc cctggaagct ccctcgtgcg 5940

ctctcctgtt ccgaccctgc cgcttaccgg atacctgtcc gcctttctcc cttcgggaag 6000

cgtggcgctt tctcaatgct cacgctgtag gtatctcagt tcggtgtagg tcgttcgctc 6060

caagctgggc tgtgtgcacg aaccccccgt tcagcccgac cgctgcgcct tatccggtaa 6120

ctatcgtctt gagtccaacc cggtaagaca cgacttatcg ccactggcag cagccactgg 6180

taacaggatt agcagagcga ggtatgtagg cggtgctaca gagttcttga agtggtggcc 6240

taactacggc tacactagaa ggacagtatt tggtatctgc gctctgctga agccagttac 6300

cttcggaaaa agagttggta gctcttgatc cggcaaacaa accaccgctg gtagcggtgg 6360

tttttttgtt tgcaagcagc agattacgcg cagaaaaaaa ggatctcaag aagatccttt 6420

gatcttttct acggggtctg acgctcagtg gaacgaaaac tcacgttaag ggattttggt 6480

catgagatta tcaaaaagga tcttcaccta gatcctttta aattaaaaat gaagttttaa 6540

atcaatctaa agtatatatg agtaaacttg gtctgacagt taccaatgct taatcagtga 6600

ggcacctatc tcagcgatct gtctatttcg ttcatccata gttgcctgac tccccgtcgt 6660

gtagataact acgatacggg agggcttacc atctggcccc agtgctgcaa tgataccgcg 6720

agacccacgc tcaccggctc cagatttatc agcaataaac cagccagccg gaagggccga 6780

gcgcagaagt ggtcctgcaa ctttatccgc ctccatccag tctattaatt gttgccggga 6840

agctagagta agtagttcgc cagttaatag tttgcgcaac gttgttgcca ttgctacagg 6900

catcgtggtg tcacgctcgt cgtttggtat ggcttcattc agctccggtt cccaacgatc 6960

aaggcgagtt acatgatccc ccatgttgtg caaaaaagcg gttagctcct tcggtcctcc 7020

gatcgttgtc agaagtaagt tggccgcagt gttatcactc atggttatgg cagcactgca 7080

taattctctt actgtcatgc catccgtaag atgcttttct gtgactggtg agtactcaac 7140

caagtcattc tgagaatagt gtatgcggcg accgagttgc tcttgcccgg cgtcaatacg 7200

ggataatacc gcgccacata gcagaacttt aaaagtgctc atcattggaa aacgttcttc 7260

ggggcgaaaa ctctcaagga tcttaccgct gttgagatcc agttcgatgt aacccactcg 7320

tgcacccaac tgatcttcag catcttttac tttcaccagc gtttctgggt gagcaaaaac 7380

aggaaggcaa aatgccgcaa aaaagggaat aagggcgaca cggaaatgtt gaatactcat 7440

actcttcctt tttcaatatt attgaagcat ttatcagggt tattgtctca tgagcggata 7500

catatttgaa tgtatttaga aaaataaaca aataggggtt ccgcgcacat ttccccgaaa 7560

agtgccacct gacgtc 7576

<210> 7

<211> 7561

<212> DNA

<213> Artificial Sequence

<220>

<223> Plasmid

<400> 7
gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctaga 900

ggtaccggat ctcaccatgg agttggggct gagctgggtt ttcctcgttg ctcttttaag 960

aggtgtccag tgtcaggagc agctggtgga gtctggggga ggcgtggtcc agcctgggag 1020

gtccctgaga ctctcctgtg cagcgtctgg attcaccttc agtacctatg gcatgcactg 1080

ggtccgccag gctccaggca aggggctgga gtgggtggca gttacatggc atgatggaag 1140

taataaatac tatgcagact ccgtgaaggg ccgattcacc atctccagag acaactccaa 1200

gaacacgctg tatctgcaaa tgaacagcct gagagccgag gacacggctg tgtattactg 1260

tgcgagagga ggagtgggag caacttacta ctactactac ggtatggacg tctggggcca 1320

agggaccacg gtcaccgtct cctcagcctc caccaagggc ccatcggtct tccccctggc 1380

accctctagc aagagcacct ctgggggcac agcggccctg ggctgcctgg tcaaggacta 1440

cttccccgaa ccggtgacgg tgtcgtggaa ctcaggcgcc ctgaccagcg gcgtgcacac 1500

cttcccggct gtcctacagt cctcaggact ctactccctc agcagcgtgg tgaccgtgcc 1560

ctccagcagc ttgggcaccc agacctacat ctgcaacgtg aatcacaagc ccagcaacac 1620

caaggtggac aagagagttg gtgagaggcc agcacaggga gggagggtgt ctgctggaag 1680

ccaggctcag cgctcctgcc tggacgcatc ccggctatgc agtcccagtc cagggcagca 1740

aggcaggccc cgtctgcctc ttcacccgga ggcctctgcc cgccccactc atgctcaggg 1800

agagggtctt ctggcttttt ccccaggctc tgggcaggca caggctaggt gcccctaacc 1860

caggccctgc acacaaaggg gcaggtgctg ggctcagacc tgccaagagc catatccggg 1920

aggaccctgc ccctgaccta agcccacccc aaaggccaaa ctctccactc cctcagctcg 1980

gacaccttct ctcctcccag attccagtaa ctcccaatct tctctctgca gagcccaaat 2040

cttgtgacaa aactcacaca tgcccaccgt gcccaggtaa gccagcccag gcctcgccct 2100

ccagctcaag gcgggacagg tgccctagag tagcctgcat ccagggacag gccccagccg 2160

ggtgctgaca cgtccacctc catctcttcc tcagcacctg aactcctggg gggaccgtca 2220

gtcttcctct tccccccaaa acccaaggac accctcatga tctcccggac ccctgaggtc 2280

acatgcgtgg tggtggacgt gagccacgaa gaccctgagg tcaagttcaa ctggtacgtg 2340

gacggcgtgg aggtgcataa tgccaagaca aagccgcggg aggagcagta caacagcacg 2400

taccgtgtgg tcagcgtcct caccgtcctg caccaggact ggctgaatgg caaggagtac 2460

aagtgcaagg tctccaacaa agccctccca gcccccatcg agaaaaccat ctccaaagcc 2520

aaaggtggga cccgtggggt gcgagggcca catggacaga ggccggctcg gcccaccctc 2580

tgccctgaga gtgaccgctg taccaacctc tgtccctaca gggcagcccc gagaaccaca 2640

ggtgtacacc ctgcccccat cccgggagga gatgaccaag aaccaggtca gcctgacctg 2700

cctggtcaaa ggcttctatc ccagcgacat cgccgtggag tgggagagca atgggcagcc 2760

ggagaacaac tacaagacca cgcctcccgt gctggactcc gacggctcct tcttcctcta 2820

tagcaagctc accgtggaca agagcaggtg gcagcagggg aacgtcttct catgctccgt 2880

gatgcatgag gctctgcaca accactacac gcagaagagc ctctccctgt ctccgggtaa 2940

atgactcgag tctagagggc ccgtttaaac ccgctgatca gcctcgactg tgccttctag 3000

ttgccagcca tctgttgttt gcccctcccc cgtgccttcc ttgaccctgg aaggtgccac 3060

tcccactgtc ctttcctaat aaaatgagga aattgcatcg cattgtctga gtaggtgtca 3120

ttctattctg gggggtgggg tggggcagga cagcaagggg gaggattggg aagacaatag 3180

caggcatgct ggggatgcgg tgggctctat ggcttctgag gcggaaagaa ccagctgggg 3240

ctctaggggg tatccccacg cgccctgtag cggcgcatta agcgcggcgg gtgtggtggt 3300

tacgcgcagc gtgaccgcta cacttgccag cgccctagcg cccgctcctt tcgctttctt 3360

cccttccttt ctcgccacgt tcgccggctt tccccgtcaa gctctaaatc ggggcatccc 3420

tttagggttc cgatttagtg ctttacggca cctcgacccc aaaaaacttg attagggtga 3480

tggttcacgt agtgggccat cgccctgata gacggttttt cgccctttga cgttggagtc 3540

cacgttcttt aatagtggac tcttgttcca aactggaaca acactcaacc ctatctcggt 3600

ctattctttt gatttataag ggattttggg gatttcggcc tattggttaa aaaatgagct 3660

gatttaacaa aaatttaacg cgaattaatt ctgtggaatg tgtgtcagtt agggtgtgga 3720

aagtccccag gctccccagg caggcagaag tatgcaaagc atgcatctca attagtcagc 3780

aaccaggtgt ggaaagtccc caggctcccc agcaggcaga agtatgcaaa gcatgcatct 3840

caattagtca gcaaccatag tcccgcccct aactccgccc atcccgcccc taactccgcc 3900

cagttccgcc cattctccgc cccatggctg actaattttt tttatttatg cagaggccga 3960

ggccgcctct gcctctgagc tattccagaa gtagtgagga ggcttttttg gaggcctagg 4020

cttttgcaaa aagctcccgg gagcttgtat atccattttc ggatctgatc agcacgtgat 4080

gaaaaagcct gaactcaccg cgacgtctgt cgagaagttt ctgatcgaaa agttcgacag 4140

cgtctccgac ctgatgcagc tctcggaggg cgaagaatct cgtgctttca gcttcgatgt 4200

aggagggcgt ggatatgtcc tgcgggtaaa tagctgcgcc gatggtttct acaaagatcg 4260

ttatgtttat cggcactttg catcggccgc gctcccgatt ccggaagtgc ttgacattgg 4320

ggaattcagc gagagcctga cctattgcat ctcccgccgt gcacagggtg tcacgttgca 4380

agacctgcct gaaaccgaac tgcccgctgt tctgcagccg gtcgcggagg ccatggatgc 4440

gatcgctgcg gccgatctta gccagacgag cgggttcggc ccattcggac cgcaaggaat 4500

cggtcaatac actacatggc gtgatttcat atgcgcgatt gctgatcccc atgtgtatca 4560

ctggcaaact gtgatggacg acaccgtcag tgcgtccgtc gcgcaggctc tcgatgagct 4620

gatgctttgg gccgaggact gccccgaagt ccggcacctc gtgcacgcgg atttcggctc 4680

caacaatgtc ctgacggaca atggccgcat aacagcggtc attgactgga gcgaggcgat 4740

gttcggggat tcccaatacg aggtcgccaa catcttcttc tggaggccgt ggttggcttg 4800

tatggagcag cagacgcgct acttcgagcg gaggcatccg gagcttgcag gatcgccgcg 4860

gctccgggcg tatatgctcc gcattggtct tgaccaactc tatcagagct tggttgacgg 4920

caatttcgat gatgcagctt gggcgcaggg tcgatgcgac gcaatcgtcc gatccggagc 4980

cgggactgtc gggcgtacac aaatcgcccg cagaagcgcg gccgtctgga ccgatggctg 5040

tgtagaagta ctcgccgata gtggaaaccg acgccccagc actcgtccga gggcaaagga 5100

atagcacgtg ctacgagatt tcgattccac cgccgccttc tatgaaaggt tgggcttcgg 5160

aatcgttttc cgggacgccg gctggatgat cctccagcgc ggggatctca tgctggagtt 5220

cttcgcccac cccaacttgt ttattgcagc ttataatggt tacaaataaa gcaatagcat 5280

cacaaatttc acaaataaag catttttttc actgcattct agttgtggtt tgtccaaact 5340

catcaatgta tcttatcatg tctgtatacc gtcgacctct agctagagct tggcgtaatc 5400

atggtcatag ctgtttcctg tgtgaaattg ttatccgctc acaattccac acaacatacg 5460

agccggaagc ataaagtgta aagcctgggg tgcctaatga gtgagctaac tcacattaat 5520

tgcgttgcgc tcactgcccg ctttccagtc gggaaacctg tcgtgccagc tgcattaatg 5580

aatcggccaa cgcgcgggga gaggcggttt gcgtattggg cgctcttccg cttcctcgct 5640

cactgactcg ctgcgctcgg tcgttcggct gcggcgagcg gtatcagctc actcaaaggc 5700

ggtaatacgg ttatccacag aatcagggga taacgcagga aagaacatgt gagcaaaagg 5760

ccagcaaaag gccaggaacc gtaaaaaggc cgcgttgctg gcgtttttcc ataggctccg 5820

cccccctgac gagcatcaca aaaatcgacg ctcaagtcag aggtggcgaa acccgacagg 5880

actataaaga taccaggcgt ttccccctgg aagctccctc gtgcgctctc ctgttccgac 5940

cctgccgctt accggatacc tgtccgcctt tctcccttcg ggaagcgtgg cgctttctca 6000

atgctcacgc tgtaggtatc tcagttcggt gtaggtcgtt cgctccaagc tgggctgtgt 6060

gcacgaaccc cccgttcagc ccgaccgctg cgccttatcc ggtaactatc gtcttgagtc 6120

caacccggta agacacgact tatcgccact ggcagcagcc actggtaaca ggattagcag 6180

agcgaggtat gtaggcggtg ctacagagtt cttgaagtgg tggcctaact acggctacac 6240

tagaaggaca gtatttggta tctgcgctct gctgaagcca gttaccttcg gaaaaagagt 6300

tggtagctct tgatccggca aacaaaccac cgctggtagc ggtggttttt ttgtttgcaa 6360

gcagcagatt acgcgcagaa aaaaaggatc tcaagaagat cctttgatct tttctacggg 6420

gtctgacgct cagtggaacg aaaactcacg ttaagggatt ttggtcatga gattatcaaa 6480

aaggatcttc acctagatcc ttttaaatta aaaatgaagt tttaaatcaa tctaaagtat 6540

atatgagtaa acttggtctg acagttacca atgcttaatc agtgaggcac ctatctcagc 6600

gatctgtcta tttcgttcat ccatagttgc ctgactcccc gtcgtgtaga taactacgat 6660

acgggagggc ttaccatctg gccccagtgc tgcaatgata ccgcgagacc cacgctcacc 6720

ggctccagat ttatcagcaa taaaccagcc agccggaagg gccgagcgca gaagtggtcc 6780

tgcaacttta tccgcctcca tccagtctat taattgttgc cgggaagcta gagtaagtag 6840

ttcgccagtt aatagtttgc gcaacgttgt tgccattgct acaggcatcg tggtgtcacg 6900

ctcgtcgttt ggtatggctt cattcagctc cggttcccaa cgatcaaggc gagttacatg 6960

atcccccatg ttgtgcaaaa aagcggttag ctccttcggt cctccgatcg ttgtcagaag 7020

taagttggcc gcagtgttat cactcatggt tatggcagca ctgcataatt ctcttactgt 7080

catgccatcc gtaagatgct tttctgtgac tggtgagtac tcaaccaagt cattctgaga 7140

atagtgtatg cggcgaccga gttgctcttg cccggcgtca atacgggata ataccgcgcc 7200

acatagcaga actttaaaag tgctcatcat tggaaaacgt tcttcggggc gaaaactctc 7260

aaggatctta ccgctgttga gatccagttc gatgtaaccc actcgtgcac ccaactgatc 7320

ttcagcatct tttactttca ccagcgtttc tgggtgagca aaaacaggaa ggcaaaatgc 7380

cgcaaaaaag ggaataaggg cgacacggaa atgttgaata ctcatactct tcctttttca 7440

atattattga agcatttatc agggttattg tctcatgagc ggatacatat ttgaatgtat 7500

ttagaaaaat aaacaaatag gggttccgcg cacatttccc cgaaaagtgc cacctgacgt 7560

c 7561

<210> 8

<211>  6082

<212>  DNA

<213>  Artificial Sequence

<220>

<223>  Plasmid

<400>  8
gacggatcgg gagatctccc gatcccctat ggtcgactct cagtacaatc tgctctgatg      60

ccgcatagtt aagccagtat ctgctccctg cttgtgtgtt ggaggtcgct gagtagtgcg     120

cgagcaaaat ttaagctaca acaaggcaag gcttgaccga caattgcatg aagaatctgc     180

ttagggttag gcgttttgcg ctgcttcgcg atgtacgggc cagatatacg cgttgacatt     240

gattattgac tagttattaa tagtaatcaa ttacggggtc attagttcat agcccatata     300

tggagttccg cgttacataa cttacggtaa atggcccgcc tggctgaccg cccaacgacc     360

cccgcccatt gacgtcaata atgacgtatg ttcccatagt aacgccaata gggactttcc     420

attgacgtca atgggtggac tatttacggt aaactgccca cttggcagta catcaagtgt     480

atcatatgcc aagtacgccc cctattgacg tcaatgacgg taaatggccc gcctggcatt     540

atgcccagta catgacctta tgggactttc ctacttggca gtacatctac gtattagtca     600

tcgctattac catggtgatg cggttttggc agtacatcaa tgggcgtgga tagcggtttg     660

actcacgggg atttccaagt ctccacccca ttgacgtcaa tgggagtttg ttttggcacc     720

aaaatcaacg ggactttcca aaatgtcgta acaactccgc cccattgacg caaatgggcg     780

gtaggcgtgt acggtgggag gtctatataa gcagagctct ctggctaact agagaaccca     840

ctgcttactg gcttatcgaa attaatacga ctcactatag ggagacccaa gctggctaga     900

aagcttggat ctcaccatga gggtccctgc tcagctcctg gggctcctgc tgctctggct     960

cccagatacc agatgtgaca tccagatgac ccagtctcca tcctccctgt ctgcatctgt    1020

aggagacaga gtcaccatca cttgccgggc gagtcagggc attagcaatt atttagcctg    1080

gtatcagcag aaaacaggga aagttcctaa gttcctgatc tatgaagcat ccactttgca    1140

atcaggggtc ccatctcggt tcagtggcgg tggatctggg acagatttca ctctcaccat    1200

cagcagcctg cagcctgaag atgttgcaac ttattactgt caaaattata acagtgcccc    1260

attcactttc ggccctggga ccaaagtgga tatcaaacga actgtggctg caccctctgt    1320

cttcatcttc ccgccatctg atgagcagtt gaaatctgga actgctagcg ttgtgtgcct    1380

gctgaataac ttctatccca gagaggccaa agtacagtgg aaggtggata acgccctcca    1440

atcgggtaac tcccaggaga gtgtcacaga gcaggacagc aaggacagca cctacagcct    1500

cagcagcacc ctgacgctga gcaaagcaga ctacgagaaa cacaaagtct acgcctgcga    1560

agtcacccat cagggcctga gctcgcccgt cacaaagagc ttcaacaggg gagagtgtta 1620

ggaattcgcg gccgctcgag tctagagggc ccgtttaaac ccgctgatca gcctcgactg 1680

tgccttctag ttgccagcca tctgttgttt gcccctcccc cgtgccttcc ttgaccctgg 1740

aaggtgccac tcccactgtc ctttcctaat aaaatgagga aattgcatcg cattgtctga 1800

gtaggtgtca ttctattctg gggggtgggg tggggcagga cagcaagggg gaggattggg 1860

aagacaatag caggcatgct ggggatgcgg tgggctctat ggcttctgag gcggaaagaa 1920

ccagctgggg ctctaggggg tatccccacg cgccctgtag cggcgcatta agcgcggcgg 1980

gtgtggtggt tacgcgcagc gtgaccgcta cacttgccag cgccctagcg cccgctcctt 2040

tcgctttctt cccttccttt ctcgccacgt tcgccggctt tccccgtcaa gctctaaatc 2100

ggggcatccc tttagggttc cgatttagtg ctttacggca cctcgacccc aaaaaacttg 2160

attagggtga tggttcacgt agtgggccat cgccctgata gacggttttt cgccctttga 2220

cgttggagtc cacgttcttt aatagtggac tcttgttcca aactggaaca acactcaacc 2280

ctatctcggt ctattctttt gatttataag ggattttggg gatttcggcc tattggttaa 2340

aaaatgagct gatttaacaa aaatttaacg cgaattaatt ctgtggaatg tgtgtcagtt 2400

agggtgtgga aagtccccag gctccccagg caggcagaag tatgcaaagc atgcatctca 2460

attagtcagc aaccaggtgt ggaaagtccc caggctcccc agcaggcaga agtatgcaaa 2520

gcatgcatct caattagtca gcaaccatag tcccgcccct aactccgccc atcccgcccc 2580

taactccgcc cagttccgcc cattctccgc cccatggctg actaattttt tttatttatg 2640

cagaggccga ggccgcctct gcctctgagc tattccagaa gtagtgagga ggcttttttg 2700

gaggcctagg cttttgcaaa aagctcccgg gagcttgtat atccattttc ggatctgatc 2760

aagagacagg atgaggatcg tttcgcatga ttgaacaaga tggattgcac gcaggttctc 2820

cggccgcttg ggtggagagg ctattcggct atgactgggc acaacagaca atcggctgct 2880

ctgatgccgc cgtgttccgg ctgtcagcgc aggggcgccc ggttcttttt gtcaagaccg 2940

acctgtccgg tgccctgaat gaactgcagg acgaggcagc gcggctatcg tggctggcca 3000

cgacgggcgt tccttgcgca gctgtgctcg acgttgtcac tgaagcggga agggactggc 3060

tgctattggg cgaagtgccg gggcaggatc tcctgtcatc tcaccttgct cctgccgaga 3120

aagtatccat catggctgat gcaatgcggc ggctgcatac gcttgatccg gctacctgcc 3180

cattcgacca ccaagcgaaa catcgcatcg agcgagcacg tactcggatg gaagccggtc 3240

ttgtcgatca ggatgatctg gacgaagagc atcaggggct cgcgccagcc gaactgttcg 3300

ccaggctcaa ggcgcgcatg cccgacggcg aggatctcgt cgtgacccat ggcgatgcct 3360

gcttgccgaa tatcatggtg gaaaatggcc gcttttctgg attcatcgac tgtggccggc 3420

tgggtgtggc ggaccgctat caggacatag cgttggctac ccgtgatatt gctgaagagc 3480

ttggcggcga atgggctgac cgcttcctcg tgctttacgg tatcgccgct cccgattcgc 3540

agcgcatcgc cttctatcgc cttcttgacg agttcttctg agcgggactc tggggttcga 3600

aatgaccgac caagcgacgc ccaacctgcc atcacgagat ttcgattcca ccgccgcctt 3660

ctatgaaagg ttgggcttcg gaatcgtttt ccgggacgcc ggctggatga tcctccagcg 3720

cggggatctc atgctggagt tcttcgccca ccccaacttg tttattgcag cttataatgg 3780

ttacaaataa agcaatagca tcacaaattt cacaaataaa gcattttttt cactgcattc 3840

tagttgtggt ttgtccaaac tcatcaatgt atcttatcat gtctgtatac cgtcgacctc 3900

tagctagagc ttggcgtaat catggtcata gctgtttcct gtgtgaaatt gttatccgct 3960

cacaattcca cacaacatac gagccggaag cataaagtgt aaagcctggg gtgcctaatg 4020

agtgagctaa ctcacattaa ttgcgttgcg ctcactgccc gctttccagt cgggaaacct 4080

gtcgtgccag ctgcattaat gaatcggcca acgcgcgggg agaggcggtt tgcgtattgg 4140

gcgctcttcc gcttcctcgc tcactgactc gctgcgctcg gtcgttcggc tgcggcgagc 4200

ggtatcagct cactcaaagg cggtaatacg gttatccaca gaatcagggg ataacgcagg 4260

aaagaacatg tgagcaaaag gccagcaaaa ggccaggaac cgtaaaaagg ccgcgttgct 4320

ggcgtttttc cataggctcc gcccccctga cgagcatcac aaaaatcgac gctcaagtca 4380

gaggtggcga aacccgacag gactataaag ataccaggcg tttccccctg gaagctccct 4440

cgtgcgctct cctgttccga ccctgccgct taccggatac ctgtccgcct ttctcccttc 4500

gggaagcgtg gcgctttctc aatgctcacg ctgtaggtat ctcagttcgg tgtaggtcgt 4560

tcgctccaag ctgggctgtg tgcacgaacc ccccgttcag cccgaccgct gcgccttatc 4620

cggtaactat cgtcttgagt ccaacccggt aagacacgac ttatcgccac tggcagcagc 4680

cactggtaac aggattagca gagcgaggta tgtaggcggt gctacagagt tcttgaagtg 4740

gtggcctaac tacggctaca ctagaaggac agtatttggt atctgcgctc tgctgaagcc 4800

agttaccttc ggaaaaagag ttggtagctc ttgatccggc aaacaaacca ccgctggtag 4860

cggtggtttt tttgtttgca agcagcagat tacgcgcaga aaaaaaggat ctcaagaaga 4920

tcctttgatc ttttctacgg ggtctgacgc tcagtggaac gaaaactcac gttaagggat 4980

tttggtcatg agattatcaa aaaggatctt cacctagatc cttttaaatt aaaaatgaag 5040

ttttaaatca atctaaagta tatatgagta aacttggtct gacagttacc aatgcttaat 5100

cagtgaggca cctatctcag cgatctgtct atttcgttca tccatagttg cctgactccc 5160

cgtcgtgtag ataactacga tacgggaggg cttaccatct ggccccagtg ctgcaatgat 5220

accgcgagac ccacgctcac cggctccaga tttatcagca ataaaccagc cagccggaag 5280

ggccgagcgc agaagtggtc ctgcaacttt atccgcctcc atccagtcta ttaattgttg 5340

ccgggaagct agagtaagta gttcgccagt taatagtttg cgcaacgttg ttgccattgc 5400

tacaggcatc gtggtgtcac gctcgtcgtt tggtatggct tcattcagct ccggttccca 5460

acgatcaagg cgagttacat gatcccccat gttgtgcaaa aaagcggtta gctccttcgg 5520

tcctccgatc gttgtcagaa gtaagttggc cgcagtgtta tcactcatgg ttatggcagc 5580

actgcataat tctcttactg tcatgccatc cgtaagatgc ttttctgtga ctggtgagta 5640

ctcaaccaag tcattctgag aatagtgtat gcggcgaccg agttgctctt gcccggcgtc 5700

aatacgggat aataccgcgc cacatagcag aactttaaaa gtgctcatca ttggaaaacg 5760

ttcttcgggg cgaaaactct caaggatctt accgctgttg agatccagtt cgatgtaacc 5820

cactcgtgca cccaactgat cttcagcatc ttttactttc accagcgttt ctgggtgagc 5880

aaaaacagga aggcaaaatg ccgcaaaaaa gggaataagg gcgacacgga aatgttgaat 5940

actcatactc ttcctttttc aatattattg aagcatttat cagggttatt gtctcatgag 6000

cggatacata tttgaatgta tttagaaaaa taaacaaata ggggttccgc gcacatttcc 6060

ccgaaaagtg ccacctgacg tc 6082

<210> 9

<211> 6082

<212> DNA

<213> Artificial Sequence

<220>

<223> Plasmid

<400> 9
gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctaga 900

aagcttggat ctcaccatga gggtccccgc tcagctcctg gggctcctgc tgctctgttt 960

cccaggtgcc agatgtgaca tccagatgac ccagtctcca tcctcactgt ctgcatctgt 1020

aggagacaga gtcaccatca cttgtcgggc gagtcagggc attaccaatt atttagcctg 1080

gtttcagcag aaaccaggga aagcccctaa gtcccttatc tatgctgcat ccagtttgca 1140

aagtggggtc ccatcaaagt tcagcggcag tggatctggg acagatttca gtctcaccat 1200

cagcagcctg cagcctgaag attttgcaac ttattactgc caacagtata atagttaccc 1260

gatcaccttc ggccaaggga cacgactgga gattaaacga actgtggctg caccatctgt 1320

cttcatcttc ccgccatctg atgagcagtt gaaatctgga actgctagcg ttgtgtgcct 1380

gctgaataac ttctatccca gagaggccaa agtacagtgg aaggtggata acgccctcca 1440

atcgggtaac tcccaggaga gtgtcacaga gcaggacagc aaggacagca cctacagcct 1500

cagcagcacc ctgacgctga gcaaagcaga ctacgagaaa cacaaagtct acgcctgcga 1560

agtcacccat cagggcctga gctcgcccgt cacaaagagc ttcaacaggg gagagtgtta 1620

ggaattcgcg gccgctcgag tctagagggc ccgtttaaac ccgctgatca gcctcgactg 1680

tgccttctag ttgccagcca tctgttgttt gcccctcccc cgtgccttcc ttgaccctgg 1740

aaggtgccac tcccactgtc ctttcctaat aaaatgagga aattgcatcg cattgtctga 1800

gtaggtgtca ttctattctg gggggtgggg tggggcagga cagcaagggg gaggattggg 1860

aagacaatag caggcatgct ggggatgcgg tgggctctat ggcttctgag gcggaaagaa 1920

ccagctgggg ctctaggggg tatccccacg cgccctgtag cggcgcatta agcgcggcgg 1980

gtgtggtggt tacgcgcagc gtgaccgcta cacttgccag cgccctagcg cccgctcctt 2040

tcgctttctt cccttccttt ctcgccacgt tcgccggctt tccccgtcaa gctctaaatc 2100

ggggcatccc tttagggttc cgatttagtg ctttacggca cctcgacccc aaaaaacttg 2160

attagggtga tggttcacgt agtgggccat cgccctgata gacggttttt cgccctttga 2220

cgttggagtc cacgttcttt aatagtggac tcttgttcca aactggaaca acactcaacc 2280

ctatctcggt ctattctttt gatttataag ggattttggg gatttcggcc tattggttaa 2340

aaaatgagct gatttaacaa aaatttaacg cgaattaatt ctgtggaatg tgtgtcagtt 2400

agggtgtgga aagtccccag gctccccagg caggcagaag tatgcaaagc atgcatctca 2460

attagtcagc aaccaggtgt ggaaagtccc caggctcccc agcaggcaga agtatgcaaa 2520

gcatgcatct caattagtca gcaaccatag tcccgcccct aactccgccc atcccgcccc 2580

taactccgcc cagttccgcc cattctccgc cccatggctg actaattttt tttatttatg 2640

cagaggccga ggccgcctct gcctctgagc tattccagaa gtagtgagga ggcttttttg 2700

gaggcctagg cttttgcaaa aagctcccgg gagcttgtat atccattttc ggatctgatc 2760

aagagacagg atgaggatcg tttcgcatga ttgaacaaga tggattgcac gcaggttctc 2820

cggccgcttg ggtggagagg ctattcggct atgactgggc acaacagaca atcggctgct 2880

ctgatgccgc cgtgttccgg ctgtcagcgc aggggcgccc ggttcttttt gtcaagaccg 2940

acctgtccgg tgccctgaat gaactgcagg acgaggcagc gcggctatcg tggctggcca 3000

cgacgggcgt tccttgcgca gctgtgctcg acgttgtcac tgaagcggga agggactggc 3060

tgctattggg cgaagtgccg gggcaggatc tcctgtcatc tcaccttgct cctgccgaga 3120

aagtatccat catggctgat gcaatgcggc ggctgcatac gcttgatccg gctacctgcc 3180

cattcgacca ccaagcgaaa catcgcatcg agcgagcacg tactcggatg gaagccggtc 3240

ttgtcgatca ggatgatctg gacgaagagc atcaggggct cgcgccagcc gaactgttcg 3300

ccaggctcaa ggcgcgcatg cccgacggcg aggatctcgt cgtgacccat ggcgatgcct 3360

gcttgccgaa tatcatggtg gaaaatggcc gcttttctgg attcatcgac tgtggccggc 3420

tgggtgtggc ggaccgctat caggacatag cgttggctac ccgtgatatt gctgaagagc 3480

ttggcggcga atgggctgac cgcttcctcg tgctttacgg tatcgccgct cccgattcgc 3540

agcgcatcgc cttctatcgc cttcttgacg agttcttctg agcgggactc tggggttcga 3600

aatgaccgac caagcgacgc ccaacctgcc atcacgagat ttcgattcca ccgccgcctt 3660

ctatgaaagg ttgggcttcg gaatcgtttt ccgggacgcc ggctggatga tcctccagcg 3720

cggggatctc atgctggagt tcttcgccca ccccaacttg tttattgcag cttataatgg 3780

ttacaaataa agcaatagca tcacaaattt cacaaataaa gcattttttt cactgcattc 3840

tagttgtggt ttgtccaaac tcatcaatgt atcttatcat gtctgtatac cgtcgacctc 3900

tagctagagc ttggcgtaat catggtcata gctgtttcct gtgtgaaatt gttatccgct 3960

cacaattcca cacaacatac gagccggaag cataaagtgt aaagcctggg gtgcctaatg 4020

agtgagctaa ctcacattaa ttgcgttgcg ctcactgccc gctttccagt cgggaaacct 4080

gtcgtgccag ctgcattaat gaatcggcca acgcgcgggg agaggcggtt tgcgtattgg 4140

gcgctcttcc gcttcctcgc tcactgactc gctgcgctcg gtcgttcggc tgcggcgagc 4200

ggtatcagct cactcaaagg cggtaatacg gttatccaca gaatcagggg ataacgcagg 4260

aaagaacatg tgagcaaaag gccagcaaaa ggccaggaac cgtaaaaagg ccgcgttgct 4320

ggcgtttttc cataggctcc gcccccctga cgagcatcac aaaaatcgac gctcaagtca 4380

gaggtggcga aacccgacag gactataaag ataccaggcg tttccccctg gaagctccct 4440

cgtgcgctct cctgttccga ccctgccgct taccggatac ctgtccgcct ttctcccttc 4500

gggaagcgtg gcgctttctc aatgctcacg ctgtaggtat ctcagttcgg tgtaggtcgt 4560

tcgctccaag ctgggctgtg tgcacgaacc ccccgttcag cccgaccgct gcgccttatc 4620

cggtaactat cgtcttgagt ccaacccggt aagacacgac ttatcgccac tggcagcagc 4680

cactggtaac aggattagca gagcgaggta tgtaggcggt gctacagagt tcttgaagtg 4740

gtggcctaac tacggctaca ctagaaggac agtatttggt atctgcgctc tgctgaagcc 4800

agttaccttc ggaaaaagag ttggtagctc ttgatccggc aaacaaacca ccgctggtag 4860

cggtggtttt tttgtttgca agcagcagat tacgcgcaga aaaaaaggat ctcaagaaga 4920

tcctttgatc ttttctacgg ggtctgacgc tcagtggaac gaaaactcac gttaagggat 4980

tttggtcatg agattatcaa aaaggatctt cacctagatc cttttaaatt aaaaatgaag 5040

ttttaaatca atctaaagta tatatgagta aacttggtct gacagttacc aatgcttaat 5100

cagtgaggca cctatctcag cgatctgtct atttcgttca tccatagttg cctgactccc 5160

cgtcgtgtag ataactacga tacgggaggg cttaccatct ggccccagtg ctgcaatgat 5220

accgcgagac ccacgctcac cggctccaga tttatcagca ataaaccagc cagccggaag 5280

ggccgagcgc agaagtggtc ctgcaacttt atccgcctcc atccagtcta ttaattgttg 5340

ccgggaagct agagtaagta gttcgccagt taatagtttg cgcaacgttg ttgccattgc 5400

tacaggcatc gtggtgtcac gctcgtcgtt tggtatggct tcattcagct ccggttccca 5460

acgatcaagg cgagttacat gatcccccat gttgtgcaaa aaagcggtta gctccttcgg 5520

tcctccgatc gttgtcagaa gtaagttggc cgcagtgtta tcactcatgg ttatggcagc 5580

actgcataat tctcttactg tcatgccatc cgtaagatgc ttttctgtga ctggtgagta 5640

ctcaaccaag tcattctgag aatagtgtat gcggcgaccg agttgctctt gcccggcgtc 5700

aatacgggat aataccgcgc cacatagcag aactttaaaa gtgctcatca ttggaaaacg 5760

ttcttcgggg cgaaaactct caaggatctt accgctgttg agatccagtt cgatgtaacc 5820

cactcgtgca cccaactgat cttcagcatc ttttactttc accagcgttt ctgggtgagc 5880

aaaaacagga aggcaaaatg ccgcaaaaaa gggaataagg gcgacacgga aatgttgaat 5940

actcatactc ttcctttttc aatattattg aagcatttat cagggttatt gtctcatgag 6000

cggatacata tttgaatgta tttagaaaaa taaacaaata ggggttccgc gcacatttcc 6060

ccgaaaagtg ccacctgacg tc 6082

<210> 10

<211> 6082

<212> DNA

<213> Artificial Sequence

<220>

<223>  Plasmid

<400>  10
gacggatcgg gagatctccc gatcccctat ggtcgactct cagtacaatc tgctctgatg      60

ccgcatagtt aagccagtat ctgctccctg cttgtgtgtt ggaggtcgct gagtagtgcg     120

cgagcaaaat ttaagctaca acaaggcaag gcttgaccga caattgcatg aagaatctgc     180

ttagggttag gcgttttgcg ctgcttcgcg atgtacgggc cagatatacg cgttgacatt     240

gattattgac tagttattaa tagtaatcaa ttacggggtc attagttcat agcccatata     300

tggagttccg cgttacataa cttacggtaa atggcccgcc tggctgaccg cccaacgacc     360

cccgcccatt gacgtcaata atgacgtatg ttcccatagt aacgccaata gggactttcc     420

attgacgtca atgggtggac tatttacggt aaactgccca cttggcagta catcaagtgt     480

atcatatgcc aagtacgccc cctattgacg tcaatgacgg taaatggccc gcctggcatt     540

atgcccagta catgacctta tgggactttc ctacttggca gtacatctac gtattagtca     600

tcgctattac catggtgatg cggttttggc agtacatcaa tgggcgtgga tagcggtttg     660

actcacgggg atttccaagt ctccacccca ttgacgtcaa tgggagtttg ttttggcacc     720

aaaatcaacg ggactttcca aaatgtcgta acaactccgc cccattgacg caaatgggcg     780

gtaggcgtgt acggtgggag gtctatataa gcagagctct ctggctaact agagaaccca     840

ctgcttactg gcttatcgaa attaatacga ctcactatag ggagacccaa gctggctaga     900

aagcttggat ctcaccatga gggtccctgc tcagctcctg gggctcctgc tgctctgttt     960

cccaggtgcc agatgtgaca tccagatgac ccagtctcca tcctcactgt ctgcatctgt    1020

aggagacaga gtcaccatca cttgtcgggc gagtcagggc attagccatt atttagcctg    1080

gtttcagcag aaaccaggga aagcccctaa gtccctgatc tatgctgcat ccagtttgca    1140

aagtggggtc ccatcaaagt tcagcggcag tggatctggg acagatttca ctctcaccat    1200

cagcagccta cagcctgaag attttgcaac ttattactgc caacagtata atagtttccc    1260

gctcactttc ggcggaggga ccaaggtgga gatcaaacga actgtggctg caccatctgt    1320

cttcatcttc ccgccatctg atgagcagtt gaaatctgga actgctagcg ttgtgtgcct    1380

gctgaataac ttctatccca gagaggccaa agtacagtgg aaggtggata acgccctcca    1440

atcgggtaac tcccaggaga gtgtcacaga gcaggacagc aaggacagca cctacagcct    1500

cagcagcacc ctgacgctga gcaaagcaga ctacgagaaa cacaaagtct acgcctgcga    1560

agtcacccat cagggcctga gctcgcccgt cacaaagagc ttcaacaggg gagagtgtta    1620

ggaattcgcg gccgctcgag tctagagggc ccgtttaaac ccgctgatca gcctcgactg    1680

tgccttctag ttgccagcca tctgttgttt gcccctcccc cgtgccttcc ttgaccctgg    1740

aaggtgccac tcccactgtc ctttcctaat aaaatgagga aattgcatcg cattgtctga 1800

gtaggtgtca ttctattctg gggggtgggg tggggcagga cagcaagggg gaggattggg 1860

aagacaatag caggcatgct ggggatgcgg tgggctctat ggcttctgag gcggaaagaa 1920

ccagctgggg ctctaggggg tatccccacg cgccctgtag cggcgcatta agcgcggcgg 1980

gtgtggtggt tacgcgcagc gtgaccgcta cacttgccag cgccctagcg cccgctcctt 2040

tcgctttctt cccttccttt ctcgccacgt tcgccggctt tccccgtcaa gctctaaatc 2100

ggggcatccc tttagggttc cgatttagtg ctttacggca cctcgacccc aaaaaacttg 2160

attagggtga tggttcacgt agtgggccat cgccctgata gacggttttt cgccctttga 2220

cgttggagtc cacgttcttt aatagtggac tcttgttcca aactggaaca acactcaacc 2280

ctatctcggt ctattctttt gatttataag ggattttggg gatttcggcc tattggttaa 2340

aaaatgagct gatttaacaa aaatttaacg cgaattaatt ctgtggaatg tgtgtcagtt 2400

agggtgtgga aagtccccag gctccccagg caggcagaag tatgcaaagc atgcatctca 2460

attagtcagc aaccaggtgt ggaaagtccc caggctcccc agcaggcaga agtatgcaaa 2520

gcatgcatct caattagtca gcaaccatag tcccgcccct aactccgccc atcccgcccc 2580

taactccgcc cagttccgcc cattctccgc cccatggctg actaattttt tttatttatg 2640

cagaggccga ggccgcctct gcctctgagc tattccagaa gtagtgagga ggcttttttg 2700

gaggcctagg cttttgcaaa aagctcccgg gagcttgtat atccattttc ggatctgatc 2760

aagagacagg atgaggatcg tttcgcatga ttgaacaaga tggattgcac gcaggttctc 2820

cggccgcttg ggtggagagg ctattcggct atgactgggc acaacagaca atcggctgct 2880

ctgatgccgc cgtgttccgg ctgtcagcgc aggggcgccc ggttcttttt gtcaagaccg 2940

acctgtccgg tgccctgaat gaactgcagg acgaggcagc gcggctatcg tggctggcca 3000

cgacgggcgt tccttgcgca gctgtgctcg acgttgtcac tgaagcggga agggactggc 3060

tgctattggg cgaagtgccg gggcaggatc tcctgtcatc tcaccttgct cctgccgaga 3120

aagtatccat catggctgat gcaatgcggc ggctgcatac gcttgatccg gctacctgcc 3180

cattcgacca ccaagcgaaa catcgcatcg agcgagcacg tactcggatg gaagccggtc 3240

ttgtcgatca ggatgatctg gacgaagagc atcaggggct cgcgccagcc gaactgttcg 3300

ccaggctcaa ggcgcgcatg cccgacggcg aggatctcgt cgtgacccat ggcgatgcct 3360

gcttgccgaa tatcatggtg gaaaatggcc gcttttctgg attcatcgac tgtggccggc 3420

tgggtgtggc ggaccgctat caggacatag cgttggctac ccgtgatatt gctgaagagc 3480

ttggcggcga atgggctgac cgcttcctcg tgctttacgg tatcgccgct cccgattcgc 3540

agcgcatcgc cttctatcgc cttcttgacg agttcttctg agcgggactc tggggttcga 3600

aatgaccgac caagcgacgc ccaacctgcc atcacgagat ttcgattcca ccgccgcctt 3660

ctatgaaagg ttgggcttcg gaatcgtttt ccgggacgcc ggctggatga tcctccagcg 3720

cggggatctc atgctggagt tcttcgccca ccccaacttg tttattgcag cttataatgg 3780

ttacaaataa agcaatagca tcacaaattt cacaaataaa gcattttttt cactgcattc 3840

tagttgtggt ttgtccaaac tcatcaatgt atcttatcat gtctgtatac cgtcgacctc 3900

tagctagagc ttggcgtaat catggtcata gctgtttcct gtgtgaaatt gttatccgct 3960

cacaattcca cacaacatac gagccggaag cataaagtgt aaagcctggg gtgcctaatg 4020

agtgagctaa ctcacattaa ttgcgttgcg ctcactgccc gctttccagt cgggaaacct 4080

gtcgtgccag ctgcattaat gaatcggcca acgcgcgggg agaggcggtt tgcgtattgg 4140

gcgctcttcc gcttcctcgc tcactgactc gctgcgctcg gtcgttcggc tgcggcgagc 4200

ggtatcagct cactcaaagg cggtaatacg gttatccaca gaatcagggg ataacgcagg 4260

aaagaacatg tgagcaaaag gccagcaaaa ggccaggaac cgtaaaaagg ccgcgttgct 4320

ggcgtttttc cataggctcc gcccccctga cgagcatcac aaaaatcgac gctcaagtca 4380

gaggtggcga aacccgacag gactataaag ataccaggcg tttccccctg gaagctccct 4440

cgtgcgctct cctgttccga ccctgccgct taccggatac ctgtccgcct ttctcccttc 4500

gggaagcgtg gcgctttctc aatgctcacg ctgtaggtat ctcagttcgg tgtaggtcgt 4560

tcgctccaag ctgggctgtg tgcacgaacc ccccgttcag cccgaccgct gcgccttatc 4620

cggtaactat cgtcttgagt ccaacccggt aagacacgac ttatcgccac tggcagcagc 4680

cactggtaac aggattagca gagcgaggta tgtaggcggt gctacagagt tcttgaagtg 4740

gtggcctaac tacggctaca ctagaaggac agtatttggt atctgcgctc tgctgaagcc 4800

agttaccttc ggaaaaagag ttggtagctc ttgatccggc aaacaaacca ccgctggtag 4860

cggtggtttt tttgtttgca agcagcagat tacgcgcaga aaaaaaggat ctcaagaaga 4920

tcctttgatc ttttctacgg ggtctgacgc tcagtggaac gaaaactcac gttaagggat 4980

tttggtcatg agattatcaa aaaggatctt cacctagatc cttttaaatt aaaaatgaag 5040

ttttaaatca atctaaagta tatatgagta aacttggtct gacagttacc aatgcttaat 5100

cagtgaggca cctatctcag cgatctgtct atttcgttca tccatagttg cctgactccc 5160

cgtcgtgtag ataactacga tacgggaggg cttaccatct ggccccagtg ctgcaatgat 5220

accgcgagac ccacgctcac cggctccaga tttatcagca ataaaccagc cagccggaag 5280

ggccgagcgc agaagtggtc ctgcaacttt atccgcctcc atccagtcta ttaattgttg 5340

ccgggaagct agagtaagta gttcgccagt taatagtttg cgcaacgttg ttgccattgc 5400

tacaggcatc gtggtgtcac gctcgtcgtt tggtatggct tcattcagct ccggttccca 5460

acgatcaagg cgagttacat gatcccccat gttgtgcaaa aaagcggtta gctccttcgg 5520

tcctccgatc gttgtcagaa gtaagttggc cgcagtgtta tcactcatgg ttatggcagc 5580

actgcataat tctcttactg tcatgccatc cgtaagatgc ttttctgtga ctggtgagta 5640

ctcaaccaag tcattctgag aatagtgtat gcggcgaccg agttgctctt gcccggcgtc 5700

aatacgggat aataccgcgc cacatagcag aactttaaaa gtgctcatca ttggaaaacg 5760

ttcttcgggg cgaaaactct caaggatctt accgctgttg agatccagtt cgatgtaacc 5820

cactcgtgca cccaactgat cttcagcatc ttttactttc accagcgttt ctgggtgagc 5880

aaaaacagga aggcaaaatg ccgcaaaaaa gggaataagg gcgacacgga aatgttgaat 5940

actcatactc ttcctttttc aatattattg aagcatttat cagggttatt gtctcatgag 6000

cggatacata tttgaatgta tttagaaaaa taaacaaata ggggttccgc gcacatttcc 6060

ccgaaaagtg ccacctgacg tc 6082

<210> 11

<211> 6085

<212> DNA

<213> Artificial Sequence

<220>

<223> Plasmid

<400> 11
gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctaga 900

aagcttggat ctcaccatga gggtccccgc tcagcttctc ttccttctgc tactctggct 960

cccagatacc actggaggaa tagtgatgac gcagtctcca gccaccctgt ctgtgtctcc 1020

aggggaaaga gccaccctct cctgcaggac cagtcagagt attggctgga acttagcctg 1080

gtaccaacag aaacctggcc aggctcccag gctcctcatc tatggtgcat cttccaggac 1140

cactggtatc ccagccaggt tcagtggcag tgggtctggg acagagttca ctctcaccat 1200

cagcagcctg cagtctgaag attctgcagt ttattactgt cagcattatg ataactggcc 1260

catgtgcagt tttggccagg ggaccgagct ggagatcaaa cgaactgtgg ctgcaccatc 1320

tgtcttcatc ttcccgccat ctgatgagca gttgaaatct ggaactgcta gcgttgtgtg 1380

cctgctgaat aacttctatc ccagagaggc caaagtacag tggaaggtgg ataacgccct 1440

ccaatcgggt aactcccagg agagtgtcac agagcaggac agcaaggaca gcacctacag 1500

cctcagcagc accctgacgc tgagcaaagc agactacgag aaacacaaag tctacgcctg 1560

cgaagtcacc catcagggcc tgagctcgcc cgtcacaaag agcttcaaca ggggagagtg 1620

ttaggaattc gcggccgctc gagtctagag ggcccgttta aacccgctga tcagcctcga 1680

ctgtgccttc tagttgccag ccatctgttg tttgcccctc ccccgtgcct tccttgaccc 1740

tggaaggtgc cactcccact gtcctttcct aataaaatga ggaaattgca tcgcattgtc 1800

tgagtaggtg tcattctatt ctggggggtg gggtggggca ggacagcaag ggggaggatt 1860

gggaagacaa tagcaggcat gctggggatg cggtgggctc tatggcttct gaggcggaaa 1920

gaaccagctg gggctctagg gggtatcccc acgcgccctg tagcggcgca ttaagcgcgg 1980

cgggtgtggt ggttacgcgc agcgtgaccg ctacacttgc cagcgcccta gcgcccgctc 2040

ctttcgcttt cttcccttcc tttctcgcca cgttcgccgg ctttccccgt caagctctaa 2100

atcggggcat ccctttaggg ttccgattta gtgctttacg gcacctcgac cccaaaaaac 2160

ttgattaggg tgatggttca cgtagtgggc catcgccctg atagacggtt tttcgccctt 2220

tgacgttgga gtccacgttc tttaatagtg gactcttgtt ccaaactgga acaacactca 2280

accctatctc ggtctattct tttgatttat aagggatttt ggggatttcg gcctattggt 2340

taaaaaatga gctgatttaa caaaaattta acgcgaatta attctgtgga atgtgtgtca 2400

gttagggtgt ggaaagtccc caggctcccc aggcaggcag aagtatgcaa agcatgcatc 2460

tcaattagtc agcaaccagg tgtggaaagt ccccaggctc cccagcaggc agaagtatgc 2520

aaagcatgca tctcaattag tcagcaacca tagtcccgcc cctaactccg cccatcccgc 2580

ccctaactcc gcccagttcc gcccattctc cgccccatgg ctgactaatt ttttttattt 2640

atgcagaggc cgaggccgcc tctgcctctg agctattcca gaagtagtga ggaggctttt 2700

ttggaggcct aggcttttgc aaaaagctcc cgggagcttg tatatccatt ttcggatctg 2760

atcaagagac aggatgagga tcgtttcgca tgattgaaca agatggattg cacgcaggtt 2820

ctccggccgc ttgggtggag aggctattcg gctatgactg ggcacaacag acaatcggct 2880

gctctgatgc cgccgtgttc cggctgtcag cgcaggggcg cccggttctt tttgtcaaga 2940

ccgacctgtc cggtgccctg aatgaactgc aggacgaggc agcgcggcta tcgtggctgg 3000

ccacgacggg cgttccttgc gcagctgtgc tcgacgttgt cactgaagcg ggaagggact 3060

ggctgctatt gggcgaagtg ccggggcagg atctcctgtc atctcacctt gctcctgccg 3120

agaaagtatc catcatggct gatgcaatgc ggcggctgca tacgcttgat ccggctacct 3180

gcccattcga ccaccaagcg aaacatcgca tcgagcgagc acgtactcgg atggaagccg 3240

gtcttgtcga tcaggatgat ctggacgaag agcatcaggg gctcgcgcca gccgaactgt 3300

tcgccaggct caaggcgcgc atgcccgacg gcgaggatct cgtcgtgacc catggcgatg 3360

cctgcttgcc gaatatcatg gtggaaaatg gccgcttttc tggattcatc gactgtggcc 3420

ggctgggtgt ggcggaccgc tatcaggaca tagcgttggc tacccgtgat attgctgaag 3480

agcttggcgg cgaatgggct gaccgcttcc tcgtgcttta cggtatcgcc gctcccgatt 3540

cgcagcgcat cgccttctat cgccttcttg acgagttctt ctgagcggga ctctggggtt 3600

cgaaatgacc gaccaagcga cgcccaacct gccatcacga gatttcgatt ccaccgccgc 3660

cttctatgaa aggttgggct tcggaatcgt tttccgggac gccggctgga tgatcctcca 3720

gcgcggggat ctcatgctgg agttcttcgc ccaccccaac ttgtttattg cagcttataa 3780

tggttacaaa taaagcaata gcatcacaaa tttcacaaat aaagcatttt tttcactgca 3840

ttctagttgt ggtttgtcca aactcatcaa tgtatcttat catgtctgta taccgtcgac 3900

ctctagctag agcttggcgt aatcatggtc atagctgttt cctgtgtgaa attgttatcc 3960

gctcacaatt ccacacaaca tacgagccgg aagcataaag tgtaaagcct ggggtgccta 4020

atgagtgagc taactcacat taattgcgtt gcgctcactg cccgctttcc agtcgggaaa 4080

cctgtcgtgc cagctgcatt aatgaatcgg ccaacgcgcg gggagaggcg gtttgcgtat 4140

tgggcgctct tccgcttcct cgctcactga ctcgctgcgc tcggtcgttc ggctgcggcg 4200

agcggtatca gctcactcaa aggcggtaat acggttatcc acagaatcag gggataacgc 4260

aggaaagaac atgtgagcaa aaggccagca aaaggccagg aaccgtaaaa aggccgcgtt 4320

gctggcgttt ttccataggc tccgcccccc tgacgagcat cacaaaaatc gacgctcaag 4380

tcagaggtgg cgaaacccga caggactata aagataccag gcgtttcccc ctggaagctc 4440

cctcgtgcgc tctcctgttc cgaccctgcc gcttaccgga tacctgtccg cctttctccc 4500

ttcgggaagc gtggcgcttt ctcaatgctc acgctgtagg tatctcagtt cggtgtaggt 4560

cgttcgctcc aagctgggct gtgtgcacga accccccgtt cagcccgacc gctgcgcctt 4620

atccggtaac tatcgtcttg agtccaaccc ggtaagacac gacttatcgc cactggcagc 4680

agccactggt aacaggatta gcagagcgag gtatgtaggc ggtgctacag agttcttgaa 4740

gtggtggcct aactacggct acactagaag gacagtattt ggtatctgcg ctctgctgaa 4800

gccagttacc ttcggaaaaa gagttggtag ctcttgatcc ggcaaacaaa ccaccgctgg 4860

tagcggtggt ttttttgttt gcaagcagca gattacgcgc agaaaaaaag gatctcaaga 4920

agatcctttg atcttttcta cggggtctga cgctcagtgg aacgaaaact cacgttaagg 4980

gattttggtc atgagattat caaaaaggat cttcacctag atccttttaa attaaaaatg 5040

aagttttaaa tcaatctaaa gtatatatga gtaaacttgg tctgacagtt accaatgctt 5100

aatcagtgag gcacctatct cagcgatctg tctatttcgt tcatccatag ttgcctgact 5160

ccccgtcgtg tagataacta cgatacggga gggcttacca tctggcccca gtgctgcaat 5220

gataccgcga gacccacgct caccggctcc agatttatca gcaataaacc agccagccgg 5280

aagggccgag cgcagaagtg gtcctgcaac tttatccgcc tccatccagt ctattaattg 5340

ttgccgggaa gctagagtaa gtagttcgcc agttaatagt ttgcgcaacg ttgttgccat 5400

tgctacaggc atcgtggtgt cacgctcgtc gtttggtatg gcttcattca gctccggttc 5460

ccaacgatca aggcgagtta catgatcccc catgttgtgc aaaaaagcgg ttagctcctt 5520

cggtcctccg atcgttgtca gaagtaagtt ggccgcagtg ttatcactca tggttatggc 5580

agcactgcat aattctctta ctgtcatgcc atccgtaaga tgcttttctg tgactggtga 5640

gtactcaacc aagtcattct gagaatagtg tatgcggcga ccgagttgct cttgcccggc 5700

gtcaatacgg gataataccg cgccacatag cagaacttta aaagtgctca tcattggaaa 5760

acgttcttcg gggcgaaaac tctcaaggat cttaccgctg ttgagatcca gttcgatgta 5820

acccactcgt gcacccaact gatcttcagc atcttttact ttcaccagcg tttctgggtg 5880

agcaaaaaca ggaaggcaaa atgccgcaaa aaagggaata agggcgacac ggaaatgttg 5940

aatactcata ctcttccttt ttcaatatta ttgaagcatt tatcagggtt attgtctcat 6000

gagcggatac atatttgaat gtatttagaa aaataaacaa ataggggttc cgcgcacatt 6060

tccccgaaaa gtgccacctg acgtc 6085

<210> 12

<211> 6097

<212> DNA

<213> Artificial Sequence

<220>

<223> Plasmid

<400> 12
gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctaga 900

aagcttggat ctcaccatga gggtccctgc tcagctcctg gggctgctaa tgctctggat 960

acctggatcc agtgcagata ttgtgatgac ccagactcca ctctctctgt ccgtcacccc 1020

tggacagccg gcctccatct cctgcaagtc tagtcagagc ctcctgcata gtgatggaaa 1080

gacctttttg tattggtatc tgcagaagcc aggccagcct ccacagctcc tgatctatga 1140

ggtttccaac cggttctctg gagtgccaga taggttcagt ggcagcgggt cagggacaga 1200

tttcacactg aaaatcagcc gggtggaggc tgaggatgtt gggctttatt actgcatgca 1260

aagtatacag cttccgctca ctttcggcgg agggaccaag gtggagatca aacgaactgt 1320

ggctgcacca tctgtcttca tcttcccgcc atctgatgag cagttgaaat ctggaactgc 1380

tagcgttgtg tgcctgctga ataacttcta tcccagagag gccaaagtac agtggaaggt 1440

ggataacgcc ctccaatcgg gtaactccca ggagagtgtc acagagcagg acagcaagga 1500

cagcacctac agcctcagca gcaccctgac gctgagcaaa gcagactacg agaaacacaa 1560

agtctacgcc tgcgaagtca cccatcaggg cctgagctcg cccgtcacaa agagcttcaa 1620

caggggagag tgttaggaat tcgcggccgc tcgagtctag agggcccgtt taaacccgct 1680

gatcagcctc gactgtgcct tctagttgcc agccatctgt tgtttgcccc tcccccgtgc 1740

cttccttgac cctggaaggt gccactccca ctgtcctttc ctaataaaat gaggaaattg 1800

catcgcattg tctgagtagg tgtcattcta ttctgggggg tggggtgggg caggacagca 1860

agggggagga ttgggaagac aatagcaggc atgctgggga tgcggtgggc tctatggctt 1920

ctgaggcgga aagaaccagc tggggctcta gggggtatcc ccacgcgccc tgtagcggcg 1980

cattaagcgc ggcgggtgtg gtggttacgc gcagcgtgac cgctacactt gccagcgccc 2040

tagcgcccgc tcctttcgct ttcttccctt cctttctcgc cacgttcgcc ggctttcccc 2100

gtcaagctct aaatcggggc atccctttag ggttccgatt tagtgcttta cggcacctcg 2160

accccaaaaa acttgattag ggtgatggtt cacgtagtgg gccatcgccc tgatagacgg 2220

tttttcgccc tttgacgttg gagtccacgt tctttaatag tggactcttg ttccaaactg 2280

gaacaacact caaccctatc tcggtctatt cttttgattt ataagggatt ttggggattt 2340

cggcctattg gttaaaaaat gagctgattt aacaaaaatt taacgcgaat taattctgtg 2400

gaatgtgtgt cagttagggt gtggaaagtc cccaggctcc ccaggcaggc agaagtatgc 2460

aaagcatgca tctcaattag tcagcaacca ggtgtggaaa gtccccaggc tccccagcag 2520

gcagaagtat gcaaagcatg catctcaatt agtcagcaac catagtcccg cccctaactc 2580

cgcccatccc gcccctaact ccgcccagtt ccgcccattc tccgccccat ggctgactaa 2640

ttttttttat ttatgcagag gccgaggccg cctctgcctc tgagctattc cagaagtagt 2700

gaggaggctt ttttggaggc ctaggctttt gcaaaaagct cccgggagct tgtatatcca 2760

ttttcggatc tgatcaagag acaggatgag gatcgtttcg catgattgaa caagatggat 2820

tgcacgcagg ttctccggcc gcttgggtgg agaggctatt cggctatgac tgggcacaac 2880

agacaatcgg ctgctctgat gccgccgtgt tccggctgtc agcgcagggg cgcccggttc 2940

tttttgtcaa gaccgacctg tccggtgccc tgaatgaact gcaggacgag gcagcgcggc 3000

tatcgtggct ggccacgacg ggcgttcctt gcgcagctgt gctcgacgtt gtcactgaag 3060

cgggaaggga ctggctgcta ttgggcgaag tgccggggca ggatctcctg tcatctcacc 3120

ttgctcctgc cgagaaagta tccatcatgg ctgatgcaat gcggcggctg catacgcttg 3180

atccggctac ctgcccattc gaccaccaag cgaaacatcg catcgagcga gcacgtactc 3240

ggatggaagc cggtcttgtc gatcaggatg atctggacga agagcatcag gggctcgcgc 3300

cagccgaact gttcgccagg ctcaaggcgc gcatgcccga cggcgaggat ctcgtcgtga 3360

cccatggcga tgcctgcttg ccgaatatca tggtggaaaa tggccgcttt tctggattca 3420

tcgactgtgg ccggctgggt gtggcggacc gctatcagga catagcgttg gctacccgtg 3480

atattgctga agagcttggc ggcgaatggg ctgaccgctt cctcgtgctt tacggtatcg 3540

ccgctcccga ttcgcagcgc atcgccttct atcgccttct tgacgagttc ttctgagcgg 3600

gactctgggg ttcgaaatga ccgaccaagc gacgcccaac ctgccatcac gagatttcga 3660

ttccaccgcc gccttctatg aaaggttggg cttcggaatc gttttccggg acgccggctg 3720

gatgatcctc cagcgcgggg atctcatgct ggagttcttc gcccacccca acttgtttat 3780

tgcagcttat aatggttaca aataaagcaa tagcatcaca aatttcacaa ataaagcatt 3840

tttttcactg cattctagtt gtggtttgtc caaactcatc aatgtatctt atcatgtctg 3900

tataccgtcg acctctagct agagcttggc gtaatcatgg tcatagctgt ttcctgtgtg 3960

aaattgttat ccgctcacaa ttccacacaa catacgagcc ggaagcataa agtgtaaagc 4020

ctggggtgcc taatgagtga gctaactcac attaattgcg ttgcgctcac tgcccgcttt 4080

ccagtcggga aacctgtcgt gccagctgca ttaatgaatc ggccaacgcg cggggagagg 4140

cggtttgcgt attgggcgct cttccgcttc ctcgctcact gactcgctgc gctcggtcgt 4200

tcggctgcgg cgagcggtat cagctcactc aaaggcggta atacggttat ccacagaatc 4260

aggggataac gcaggaaaga acatgtgagc aaaaggccag caaaaggcca ggaaccgtaa 4320

aaaggccgcg ttgctggcgt ttttccatag gctccgcccc cctgacgagc atcacaaaaa 4380

tcgacgctca agtcagaggt ggcgaaaccc gacaggacta taaagatacc aggcgtttcc 4440

ccctggaagc tccctcgtgc gctctcctgt tccgaccctg ccgcttaccg gatacctgtc 4500

cgcctttctc ccttcgggaa gcgtggcgct ttctcaatgc tcacgctgta ggtatctcag 4560

ttcggtgtag gtcgttcgct ccaagctggg ctgtgtgcac gaaccccccg ttcagcccga 4620

ccgctgcgcc ttatccggta actatcgtct tgagtccaac ccggtaagac acgacttatc 4680

gccactggca gcagccactg gtaacaggat tagcagagcg aggtatgtag gcggtgctac 4740

agagttcttg aagtggtggc ctaactacgg ctacactaga aggacagtat ttggtatctg 4800

cgctctgctg aagccagtta ccttcggaaa aagagttggt agctcttgat ccggcaaaca 4860

aaccaccgct ggtagcggtg gtttttttgt ttgcaagcag cagattacgc gcagaaaaaa 4920

aggatctcaa gaagatcctt tgatcttttc tacggggtct gacgctcagt ggaacgaaaa 4980

ctcacgttaa gggattttgg tcatgagatt atcaaaaagg atcttcacct agatcctttt 5040

aaattaaaaa tgaagtttta aatcaatcta aagtatatat gagtaaactt ggtctgacag 5100

ttaccaatgc ttaatcagtg aggcacctat ctcagcgatc tgtctatttc gttcatccat 5160

agttgcctga ctccccgtcg tgtagataac tacgatacgg gagggcttac catctggccc 5220

cagtgctgca atgataccgc gagacccacg ctcaccggct ccagatttat cagcaataaa 5280

ccagccagcc ggaagggccg agcgcagaag tggtcctgca actttatccg cctccatcca 5340

gtctattaat tgttgccggg aagctagagt aagtagttcg ccagttaata gtttgcgcaa 5400

cgttgttgcc attgctacag gcatcgtggt gtcacgctcg tcgtttggta tggcttcatt 5460

cagctccggt tcccaacgat caaggcgagt tacatgatcc cccatgttgt gcaaaaaagc 5520

ggttagctcc ttcggtcctc cgatcgttgt cagaagtaag ttggccgcag tgttatcact 5580

catggttatg gcagcactgc ataattctct tactgtcatg ccatccgtaa gatgcttttc 5640

tgtgactggt gagtactcaa ccaagtcatt ctgagaatag tgtatgcggc gaccgagttg 5700

ctcttgcccg gcgtcaatac gggataatac cgcgccacat agcagaactt taaaagtgct 5760

catcattgga aaacgttctt cggggcgaaa actctcaagg atcttaccgc tgttgagatc 5820

cagttcgatg taacccactc gtgcacccaa ctgatcttca gcatctttta ctttcaccag 5880

cgtttctggg tgagcaaaaa caggaaggca aaatgccgca aaaaagggaa taagggcgac 5940

acggaaatgt tgaatactca tactcttcct ttttcaatat tattgaagca tttatcaggg 6000

ttattgtctc atgagcggat acatatttga atgtatttag aaaaataaac aaataggggt 6060

tccgcgcaca tttccccgaa aagtgccacc tgacgtc 6097

<210> 13

<211> 6094

<212> DNA

<213> Artificial Sequence

<220>

<223> Plasmid

<400> 13
gacggatcgg gagatctccc gatcccctat ggtcgactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggac tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctaga 900

aagcttggat ctcaccatgg tgttgcagac ccaggtcttc atttctctgt tactctggat 960

ctctggtgcc tacggggaca tcgtgatgac ccagtctcca gactccctgg ctgtgtctct 1020

gggcgagagg gccaccatca actgcaagtc caaccagagt gtcttacaca gctccaacaa 1080

taagaactat ttagcttggt accagcagaa accaggacag cctcctaaat tgctcattta 1140

ttgggcattc ctccgggaat ccggggtccc tgaccgcttc agtggcagcg ggtctgggac 1200

agatttcact ctcaccatca gcagcctgca ggctgaagat gtggcagttt attactgtca 1260

ccaatattat tctactttat atactttcgg cggagggacc aaggtagaga tcaaacgaac 1320

ygtggctgca ccatctgtct tcatcttccc gccatctgat gagcagttga aatctggaac 1380

tgctagcgtt gtgtgcctgc tgaataactt ctatcccaga gaggccaaag tacagtggaa 1440

ggtggataac gccctccaat cgggtaactc ccaggagagt gtcacagagc aggacagcaa 1500

ggacagcacc tacagcctca gcagcaccct gacgctgagc aaagcagact acgagaaaca 1560

caaagtctac gcctgcgaag tcacccatca gggcctgagc tcgcccgtca caaagagctt 1620

caacagggga gagtgttagg cggccgctcg agtctagagg gcccgtttaa acccgctgat 1680

cagcctcgac tgtgccttct agttgccagc catctgttgt ttgcccctcc cccgtgcctt 1740

ccttgaccct ggaaggtgcc actcccactg tcctttccta ataaaatgag gaaattgcat 1800

cgcattgtct gagtaggtgt cattctattc tggggggtgg ggtggggcag gacagcaagg 1860

gggaggattg ggaagacaat agcaggcatg ctggggatgc ggtgggctct atggcttctg 1920

aggcggaaag aaccagctgg ggctctaggg ggtatcccca cgcgccctgt agcggcgcat 1980

taagcgcggc gggtgtggtg gttacgcgca gcgtgaccgc tacacttgcc agcgccctag 2040

cgcccgctcc tttcgctttc ttcccttcct ttctcgccac gttcgccggc tttccccgtc 2100

aagctctaaa tcgggggctc cctttagggt tccgatttag tgctttacgg cacctcgacc 2160

ccaaaaaact tgattagggt gatggttcac gtagtgggcc atcgccctga tagacggttt 2220

ttcgcccttt gacgttggag tccacgttct ttaatagtgg actcttgttc caaactggaa 2280

caacactcaa ccctatctcg gtctattctt ttgatttata agggattttg gggatttcgg 2340

cctattggtt aaaaaatgag ctgatttaac aaaaatttaa cgcgaattaa ttctgtggaa 2400

tgtgtgtcag ttagggtgtg gaaagtcccc aggctcccca gcaggcagaa gtatgcaaag 2460

gcatgcatct caattagtca gcaaccaggt gtggaaagtc cccaggctcc ccagcaggca 2520

gaagtatgca aagcatgcat ctcaattagt cagcaaccat agtcccgccc ctaactccgc 2580

ccatcccgcc cctaactccg cccagttccg cccattctcc gccccatggc tgactaattt 2640

tttttattta tgcagaggcc gaggccgcct ctgcctctga gctattccag aagtagtgag 2700

gaggcttttt tggaggccta ggcttttgca aaaagctccc gggagcttgt atatccattt 2760

tcggatctga tcaagagaca ggatgaggat cgtttcgcat gattgaacaa gatggattgc 2820

acgcaggttc tccggccgct tgggtggaga ggctattcgg ctatgactgg gcacaacaga 2880

caatcggctg ctctgatgcc gccgtgttcc ggctgtcagc gcaggggcgc ccggttcttt 2940

ttgtcaagac cgacctgtcc ggtgccctga atgaactgca ggacgaggca gcgcggctat 3000

cgtggctggc cacgacgggc gttccttgcg cagctgtgct cgacgttgtc actgaagcgg 3060

gaagggactg gctgctattg ggcgaagtgc cggggcagga tctcctgtca tctcaccttg 3120

ctcctgccga gaaagtatcc atcatggctg atgcaatgcg gcggctgcat acgcttgatc 3180

cggctacctg cccattcgac caccaagcga aacatcgcat cgagcgagca cgtactcgga 3240

tggaagccgg tcttgtcgat caggatgatc tggacgaaga gcatcagggg ctcgcgccag 3300

ccgaactgtt cgccaggctc aaggcgcgca tgcccgacgg cgaggatctc gtcgtgaccc 3360

atggcgatgc ctgcttgccg aatatcatgg tggaaaatgg ccgcttttct ggattcatcg 3420

actgtggccg gctgggtgtg gcggaccgct atcaggacat agcgttggct acccgtgata 3480

ttgctgaaga gcttggcggc gaatgggctg accgcttcct cgtgctttac ggtatcgccg 3540

ctcccgattc gcagcgcatc gccttctatc gccttcttga cgagttcttc tgagcgggac 3600

tctggggttc gaaatgaccg accaagcgac gcccaacctg ccatcacgag atttcgattc 3660

caccgccgcc ttctatgaaa ggttgggctt cggaatcgtt ttccgggacg ccggctggat 3720

gatcctccag cgcggggatc tcatgctgga gttcttcgcc caccccaact tgtttattgc 3780

agcttataat ggttacaaat aaagcaatag catcacaaat ttcacaaata aagcattttt 3840

ttcactgcat tctagttgtg gtttgtccaa actcatcaat gtatcttatc atgtctgtat 3900

accgtcgacc tctagctaga gcttggcgta atcatggtca tagctgtttc ctgtgtgaaa 3960

ttgttatccg ctcacaattc cacacaacat acgagccgga agcataaagt gtaaagcctg 4020

gggtgcctaa tgagtgagct aactcacatt aattgcgttg cgctcactgc ccgctttcca 4080

gtcgggaaac ctgtcgtgcc agctgcatta atgaatcggc caacgcgcgg ggagaggcgg 4140

tttgcgtatt gggcgctctt ccgcttcctc gctcactgac tcgctgcgct cggtcgttcg 4200

gctgcggcga gcggtatcag ctcactcaaa ggcggtaata cggttatcca cagaatcagg 4260

ggataacgca ggaaagaaca tgtgagcaaa aggccagcaa aaggccagga accgtaaaaa 4320

ggccgcgttg ctggcgtttt tccataggct ccgcccccct gacgagcatc acaaaaatcg 4380

acgctcaagt cagaggtggc gaaacccgac aggactataa agataccagg cgtttccccc 4440

tggaagctcc ctcgtgcgct ctcctgttcc gaccctgccg cttaccggat acctgtccgc 4500

ctttctccct tcgggaagcg tggcgctttc tcaatgctca cgctgtaggt atctcagttc 4560

ggtgtaggtc gttcgctcca agctgggctg tgtgcacgaa ccccccgttc agcccgaccg 4620

ctgcgcctta tccggtaact atcgtcttga gtccaacccg gtaagacacg acttatcgcc 4680

actggcagca gccactggta acaggattag cagagcgagg tatgtaggcg gtgctacaga 4740

gttcttgaag tggtggccta actacggcta cactagaagg acagtatttg gtatctgcgc 4800

tctgctgaag ccagttacct tcggaaaaag agttggtagc tcttgatccg gcaaacaaac 4860

caccgctggt agcggtggtt tttttgtttg caagcagcag attacgcgca gaaaaaaagg 4920

atctcaagaa gatcctttga tcttttctac ggggtctgac gctcagtgga acgaaaactc 4980

acgttaaggg attttggtca tgagattatc aaaaaggatc ttcacctaga tccttttaaa 5040

ttaaaaatga agttttaaat caatctaaag tatatatgag taaacttggt ctgacagtta 5100

ccaatgctta atcagtgagg cacctatctc agcgatctgt ctatttcgtt catccatagt 5160

tgcctgactc cccgtcgtgt agataactac gatacgggag ggcttaccat ctggccccag 5220

tgctgcaatg ataccgcgag acccacgctc accggctcca gatttatcag caataaacca 5280

gccagccgga agggccgagc gcagaagtgg tcctgcaact ttatccgcct ccatccagtc 5340

tattaattgt tgccgggaag ctagagtaag tagttcgcca gttaatagtt tgcgcaacgt 5400

tgttgccatt gctacaggca tcgtggtgtc acgctcgtcg tttggtatgg cttcattcag 5460

ctccggttcc caacgatcaa ggcgagttac atgatccccc atgttgtgca aaaaagcggt 5520

tagctccttc ggtcctccga tcgttgtcag aagtaagttg gccgcagtgt tatcactcat 5580

ggttatggca gcactgcata attctcttac tgtcatgcca tccgtaagat gcttttctgt 5640

gactggtgag tactcaacca agtcattctg agaatagtgt atgcggcgac cgagttgctc 5700

ttgcccggcg tcaatacggg ataataccgc gccacatagc agaactttaa aagtgctcat 5760

cattggaaaa cgttcttcgg ggcgaaaact ctcaaggatc ttaccgctgt tgagatccag 5820

ttcgatgtaa cccactcgtg cacccaactg atcttcagca tcttttactt tcaccagcgt 5880

ttctgggtga gcaaaaacag gaaggcaaaa tgccgcaaaa aagggaataa gggcgacacg 5940

gaaatgttga atactcatac tcttcctttt tcaatattat tgaagcattt atcagggtta 6000

ttgtctcatg agcggataca tatttgaatg tatttagaaa aataaacaaa taggggttcc 6060

gcgcacattt ccccgaaaag tgccacctga cgtc 6094

<210> 14

<211> 481

<212> DNA

<213> Artificial Sequence

<220>

<223> Includes BamHI/Bg1II cloning junction, signal peptide, V region,
portion of C region and 3\222XbaI/NheI (heavy) or NheI (light) cloning
junction

<400> 14
ggatctcacc atggagttgg gactgcgctg gggcttcctc gttgctcttt taagaggtgt 60

ccagtgtcag gtgcaattgg tggagtctgg gggaggcgtg gtccagcctg ggaggtccct 120

gagactctcc tgtgcagcgt ctggattcgc cttcagtaga tatggcatgc actgggtccg 180

ccaggctcca ggcaaggggc tggagtgggt ggcagttata tggtatgatg gaagtaataa 240

atactatgca gactccgtga agggccgatt caccatctcc agagacaatt ccaagaacac 300

gcagtatctg caaatgaaca gcctgagagc cgaggacacg gctgtgtatt actgtgcgag 360

aggcggtgac ttcctctact actactatta cggtatggac gtctggggcc aagggaccac 420

ggtcaccgtc tcctcagcct ccaccaaggg cccatcggtc ttccccctgg caccctctag 480

c 481

<210> 15

<211> 142

<212> PRT

<213> Homo sapiens

<400> 15

Met Glu Leu Gly Leu Arg Trp Gly Phe Leu Val Ala Leu Leu Arg Gly
1               5                   10                  15

Val Gln Cys Gln Val Gln Leu Val Glu Ser Gly Gly Gly Val Val Gln
            20                  25                  30

Pro Gly Arg Ser Leu Arg Leu Ser Cys Ala Ala Ser Gly Phe Ala Phe
        35                  40                  45

Ser Arg Tyr Gly Met His Trp Val Arg Gln Ala Pro Gly Lys Gly Leu
    50                  55                  60

Glu Trp Val Ala Val Ile Trp Tyr Asp Gly Ser Asn Lys Tyr Tyr Ala
65                  70                  75                  80

Asp Ser Val Lys Gly Arg Phe Thr Ile Ser Arg Asp Asn Ser Lys Asn
                85                  90                  95

Thr Gln Tyr Leu Gln Met Asn Ser Leu Arg Ala Glu Asp Thr Ala Val
            100                 105                 110

Tyr Tyr Cys Ala Arg Gly Gly Asp Phe Leu Tyr Tyr Tyr Tyr Tyr Gly
        115                 120                 125

Met Asp Val Trp Gly Gln Gly Thr Thr Val Thr Val Ser Ser
    130                 135                 140

<210> 16

<211> 463

<212> DNA

<213> Artificial Sequence

<220>

<223>  Includes BamHI/BglII cloning junction, signal peptide, V region,
portion of C region and 3\222XbaI/NheI (heavy) or NheI (light) cloning
junction

<400>  16
ggatctcacc atgagggtcc ctgctcagct cctgggactc ctgctgctct ggctcccaga      60

taccagatgt gacatccaga tgacccagtc tccatcctcc ctgtctgcat ctgtaggaga     120

cagagtcacc atcacttgcc gggcgagtca gggcattagc aattatttag cctggtatca     180

gcagaaaaca gggaaagttc ctaagttcct gatctatgaa gcatccactt tgcaatcagg     240

ggtcccatct cggttcagtg gcggtggatc tgggacagat ttcactctca ccatcagcag     300

cctgcagcct gaagatgttg caacttatta ctgtcaaaat tataacagtg ccccattcac     360

tttcggccct gggaccaaag tggatatcaa acgaactgtg gctgcaccct ctgtcttcat     420

cttcccgcca tctgatgagc agttgaaatc tggaactgct agc                       463


<210>  17

<211>  127

<212>  PRT

<213>  Homo sapiens

<400>  17

Met Arg Val Pro Ala Gln Leu Leu Gly Leu Leu Leu Leu Trp Leu Pro
1               5                   10                  15

Asp Thr Arg Cys Asp Ile Gln Met Thr Gln Ser Pro Ser Ser Leu Ser
            20                  25                  30

Ala Ser Val Gly Asp Arg Val Thr Ile Thr Cys Arg Ala Ser Gln Gly
        35                  40                  45

Ile Ser Asn Tyr Leu Ala Trp Tyr Gln Gln Lys Thr Gly Lys Val Pro
    50                  55                  60

Lys Phe Leu Ile Tyr Glu Ala Ser Thr Leu Gln Ser Gly Val Pro Ser
65                  70                  75                  80

Arg Phe Ser Gly Gly Gly Ser Gly Thr Asp Phe Thr Leu Thr Ile Ser
                85                  90                  95

Ser Leu Gln Pro Glu Asp Val Ala Thr Tyr Tyr Cys Gln Asn Tyr Asn
            100                 105                 110

Ser Ala Pro Phe Thr Phe Gly Pro Gly Thr Lys Val Asp Ile Lys
        115                 120                 125

<210>  18

<211>  508

<212>  DNA

<213>  Artificial Sequence

<220>

<223>  Includes BamHI/BglII cloning junction, signal peptide, V region,
portion of C region and 3\222XbaI/NheI (heavy) or NheI (light) cloning
junction

<400>  18
ggatctcacc atggggtcaa ccgccatcct caccatggag ttggggctgc gctgggttct      60

cctcgttgct cttttaagag gtgtccagtg tcaggtgcag ctggtggagt ctgggggagg     120

cgtggtccag cctgggaggt ccctgagact ctcctgtgca gcgtctggat tcaccttcag     180

taactatgtc atgcactggg tccgccaggc tccaggcaag gggctggagt gggtggcaat     240

tatatggtat gatggaagta ataaatacta tgcagactcc gtgaagggcc gattcaccat     300

ctccagagac aattccaaga acacgctgta tctgcaaatg aacagcctga gagccgagga     360

cacggctgtg tattactgtg cgggtggata taactggaac tacgagtacc actactacgg     420

tatggacgtc tggggccaag ggaccacggt caccgtctcc tcagcctcca ccaagggccc     480

atcggtcttc cccctggcac cctctagc                                        508

<210>  19

<211>  143

<212>  PRT

<213>  Homo sapiens

<400>  19

Met Glu Leu Gly Leu Arg Trp Val Leu Leu Val Ala Leu Leu Arg Gly
1               5                   10                  15

Val Gln Cys Gln Val Gln Leu Val Glu Ser Gly Gly Gly Val Val Gln
            20                  25                  30

Pro Gly Arg Ser Leu Arg Leu Ser Cys Ala Ala Ser Gly Phe Thr Phe
        35                  40                  45

Ser Asn Tyr Val Met His Trp Val Arg Gln Ala Pro Gly Lys Gly Leu
    50                  55                  60

Glu Trp Val Ala Ile Ile Trp Tyr Asp Gly Ser Asn Lys Tyr Tyr Ala
65                  70                  75                  80

Asp Ser Val Lys Gly Arg Phe Thr Ile Ser Arg Asp Asn Ser Lys Asn
                85                  90                  95

Thr Leu Tyr Leu Gln Met Asn Ser Leu Arg Ala Glu Asp Thr Ala Val
            100                 105                 110

Tyr Tyr Cys Ala Gly Gly Tyr Asn Trp Asn Tyr Glu Tyr His Tyr Tyr
        115                 120                 125

Gly Met Asp Val Trp Gly Gln Gly Thr Thr Val Thr Val Ser Ser

130                 135                 140

<210>  20

<211>  463

<212>  DNA

<213>  Artificial Sequence
<220>

<223>  Includes BamHI/Bg1II cloning junction, signal peptide, V region,
portion of C region and 3\222XbaI/NheI (heavy) or NheI (light) cloning
junction

<400>  20
ggatctcacc atgagggtcc ccgctcagct cctggggctc ctgctgctct gtttcccagg      60

tgccagatgt gacatccaga tgacccagtc tccatcctca ctgtctgcat ctgtaggaga     120

cagagtcacc atcacttgtc gggcgagtca gggcattacc aattatttag cctggtttca     180

gcagaaacca gggaaagccc ctaagtccct tatctatgct gcatccagtt tgcaaagtgg     240

ggtcccatca aagttcagcg gcagtggatc tgggacagat ttcagtctca ccatcagcag     300

cctgcagcct gaagattttg caacttatta ctgccaacag tataatagtt acccgatcac     360

cttcggccaa gggacacgac tggagattaa acgaactgtg gctgcaccat ctgtcttcat     420

cttcccgcca tctgatgagc agttgaaatc tggaactgct agc                       463

<210>  21

<211>  127

<212>  PRT

<213>  Homo sapiens

<400>  21

Met Arg Val Pro Ala Gln Leu Leu Gly Leu Leu Leu Leu Cys Phe Pro
1               5                   10                  15

Gly Ala Arg Cys Asp Ile Gln Met Thr Gln Ser Pro Ser Ser Leu Ser
            20                  25                  30

Ala Ser Val Gly Asp Arg Val Thr Ile Thr Cys Arg Ala Ser Gln Gly
        35                  40                  45

Ile Thr Asn Tyr Leu Ala Trp Phe Gln Gln Lys Pro Gly Lys Ala Pro
    50                  55                  60

Lys Ser Leu Ile Tyr Ala Ala Ser Ser Leu Gln Ser Gly Val Pro Ser
65                  70                  75                  80

Lys Phe Ser Gly Ser Gly Ser Gly Thr Asp Phe Ser Leu Thr Ile Ser
                85                  90                  95

Ser Leu Gln Pro Glu Asp Phe Ala Thr Tyr Tyr Cys Gln Gln Tyr Asn
            100                 105                 110

Ser Tyr Pro Ile Thr Phe Gly Gln Gly Thr Arg Leu Glu Ile Lys
        115                 120                 125

<210>  22

<211>  490

<212>  DNA

<213>  Artificial Sequence

<220>

<223>  Includes BamHI/Bg1II cloning junction, signal peptide, V region,
portion of C region and 3\222XbaI/NheI (heavy) or NheI (light) cloning
junction

<400>  22
ggatctcacc atggagttgg gacttagctg ggttttcctc gttgctcttt taagaggtgt      60

ccagtgtcag gtccagctgg tggagtctgg gggaggcgtg gtccagcctg ggaggtccct     120

gagactctcc tgtgcagcgt ctggattcac cttcagtagc tatggcatgc actgggtccg     180

ccaggctcca ggcaaggggc tggactgggt ggcaattatt tggcatgatg gaagtaataa     240

atactatgca gactccgtga agggccgatt caccatctcc agagacaatt ccaagaagac     300

gctgtacctg caaatgaaca gtttgagagc cgaggacacg gctgtgtatt actgtgcgag     360

agcttgggcc tatgactacg gtgactatga atactacttc ggtatggacg tctggggcca     420

agggaccacg gtcaccgtct cctcagcctc caccaagggc ccatcggtct tccccctggc     480

accctctagc                                                            490

<210>  23

<211>  145

<212>  PRT

<213>  Homo sapiens

<400>  23

Met Glu Leu Gly Leu Ser Trp Val Phe Leu Val Ala Leu Leu Arg Gly
1               5                   10                  15

Val Gln Cys Gln Val Gln Leu Val Glu Ser Gly Gly Gly Val Val Gln
            20                  25                  30

Pro Gly Arg Ser Leu Arg Leu Ser Cys Ala Ala Ser Gly Phe Thr Phe
        35                  40                  45

Ser Ser Tyr Gly Met His Trp Val Arg Gln Ala Pro Gly Lys Gly Leu
    50                  55                  60

Asp Trp Val Ala Ile Ile Trp His Asp Gly Ser Asn Lys Tyr Tyr Ala
65                  70                  75                  80

Asp Ser Val Lys Gly Arg Phe Thr Ile Ser Arg Asp Asn Ser Lys Lys
                85                  90                  95

Thr Leu Tyr Leu Gln Met Asn Ser Leu Arg Ala Glu Asp Thr Ala Val
            100                 105                 110

Tyr Tyr Cys Ala Arg Ala Trp Ala Tyr Asp Tyr Gly Asp Tyr Glu Tyr
        115                 120                 125

Tyr Phe Gly Met Asp Val Trp Gly Gln Gly Thr Thr Val Thr Val Ser
    130                 135                 140

Ser
145

<210>  24

<211>  463

<212>  DNA

<213>  Artificial Sequence

<220>

<223>  Includes BamHI/Bg1II cloning junction, signal peptide, V region,
portion of C region and 3\222XbaI/NheI (heavy) or NheI (light) cloning
junction

<400>  24
ggatctcacc atgagggtcc ctgctcagct cctggggctc ctgctgctct gtttcccagg      60

tgccagatgt gacatccaga tgacccagtc tccatcctca ctgtctgcat ctgtaggaga     120

cagagtcacc atcacttgtc gggcgagtca gggcattagc cattatttag cctggtttca     180

gcagaaacca gggaaagccc ctaagtccct gatctatgct gcatccagtt tgcaaagtgg     240

ggtcccatca aagttcagcg gcagtggatc tgggacagat ttcactctca ccatcagcag     300

cctacagcct gaagattttg caacttatta ctgccaacag tataatagtt tcccgctcac     360

tttcggcgga gggaccaagg tggagatcaa acgaactgtg gctgcaccat ctgtcttcat     420

cttcccgcca tctgatgagc agttgaaatc tggaactgct agc                       463

<210>  25

<211>  127

<212>  PRT

<213>  Homo sapiens

<400>  25

Met Arg Val Pro Ala Gln Leu Leu Gly Leu Leu Leu Leu Cys Phe Pro
1               5                   10                  15

Gly Ala Arg Cys Asp Ile Gln Met Thr Gln Ser Pro Ser Ser Leu Ser
            20                  25                  30

Ala Ser Val Gly Asp Arg Val Thr Ile Thr Cys Arg Ala Ser Gln Gly
        35                  40                  45

Ile Ser His Tyr Leu Ala Trp Phe Gln Gln Lys Pro Gly Lys Ala Pro
    50                  55                  60

Lys Ser Leu Ile Tyr Ala Ala Ser Ser Leu Gln Ser Gly Val Pro Ser
65                  70                  75                  80

Lys Phe Ser Gly Ser Gly Ser Gly Thr Asp Phe Thr Leu Thr Ile Ser
                85                  90                  95

Ser Leu Gln Pro Glu Asp Phe Ala Thr Tyr Tyr Cys Gln Gln Tyr Asn
            100                 105                 110

Ser Phe Pro Leu Thr Phe Gly Gly Gly Thr Lys Val Glu Ile Lys
        115                 120                 125

<210>  26

<211>  469

<212>  DNA

<213>  Artificial Sequence

<220>

<223>  Includes BamHI/Bg1II cloning junction, signal peptide, V region,
portion of C region and 3\222XbaI/NheI (heavy) or NheI (light) cloning
junction

<400>  26
ggatcccacc atggggtcaa ccgtcatcct cgccctcctc ctggctgttc tccaaggagt      60

ctgtgccgag gtgcagctgg tgcagtctgg agcagaggtg aaaaagcccg gggagtctct     120

gaagatctcc tgtaagggtt ctggatacag ctttaccagt tactggatcg gctgggtgcg     180

ccagatgccc gggaaaggcc tggagtggat ggggatcatc tatcctggtg actctgatac     240

cagatacagc ccgtccttcc aaggccaggt caccatctca gccgacaagt ccatcagcac     300

cgcctacctg cagtggagca gcctgaaggc ctcggacacc gccatgtatt actgtgcgag     360

acggatggca gcagctggcc cctttgacta ctggggccag ggaaccctgg tcaccgtctc     420

ctcagcctcc accaagggcc catcggtctt ccccctggca ccctctagc                 469

<210>  27

<211>  138

<212>  PRT

<213>  Homo sapiens

<400>  27

Met Gly Ser Thr Val Ile Leu Ala Leu Leu Leu Ala Val Leu Gln Gly
1               5                   10                  15

Val Cys Ala Glu Val Gln Leu Val Gln Ser Gly Ala Glu Val Lys Lys
            20                  25                  30

Pro Gly Glu Ser Leu Lys Ile Ser Cys Lys Gly Ser Gly Tyr Ser Phe
        35                  40                  45

Thr Ser Tyr Trp Ile Gly Trp Val Arg Gln Met Pro Gly Lys Gly Leu
    50                  55                  60

Glu Trp Met Gly Ile Ile Tyr Pro Gly Asp Ser Asp Thr Arg Tyr Ser
65                  70                  75                  80

Pro Ser Phe Gln Gly Gln Val Thr Ile Ser Ala Asp Lys Ser Ile Ser
                85                  90                  95

Thr Ala Tyr Leu Gln Trp Ser Ser Leu Lys Ala Ser Asp Thr Ala Met
            100                 105                 110

Tyr Tyr Cys Ala Arg Arg Met Ala Ala Ala Gly Pro Phe Asp Tyr Trp
        115                 120                 125

Gly Gln Gly Thr Leu Val Thr Val Ser Ser
    130                 135

<210>  28

<211>  466

<212>  DNA

<213>  Artificial Sequence

<220>

<223>  Includes BamHI/Bg1II cloning junction, signal peptide, V region,
portion of C region and 3\222XbaI/NheI (heavy) or NheI (light) cloning
junction

<400>  28
ggatctcacc atgagggtcc ccgctcagct tctcttcctt ctgctactct ggctcccaga      60

taccactgga ggaatagtga tgacgcagtc tccagccacc ctgtctgtgt ctccagggga     120

aagagccacc ctctcctgca ggaccagtca gagtattggc tggaacttag cctggtacca     180

acagaaacct ggccaggctc ccaggctcct catctatggt gcatcttcca ggaccactgg     240

tatcccagcc aggttcagtg gcagtgggtc tgggacagag ttcactctca ccatcagcag     300

cctgcagtct gaagattctg cagtttatta ctgtcagcat tatgataact ggcccatgtg     360

cagttttggc caggggaccg agctggagat caaacgaact gtggctgcac catctgtctt     420

catcttcccg ccatctgatg agcagttgaa atctggaact gctagc                    466

<210>  29

<211>  128

<212>  PRT

<213>  Homo sapiens

<400>  29

Met Arg Val Pro Ala Gln Leu Leu Phe Leu Leu Leu Leu Trp Leu Pro
1               5                   10                  15

Asp Thr Thr Gly Gly Ile Val Met Thr Gln Ser Pro Ala Thr Leu Ser
            20                  25                  30

Val Ser Pro Gly Glu Arg Ala Thr Leu Ser Cys Arg Thr Ser Gln Ser
        35                  40                  45

Ile Gly Trp Asn Leu Ala Trp Tyr Gln Gln Lys Pro Gly Gln Ala Pro
    50                  55                  60

Arg Leu Leu Ile Tyr Gly Ala Ser Ser Arg Thr Thr Gly Ile Pro Ala
65                  70                  75                  80

Arg Phe Ser Gly Ser Gly Ser Gly Thr Glu Phe Thr Leu Thr Ile Ser
                85                  90                  95

Ser Leu Gln Ser Glu Asp Ser Ala Val Tyr Tyr Cys Gln His Tyr Asp
            100                 105                 110

Asn Trp Pro Met Cys Ser Phe Gly Gln Gly Thr Glu Leu Glu Ile Lys
        115                 120                 125

<210>  30

<211>  487

<212>  DNA

<213>  Artificial Sequence

<220>

<223>  Includes BamHI/Bg1II cloning junction, signal peptide, V region,
portion of C region and 3\222XbaI/NheI (heavy) or NheI (light) cloning
junction

<400>  30
ggatctcacc atggagtttg ggctgtgctg gattttcctc gttgctcttt taagaggtgt      60

ccagtgtcag gtgcagctgg tggagtctgg gggaggcgtg gtccagcctg ggaggtccct     120

gagactctcc tgtgcagcct ctggattcac cttcattagc tatggcatgc actgggtccg     180

ccaggctcca ggcaaggggc tggagtgggt ggcagttata tcatatgatg gaagtaataa     240

atactatgca gactccgtga agggccgatt caccatctcc agagacaatt ccaagaacac     300

gctgtatctg caaatgaaca gcctgagagc tgaggacacg gctgtgtatt actgtgcgag     360

agtattagtg ggagctttat attattataa ctactacggg atggacgtct ggggccaagg     420

gaccacggtc accgtctcct cagcctccac caagggccca tcggtcttcc ccctggcacc     480

ctctagc                                                               487

<210>  31

<211>  144

<212>  PRT

<213>  Homo sapiens

<400>  31

Met Glu Phe Gly Leu Cys Trp Ile Phe Leu Val Ala Leu Leu Arg Gly
1               5                   10                  15

Val Gln Cys Gln Val Gln Leu Val Glu Ser Gly Gly Gly Val Val Gln
            20                  25                  30

Pro Gly Arg Ser Leu Arg Leu Ser Cys Ala Ala Ser Gly Phe Thr Phe
        35                  40                  45

Ile Ser Tyr Gly Met His Trp Val Arg Gln Ala Pro Gly Lys Gly Leu
    50                  55                  60

Glu Trp Val Ala Val Ile Ser Tyr Asp Gly Ser Asn Lys Tyr Tyr Ala
65                  70                  75                  80

Asp Ser Val Lys Gly Arg Phe Thr Ile Ser Arg Asp Asn Ser Lys Asn
                85                  90                  95

Thr Leu Tyr Leu Gln Met Asn Ser Leu Arg Ala Glu Asp Thr Ala Val
            100                 105                 110

Tyr Tyr Cys Ala Arg Val Leu Val Gly Ala Leu Tyr Tyr Tyr Asn Tyr
        115                 120                 125

Tyr Gly Met Asp Val Trp Gly Gln Gly Thr Thr Val Thr Val Ser Ser
    130                 135                 140

<210>  32

<211>  478

<212>  DNA

<213>  Artificial Sequence

<220>

<223>  Includes BamHI/BglII cloning junction, signal peptide, V region, portion of C region and 3\222XbaI/NheI (heavy) or NheI (light) cloning junction

<400>  32
ggatctcacc atgagggtcc ctgctcagct cctggggctg ctaatgctct ggatacctgg      60

atccagtgca gatattgtga tgacccagac tccactctct ctgtccgtca cccctggaca     120

gccggcctcc atctcctgca agtctagtca gagcctcctg catagtgatg gaaagacctt     180

tttgtattgg tatctgcaga agccaggcca gcctccacag ctcctgatct atgaggtttc     240

caaccggttc tctggagtgc cagataggtt cagtggcagc gggtcaggga cagatttcac     300

actgaaaatc agccgggtgg aggctgagga tgttgggctt tattactgca tgcaaagtat     360

acagcttccg ctcactttcg gcggagggac caaggtggag atcaaacgaa ctgtggctgc     420

accatctgtc ttcatcttcc cgccatctga tgagcagttg aaatctggaa ctgctagc 478

<210> 33

<211> 132

<212> PRT

<213> Homo sapiens

<400> 33

Met Arg Val Pro Ala Gln Leu Leu Gly Leu Leu Met Leu Trp Ile Pro
1               5                   10                  15

Gly Ser Ser Ala Asp Ile Val Met Thr Gln Thr Pro Leu Ser Leu Ser
            20                  25                  30

Val Thr Pro Gly Gln Pro Ala Ser Ile Ser Cys Lys Ser Ser Gln Ser
        35                  40                  45

Leu Leu His Ser Asp Gly Lys Thr Phe Leu Tyr Trp Tyr Leu Gln Lys
    50                  55                  60

Pro Gly Gln Pro Pro Gln Leu Leu Ile Tyr Glu Val Ser Asn Arg Phe
65                  70                  75                  80

Ser Gly Val Pro Asp Arg Phe Ser Gly Ser Gly Ser Gly Thr Asp Phe
                85                  90                  95

Thr Leu Lys Ile Ser Arg Val Glu Ala Glu Asp Val Gly Leu Tyr Tyr
            100                 105                 110

Cys Met Gln Ser Ile Gln Leu Pro Leu Thr Phe Gly Gly Gly Thr Lys
        115                 120                 125

Val Glu Ile Lys
    130

## Description

### RELATED APPLICATIONS

This application claims the benefit under 35 U.S.C. §119 of United States provisional applications 61/893145, filed October 18, 2013 and 61/903589, filed November 13, 2013, the entire contents of each of which are incorporated herein by reference. Foreign priority benefits are claimed under 35 U.S.C. §119(a)-(d) or 35 U.S.C. §365(b) to Japanese application number 2013-235506, filed November 13, 2013, the entire contents of which are also incorporated by reference herein.

### BACKGROUND

Prostate cancer is the most common malignancy and the second leading cause of cancer death in men in the United States (Jemal A, et al., CA Cancer J Clin 2005;55:10-30). Localized prostate cancer typically is treated with surgery or radiation, and recurrent disease can be controlled temporarily with androgen ablation (Klein EA, et al., Urol Clin North Am 2003;30:315-30). However, almost all prostate carcinomas eventually become hormone refractory and then rapidly progress (Denmeade SR, et al., Nat Rev Cancer 2002;2:389-96). Hormone-refractory or androgen-independent prostate cancer has proven to be largely resistant to conventional chemotherapy. Therapies that have been shown to prolong life in subjects with metastatic, castration-resistant prostate cancer (mCRPC) include: Taxotere® (docetaxel), Jevtana® (cabazitaxel), Zytiga® (abiraterone acetate), Provenge® (sipuleucel-T), Xtandi® (enzalutamide) and Xofigo® (radium Ra-223 dichloride). The survival benefits may be modest as in the case of Jevtana®, which offer 2.4 month survival benefit (Gulley J, et al., Am J Ther. 2004;351:1513-20; Petrylak DP, et al., New Engl J Med 2004;351:1513-20). New therapies are needed to expand therapeutic options, such as for subjects with mCRPC.

### SUMMARY OF THE INVENTION

The present invention relates, at least in part, to the surprising discovery that antiandrogens and prostate-specific membrane antigen (PSMA) ligands bound to therapeutic agents (referred to herein as "PSMA ligand conjugates"), such as anticancer agents or cytotoxic agents (referred to herein as, when bound to a PSMA ligand, "PSMA ligand-anticancer agent conjugates" or "PSMA ligand-cytotoxic agent conjugates", respectively), can act synergistically to inhibit proliferation of PSMA-expressing cancer cells. Unexpectedly, this synergism occurs in both androgen-dependent and androgen-independent cells. It was also discovered that mTOR inhibitors and PSMA ligand conjugates also act synergistically to inhibit proliferation of PSMA-expressing cancer cells, such as androgen-independent cells in particular. It was also discovered that prednisone and PSMA ligand conjugates also acted synergistically in combination. Thus, provided herein are methods, compositions and kits for inhibiting proliferation of, or killing, PSMA-expressing cells (*e.g.,* cancer cells such as prostate cancer cells) as well as for sensitizing or resensitizing cells that can express PSMA, such as androgen-independent PSMA-expressing cells, to androgen therapy.

Some aspects of the disclosure provide methods of inhibiting proliferation of prostate-specific membrane antigen (PSMA)-expressing cancer cells. The methods may comprise contacting PSMA-expressing cancer cells with a compound that increases cell surface expression of PSMA, and contacting the PSMA-expressing cancer cells with a PSMA ligand-anticancer agent conjugate.

In some embodiments, the compound that increases cell surface expression of PSMA is an antiandrogen.

In some embodiments, the antiandrogen blocks enzyme cytochrome CYP17. In some embodiments, the antiandrogen blocks enzyme cytochrome CYP17A1.

In some embodiments, the antiandrogen is an androgen receptor antagonist.

In some embodiments, the antiandrogen is abiraterone, enzalutamide, nilutamide, flutamide, bicalutamide, ARN 509, galeterone or orteronel. In some embodiments, the antiandrogen is enzalutamide or abiraterone.

In some embodiments, the antiandrogen is in an amount effective to upregulate PSMA expression.

In some embodiments, the step of contacting the PSMA-expressing cancer cells with the compound that increases cell surface expression of PSMA, such as an antiandrogen, and the step of contacting the PSMA-expressing cancer cells with the PSMA ligand-anticancer agent conjugate are concurrent. In other embodiments, the step of contacting the PSMA-expressing cancer cells with the compound, such as an antiandrogen, and the step of contacting the PSMA-expressing cancer cells with the PSMA ligand-anticancer agent conjugate are sequential.

In some embodiments, the step of contacting the PSMA-expressing cancer cells with the compound, such as an antiandrogen, is prior to the step of contacting the PSMA-expressing cancer cells with the PSMA ligand-anticancer agent conjugate.

In some embodiments, the step of contacting the PSMA-expressing cancer cells with the PSMA ligand-anticancer agent conjugate occurs within one week of the step of contacting the PSMA-expressing cancer cells with the compound, such as an antiandrogen.

In some embodiments, the PSMA-expressing cancer cells are in contact with the compound, such as an antiandrogen, for at least 3 days. In other embodiments, the PSMA-expressing cancer cells are in contact with the compound, such as an antiandrogen, for at least 7 days. In yet other embodiments, the PSMA-expressing cancer cells are in contact with the compound, such as an antiandrogen, for at least 14 days. In still other embodiments, the PSMA-expressing cancer cells are in contact with the compound, such as an antiandrogen, for at least 21 days. In still other embodiments, the PSMA-expressing cancer cells are in contact with the compound, such as an antiandrogen, for at least 28 days.

In some embodiments, the PSMA-expressing cancer cells comprise androgen-independent PSMA-expressing cancer cells.

In some embodiments, the PSMA-expressing cancer cells comprise PSMA-expressing cancer cells insensitive to antiandrogen therapy.

In some embodiments, the PSMA-expressing cancer cells insensitive to antiandrogen therapy are re-sensitized to antiandrogen therapy after the step of contacting the PSMA-expressing cancer cells with the compound, such as an antiandrogen.

In some embodiments, the PSMA-expressing cancer cells are of a tumor.

In some embodiments, the PSMA-expressing cancer cells are PSMA-expressing prostate cancer cells. In other embodiments, the PSMA-expressing cancer cells are PSMA-expressing non-prostate cancer cells. In yet other embodiments, the PSMA-expressing cancer cells are of the neovasculature of a non-prostate cancer or non-prostate tumor.

In some embodiments, the PSMA-expressing cancer cells are of a subject. In some embodiments, the subject has progressive metastatic castration-resistant prostate cancer. In some embodiments, the subject has had prior chemotherapy with one or more taxanes. In some embodiments, the subject has had prior treatment with one or more antiandrogens. In some embodiments, the subject has prostate cancer that has progressed despite prior treatment, such as with one or more antiandrogens. In some embodiments, the subject has prostate cancer that is starting to progress despite treatment, such as with one or more antiandrogens. In some embodiments, the subject has not had prior cytotoxic chemotherapy. In some embodiments, the subject has not had prior treatment with one or more antiandrogens. In some embodiments, the subject has not had prior treatment with enzalutamide or abiraterone. In some embodiments, the subject that has not had prior treatment with antiandrogens, such as enzalutamide or abiraterone, is one with metastatic castration-resistant prostate cancer. In some embodiments, the subject is any one of the subjects described herein.

Other aspects of the disclosure provide methods of inhibiting proliferation of prostate-specific membrane antigen (PSMA)-expressing cancer cells. The methods may comprise contacting PSMA-expressing cancer cells with an mTOR inhibitor, and contacting the PSMA-expressing cancer cells with a PSMA ligand-anticancer agent conjugate.

In some embodiments, the mTOR inhibitor is rapamycin.

In some embodiments, the mTOR inhibitor is in an amount effective to upregulate PSMA expression.

In some embodiments, the step of contacting the PSMA-expressing cancer cells with the mTOR inhibitor and the step of contacting the PSMA-expressing cancer cells with the PSMA ligand-anticancer agent conjugate are concurrent.

In some embodiments, the step of contacting the PSMA-expressing cancer cells with the mTOR inhibitor and the step of contacting the PSMA-expressing cancer cells with the PSMA ligand-anticancer agent conjugate are sequential.

In some embodiments, the step of contacting the PSMA-expressing cancer cells with the mTOR inhibitor is prior to the step of contacting the PSMA-expressing cancer cells with the PSMA ligand-anticancer agent conjugate.

In some embodiments, the PSMA-expressing cancer cells are in contact with the mTOR inhibitor for at least 7 days.

In some embodiments, the PSMA-expressing cancer cells comprise androgen-independent PSMA-expressing cancer cells.

In some embodiments, the PSMA-expressing cancer cells comprise PSMA-expressing cancer cells insensitive to antiandrogen therapy.

In some embodiments, the PSMA-expressing cancer cells insensitive to antiandrogen therapy are re-sensitized to antiandrogen therapy after the step of contacting the PSMA-expressing cancer cells with the mTOR inhibitor.

In some embodiments, the PSMA-expressing cancer cells may be of a tumor.

In some embodiments, the PSMA-expressing cancer cells are PSMA-expressing prostate cancer cells. In other embodiments, the PSMA-expressing cancer cells are PSMA-expressing non-prostate cancer cells. In yet other embodiments, the PSMA-expressing cancer cells are of the neovasculature of a non-prostate cancer or non-prostate tumor.

In some embodiments, the PSMA-expressing cancer cells are of a subject. In some embodiments, the subject has progressive metastatic castration-resistant prostate cancer. In some embodiments, the subject has had prior chemotherapy with one or more taxanes. In some embodiments, the subject has had prior treatment with one or more antiandrogens. In some embodiments, the subject has prostate cancer that has progressed despite prior treatment, such as with one or more antiandrogens. In some embodiments, the subject has prostate cancer that is starting to progress despite treatment, such as with one or more antiandrogens. In some embodiments, the subject has not had prior cytotoxic chemotherapy. In some embodiments, the subject has not had prior treatment with one or more antiandrogens. In some embodiments, the subject has not had prior treatment with enzalutamide or abiraterone. In some embodiments, the subject that has not had prior treatment with antiandrogens, such as enzalutamide or abiraterone, is one with metastatic castration-resistant prostate cancer. In some embodiments, the subject is any one of the subjects described herein.

In still other aspects of the disclosure provide methods of inhibiting proliferation of prostate-specific membrane antigen (PSMA)-expressing cancer cells. The methods may comprise contacting PSMA-expressing cancer cells with prednisone, and contacting the PSMA-expressing cancer cells with a PSMA ligand conjugate. In some embodiments, the method further comprises contacting the PSMA-expressinsg cancer cells with a compound that increases cell surface expression of PSMA, such as an antiandrogen.

In some embodiments, the antiandrogen blocks enzyme cytochrome CYP17. In some embodiments, the antiandrogen blocks enzyme cytochrome CYP17A1.

In some embodiments, the antiandrogen is an androgen receptor antagonist.

In some embodiments, the antiandrogen is abiraterone, enzalutamide, nilutamide, flutamide, bicalutamide, ARN 509, galeterone or orteronel. In some embodiments, the antiandrogen is enzalutamide or abiraterone.

In some embodiments, the antiandrogen is in an amount effective to upregulate PSMA expression.

In some embodiments, the step of contacting the PSMA-expressing cancer cells with prednisone and/or the compound that increases cell surface expression of PSMA, such as an antiandrogen, and the step of contacting the PSMA-expressing cancer cells with the PSMA ligand conjugate are concurrent. In other embodiments, the step of contacting the PSMA-expressing cancer cells with prednisone and/or the compound, such as an antiandrogen, and the step of contacting the PSMA-expressing cancer cells with the PSMA ligand conjugate are sequential.

In some embodiments, the step of contacting the PSMA-expressing cancer cells with prednisone and/or the compound, such as an antiandrogen, is prior to the step of contacting the PSMA-expressing cancer cells with the PSMA ligand conjugate.

In some embodiments, the step of contacting the PSMA-expressing cancer cells with the PSMA ligand conjugate occurs within one week of the step of contacting the PSMA-expressing cancer cells with the compound, such as an antiandrogen.

In some embodiments, the PSMA-expressing cancer cells are in contact with the compound, such as an antiandrogen, for at least 3 days. In other embodiments, the PSMA-expressing cancer cells are in contact with the compound, such as an antiandrogen, for at least 7 days. In yet other embodiments, the PSMA-expressing cancer cells are in contact with the compound, such as an antiandrogen, for at least 14 days. In still other embodiments, the PSMA-expressing cancer cells are in contact with the compound, such as an antiandrogen, for at least 21 days. In still other embodiments, the PSMA-expressing cancer cells are in contact with the compound, such as an antiandrogen, for at least 28 days.

In some embodiments, the PSMA-expressing cancer cells comprise androgen-independent PSMA-expressing cancer cells.

In some embodiments, the PSMA-expressing cancer cells comprise PSMA-expressing cancer cells insensitive to antiandrogen therapy.

In some embodiments, the PSMA-expressing cancer cells insensitive to antiandrogen therapy are re-sensitized to antiandrogen therapy after the step of contacting the PSMA-expressing cancer cells with the compound, such as an antiandrogen.

In some embodiments, the PSMA-expressing cancer cells are of a tumor.

In some embodiments, the PSMA-expressing cancer cells are PSMA-expressing prostate cancer cells. In other embodiments, the PSMA-expressing cancer cells are PSMA-expressing non-prostate cancer cells. In yet other embodiments, the PSMA-expressing cancer cells are of the neovasculature of a non-prostate cancer or non-prostate tumor.

In some embodiments, the PSMA-expressing cancer cells are of a subject. In some embodiments, the subject has progressive metastatic castration-resistant prostate cancer. In some embodiments, the subject has had prior chemotherapy with one or more taxanes. In some embodiments, the subject has had prior treatment with one or more antiandrogens. In some embodiments, the subject has prostate cancer that has progressed despite prior treatment, such as with one or more antiandrogens. In some embodiments, the subject has prostate cancer that is starting to progress despite treatment, such as with one or more antiandrogens. In some embodiments, the subject has not had prior cytotoxic chemotherapy. In some embodiments, the subject has not had prior treatment with one or more antiandrogens. In some embodiments, the subject has not had prior treatment with enzalutamide or abiraterone. In some embodiments, the subject that has not had prior treatment with antiandrogens, such as enzalutamide or abiraterone, is one with metastatic castration-resistant prostate cancer. In some embodiments, the subject is any one of the subjects described herein.

Yet other aspects of the disclosure provide methods of specific delivery of a cytotoxic agent to PSMA-expressing cells in a subject. The methods may comprise administering to a subject a compound that increases cell surface expression of PSMA, and administering to the subject a PSMA ligand-cytotoxic agent conjugate.

In some embodiments, the compound that increases cell surface expression of PSMA is an antiandrogen.

In some embodiments, the antiandrogen blocks enzyme cytochrome CYP17. In some embodiments, the antiandrogen blocks enzyme cytochrome CYP17A1.

In some embodiments, the antiandrogen is an androgen receptor antagonist. In some embodiments, the antiandrogen is abiraterone, enzalutamide, nilutamide, flutamide, bicalutamide, ARN 509, galeterone or orteronel. In some embodiments, the antiandrogen is enzalutamide or abiraterone.

In some embodiments, the compound that increases cell surface expression of PSMA is an mTOR inhibitor. In some embodiments, the mTOR inhibitor is rapamycin.

In some embodiments, the step of administering the compound and the step of administering the PSMA ligand-cytotoxic agent conjugate are concurrent. In other embodiments, the step of administering the compound and the step of administering the PSMA ligand-cytotoxic agent conjugate are sequential. In some embodiments, the step of administering the compound is prior to the step of administering the PSMA ligand-cytotoxic agent conjugate.

In some embodiments, the step of administering the PSMA ligand-cytotoxic agent conjugate occurs within one week of the step of administering the compound.

In some embodiments, the PSMA-expressing cells are in contact with the compound for at least 3 days. In other embodiments, the PSMA-expressing cells are in contact with the compound for at least 7 days. In yet other embodiments, the PSMA-expressing cells are in contact with the compound for at least 14 days. In still other embodiments, the PSMA-expressing cells are in contact with the compound for at least 21 days. In yet other embodiments, the PSMA-expressing cells are in contact with the compound for at least 28 days.

In some embodiments, the PSMA-expressing cells comprise androgen-independent PSMA-expressing cancer cells.

In some embodiments, the PSMA-expressing cells comprise PSMA-expressing cancer cells insensitive to antiandrogen therapy.

In some embodiments, the PSMA-expressing cells insensitive to antiandrogen therapy are re-sensitized to antiandrogen therapy after the step of administering the compound.

In some embodiments, the PSMA-expressing cells are of a tumor.

In some embodiments, the PSMA-expressing cells are PSMA-expressing prostate cancer cells. In other embodiments, the PSMA-expressing cells are PSMA-expressing non-prostate cancer cells. In still other embodiments, the PSMA-expressing cells are of the neovasculature of a non-prostate cancer or non-prostate tumor.

In some embodiments, the PSMA-expressing cancer cells are of a subject. In some embodiments, the subject has progressive metastatic castration-resistant prostate cancer. In some embodiments, the subject has had prior chemotherapy with one or more taxanes. In some embodiments, the subject has had prior treatment with one or more antiandrogens. In some embodiments, the subject has prostate cancer that has progressed despite prior treatment, such as with one or more antiandrogens. In some embodiments, the subject has prostate cancer that is starting to progress despite treatment, such as with one or more antiandrogens. In some embodiments, the subject has not had prior cytotoxic chemotherapy. In some embodiments, the subject has not had prior treatment with one or more antiandrogens. In some embodiments, the subject has not had prior treatment with enzalutamide or abiraterone. In some embodiments, the subject that has not had prior treatment with antiandrogens, such as enzalutamide or abiraterone, is one with metastatic castration-resistant prostate cancer. In some embodiments, the subject is any one of the subjects described herein.

Still other aspects of the disclosure provide compounds that increase cell surface expression of PSMA and a PSMA ligand-anticancer agent conjugate or PSMA ligand-cytotoxic agent conjugate. In yet other aspects, compositions are provided the comprise prednisone and a PSMA ligand conjugates as provided herein. In some embodimebts, the compositions further comprise a compound that increases cell surface expression of PSMA.

In some embodiments, the compound that increases cell surface expression of PSMA is an antiandrogen.

In some embodiments, the antiandrogen blocks enzyme cytochrome CYP17. In some embodiments, the antiandrogen blocks enzyme cytochrome CYP17A1.

In some embodiments, the antiandrogen is an androgen receptor antagonist. In some embodiments, the antiandrogen is abiraterone, enzalutamide, nilutamide, flutamide, bicalutamide, ARN 509, galeterone or orteronel. In some embodiments, the antiandrogen is enzalutamide or abiraterone.

In some embodiments, the compound that increases cell surface expression of PSMA is an mTOR inhibitor. In some embodiments, the mTOR inhibitor is rapamycin.

In some embodiments, the compositions further comprise a pharmaceutically acceptable carrier and/or excipient.

Still other aspects of the disclosure provide kits that may comprise a container containing a compound that increases cell surface expression of PSMA in a PSMA-expressing cell, and a container containing a PSMA ligand-anticancer agent conjugate or PSMA ligand-cytotoxic agent conjugate. In other aspects, kits are provided that comprise a container containing prednisone, and a container containing a PSMA ligand conjugate. In some embodiments, the kit further comprises a container that comprises a compound that increases cell surface expression of PSMA.

In some embodiments, the compound that increases cell surface expression of PSMA is an antiandrogen.

In some embodiments, the antiandrogen blocks enzyme cytochrome CYP17. In some embodiments, the antiandrogen blocks enzyme cytochrome CYP17A1.

In some embodiments, the antiandrogen is an androgen receptor antagonist. In some embodiments, the antiandrogen is abiraterone, enzalutamide, nilutamide, flutamide, bicalutamide, ARN 509, galeterone or orteronel. In some embodiments, the antiandrogen is enzalutamide or abiraterone.

In some embodiments, the compound that increases cell surface expression of PSMA is an mTOR inhibitor. In some embodiments, the mTOR inhibitor is rapamycin.

In some embodiments, the kits further comprise a pharmaceutically acceptable carrier and/or excipient.

In some embodiments, the compound and/or the PSMA ligand conjugate is in an aqueous medium. In other embodiments, the compound and/or the PSMA ligand conjugate are/ is lyophilized.

In some embodiments, the kits further comprise a diluent.

In some embodiments, the kits further comprise instructions for reconstituting the compound and/or the PSMA ligand conjugate. In other embodiments, the kits further comprise instructions for reconstituting prednisone and/or the PSMA ligand conjugate and/or the compound (when such kits further comprise the compound).

In some embodiments, the kits further comprise instructions for combining the compound and/or the PSMA ligand conjugate. In other embodiments, the kits further comprise instructions for combining prednisone and/or the PSMA ligand conjugate. In yet other embodiments, the kits further comprise instructions for combinding the compound with prednisone and/or the PSMA ligand conjugate (when such kits further comprise the compound).

In some aspects, a method of inhibiting proliferation of prostate-specific membrane antigen (PSMA)-expressing cancer cells is provided. In some embodiments, the method comprises contacting PSMA-expressing cancer cells with prednisone; and contacting the PSMA-expressing cancer cells with a PSMA ligand conjugate. In embodiments of these methods, the PSMA-expressing cancer cells may be any one of the cells provided herein. In other embodiments, the PSMA ligand conjugate is any one of the PSMA ligand conjugates provided herein. In other aspects, a composition comprising prednisone and a PSMA ligand conjugate is provided. In still other aspects, a kit comprising a container containing prednisone, and a container containing a PSMA ligand conjugate is provided.

In some emboiments of any one of these methods, compositions or kits, the PSMA ligand of the conjugate comprises an antibody, or antigen-binding fragment thereof, that binds specifically PSMA. In such embodiments, the PSMA ligand is any one of the antibodies or antigen-binding fragments provided herein.

In some embodiments of any one of these methods, compositions or kits, the PSMA ligand of the conjugate comprises a small molecule ligand that binds specifically PSMA. In such embodiments, the PSMA ligand is any one of the small molecule ligands provided herein.

In some embodiments of any one of these methods, compositions or kits, the PSMA ligand conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.

In some embodiments of any one of these methods, compositions or kits, the anticancer or cytotoxic agent of the PSMA ligand conjugate is any one of the anticancer or cytotoxic agents provided herein. In some embodiments, the agent comprises an auristatin, tubulysin,a pyrrolobenzodiazepine dimer, calicheamicin, colchicine, ispinesib, combrestatin A4, maytansinoid DM1, maytansinoid DM4, doxorubicin, or a cytotoxic radionuclide. In some embodiments, the auristatin comprises monomethylauristatin norephedrine or monomethylauristatin phenylalanine.

In some embodiments of any one of these methods, the method further comprises contacting the PSMA expressing cells with a compound that increases cell surface expression of PSMA. In some embodiments of any one of these compositions or kits, the compositions or kits further comprise a compound that increases cell surface expression of PSMA. In some embodiments, the compound is an antiandrogen or an mTOR inhibitor. In such embodiments, the antiandrogen can be any one of the antiandrogens provided herein. In some embodiments, the mTOR inhibitor is any one of the mTOR inhibitors provided herein

In some embodiments of any one of these methods, compositions or kits, the antiandrogen is abiraterone, enzalutamide, nilutamide, flutamide, bicalutamide, ARN 509, galeterone or orteronel. In some embodiments, the antiandrogen is enzalutamide, abiraterone or ARN 509.

In some embodiments of any one of the methods provided, the steps of contacting are concurrent. In other embodiments, the steps of contacting are sequential. In some embodiments, the step of contacting the PSMA-expressing cancer cells with the prednisone and/or compound is prior to the step of contacting the PSMA-expressing cancer cells with the PSMA ligand conjugate.

In some embodiments of any one of these methods, compositions or kits, the PSMA-expressing cancer cells comprise androgen-independent PSMA-expressing cancer cells. In some embodiments, the PSMA-expressing cancer cells comprise PSMA-expressing cancer cells insensitive to antiandrogen therapy.

In some embodiments of any one of these methods, the PSMA-expressing cancer cells are of a subject. In such embodiments, the subject can be any one of the subjects provided herein.

In some embodiments of any one of the foregoing methods or compositions or kits, the antiandrogen is enzalutamide, abiraterone or ARN 509, and the PSMA ligand-anticancer agent conjugate is a PSMA ADC.

In some embodiments of any one of the foregoing compositions or kits, the compositions or kits further comprise a pharmaceutically acceptable carrier and/or excipient.

In some embodiments of any one of the foregoing kits, any one or all of the components of the kits are in an aqueous medium. In some embodiments of any one of the foregoing kits, any one or all of the components of the kits are lyophilized.

In some embodiments of any one of the foregoing kits, the kits further comprise instructions for reconstituting any one or all of the components of the kits. In some embodiments, of any one of the foregoing kits, the kits further compise instructions for combining any one or all of the components of the kits.

In some embodiments of any one of the foregoing compositions or kits, the compositions or kits further comprise a diluent.

In any one of the foregoing aspects or embodiments, the PSMA ligand of the conjugate may comprise an antibody, or antigen-binding fragment thereof, that binds specifically PSMA. In an embodiment, the PSMA ligand is an antibody or antigen-binding fragment thereof that binds the extracellular portion of PSMA. In another embodiment, the PSMA ligand is an antibody or antigen-binding fragment thereof that binds a PSMA protein dimer. In still another embodiment, the PSMA ligand is an antibody or antigen-binding fragment thereof that binds preferentially a PSMA protein dimer not a PSMA protein monomer. In a further embodiment, the antibody or antigen-binding fragment thereof binds with at least a 2-fold, 3-fold, 4-fol, 5-fold, 10-fold or more greater affinity to a PSMA protein dimer as compared to a PSMA protein monomer. In yet another embodiment, the PSMA protein dimer and PSMA protein monomer are in a non-denatured form, such as a native conformation.

In any one of the foregoing aspects or embodiments, the antibody or antigen-binding fragment thereof may be an antibody selected from the group consisting of: PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1 and Abgenix 4.152.1, or an antigen-binding fragment thereof.

In any one of the foregoing aspects or embodiments, the antibody or antigen-binding fragment thereof may comprise (i) the three complementarity determining regions of a heavy chain variable region comprising an amino acid sequence set forth as SEQ ID NO:15 and (ii) the three complementarity determining regions of a light chain variable region comprising an amino acid sequence set forth as SEQ ID NO: 17.

In any one of the foregoing aspects or embodiments, the antibody or antigen-binding fragment thereof may comprise (i) the three complementarity determining regions of a heavy chain variable region comprising an amino acid sequence set forth as SEQ ID NO:19 and (ii) the three complementarity determining regions of a light chain variable region comprising an amino acid sequence set forth as SEQ ID NO: 21.

In any one of the foregoing aspects or embodiments, the antibody or antigen-binding fragment thereof may comprise (i) the three complementarity determining regions of a heavy chain variable region comprising an amino acid sequence set forth as SEQ ID NO:23 and (ii) the three complementarity determining regions of a light chain variable region comprising an amino acid sequence set forth as SEQ ID NO: 25.

In any one of the foregoing aspects or embodiments, the antibody or antigen-binding fragment thereof may comprise (i) the three complementarity determining regions of a heavy chain variable region comprising an amino acid sequence set forth as SEQ ID NO:27 and (ii) the three complementarity determining regions of a light chain variable region comprising an amino acid sequence set forth as SEQ ID NO: 29.

In any one of the foregoing aspects or embodiments, the antibody or antigen-binding fragment thereof may comprise (i) the three complementarity determining regions of a heavy chain variable region comprising an amino acid sequence set forth as SEQ ID NO: 31 and (ii) the three complementarity determining regions of a light chain variable region comprising an amino acid sequence set forth as SEQ ID NO: 33.

In any one of the foregoing aspects or embodiments, the antibody or antigen-binding fragment thereof may be antibody PSMA 10.3, AB-PG1-XG1-006, AB-PG1-XG1-026, AB-PG1-XG1-051, AB-PG1-XG1-069, AB-PG1-XG1-077, or an antigen-binding fragment thereof.

In any one of the foregoing aspects or embodiments, the antibody or antigen-binding fragment thereof may be antibody E99, J415, J533 or J591 or an antibody produced by a hybridoma having ATCC Accession Number HB-12101, HB-12109, HB-12127or HB-12126, or an antigen -binding fragment thereof.

In any one of the foregoing aspects or embodiments, the antibody or antigen-binding fragment thereof may be an antibody produced by a hybidoma having ATCC Accession Number HB12060 (3F5.4G6), HB12309 (3D7-1.1), HB12310 (4E10-1.14), HB12489 (1G3), HB12495 (1G9), HB 12490 (2C7), HB 12494 (3C4), HB 12491 (3C6), HB 12484 (3C9), HB 12486 (3E6), HB 12488 (3E11), HB 12485 (3G6), HB 12493 (4D4), HB 12487 (4D8), HB 12492 (4C8B9), HB 12664 (3F6), HB 12678 (2E4), HB 12665 (3C2), HB 12672 (2D4), HB12660 (4C8G8), HB12675 (2C4), HB12663 (4C11), HB12661 (1D11), HB12667 (4E8), HB 12674 (2G5), HB 12620 (4E6), HB 12677 (1F4), HB 12666 (2E3), HB 12662 (3D8), HB12668 (4F8), HB12673 (3D2), HB12676 (1G7), HB12669 (3D4), HB12679 (5G10) or HB 12671 (5E9), or an antigen-binding fragment thereof.

In any one of the foregoing aspects or embodiments, the PSMA ligand of the conjugate may comprise a small molecule ligand that binds specifically PSMA. In an embodiment, the small molecule ligand binds or inhibits an enzymatic site of PSMA. In a particular embodiment, the small molecule ligand binds or inhibits the glutamate carboxypeptidase II (CCPII) site on PSMA.

In any one of the foregoing aspects or embodiments, the small molecule ligand may comprise MIP-1095, MIP-1072, GL2 or DUPA.

In any one of the foregoing aspects or embodiments, the PSMA ligand-anticancer agent conjugate may comprise EC1069 or EC1719.

In any one of the foregoing aspects or embodiments, the PSMA ligand-anticancer agent conjugate may comprise BIND-014.

In any one of the foregoing aspects or embodiments, the PSMA ligand conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.

In any one of the foregoing aspects or embodiments, the PSMA ligand conjugate may comprise ¹²³I-MIP-1095 or ¹²³I-MIP-1072.

In any one of the foregoing aspects or embodiments, the anticancer agent may comprise an auristatin, tubulysin,a pyrrolobenzodiazepine dimer, calicheamicin, colchicine, ispinesib, combrestatin A4, maytansinoid DM1, maytansinoid DM4, doxorubicin, or a cytotoxic radionuclide.

In any one of the foregoing aspects or embodiments, the auristatin may comprise monomethylauristatin norephedrine or monomethylauristatin phenylalanine.

In some embodiments of any one of the methods provided, the subject has received treatment with an antiandrogen and has experienced no or minimal sensitivity to the antiandrogen therapy. In some embodiments of any one of the methods provided, the subject has experienced a loss of sensitivity to an antiandrogen therapy. A subject that is sensitive to antiandrogen therapy is one that experiences an appreciable reduction in the subject's cancer, such as a decrease in the number of cancer cells, such as a decrease in the number of circulating cancer cells or tumor size, or symptoms associated with the cancer. Sensitivity to antiandrogen therapy may be measured by, for example, determining the level of proliferation of the subject's cancer cells after treatment and, in some embodiments, may be compared to the level of proliferation of the cancer cells prior to the treatment. Sensitivity to antiandrogen therapy may be determined during a course of a treatment with an antiandrogen, and when the level of reduction in the subject's cancer no longer appreciably changes or the subject experiences an undesired increase in the progression of the cancer, the subject may be considered to have experienced a loss of sensitivity to the antiandrogen therapy. In some embodiments, an increase in a subject's cancer refers to an increase in the number of cancer cells or tumor size or to an increase in the symptoms associate with the cancer. Sensitivity to antiandrogen therapy may be determined using routine methods by a clinician.

In embodiments of any one of the methods provided herein the subject may be one that has no or minimal sensitivity to an antiandrogen therapy. Additionally, in embodiments of any one of the methods provided herein the subject is one that has experienced a loss of sensitivity to antiandrogen therapy. Any one of the methods and compositions provided herein can be used to sensitize or resensitize such subjects to treatment with an antiandrogen when administered concurrently or sequentially with the PSMA ligand conjugates provided herein.

Any one of the methods provided herein can, in some embodiments, include the step of identifying a subject that has no or minimal sensitivity to antiandrogen therapy or that has experienced a loss of sensitivity to antiandrogen therapy. Any one of the methods provided herein, in some embodiments, can include the step of assessing the level of sensitivity to antiandrogen therapy in a subject. Any one of the methods provided herein, in some embodiments, can include the step of administering an antiandrogen until there is a loss of sensitivity thereto as well as a step of administering an antiandrogen concurrently or sequentially with a PSMA ligand conjugate as provided herein.

Any one of the methods provided herein can include a step of assessing the progression of the cancer in the subject. Progression can be assessed in a number of ways. In some embodiments, progression is assessed by any one or more of the following: determining the level of PSA, assessing bone metastases and assessing measurable disease. In some embodiments, progression of bone metastases is the appearance of 2 or more new bone lesions on a bone scan (CT scan, MRI), or new radiographic lesions. Progression may also be determined by RECIST (Eisenhauer EA et al Eur J Cancer 2009:45(2):228-47). In other embodiments, progression of the cancer is occurring when there is an increase in pain, such as bone pain. In any one of the methods provided herein, assessing progression can comprise assessing any one or more of the foregoing.

In some embodiments of any one of the methods provided herein, the PSMA-expressing cancer cells are of a subject. In some embodiments, the subject has progressive metastatic castration-resistant prostate cancer. In some embodiments, the subject has had prior chemotherapy with one or more taxanes. In some embodiments, the subject has had prior treatment with one or more antiandrogens. In some embodiments, the subject has prostate cancer that has progressed despite prior treatment, such as with one or more antiandrogens. In some embodiments, the subject has prostate cancer that is starting to progress despite treatment, such as with one or more antiandrogens. In some embodiments, the subject has not had prior cytotoxic chemotherapy. In some embodiments, the subject has not had prior treatment with one or more antiandrogens. In some embodiments, the subject has not had prior treatment with enzalutamide or abiraterone. In some embodiments, the subject that has not had prior treatment with antiandrogens, such as enzalutamide or abiraterone, is one with metastatic castration-resistant prostate cancer. In some embodiments, the subject is any one of the subjects described herein.

### BRIEF DESCRIPTION OF THE DRAWINGS

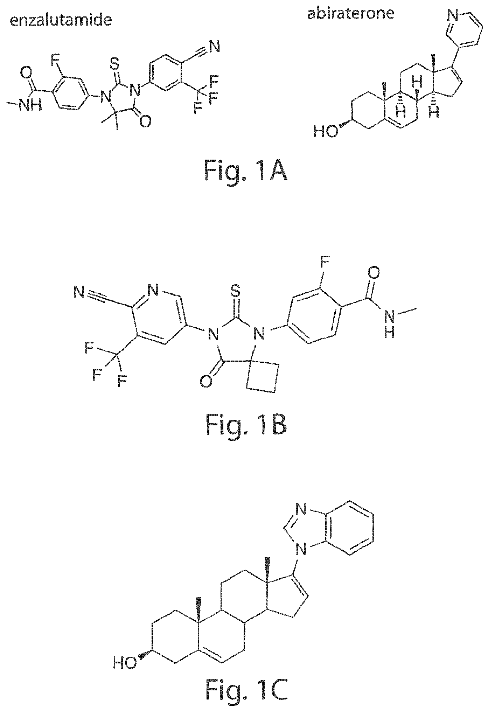

**FIG. 1A** presents a schematic showing the structure of two examples of antiandrogens that increase the expression of PSMA, enzalutamide and abiraterone; **FIG. 1B** presents a schematic showing the chemical structure of ARN-509 (MW=477.43); **FIG. 1C** presents a schematic showing the chemical structure of galeterone (TOK-001) (MW=388.55); **FIG. ID** presents a schematic showing the structure of a PSMA ligand conjugate that includes a PSMA antibody conjugated to monomethyl auristatin E (MMAE) (PSMA antibody-drug conjugate (PSMA ADC)), an average of four molecules of MMAE in this embodiment.

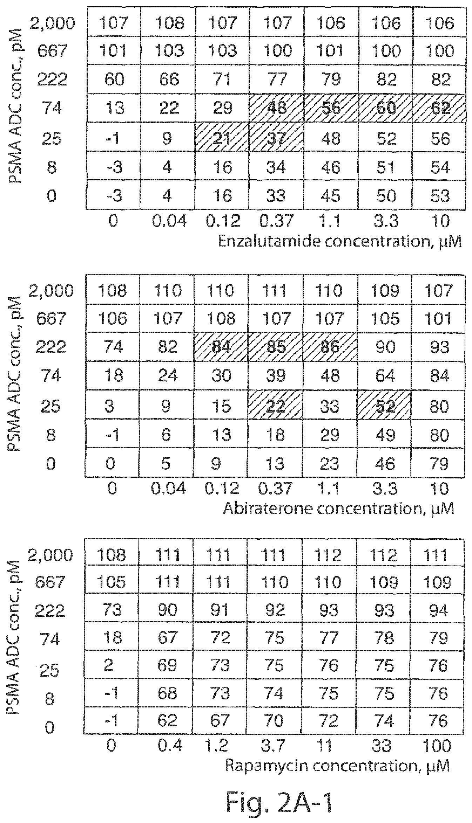

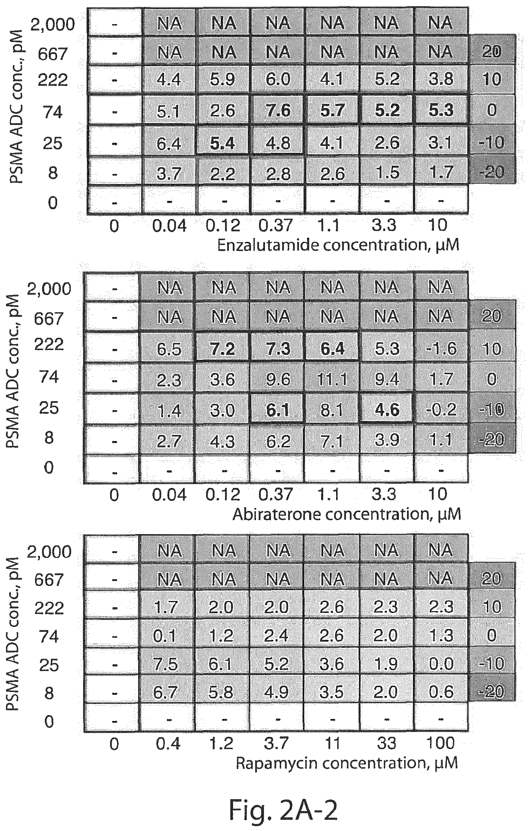

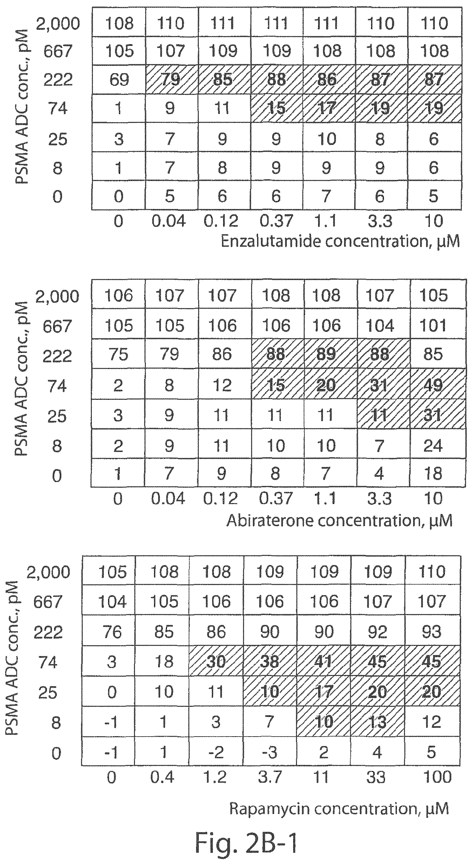

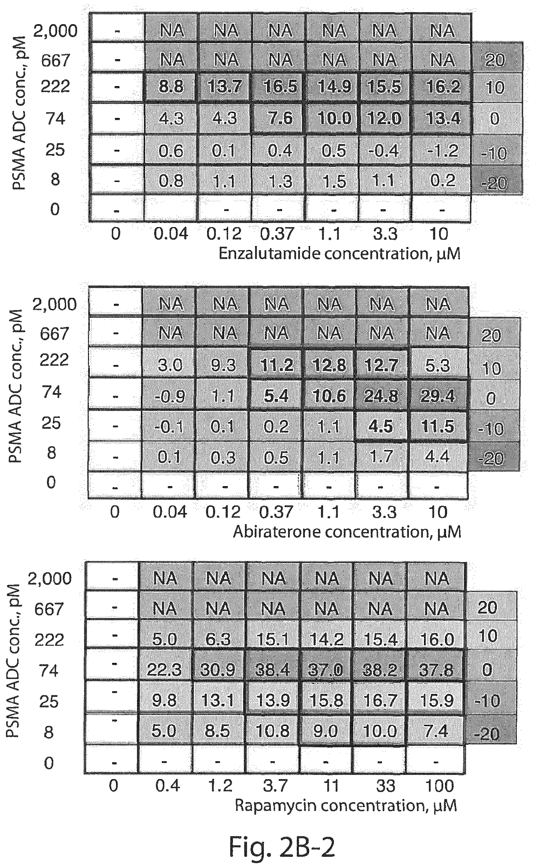

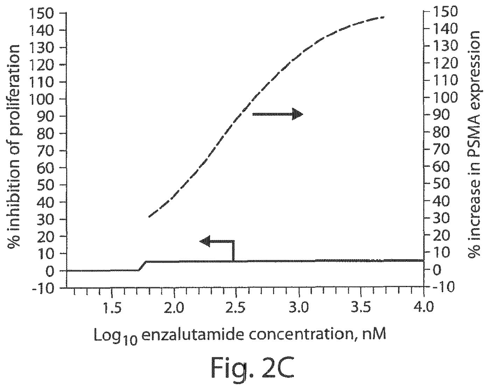

**FIG. 2** shows percent cell proliferation inhibition values and Bliss differences for LNCaP cells (A) and C4-2 cells (B) contacted with combinations of PSMA ligand conjugate and enzalutamide, abiraterone or rapamycin. Each data point represents the mean of three to seven independent assays. Bliss differences are depicted via heat maps of values that are positive (medium gray), negative (dark gray), or near zero (light gray). Bliss parameters that are significantly different from zero (P<0.05) are highlighted via bold text and dark gray borders, and the corresponding percent inhibition values are highlighted with shading and dark gray text. NA = not applicable. (C) Graph showing increase in PSMA expression and inhibition of cell proliferation as a function of increasing concentration of enzalutamide.

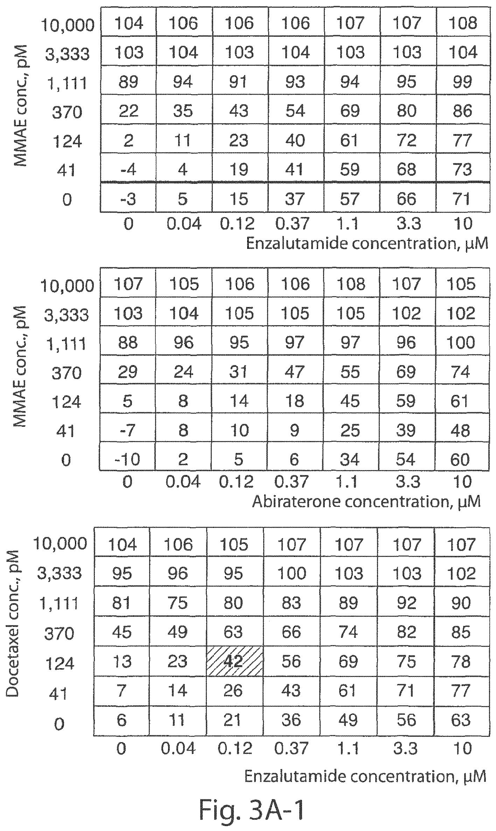

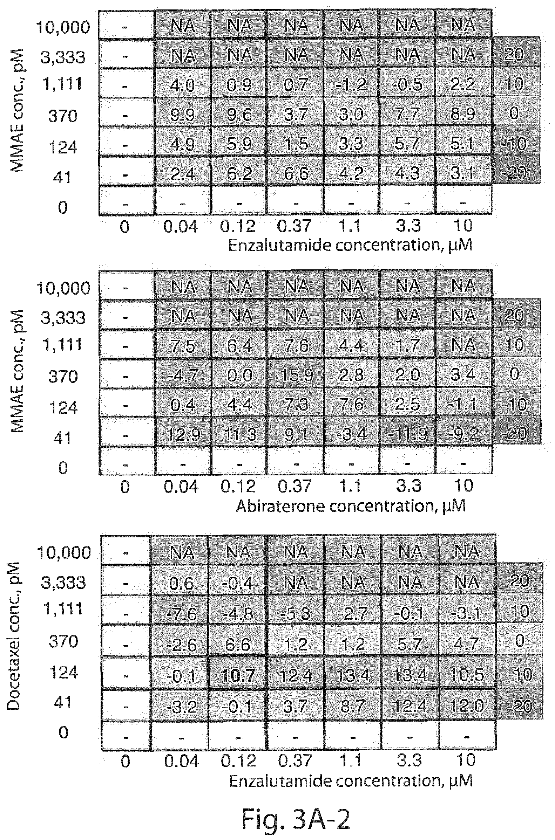

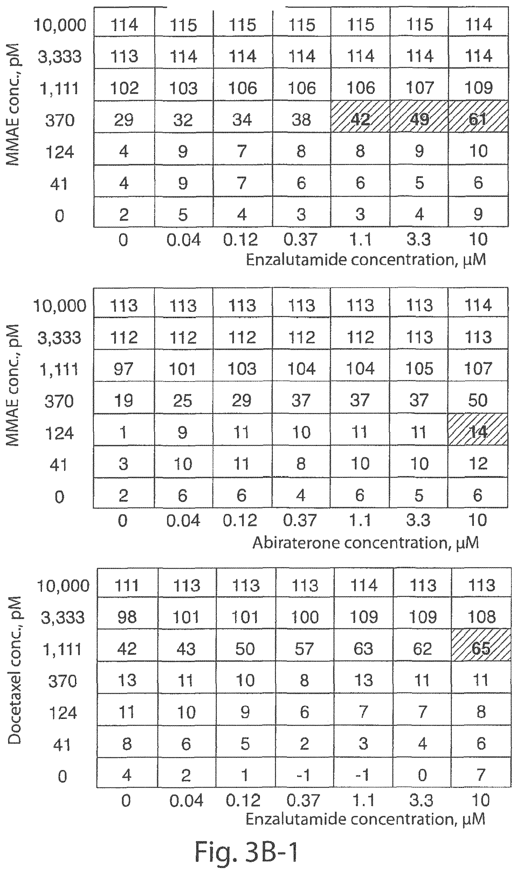

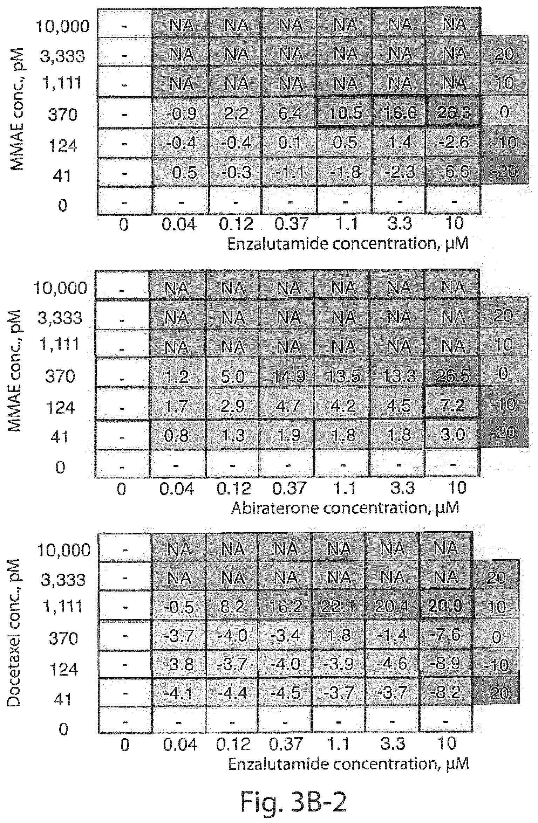

**FIG. 3** shows percent cell proliferation inhibition values and Bliss differences for LNCaP cells (A) and C4-2 cells (B) contacted with combinations of microtubule inhibitors and antiandrogens. Each data point represents the mean of three to five independent assays. Bliss differences are depicted via heat maps of values that are positive (medium gray), negative (dark gray), or near zero (light gray). Bliss parameters that are significantly different from zero (P<0.05) are highlighted via bold text and dark gray borders, and the corresponding percent inhibition values are highlighted with shading and dark gray text. NA = not applicable.

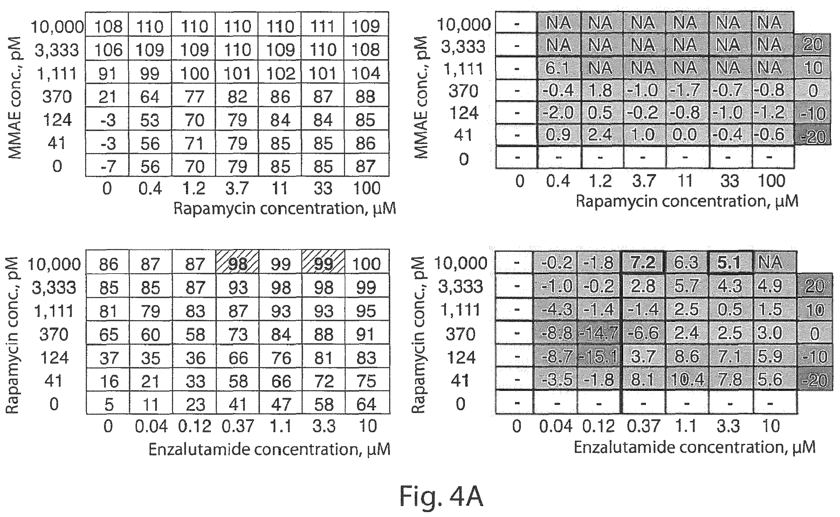

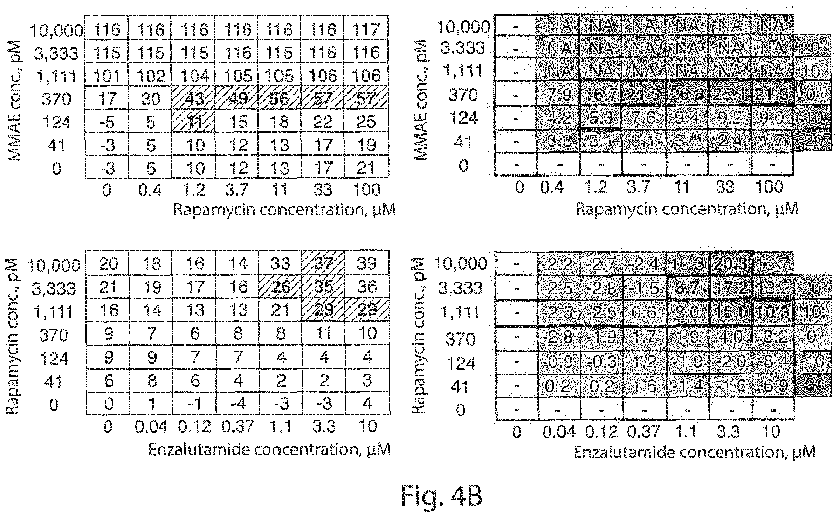

**FIG. 4** shows percent cell proliferation inhibition values and Bliss differences for LNCaP cells (A) and C4-2 cells (B) contacted with combinations of rapamycin with MMAE or enzalutamide. Each data point represents the mean of three independent assays. Bliss differences are depicted via heat maps of values that are positive (medium gray), negative (dark gray), or near zero (light gray). Bliss parameters that are significantly different from zero (P<0.05) are highlighted via bold text and dark gray borders, and the corresponding percent inhibition values are highlighted with shading and dark gray text. NA = not applicable.

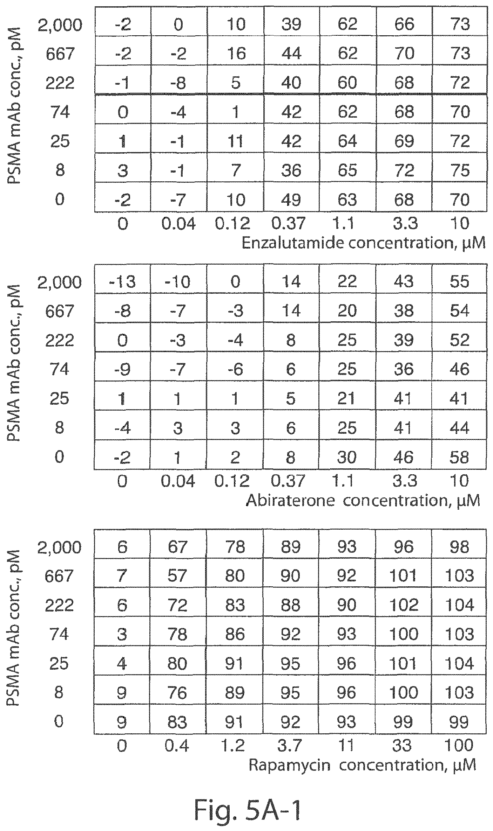

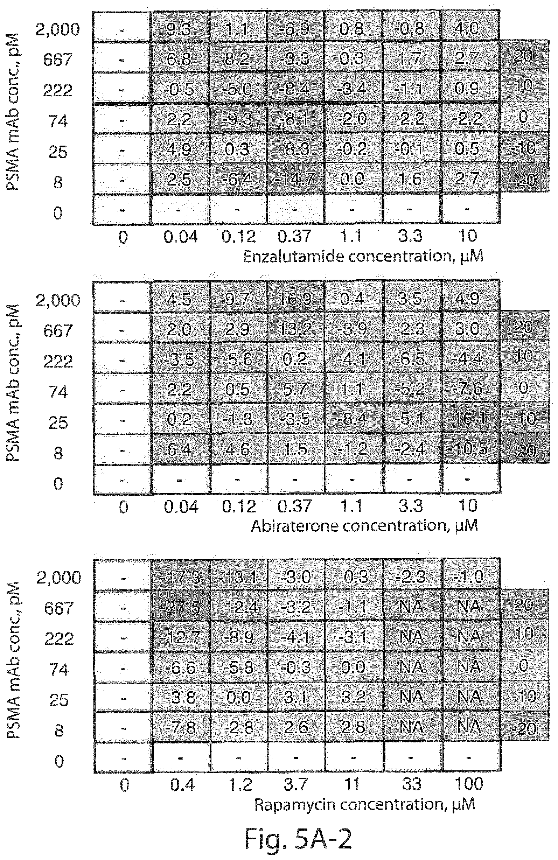

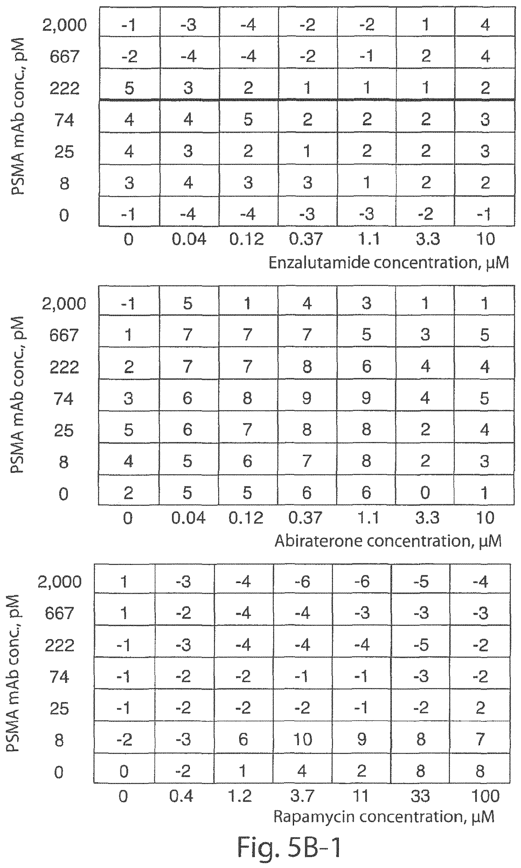

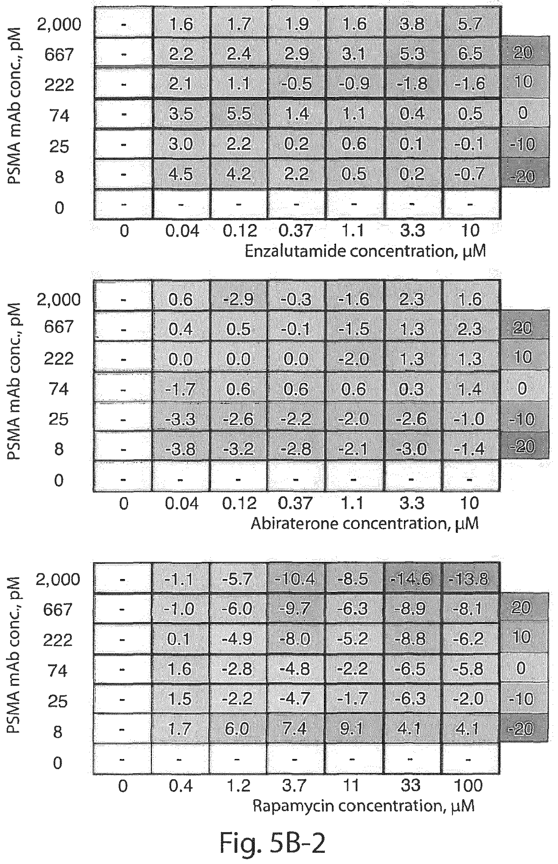

**FIG. 5** shows percent cell proliferation inhibition values and Bliss differences for LNCaP cells (A) and C4-2 cells (B) contacted with combinations of PSMA monoclonal antibody and enzalutamide, abiraterone or rapamycin. Each data point represents the mean of two or three independent assays. Bliss differences are depicted via heat maps of values that are positive (medium gray), negative (dark gray), or near zero (light gray). No Bliss parameters were significantly different from zero (P<0.05). NA = not applicable.

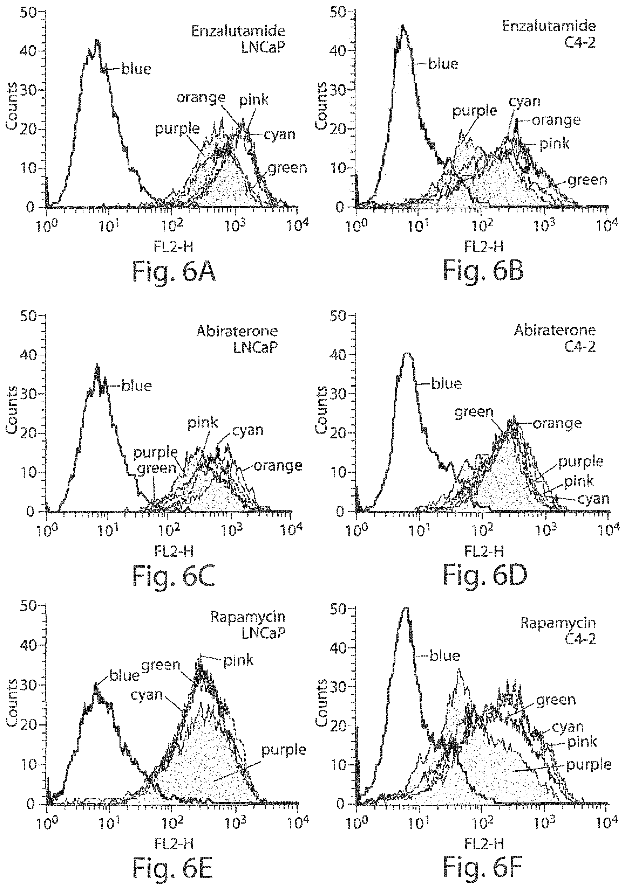

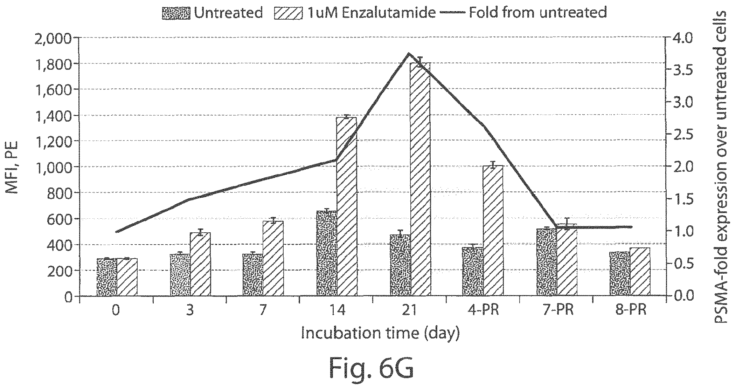

**FIG. 6** shows a flow cytometry analysis of PSMA expression. PSMA expression was measured by flow cytometry on cells treated for 7 days with varying concentrations of enzalutamide, abiraterone or rapamycin (A-F) or treated with 1 µM enzalutamide for varying times (G). In A-D, anti-PSMA staining is shown as a function of antiandrogen concentrations as follows: green (0.125 µM), pink (0.5 µM), cyan (1 µM) and orange (5 µM). In E-F, the green, pink and cyan histograms represent rapamycin concentrations of 1, 10 and 100 nM, respectively. Filled purple histograms denote untreated cells, and the blue line depicts background staining with isotype-control antibody. (G) Time-course of PSMA expression in enzalutamide-treated C4-2 cells. Vertical bars depict mean fluorescence intensity (MFI) values on the left axis, and the red line represents the fold increase in PSMA expression in treated cells relative to untreated cells on the right axis. (PR = days post enzalutamide removal.)

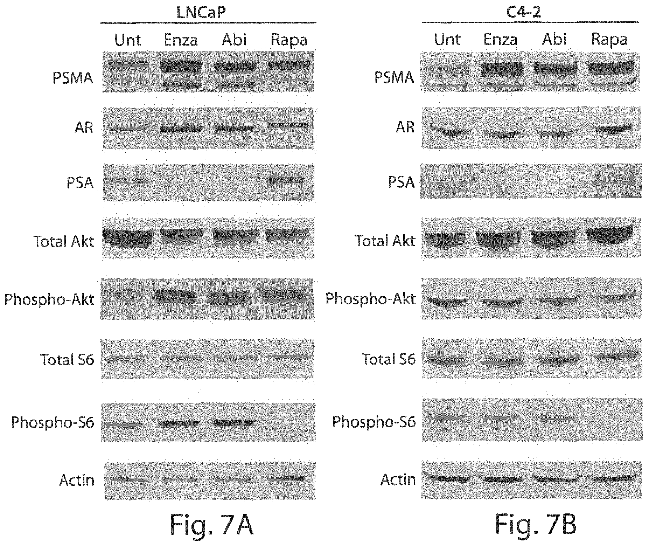

**FIG. 7** shows a Western blot protein expression analysis of whole-cell extracts of LNCaP (A) or C4-2 (B) cells left untreated (Unt) or treated for 7 days with enzalutamide (Enza), abiraterone (Abi) or rapamycin (Rapa) prior to the analysis.

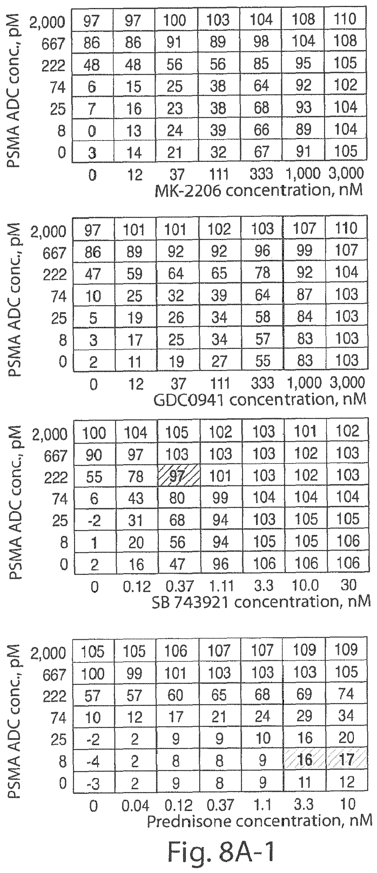

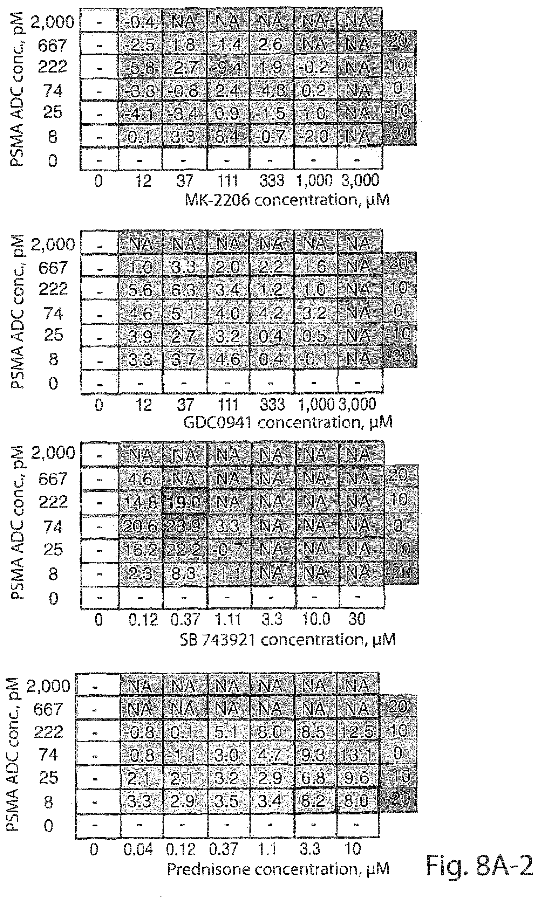

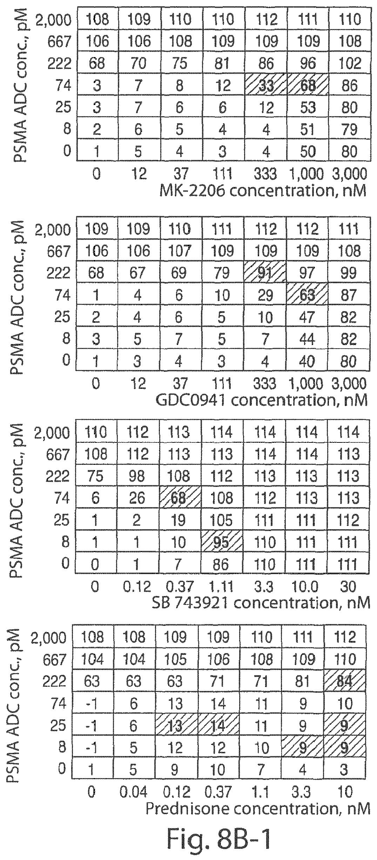

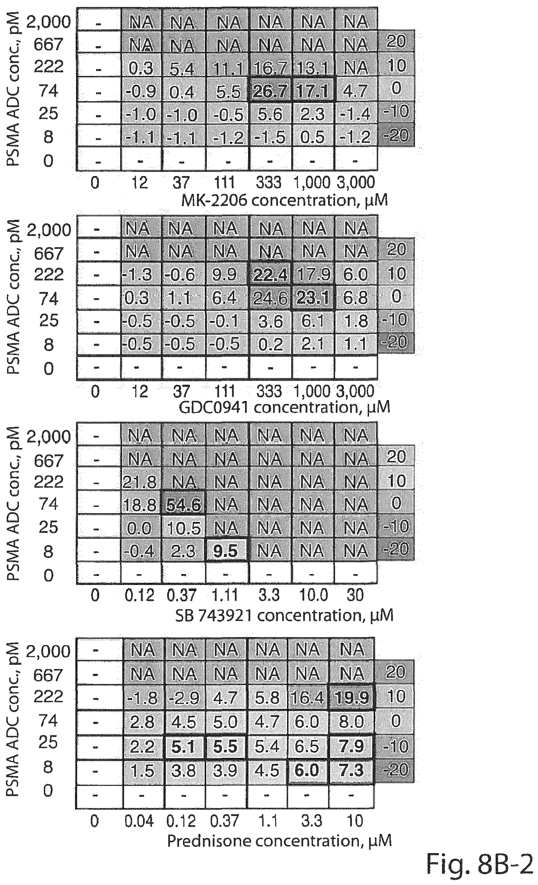

**FIG. 8** shows percent cell proliferation inhibition values and Bliss differences for LNCaP cells (A) and C4-2 cells (B) contacted with combinations of PSMA ligand conjugate and prednisone or inhibitors of Akt, PI3K or kinesin spindle protein (inhibitors MK-2206, GDC-0941 and SB743921, respectively). Each data point represents the mean of four or five independent assays. Bliss differences are depicted via heat maps of values that are positive (medium gray), negative (dark gray), or near zero (light gray). Bliss parameters that are significantly different from zero (P<0.05) are highlighted via bold text and dark gray borders, and the corresponding percent inhibition values are highlighted with shading and dark gray text. NA = not applicable.

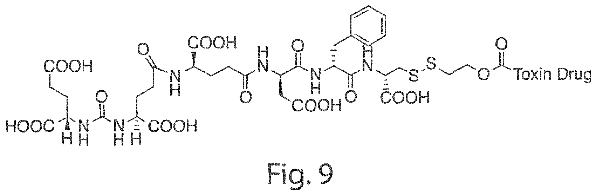

**FIG. 9** presents a schematic showing a chemical structure with a D-γ-Glu D-Asp-D-Phe-D-Cys linker.

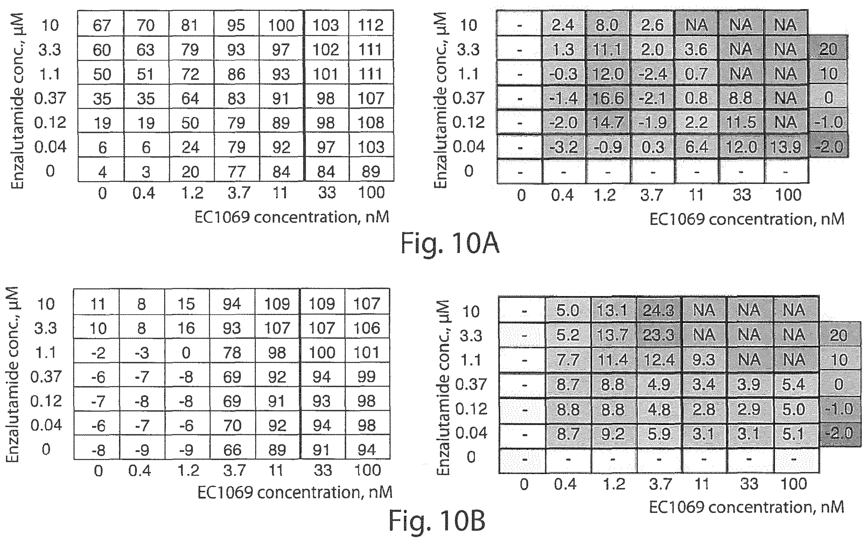

**FIG. 10** shows percent cell proliferation inhibition values and Bliss differences for LNCaP cells (A) and C4-2 cells (B) contacted with combinations of PSMAsmall molecule ligand conjugate, EC1069 and enzalutamide. Each data point represents one independent assay. Bliss differences are depicted via heat maps of values that are positive (medium gray), negative (dark gray), or near zero (light gray). NA = not applicable.

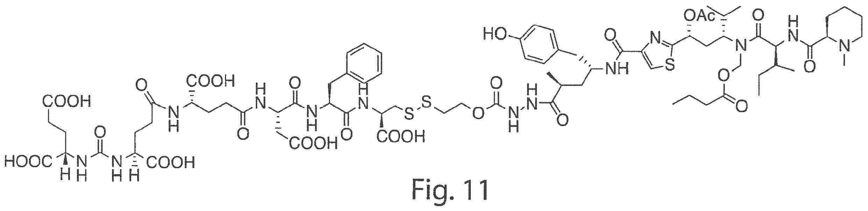

**FIG. 11** presents a schematic showing the structure of PSMA small molecule ligand conjugate that comprises an anticancer agent, tubulysin (also referred to herein as EC1069).

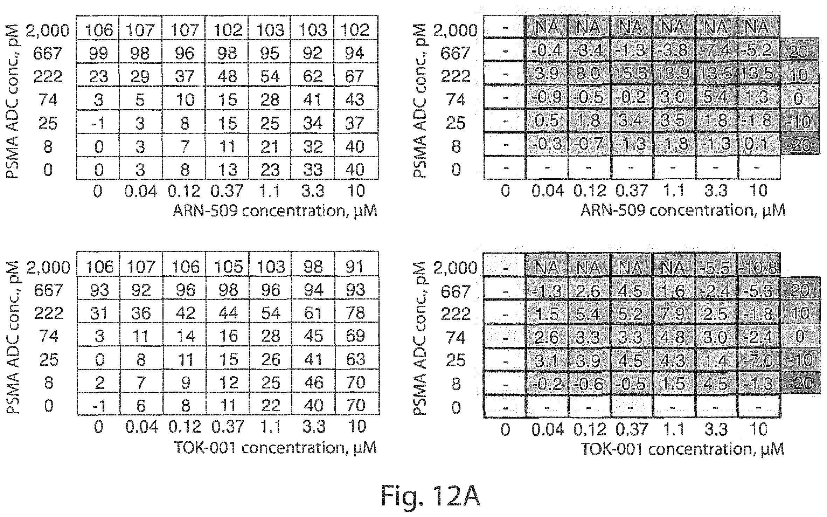

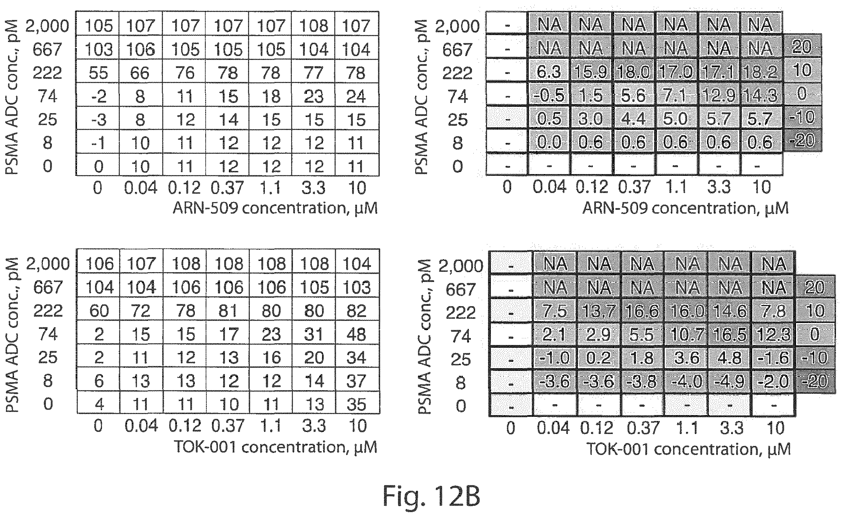

**FIG. 12** presents data from combinations of PSMA ADC with ARN-509 (Aragon Pharmaceuticals, androgen receptor inhibitor) and TOK-001 (Tokai Pharmaceuticals , CYP17 inhibitor and androgen receptor antagonist). Percent inhibition values and Bliss differences are shown for LNCaP cells (A) and C4-2 cells (B). Bliss differences are depicted via heat maps of values that are positive (medium gray), negative (dark gray), or near zero (light gray). NA = not applicable. ARN-509 and TOK-001 were purchased from Selleck Chemicals.

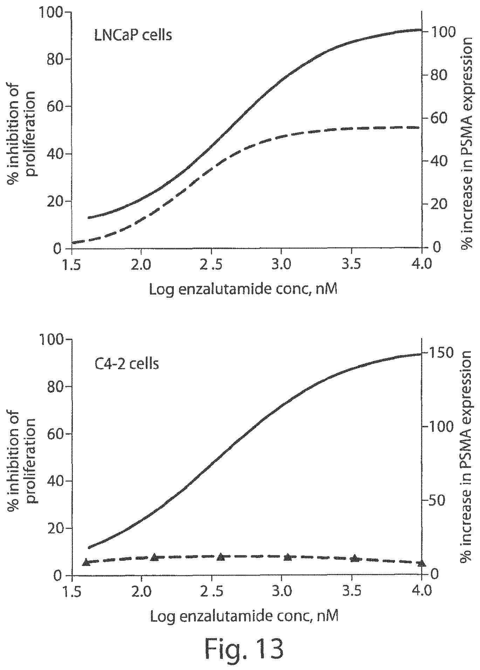

**FIG. 13** demonstrates the effects of enzalutamide on proliferation and PSMA expression. These data show that the effects on proliferation and PSMA expression occur at similar concentrations in LNCaP cells (but are uncoupled in C4-2 cells).

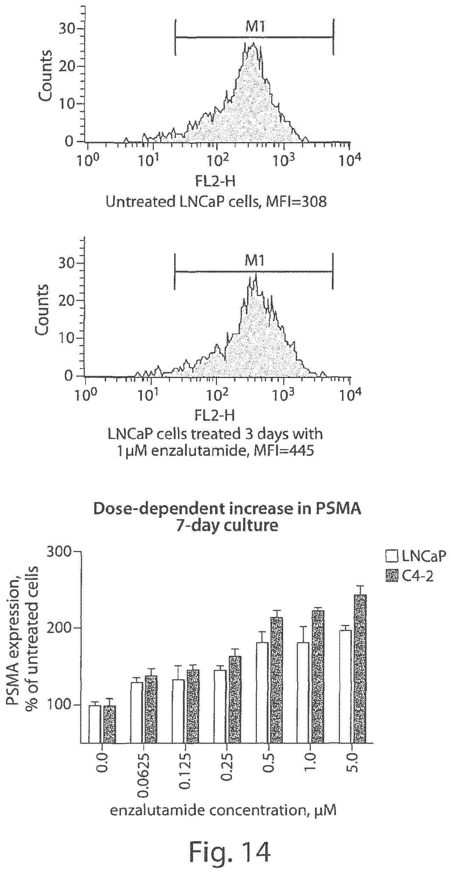

**FIG. 14** demonstrates the rapid, significant and dose-dependent increases in PSMA expression with enzalutamide. The effects were exhibited in a clinically meaningful dose range and were seen for androgen-dependent (LNCaP) and androgen-independent (C4-2) cells.

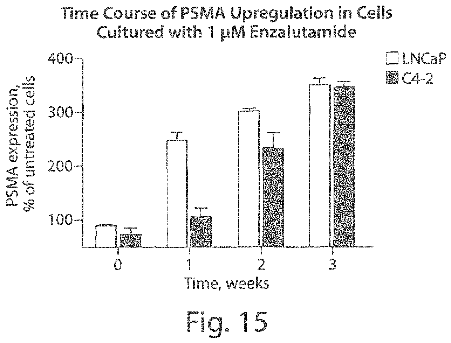

**FIG. 15** also demonstrates the increased expression of PSMA with enzalutamide. PSMA expression was maximal after 3 to 4 weeks of exposure but returned to basal levels one week after removal of enzalutamide.

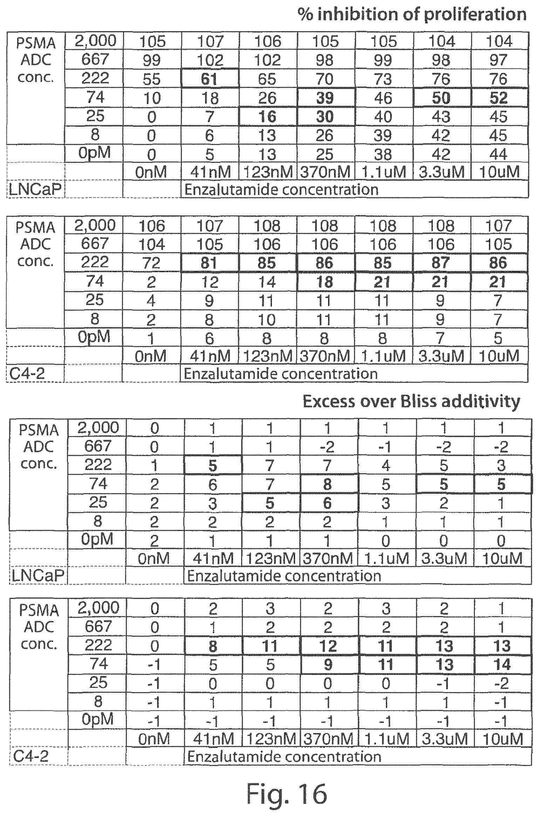

**FIG. 16** shows the synergistic anticancer activity of enzalutamide and PSMA ADC *in vitro.* Results are the average of four repetitions for each condition. Values in bold indicate statistically significant synergy.

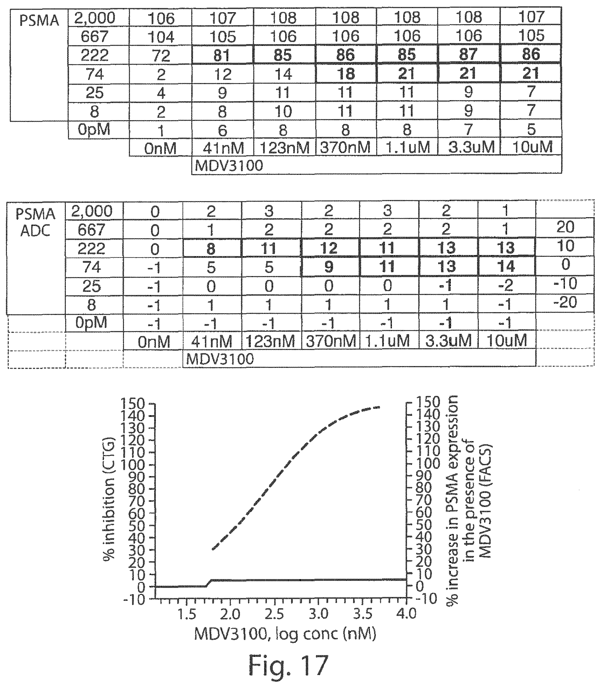

**FIG. 17** shows that PSMA ADC synergizes with enzalutamide *in vitro.* The synergy was shown in both androgen-dependent and androgen-independent cells and was associated with the increased expression of PSMA. Surprisingly, the synergy was observed even when enzalutamide alone had minimal antitumor activity.

### DETAILED DESCRIPTION OF THE INVENTION

The invention relates, at least in part, to combination treatments for PSMA-expressing cancer, such as prostate cancer and, in particular, progressive, metastatic prostate cancer. The treatments can include administration of at least one agent that targets prostate specific membrane antigen (PSMA) in combination with an agent that targets androgen receptors (ARs). The invention is based, at least in part, on the surprising discovery that antiandrogens (*e.g*., enzalutamide or abiraterone) significantly and reversibly augment PSMA expression and potentiate the activity of PSMA ligand conjugates. In androgen-independent cells, the effects on PSMA expression and conjugate activity were synergistic and uncoupled from any antiproliferative effect of the antiandrogens. Synergistic inhibition of tumor cell growth was associated with an upregulation of PSMA expression by antiandrogens, even in cells that were refractory to treatment with antiandrogens alone. Co-treatment, in effect, can re-sensitize the cells to antiandrogen therapy.

Both enzalutamide and abiraterone potently synergized with PSMA ligand conjugates over a range of concentrations. Synergy was observed on cells that were both responsive and unresponsive to treatment with antiandrogens alone. In androgen-dependent LNCaP cells, the pharmacologic effects of antiandrogens (*e.g*., antiproliferative effects, effects on gene expression, and synergy with PSMA ligand conjugates) were observed over a similar range of clinically relevant concentrations. In androgen-independent C4-2 cells, effects on gene expression and synergy were uncoupled from any appreciable antiproliferative activity of either enzalutamide or abiraterone. Thus, these antiandrogens remain pharmacologically active for C4-2 cells; however, the cells have adopted compensatory survival mechanisms that allow them to proliferate in the presence of ongoing androgen receptor (AR) blockade.

Little to no synergy was observed between the antiandrogens and the components of the conjugates (*i.e.*, free MMAE and unmodified PSMA mAb). Free MMAE showed additive to weakly synergistic effects when combined with the antiandrogens. There was modest synergy between the antiandrogens and docetaxel. The unmodified mAb showed no antiproliferative activity either alone or in combination with antiandrogens. Therefore, the strong synergy observed with PSMA ligand conjugates is specific to the conjugates and is neither a pass-through effect of its components nor generalizable to microtubule inhibitors as a class.

PSMA expression was doubled after 7 days treatment with enzalutamide, and 4-fold higher after 21 days. The magnitude of PSMA upregulation by enzalutamide or abiraterone approached that induced by charcoal-stripped serum, which is depleted in a range of hormones, cytokines, and growth factors (40). The findings suggest near-maximal androgen suppression in our system. The time course of expression was monitored in cells treated with enzalutamide. PSMA expression increased with continued treatment over three weeks and then rapidly returned to baseline upon removal of enzalutamide. The findings have implications for combining and sequencing potent antiandrogens and PSMA-targeted therapies in the clinic.

In addition, PSMA ligand conjugates synergized with a PI3K/mTOR pathway inhibitor via a multimodal mechanism involving increased PSMA expression and disruption of microtubule function. In C4-2 cells, rapamycin exhibited minimal single-agent activity but potentiated the activity of PSMA ligand conjugates, suggesting that PI3K pathway activation is an adaptive response to ADC treatment in C4-2 cells. Thus, the invention is also based, in part, on the surprising discovery that, in some instances mTOR inhibitor, such as rapamycin, activity synergized with the activity of PSMA ligand conjugates.

As used herein, a "PSMA ligand conjugate" comprises a molecule that binds specifically PSMA, such as an extracellular domain of PSMA, and is conjugated to a therapeutic agent. The therapeutic agent may be an anticancer agent or a cytotoxic agent. A "PSMA ligand," therefore, herein refers to a molecule that specifically binds PSMA, as described herein. When a PSMA ligand is conjugated to an anticancer agent, the PSMA ligand conjugate is also referred to herein as a "PSMA ligand-anticancer agent conjugate". When a PSMA ligand is conjugated to a cytotoxic agent, the PSMA ligand conjugate is also referred to herein as a "PSMA ligand-cytotoxic agent conjugate."

As used herein, "PSMA-expressing cells" refers to cells that express PSMA or that can express PSMA (*e.g*., human PSMA). PSMA is a 100 kD Type II membrane glycoprotein expressed in prostate tissues (Horoszewicz et al., 1987, Anticancer Res. 7:927-935; U.S. Pat. No. 5,162,504). PSMA was characterized as a type II transmembrane protein having sequence homology with the transferrin receptor (Israeli et al., 1994, Cancer Res. 54:1807-1811) and with NAALADase activity (Carter et al., 1996, Proc. Natl. Acad. Sci. U.S.A. 93:749-753). PSMA is expressed in increased amounts in prostate cancer (Horoszewicz et al., 1987, Anticancer Res. 7:927-935; ; Rochon et al., 1994, Prostate 25:219-223; Murphy et al., 1995, Prostate 26:164-168; and Murphy et al., 1995, Anticancer Res. 15:1473-1479). PSMA expression in cancerous prostate is approximately 10-fold greater than that in normal prostate. Expression in normal prostate is approximately 10-fold greater than that in the brain and is 50-to 100-fold greater than that of the liver or kidney. In most normal tissues, no expression of PSMA is observed.

PSMA expression increases with disease progression, becoming highest in metastatic, hormone-refractory disease. In addition, PSMA is also abundantly expressed on the neovasculature of a variety of non-prostate tumors, including bladder, breast, colon, pancreas, sarcoma, melanoma, renal, liver, lung (*e.g*., non-small cell lung carcinoma), and kidney tumors, but not on normal vasculature. "PSMA-expressing cells," therefore, include PSMA-expressing cancer cells such as prostate cancer cells as well as PSMA-expressing cells (*e.g.*, endothelial cells) of the neovasculature of a number of non-prostate cancers or non-prostate tumors.

As used herein, an "androgen-dependent PSMA-expressing cell" refers to a PSMA-expressing cell, such as a cancer cell, that is responsive to antiandrogens. A cell is herein considered to be responsive to an antiandrogen if proliferation of the cell can be substantially inhibited by the antiandrogen.

As used herein, an "androgen-independent PSMA-expressing cell" refers to a PSMA-expressing cell, such as a cancer cell, that is non-responsive to antiandrogens, also referred to herein as a "PSMA-expressing cell insensitive to antiandrogen therapy." A cell is herein considered to be non-responsive to an antiandrogen if proliferation of the cell is not substantially inhibited by the antiandrogen.

In some embodiments, PSMA-expressing cells insensitive to antiandrogen therapy are sensitized to antiandrogen therapy after the step of contacting the PSMA-expressing cells with a compound that increases cell surface expression of PSMA (*e.g*., antiandrogen or mTOR inhibitor). Such cells can then be further contacted with a PSMA ligand conjugate as provided herein, and such contact can occur concurrently or sequentially. Thus, in some embodiments, androgen-independent PSMA-expressing cells (*e.g.,* cancer cells) that are otherwise non-responsive to antiandrogens may become responsive, and, therefore, sensitized to antiandrogens when contacted with an antiandrogen, or an mTOR inhibitor, and can be further contacted with a PSMA ligand conjugate to effect cell proliferation inhibition or cell killing. Proliferation of such "sensitized" PSMA-expressing cancer cells may be substantially inhibited as a result. Sensitization of androgen-independent PSMA-expressing cells to antiandrogens may occur, in some embodiments, within 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days or more of being contacted with a compound that increases cell surface expression of PSMA. In some embodiments, sensitization of androgen-independent PSMA-expressing cells to antiandrogens may occur in less than 2 days.

In some embodiments, PSMA-expressing cells are contacted with a compound that increases cell surface expression of PSMA for a period of time sufficient to increase (*e.g.*, upregulate) expression of PSMA on the surface of the cells. A time sufficient to upregulate cell surface expression of PSMA can be determined by myriad protein expression assays, which are known in the art, including for example enzyme-linked immunosorbent assays (ELISAs) and Western blotting. In some embodiments, a time sufficient to upregulate cell surface expression of PSMA may be 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days or more. In some embodiments, a time sufficient to upregulate cell surface expression of PSMA may be less than 1 day.

Examples of PSMA ligands for use as provided herein include, without limitation, antibodies or antigen binding fragments thereof as well as small molecule ligands that bind specifically PSMA and may act as substrate mimicks of enzymatic sites on PSMA. Antibodies that bind specifically to PSMA may be referred to herein as "PSMA antibodies." Likewise, small molecule ligands that bind specifically PSMA may be referred to herein as "PSMA small molecule ligands."

As used herein, "specific binding" refers to molecule (*e.g*., antibody) binding to a predetermined target (*e.g.,* antigen), in this case PSMA (*e.g.,* human PSMA). In some embodiments, that sequence of PSMA is set forth as SEQ ID NO: 1. Typically, the molecule binds with an affinity that is at least two-fold greater than its affinity for binding to a nonspecific target (*e.g.,* BSA, casein), which is a target other than PSMA, an isoform or variant of PSMA, or a closely-related target.

An antibody or an antigen-binding fragment thereof of a PSMA ligand conjugate may be any antibody or antigen-binding fragment thereof that binds PSMA (*e.g*., binds specifically to an epitope of PSMA). Examples of PSMA antibodies for use as provided herein include, without limitation, those listed in **Table 1.** Antigen-binding fragments of these antibodies are also examples of antigen-binding fragments for use in the methods and compositions as provided herein.

In some embodiments, the antibody is produced by hybridomas referred to herein. These hybridomas were deposited pursuant to, and in satisfaction of, the requirements of the Budapest Treaty on the International Recognition of the Deposit of Microorganisms for the Purposes of Patent Procedure with the American Type Culture Collection ("ATCC"), having the address 10801 University Boulevard, Manassas, Va. 20110-2209, as an International Depository Authority and given the Patent Deposit Designations **(Table 1**):

**Table 1**

| Antibody | Hybridoma/Plasmid | Patent Deposit Designation | Date of Deposit |
|---|---|---|---|
| PSMA 3.7 | PSMA 3.7 | PTA-3257 | April 5, 2001 |
| PSMA 3.9 | PSMA 3.9 | PTA-3258 | April 5, 2001 |
| PSMA 3.11 | PSMA 3.11 | PTA-3269 | April 10, 2001 |
| PSMA 5.4 | PSMA 5.4 | PTA-3268 | April 10, 2001 |
| PSMA 7.1 | PSMA 7.1 | PTA-3292 | April 18, 2001 |
| PSMA 7.3 | PSMA 7.3 | PTA-3293 | April 18, 2001 |
| PSMA 10.3 | PSMA 10.3 | PTA-3347 | May 1, 2001 |
| | PSMA 10.3 HC in pcDNA (SEQ ID NO: 7) | PTA-4413 | May 29, 2002 |
| | PSMA 10.3 Kappa in pcDNA (SEQ ID NO: 13) | PTA-4414 | May 29, 2002 |
| PSMA 1.8.3 | PSMA 1.8.3 | PTA-3906 | Dec. 5, 2001 |
| PSMA A3.1.3 | PSMA A3.1.3 | PTA-3904 | Dec. 5, 2001 |
| PSMA A3.3.1 | PSMA A3.3.1 | PTA-3905 | Dec. 5, 2001 |
| Abgenix 4.248.2 | Abgenix 4.248.2 | PTA-4427 | June 4, 2002 |
| Abgenix 4.360.3 | Abgenix 4.360.3 | PTA-4428 | June 4, 2002 |
| Abgenix 4.7.1 | Abgenix 4.7.1 | PTA-4429 | June 4, 2002 |
| Abgenix 4.4.1 | Abgenix 4.4.1 | PTA-4556 | July 18, 2002 |
| Abgenix 4.177.3 | Abgenix 4.177.3 | PTA-4557 | July 18, 2002 |
| Abgenix 4.16.1 | Abgenix 4.16.1 | PTA-4357 | May 16, 2002 |
| Abgenix 4.22.3 | Abgenix 4.22.3 | PTA-4358 | May 16, 2002 |
| Abgenix 4.28.3 | Abgenix 4.28.3 | PTA-4359 | May 16, 2002 |
| Abgenix 4.40.2 | Abgenix 4.40.2 | PTA-4360 | May 16, 2002 |
| Abgenix 4.48.3 | Abgenix 4.48.3 | PTA-4361 | May 16, 2002 |
| Abgenix 4.49.1 | Abgenix 4.49.1 | PTA-4362 | May 16, 2002 |
| Abgenix 4.209.3 | Abgenix 4.209.3 | PTA-4365 | May 16, 2002 |
| Abgenix 4.219.3 | Abgenix 4.219.3 | PTA-4366 | May 16, 2002 |
| Abgenix 4.288.1 | Abgenix 4.288.1 | PTA-4367 | May 16, 2002 |
| Abgenix 4.333.1 | Abgenix 4.333.1 | PTA-4368 | May 16, 2002 |
| Abgenix 4.54.1 | Abgenix 4.54.1 | PTA-4363 | May 16, 2002 |
| Abgenix 4.153.1 | Abgenix 4.153.1 | PTA-4388 | May 23, 2002 |
| Abgenix 4.232.3 | Abgenix 4.232.3 | PTA-4389 | May 23, 2002 |
| Abgenix 4.292.3 | Abgenix 4.292.3 | PTA-4390 | May 23, 2002 |
| Abgenix 4.304.1 | Abgenix 4.304.1 | PTA-4391 | May 23, 2002 |
| AB-PG1-XG1-006 | AB-PG1-XG1-006 Heavy Chain (SEQ ID NO: 2) | PTA-4403 | May 29, 2002 |
| | AB-PG1-XG1-006 Light Chain (SEQ ID NO: 8) | PTA-4404 | |
| AB-PG1-XG1-026 | AB-PG1-XG1-026 Heavy Chain (SEQ ID NO: 3) | PTA-4405 | May 29, 2002 |
| | AB-PG1-XG1-026 Light Chain (SEQ ID NO: 9) | PTA-4406 | |
| AB-PG1-XG1-051 | AB-PG1-XG1-051 Heavy Chain (SEQ ID NO: 4) | PTA-4407 | May 29, 2002 |
| | AB-PG1-XG1-051 Light Chain (SEQ ID NO: 10) | PTA-4408 | |
| AB-PG1-XG1-069 | AB-PG1-XG1-069 Heavy Chain (SEQ ID NO: 5) | PTA-4409 | May 29, 2002 |
| | AB-PG1-XG1-069 Light Chain (SEQ ID NO: 11) | PTA-4410 | |
| AB-PG1-XG1-077 | AB-PG1-XG1-077 Heavy Chain (SEQ ID NO: 6) | PTA-4411 | May 29, 2002 |
| | AB-PG1-XG1-077 Light Chain (SEQ ID NO: 12) | PTA-4412 | |

An antibody or an antigen-binding fragment thereof of a PSMA ligand conjugate, in some embodiments, may comprise: (i) any one of the heavy chains provided herein and (ii) any one of the light chains provided herein. In some embodiments, a PSMA antibody as provided herein may include (i) any one of the heavy chain variable regions provided herein and (ii) any one of the light chain variable regions provided herein. In some embodiments, PSMA antibodies include (i) the three complementarity determining regions of any one of the heavy chain variable regions provided herein and (ii) the three complementarity determining regions of any one of the light chain variable regions provided herein.

Plasmids encoding exemplary heavy and light chains of antibodies were also deposited with the ATCC and are shown in **Table 1** above.

Also provided are antigen-binding fragments of any one of the foregoing antibodies for use as the PSMA ligands of the PSMA ligand conjugates provided herein.

As used herein, the names of the deposited hybridomas or plasmids may be used interchangeably with the names of the antibodies. It would be clear to one of skill in the art when the name is intended to refer to the antibody or when it refers to the plasmids or hybridomas that encode or produce the antibodies, respectively. Additionally, antibody names may be an abbreviated form of the name shown in **Table 1.** For instance, antibody AB-PG1-XG1-006 may be referred to as "AB-PG1-XG1-006," "PG1-XG1-006," " XG1-006," or "006." In another example, the antibody PSMA 4.232.3 may be referred to as "PSMA 4.232.3," " 4.232.3," "4.232.3," or "4.232." It is intended that all of the variations in the name of the antibody refer to the same antibody and not a different one.

As used herein, a "coding region" refers to a region of a nucleotide sequence that encodes a polypeptide sequence; the coding region can include a region coding for a portion of a protein that is later cleaved off, such as a signal peptide.

Those of skill in the art will appreciate that nucleic acids and polypeptides as provided herein include nucleotide sequences and amino acid sequences as provided herein. In some instances, the nucleotide sequences and amino acid sequences may include sequences that encode, or that are, signal peptides. Each of these sequences with, or without, the portion of the sequence that encodes or is a signal peptide is contemplated herein.

In some embodiments, a PSMA antibody is encoded by a nucleic acid molecule or amino acid molecule that is highly homologous to the nucleic acid molecules or amino acid molecules provided herein, respectively. For example, homologous nucleic acid molecule or amino acid molecule may comprise a nucleotide sequence or amino acid sequence that is at least about 90% identical to a nucleotide sequence or amino acid sequence provided herein. As another example, a nucleotide sequence or amino acid sequence is at least about 95% identical, at least about 97% identical, at least about 98% identical, or at least about 99% identical to a nucleotide sequence or amino acid sequence as provided herein. Homology can be calculated using various, publicly available software, which are well known to one of ordinary skill in the art. Exemplary tools include the BLAST system available from the website of the National Center for Biotechnology Information (NCBI) at the National Institutes of Health.

In some embodiments, PSMA antibodies include the antibodies provided in U.S. Patents 6,107,090, 6,649,163 and 6,962,981. Such antibodies are incorporated herein by reference. PSMA antibodies, therefore, include E99, J415, J533, and J591 monoclonal antibodies; monoclonal antibodies produced by hybridomas having ATCC Accession Numbers HB-12101, HB-12109, HB-12127 and HB-12126; and monoclonal antibodies produced by hybidomas having ATCC Accession Numbers HB12060 (3F5.4G6), HB12309 (3D7-1.1), HB12310 (4E10-1.14), HB12489 (1G3), HB12495 (1G9), HB12490 (2C7), HB12494 (3C4), HB12491 (3C6), HB12484 (3C9), HB12486 (3E6), HB12488 (3E11), HB12485 (3G6), HB12493 (4D4), HB12487 (4D8), HB12492 (4C8B9), HB12664 (3F6), HB12678 (2E4), HB12665 (3C2), HB12672 (2D4), HB12660 (4C8G8), HB12675 (2C4), HB12663 (4C11), HB12661 (1D11), HB12667 (4E8), HB12674 (2G5), HB12620 (4E6), HB12677 (1F4), HB12666 (2E3), HB12662 (3D8), HB12668 (4F8), HB12673 (3D2), HB12676 (1G7), HB12669 (3D4), HB12679 (5G10), and HB12671 (5E9). Antigen-binding fragments of these antibodies are also contemplated for use as PSMA ligands of the PSMA ligand conjugates provided herein.

Also provided herein are nucleic acid molecules encoding PSMA antibodies and antigen-binding fragments thereof and vectors comprising the nucleic acid molecules as described herein. The vectors provided can be used to transform or transfect host cells for producing PSMA antibodies and antigen-binding fragments thereof with the specificity described herein.

As used herein, "antibody" refers to a glycoprotein comprising at least two heavy (H) chains and two light (L) chains inter-connected by disulfide bonds. Each heavy chain is comprised of a heavy chain variable region (abbreviated herein as HCVR or V_{H}) and a heavy chain constant region. The heavy chain constant region is comprised of three domains, C_{H}1, C_{H}2 and C_{H}3. Each light chain is comprised of a light chain variable region (abbreviated herein as LCVR or V_{L}) and a light chain constant region. The light chain constant region is comprised of one domain, CL. The V_{H} and V_{L} regions can be further subdivided into regions of hypervariability, termed complementarity determining regions (CDRs), interspersed with regions that are more conserved, termed framework regions (FRs). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

As used herein, "antigen-binding fragment" of an antibody refers to one or more portions of an antibody that retain the ability to bind specifically to an antigen (*e.g*., PSMA). The antigen-binding function of an antibody can be performed by fragments of a full-length antibody. Examples of binding fragments encompassed within the term "antigen-binding fragment" of an antibody include (i) a Fab fragment, a monovalent fragment consisting of the V_{L}, V_{H}, C_{L} and C_{H}1 domains; (ii) a F(ab')₂ fragment, a bivalent fragment comprising two Fab fragments linked by a disulfide bridge at the hinge region; (iii) a Fd fragment consisting of the V_{H} and CH1 domains; (iv) a Fv fragment consisting of the V_{L} and V_{H} domains of a single arm of an antibody, (v) a dAb fragment (Ward et al., (1989) Nature 341:544-546), which consists of a V_{H} domain; and (vi) an isolated complementarity determining region (CDR). Furthermore, although the two domains of the Fv fragment, V and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody.

As used herein, "isolated antibody" refers to an antibody that is substantially free of other antibodies having different antigenic specificities (*e.g*., an isolated antibody that specifically binds to PSMA is substantially free of antibodies that specifically bind antigens other than PSMA). An isolated antibody that specifically binds to an epitope, isoform or variant of PSMA may, however, in some embodiments, have cross-reactivity to other related antigens, *e.g.,* from other species (*e.g.,* PSMA species homologs). Moreover, an isolated antibody may, in some embodiments, be substantially free of other cellular material and/or chemicals.

Isolated antibodies of the invention encompass various antibody isotypes, such as IgG1, IgG2, IgG3, IgG4, IgM, IgA1, IgA2, IgAsec, IgD, IgE. As used herein, "isotype" refers to the antibody class (*e.g.,* IgM or IgG1) that is encoded by heavy chain constant region genes. Antibodies can be full length or can include only an antigen-binding fragment such as the antibody constant and/or variable domain of IgG1, IgG2, IgG3, IgG4, IgM, IgA1, IgA2, IgAsec, IgD or IgE or could consist of a Fab fragment, a F(ab')₂ fragment, and a Fv fragment.

Antibodies, in some embodiments, may be polyclonal, monoclonal, or a mixture of polyclonal and monoclonal antibodies. Antibodies can be produced by a variety of techniques well known in the art. Procedures for raising polyclonal antibodies are well known. Monoclonal antibody production may be effected by techniques that are also well known in the art.

As used herein, "monoclonal antibody" refers to a preparation of antibody molecules of single molecular composition. A monoclonal antibody displays a single binding specificity and affinity for a particular epitope.

In some embodiments, recombinant antibodies are contemplated for use as provided herein. As used herein, a "recombinant antibody" refers to an antibody that is prepared, expressed, created or isolated by recombinant means, such as an antibody isolated from an animal (*e.g.,* a mouse) that is transgenic for another species' immunoglobulin genes, an antibody expressed using a recombinant expression vector transfected into a host cell, an antibody isolated from a recombinant, combinatorial antibody library, or an antibody prepared, expressed, created or isolated by any other means that involves splicing of immunoglobulin gene sequences to other DNA sequences.

In yet other embodiments, an antibody may be a chimeric or humanized antibody. As used herein, a "chimeric antibody" refers to an antibody that combines the murine variable or hypervariable regions with the human constant region or constant and variable framework regions. As used herein, "humanized antibody" refers to an antibody that retains only the antigen-binding CDRs from the parent antibody in association with human framework regions (*see,* Waldmann, 1991, Science 252:1657). Such chimeric or humanized antibodies retaining binding specificity of the murine antibody are expected to have reduced immunogenicity when administered *in vivo* for diagnostic, prophylactic or therapeutic applications as provided herein.

In still other embodiments, monoclonal antibodies provided here may be modified to be in the form of a bispecific antibody or a multispecific antibody. As used herein, a "bispecific antibody" includes any agent, *e.g.,* a protein, peptide, or protein or peptide complex, that has two different binding specificities which bind to, or interact with (a) a cell surface antigen and (b) an Fc receptor on the surface of an effector cell. As used herein, a "multispecific antibody" includes any agent, *e.g.,* a protein, peptide, or protein or peptide complex, that has more than two different binding specificities that bind to, or interact with, (a) a cell surface antigen, (b) an Fc receptor on the surface of an effector cell, and (c) at least one other component. Accordingly, antibodies provided herein, in some embodiments, may be bispecific, trispecific, tetraspecific, or other multispecific antibodies directed to cell surface antigens, such as PSMA, and to Fc receptors on effector cells. "Bispecific antibodies" also includes diabodies. Diabodies are bivalent, bispecific antibodies in which the V_{H} and V_{L} domains are expressed on a single polypeptide chain and use a linker that is too short to allow for pairing between the two domains on the same chain, thereby forcing the domains to pair with complementary domains of another chain and creating two antigen-binding sites (*see, e.g.,* Holliger, P., et al. (1993) Proc. Natl. Acad. Sci. USA 90:6444-6448; Poijak, R. J., et al. (1994) Structure 2:1121-1123).

A bispecific antibody can be formed of an antigen-binding region specific for the extracellular domain of PSMA and an antigen-binding region specific for an effector cell that has tumoricidal or tumor inhibitory activity. The two antigen-binding regions of the bispecific antibody are either chemically linked or can be expressed by a cell genetically engineered to produce the bispecific antibody. (*See* generally, Fanger et al., 1995 Drug News & Perspec. 8(3): 133-137). Suitable effector cells having tumoricidal activity include, without limitation, cytotoxic T-cells (primarily CD8⁺ cells), and natural killer cells.

In some embodiments, antibodies provided herein are human antibodies. As used herein, "human antibodies" include antibodies having variable and constant regions derived from human germline immunoglobulin sequences. Human antibodies of the invention may include amino acid residues not encoded by human germline immunoglobulin sequences (*e.g*., mutations introduced by random or site-specific mutagenesis *in vitro* or by somatic mutation *in vivo*). The term "human antibodies", as used herein, is not intended to include antibodies in which CDR sequences derived from the germline of another mammalian species, such as a mouse have been grafted onto human framework sequences (otherwise referred to herein as "humanized antibodies"). Human antibodies directed against PSMA can be generated using transgenic mice carrying parts of the human immune system rather than the mouse system. Human PSMA antibodies provided herein specifically bind cell-surface PSMA and/or rsPSMA (recombinant soluble PSMA, *i.e.,* with the sequence of the extracellular portion of PSMA) with sub-nanomolar affinity. Human PSMA antibodies provided herein may have binding affinities of about 1×10⁻⁹ M or less, about 1×10⁻¹⁰ M or less, or 1×10⁻¹¹ M or less. In some embodiments, the binding affinity of human PSMA antibodies as provided herein is less than about 5×10⁻¹⁰ M.

Antibodies or antigen-binding fragments thereof can be selected, for example, based on the following criteria, which are not intended to be exclusive:
∘ 1) binding to live cells expressing PSMA;
∘ 2) high affinity of binding to PSMA;
∘ 3) binding to a unique epitope on PSMA (to eliminate the possibility that antibodies with complimentary activities when used in combination would compete for binding to the same epitope);
∘ 4) opsonization of cells expressing PSMA;
∘ 5) mediation of growth inhibition, phagocytosis and/or killing of cells expressing PSMA in the presence of effector cells;
∘ 6) modulation (inhibition or enhancement) of NAALADase, folate hydrolase, dipeptidyl peptidase IV and/or γ-glutamyl hydrolase activities;
∘ 7) growth inhibition, cell cycle arrest and/or cytotoxicity in the absence of effector cells;
∘ 8) internalization of PSMA;
∘ 9) binding to a conformational epitope on PSMA;
∘ 10) minimal cross-reactivity with cells or tissues that do not express PSMA; and
∘ 11) preferential binding to dimeric forms of PSMA rather than monomeric forms of PSMA.

PSMA antibodies and antigen-binding fragments thereof provided herein typically meet one or more, and in some instances, all of the foregoing criteria.

In some embodiments, an isolated antibody or antigen-binding fragment thereof may be selected for its ability to bind live cells expressing PSMA. In order to demonstrate binding of monoclonal antibodies to live cells expressing the PSMA, flow cytometry can be used. Binding of the antibody or antigen-binding fragment thereof to live cells expressing PSMA can inhibit the growth of the cells or mediate cytolysis of the cells. Cytolysis can be complement mediated or can be mediated by effector cells. In some embodiments, the cytolysis is carried out in a living organism, preferably a mammal, and the live cell is a tumor cell. Examples of tumors that can be targeted by PSMA antibodies (*e.g*., PSMA antibody conjugates) provided herein, include any tumor that expresses PSMA such as, *e.g*., prostate, bladder, pancreas, lung, colon, kidney, melanomas and sarcomas. A tumor that expresses PSMA includes tumors with neovasculature expressing PSMA.

In some embodiments, a PSMA antibody, or antigen-binding fragment thereof, binds to and is internalized with PSMA expressed on cells. Thus, a PSMA ligand conjugate comprising a PSMA antibody may be internalized with PSMA expressed on cells. The mechanism by which this internalization occurs is not critical to the practice of the present invention. For example, the antibody or antigen-binding fragment thereof can induce internalization of PSMA.

In some embodiments, a PSMA antibody, or antigen-binding fragment thereof, binds to a conformational epitope within the extracellular domain of the PSMA molecule. Antibodies that bind to native protein but not denatured protein are those antibodies that bind conformational epitopes, and are preferred antibodies in some embodiments.

In other embodiments, a PSMA antibody, or antigen-binding fragment thereof, binds to a dimer-specific epitope on PSMA. Generally, antibodies or antigen-binding fragments thereof that bind to a dimer-specific epitope preferentially bind the PSMA dimer rather than the PSMA monomer. Antibodies that bind to the PSMA dimer but not to the monomeric PSMA protein are preferred antibodies in some embodiments.

Other PSMA antibodies, or antigen-binding fragments thereof, provided herein include antibodies that bind specifically to an epitope on PSMA defined by a second antibody. To determine the epitope, one can use standard epitope mapping methods known in the art.

In some embodiments, the PSMA antibody of a PSMA ligand conjugate is a monoclonal antibody that binds prostate specific membrane antigen (PSMA) protein dimer, PSMA protein dimer being a homodimer of PSMA protein monomer having the sequence of SEQ ID NO: 1, or an antigen-binding fragment thereof, wherein the antibody, or the antigen-binding fragment, (i) binds live cells and (ii) binds with at least a two-fold greater affinity to PSMA protein dimer than to PSMA protein monomer, as described in U.S. Patent No. 8,114,965. The antibodies and antigen-binding fragments provided in U. S. Patent No. 8,114,965 are specifically incorporated by reference herein, any one of which antibodies or antigen-binding fragments thereof may be the PSMA antibody or antigen-binding fragment of a PSMA ligand conjugate as provide herein.

In some embodiments, PSMA antibodies are conjugated to radioactive molecules. An example of such a PSMA ligand conjugate, thus, includes ¹⁷⁷Lu-J591, which contains monoclonal PSMA antibody J591 conjugated through 1,4,7,10-tetraazacyclododecane-1,4,7,10-tetraacetic acid (DOTA) to ¹⁷⁷Lutetium (¹⁷⁷Lu).

The PSMA ligand of a PSMA ligand conjugate may be any small molecule ligand that binds specifically PSMA. Such small molecule ligands may bind to the enzymatic site of PSMA in its native conformation. Also, such small molecule ligands may possess any one or more of the characteristics described above for PSMA antibody ligands.

In some embodiments, the small molecule ligand is based on a glutamate-urea-lysine heterodimer (*e.g*., glutamate-urea-lysine analog), or a glutamate-urea-glutamate based dimer, that binds specifically to an enzymatic site on PSMA. In some embodiments, such small molecule ligands are conjugated to a radionuclide as the anticancer, or cytotoxic, agent (*e.g.,* cytotoxic radionuclide or radiotherapeutic isotope). Examples of PSMA ligand conjugates, thus, include glutamate-urea-amino acid based small molecule ligands conjugated to a radionuclide through an intervening linker such as ¹²³I-MIP-I095 (also referred to as ¹²³I-MIP-1466) and ¹²³I-MIP-1072 (Molecular Insight Pharmaceuticals, Inc.). Other examples of PSMA small molecule ligands and PSMA ligand conjugates can be found in U.S. Patent 8,465,725 and U.S. 8,487,129 and are incorporated herein by reference. In some embodiments, I¹²³ may be substituted with other radiohalogens including those selected from the group consisting of I¹²⁵, I¹³¹, I¹²⁴, BR⁷⁵, BR⁷⁷ and F¹⁸.

The chemical structure of ¹²³I-MIP-1095 (*i.e.,* ¹²³I-(S)-2-(3-((S)-1-carboxy-5-(3-(4-iodophenyl)ureido)pentyl)ureido)pentanedioic acid) is :

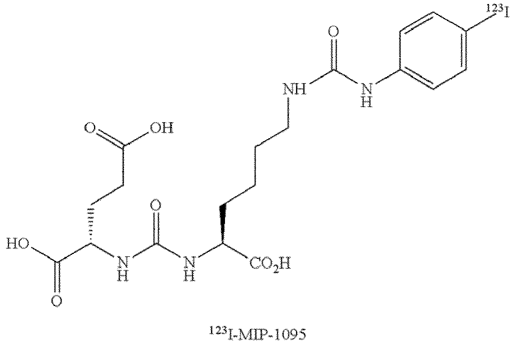

In another embodiment, the PSMA ligand conjugate is ¹²⁴I-MIP-1095. In another embodiment, the PSMA ligand conjugate is ¹³¹I-MIP-1095.

The chemical structure of ¹²³I-MIP-1072 (*i.e.,* ¹²³I-(S)-2-(3-((S)-1-carboxy-5-(4-iodobenzylamino)pentyl)ureido)pentanedioic acid) is:

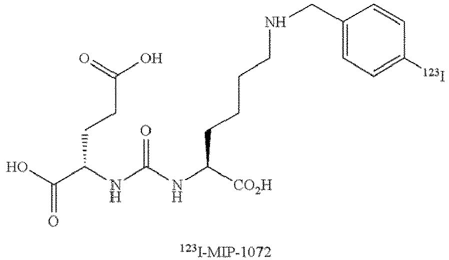

In some embodiments, the small molecule ligand of a PSMA ligand conjugate is a GL2 molecule as described in International Publication No. WO2010/005723. Any of the small molecules ligands provided herein, including a GL2 molecule, may be conjugated to a therapeutic agent by way of a nanoparticle (*e.g*., polymer-based, lipid-based and/or nucleic acid-based nanoparticles). In such embodiments, the nanoparticle may contain the therapeutic agent. Thus, in some embodiments, the PSMA ligand conjugate comprises a small molecule ligand conjugated to a nanoparticle that contains an anticancer or cytotoxic agent. Examples of such PSMA ligand conjugates include, without limitation, BIND-014 (Bind Biosciences, Inc.) described in International Publication No. WO2010/005723. The PSMA ligands and PSMA ligand conjugates of which are incorporated by reference herein.

PSMA small molecule ligands, in some embodiments, may be selected from the group consisting of compounds I, II, III and IV: 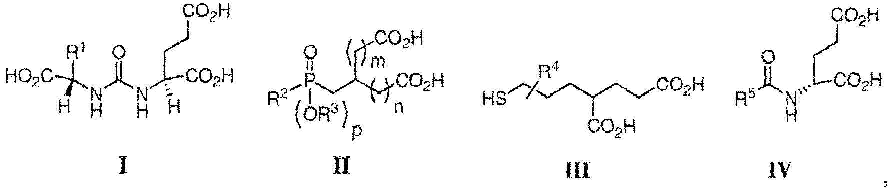 and enantiomers, stereoisomers, rotamers, tautomers, diastereomers, or racemates thereof; wherein m and n are each, independently, 0, 1, 2 or 3; p is 0 or 1; R1, R2, R4 and R5 are each, independently, selected from the group consisting of substituted or unsubstituted alkyl, substituted or unsubstituted aryl, and any combination thereof; and R3 is H or CH3; wherein R1, R2, R4 or R5 comprise a point of covalent attachment to the nanoparticle. For example, R1, R2, R4 and R5 may be each, independently, Ci₁₋₆-alkyl or phenyl, or any combination of Ci₁₋₆-alkyl or phenyl, which are independently substituted one or more times with OH, SH, NH2, or CO2H, and wherein the alkyl group may be interrupted by N(H), S or O. In some embodiments, for example, R1, R2, R4 and R5 are each, independently, CH₂-Ph, (CH₂)₂-SH, CH₂-SH, (CH₂)₂C(H)(NH₂)CO₂H, CH₂C(H)(NH₂)CO₂H, CH(NH₂)CH₂CO₂H, (CH₂)₂C(H)(SH)CO₂H, CH₂-N(H)-Ph, O-CH₂-Ph, or O-(CH₂)₂-Ph, wherein each Ph may be independently substituted one or more times with OH, NH₂, CO₂H or SH.

PSMA small molecule ligands, in other embodiments, may be selected from the group consisting of: 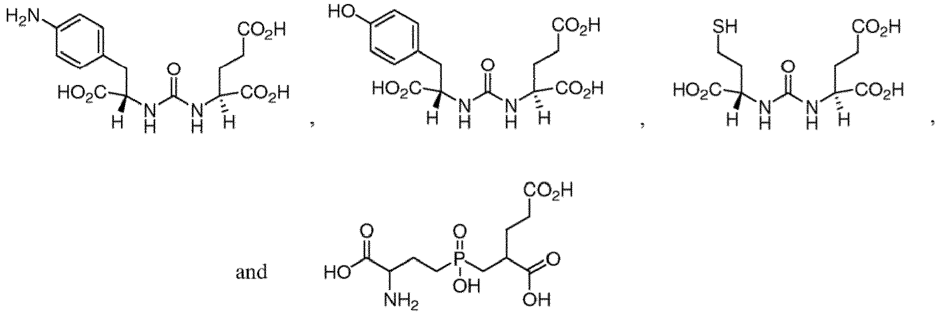 and enantiomers, stereoisomers, rotamers, tautomers, diastereomers, or racemates thereof; and wherein the NH₂, OH or SH groups serve as a point of covalent attachment, or may be selected from the group consisting of 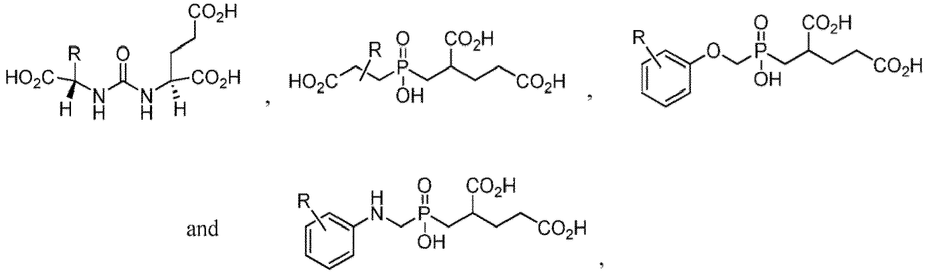 and enantiomers, stereoisomers, rotamers, tautomers, diastereomers, or racemates thereof; wherein R is independently selected from the group consisting of NH₂, SH, OH, CO₂H, Ci_6-alkyl that is substituted with NH₂, SH, OH or CO₂H, and phenyl that is substituted with NH₂, SH, OH or CO₂H, and wherein R serves as the point of covalent attachment.

PSMA small molecule ligands, in yet other embodiments, may be selected from the group consisting of: 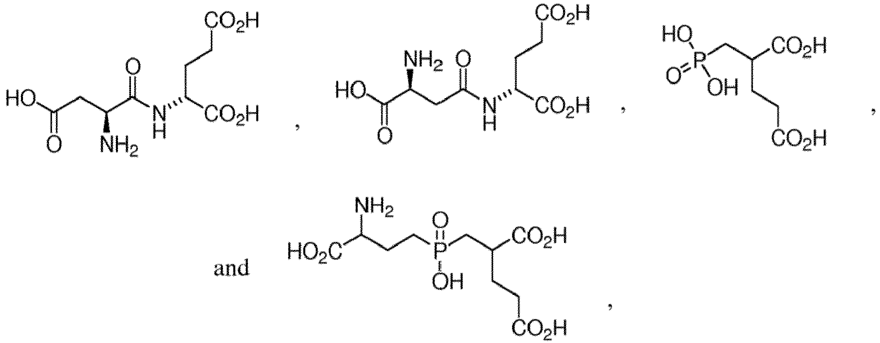 and enantiomers, stereoisomers, rotamers, tautomers, diastereomers, or racemates thereof; any of which may be further substituted with NH2, SH, OH, CO₂H, Ci₁₋₆-alkyl that is substituted with NH₂, SH, OH or CO₂H, or phenyl that is substituted with NH₂, SH, OH or CO₂H, wherein these functional groups serve as the point of covalent attachment. For example, a low-molecular weight PSMA ligand may be 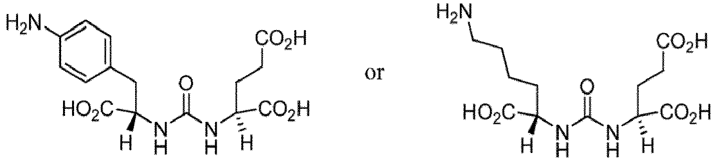 and enantiomers, stereoisomers, rotamers, tautomers, diastereomers, or racemates thereof; wherein the NH₂ groups serve as the point of covalent attachment. Attachment may be to a linker, polymer, particle, etc.

In some embodiments, the PSMA small molecule ligand that comprises a molecule that is or mimics a substrate that binds the enzymatic site on PSMA includes 2-[3-(1,3-dicarboxypropyl)ureido] pentanedioic acid (DUPA). In some embodiments, such small molecule ligands are conjugated to a chemotherapeutic agent, such as tubulysin hydrazide (TubH). The synthesis and uses of an example of such a PSMA ligand conjugate (EC1069) are described in Kularatne, SA et al J Med Chem 2010, 53, 7767-7777; Kularatne, SA et al Mol Phramaceutics Vol 6, no 3, 780-789, 2009. EC1719 is another example of a PSMA ligand conjugate that includes TubH. EC1069 and EC1719 can target the chemotherapy drug to PSMA receptors expressed on prostate cancer cells (Endocyte). Other EC1069 and EC1719 analogs linking DUPA and TubH can also target PSMA receptors expressed on prostate cancer cells. Thus, also contemplated herein are analogs of EC1069 and analogs of EC1719. The terms "EC1069" and "EC1719," therefore, encompasses EC 1069, EC1719 and analogs thereof. The linkers of the analogs, in some embodiments, may be peptides with D-amino-acid(s), or peptides attached with sugar moieties, amides or esters. An example of a linker, therefore, is D-γ-Glu D-Asp-D-Phe-D-Cys (**FIG. 9**). Other linkers may be used as provided herein.

Conjugation of one or more therapeutic agents to a PSMA ligand can include many chemical mechanisms, for instance covalent binding, affinity binding, intercalation, coordinate binding, electrostatic binding and complexation. Conjugation may also include encapsulation and is intended to refer to any mechanism by which one component may be associated with another component. Conjugation may be direct conjugation of the therapeutic agent to the PSMA ligand or it may be indirect, such as via a linker, polymer, particle etc., and it is the linker, polymer, particle, etc. to which the therapeutic agent is bound.

Covalent binding can be achieved either by direct condensation of existing side chains or by the incorporation of external bridging molecules. Many bivalent or polyvalent agents are useful in coupling protein molecules to other proteins, peptides or amine functions, etc. For example, the literature is replete with coupling agents such as carbodiimides, diisocyanates, glutaraldehyde, diazobenzenes, and hexamethylene diamines. This list is not intended to be exhaustive of the various coupling agents known in the art but, rather, is exemplary of the more common coupling agents.

In some embodiments, wherein the PSMA ligand is an antibody, it is contemplated the antibody is first derivatized, and then the therapeutic agent is attached to the derivatized product. Suitable cross-linking agents for use in this manner include, for example, SPDP (N-succinimidyl-3-(2-pyridyldithio)propionate), and SMPT, 4-succinimidyl-oxycarbonyl-methyl-(2-pyridyldithio)toluene.

In some embodiments, where the agent is a protein toxin, it may be fused to the PSMA ligand by genetic methods to form a hybrid immunotoxin fusion protein. The fusion proteins can include additional peptide sequences, such as peptide spacers that operatively attach, for example, the PSMA ligand and toxin, as long as such additional sequences do not appreciably affect the targeting or toxin activities of the fusion protein. The proteins, in some embodiments, may be attached by a peptide linker or spacer, such as a glycine-serine spacer peptide, or a peptide hinge, as is well known in the art. Thus, for example, if the PSMA ligand is a PSMA antibody, the C-terminus of PSMA antibody can be fused to the N-terminus of the protein toxin molecule to form an immunotoxin that retains the binding properties of the PSMA antibody. Other fusion arrangements will be known to one of ordinary skill in the art. To express the fusion immunotoxin, the nucleic acid encoding the fusion protein is inserted into an expression vector in accordance with standard methods, for stable expression of the fusion protein, such as in mammalian cells, such as CHO cells. The fusion protein can be isolated and purified from the cells or culture supernatant using standard methodology, such as a PSMA affinity column.

Examples of anticancer agents for use as provided herein include, without limitation, cytotoxic agents, chemotherapeutic agents and agents that act on tumor neovasculature. Cytotoxic agents include, but are not limited to, cytotoxic radionuclides, chemical toxins and protein toxins. Cytotoxic radionuclides or radiotherapeutic isotopes include alpha-emitting isotopes such as, for example, ²²⁵Ac, ²¹¹At, ²¹²Bi, ²¹³Bi, ²¹²Pb, ²²⁴Ra, ²²³Ra. Cytotoxic radionuclides or radiotherapeutic isotopes include beta-emitting isotopes such as, for example, ¹⁸⁶Rh, ¹⁸⁸Rh, ¹⁷⁷Lu, ⁹⁰Y, ¹³¹I, ⁶⁷Cu, ⁶⁴Cu ¹⁵³Sm, ¹⁶⁶Ho. In some instances, cytotoxic radionuclides may emit Auger and/or low energy electrons and include the isotopes ¹²³I, ¹²⁴I, ¹²⁵I, ¹³¹I, ⁷⁵Br, ⁷⁷Br and ¹⁸F.

Radionuclides typically are coupled to an antibody or antigen-binding fragment thereof by chelation. For example, in the case of metallic radionuclides, a bifunctional chelator is commonly used to link the isotope to the antibody or other protein of interest. Typically, the chelator is first attached to the antibody, and the chelator-antibody conjugate is contacted with the metallic radioisotope. A number of bifunctional chelators have been developed for this purpose, including the diethylenetriamine pentaacetic acid (DTPA) series of amino acids described in U.S. patents 5,124,471, 5,286,850 and 5,434,287, which are incorporated herein by reference. As another example, hydroxamic acid-based bifunctional chelating agents are described in U.S. patent 5,756,825, the contents of which are incorporated herein. Another example is the chelating agent termed p-SCN-Bz-HEHA (1,4,7,10,13,16-hexaazacyclo-octadecane- N,N',N",N''',N'''',N'''''-hexaacetic acid) (Deal et al., J. Med. Chem. 42:2988, 1999), which is an effective chelator of radiometals such as ²²⁵Ac. Yet another example is DOTA (1,4,7,10-tetraazacyclododecane N,N',N",N'''-tetraacetic acid), which is a bifunctional chelating agent (*see* McDevitt et al., Science 294:1537-1540, 2001) that can be used in a two-step method for labeling followed by conjugation.

Chemical toxins or chemotherapeutic agents include, but are not limited to, members of the enediyne family of molecules, such as calicheamicin and esperamicin. Chemical toxins or chemotherapeutic agents can also include pyrrolobenzodiazepine (PBD) dimers (e.g., SJG-136, SG2000, SG2202, SG2285 as described in Hartley JA et al., Cancer Res. 2010, 70(17):6849-58), calicheamicins, colchicine, ispinesib (a novel small molecule inhibitor of kinesin spindle protein), combrestatin (e.g., combrestatin A4), maytansine derivatives such as maytansinoid DM4 (*N*2'-deacetyl-*N*2'-(4-mercapto-4-methyl-1-oxopentyl)maytansine) and maytansinoid DM1 (mertansine), methotrexate, doxorubicin, melphalan, chlorambucil, ARA-C, vindesine, mitomycin C, cis-platinum, etoposide, bleomycin and/or 5-fluorouracil. Other antineoplastic agents include dolastatins (U.S. Patent Nos. 6,034,065 and 6,239,104) and derivatives thereof. Dolastatins and derivatives thereof include dolastatin 10 (dolavaline-valine-dolaisoleuine-dolaproine-dolaphenine) and the derivatives auristatin PHE (dolavaline-valine-dolaisoleuine-dolaproine-phenylalanine-methyl ester) (Pettit, G.R. et al., Anticancer Drug Des. 13(4):243-277, 1998; Woyke, T. et al., Antimicrob. Agents Chemother. 45(12):3580-3584, 2001), aurastatin E (e.g., monomethylauristatin norephedrine), aurastatin F (*e.g.,* monomethylauristatin phenylalanine) and the like. Toxins also include poisonous lectins, plant toxins such as ricin, abrin, modeccin, botulina and diphtheria toxins. Other chemotherapeutic agents are known to those skilled in the art and may be used as provided herein.

Agents that act on the tumor vasculature include, but are not limited to, tubulin-binding agents *(e.g.,* anti-tubulin agents) such as tubulysin and derivatives thereof (Kaur et al., Biochem J. 396(Pt 2):235-242, 2006), combrestatin A4 (Griggs et al., Lancet Oncol. 2:82, 2001), angiostatin and endostatin (reviewed in Rosen, Oncologist 5:20, 2000, incorporated by reference herein) and interferon inducible protein 10 (U.S. Patent No. 5,994,292). A number of other antiangiogenic agents are also contemplated and include: 2ME2, angiostatin, angiozyme, anti-VEGF RhuMAb, Apra (CT-2584), avicine, benefin, BMS275291, carboxyamidotriazole, CC4047, CC5013, CC7085, CDC801, CGP-41251 (PKC 412), CM101, combretastatin A-4 prodrug, EMD 121974, endostatin, flavopiridol, genistein (GCP), IM-862, ImmTher, interferon alpha, interleukin-12, gefitinib (ZD1839), marimastat, metastat (Col-3), neovastat, octreotide, paclitaxel, penicillamine, photofrin, photopoint, PI-88, prinomastat (AG-3340), PTK787 (ZK22584), R0317453, solimastat, squalamine, SU 101, SU 5416, SU-6668, suradista (FCE 26644), suramin (metaret), tetrathiomolybdate, thalidomide, TNP-470 and vitaxin. Additional antiangiogenic agents are described by Kerbel, J. Clin. Oncol. 19(18s):45s-51s, 2001, which is incorporated by reference herein. Such agents are contemplated for use in the PSMA ligand conjugates provided herein.

In some embodiments, a PSMA ligand conjugate is a PSMA antibody-drug conjugate. Non-limiting examples of PSMA antibody-drug conjugates are described in US-2007-0160617-A1 and US-2011-0250216-A, such examples of each of which are incorporated by reference herein. In some embodiments, a PSMA antibody-drug conjugate comprises an antibody or antigen-binding fragment thereof that specifically binds PSMA and is conjugated to a dolastatin 10 derivative, in particular auristatins such as, MMAE (also referred to herein as monomethylauristatin E or monomethylauristatin norephedrine) or MMAF (also referred to herein as monomethylauristatin F or monomethylauristatin phenylalanine).

The antibody or antigen-binding fragment thereof can be, in some embodiments, conjugated to MMAE or MMAF with a compound of the following formula **(Formula 1**): - An-Ym-Zm-Xn-Wn-, wherein A is a carboxylic acyl unit; Y is an amino acid; Z is an amino acid; X and W are each a self-immolative spacer; n is an integer of 0 or 1; and m is an integer of 0 or 1, 2, 3, 4, 5 or 6. The ADC, in some embodiments, is represented by the formula **(Formula 2**): L-{Aₙ-Yₘ-Zₘ-Xₙ-Wn-D}ₚ wherein L is an antibody or antigen-binding fragment thereof that binds PSMA, D is MMAE or MMAF and p is an integer of 1, 2, 3, 4, 5, 6, 7 or 8. The other components are as described above. In one embodiment, the carboxylic unit "Aₙ" is linked to the antibody or antigen-binding fragment via a sulfur atom derived from the antibody or antigen-binding fragment:

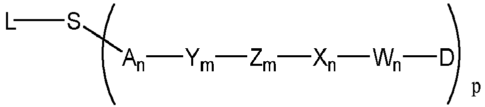

In one embodiment, A is 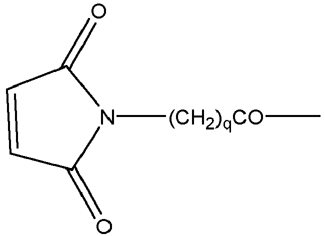 in which q is 1-10. Therefore, in one embodiment, the conjugate is: 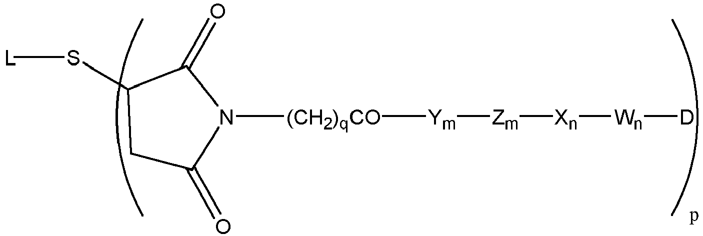 wherein L, Y, Z, X, W, D, n, m, q and p are as previously defined.

In another embodiment, A is 4-(N-succinimidomethyl)cyclohexane-1-carbonyl, m-succinimidobenzoyl, 4-(p-succinimidophenyl) -butyryl, 4-(2-acetamido)benzoyl, 3-thiopropionyl, 4-(1-thioethyl)-benzoyl, 6-(3-thiopropionylamido)-hexanoyl or maleimide caproyl. In a further embodiment, A is maleimide caproyl. Representative examples of various carboxylic acyl units and methods for their synthesis and attachment are described in US Pat. No. 6,214,345, the entire contents of which, but in particular the examples and methods for their synthesis and attachment, are herein incorporated by reference.

In another embodiment, Y is alanine, valine, leucine, isoleucine, methionine, phenylalanine, tryptophan or proline. In yet another embodiment, Y is valine. In a further embodiment, Z is lysine, lysine protected with acetyl or formyl, arginine, arginine protected with tosyl or nitro groups, histidine, ornithine, ornithine protected with acetyl or formyl, or citrulline. In still a further embodiment, Z is citrulline. In one embodiment Yₘ-Zₘ is valine-citrulline. In another embodiment, Yₘ-Zₘ is a protein sequence which is selectively cleavable by a protease.

In a further embodiment, X is a compound having the formula 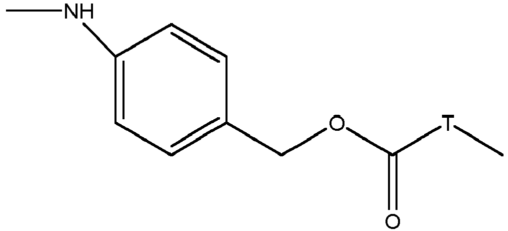 in which T is O, N, or S. In another embodiment, X is a compound having the formula -HN-R¹ -COT in which R¹ is C₁ -C₅ alkyl, T is O, N or S. In a further embodiment, X is a compound having the formula 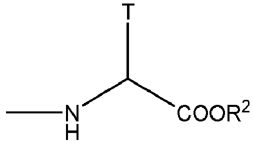 in which T is O, N, or S, R² is H or C₁ -C₅ alkyl. In one embodiment, X is p-aminobenzylcarbamoyloxy. In another embodiment, X is p-aminobenzylalcohol. In a further embodiment, X is p-aminobenzylcarbamate. In yet a further embodiment, X is p-aminobenzyloxycarbonyl. In another embodiment, X is γ-aminobutyric acid; α,α-dimethyl γ-aminobutyric acid or β,β-dimethyl γ-aminobutyric acid.

In some embodiments, W is 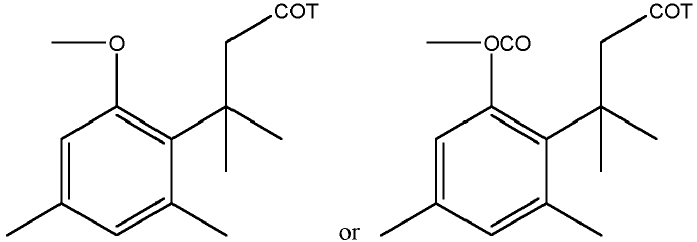 in which T is O, S or N.

In one embodiment, the compound of **Formula 1** is maleimidocaproyl. Maleimidocaproyl has been used for conjugation of two specific auristatins to an anti-CD30 mAb (AC10) (Doronina, Svetlana et al. "Novel Linkers for Monoclonal Antibody-Mediated Delivery of Anticancer Agents", AACR, Anaheim, CA, Abstract No. 1421, April 16-20, 2005). Maleimidocaproyl reacts with thiol groups to form a thioether.

MMAE or MMAF can be conjugated to an antibody or antigen-binding fragment thereof using methods known to those of ordinary skill in the art (*e.g., See,* Niemeyer, CM, *Bioconjugation Protocols, Strategies and Methods,* Humana Press, 2004) or as described herein. In some embodiments, more than one MMAE or MMAF molecule is conjugated to the antibody or antigen-binding fragment thereof. In other embodiments, 1, 2, 3, 4, 5, 6, 7 or 8 MMAE or MMAF molecules are conjugated to the antibody or antigen-binding fragment thereof. In still other embodiments, at least 2, 3, 4 or 5 MMAE or MMAF molecules are conjugated to the antibody or antigen-binding fragment thereof. In further embodiments, 2, 3, 4 or 5 MMAE or MMAF molecules are conjugated to the antibody or antigen-binding fragment thereof.

In some embodiments, the PSMA ligand conjugate is PSMA antibody (or antigen-binding fragment thereof)-maleimide caproyl-valine-citrulline-p-aminobenzyloxycarbonyl-monomethylauristatin norephedrine, PSMA antibody (or antigen-binding fragment thereof)-maleimide caproyl-valine-citrulline-p-aminobenzylcarbamate-monomethylauristatin norephedrine, PSMA antibody (or antigen-binding fragment thereof) -maleimide caproyl-monomethylauristatin norephedrine, PSMA antibody (or antigen-binding fragment thereof) - maleimide caproyl-valine-citrulline-p-aminobenzyloxycarbonyl-monomethylauristatin phenylalanine, PSMA antibody (or antigen-binding fragment thereof) -maleimide caproyl-valine-citrulline-p-aminobenzylcarbamate-monomethylauristatin phenylalanine or PSMA antibody (or antigen-binding fragment thereof) -maleimide caproyl-monomethylauristatin phenylalanine. In any of the foregoing, the PSMA antibody or antigen-binding fragment thereof may be any of the antibodies or antigen-binding fragments provided herein.

PSMA antibody-drug conjugates, for example, have been found to have particularly high levels of selectivity when killing of non-PSMA-expressing cells is compared to killing of PSMA-expressing cells. Therefore, in some embodiments, PSMA antibody-MMAE or PSMA antibody-MMAF conjugates have a PC-3™ cell to C4-2 cell or LNCaP™ cell selectivity of at least 250. In other embodiments, the selectivity is at least 300, 350, 400, 450, 500, 600, 700, 800, 900, 1000, 1100, 1200, 1300, 1400, 1500, 1600, 1700, 1800, 1900, 2000, 2250, 2500, 2750, 3000, 3500, 4000, 4500, 5000, 5500, 6000, 6500, 7000, 7500, 8000, 8500, 9000, 9500, 10000, 11000, 12000, 13000, 14000, 15000, 17500, 20000 or more. In some embodiments, the selectivity is between 250-500, 500-750, 750-1000, 1000-2000, 2000-5000, 5000-10000, 10000-15000 or 15000-20000. "Selectivity", as defined herein, refers to the ratio of IC₅₀ values of a PSMA antibody-MMAE conjugate or PSMA antibody-MMAF conjugate on PC-3™ cells (non-PSMA-expressing cells) to C4-2 cells or LNCaP™ cells (PSMA-expressing cells).

PSMA antibody-drug conjugates, in some embodiments, mediate PSMA-expressing cell specific killing at very low concentrations, such as at or near picomolar concentrations. PSMA antibody-drug conjugates, in some embodiments, exhibit IC₅₀s at concentrations of less than about 1 X 10⁻¹⁰M, less than about 1 X 10⁻¹¹M, or less than about 1 X 10⁻¹²M. In some embodiments, an IC₅₀ is achieved at a concentration of less than about 1.5 X 10⁻¹¹M. In other embodiments, PSMA antibody-drug conjugates exhibit IC₅₀s of between 10-210, 40-210, 60-210 or 65-210 picomoles (pM). In yet other embodiments, PSMA antibody-drug conjugates exhibit IC₅₀s of about 10, 40, 60 or 80 pM. In still other embodiments, PSMA antibody-drug conjugates exhibit IC₅₀s of about 11, 42, 60 or 83 pM.

The level of cell killing can be determined by any of the methods provided herein or otherwise known to those of ordinary skill in the art.

An "antiandrogen," as used herein, refers to an agent that blocks (*e.g*., inhibits) the action of androgen hormones and androgen-regulated molecules. Adrenergic receptor antagonists are herein considered to be antiandrogens. The term "antiandrogen" includes antiandrogens, antiandrogen analogs, and antiandrogen derivatives. In prostate cancer, antiandrogens block the activity of testosterone, which typically slows prostate cancer growth. In some embodiments, an antiandrogen blocks enzyme cytochrome P450 17A1, encoded by the *CYP17A* gene. Antiandrogens may be steroidal or non-steroidal (also referred to as "pure"). Examples of antiandrogens for use as provided herein include, without limitation, abiraterone (ZYTIGA®), enzalutamide (XTANDI®), nilutamide (NILANDRON®), flutamide (EULEXIN®), bicalutamide (CASODEX®), orteronel (TAK-700, Tokai Pharmaceuticals, Inc.) Potent antiandrogens such as, for example, enzalutamide, abiraterone, ARN 509 (Aragon Pharmaceuticals, Inc.) and galeterone (TOK-001 or VN/124-1, Tokai Pharmaceuticals, Inc.), which are typically used in progressive, metatstatic castration-resistant prostate cancer and which affect expression of a host of androgen-regulated molecules, such as PSMA expression.

"Potent" antiandrogens, or "second-generation" antiandrogens herein include antiandrogens that retain activity in a cellular environment of increased androgen receptor expression relative to wild-type expression. Potent antiandrogens include those that are active in subjects with castration-resistant prostate cancer. Potent antiandrogens also include antiandrogens that bind to the androgen receptor with greater relative affinity than common, clinically used antiandrgogens (*e.g*., bicalutamide, nilutamide, flutamide). Examples of potent antiandrogens include, without limitation, enzalutamide, abiraterone, ARN-509, galeterone, and diarylthiohydantoins RD162 and MDV310, 3beta-hydroxyandrosta-5,16-diene (HAD) and androsta-1,4-diene-3,17-dione-17-ethylene ketal (OAK) (Miyamoto H et al., Int J Cancer 2005, 117(5):866-72, incorporated by reference herein). Abiraterone, for example, inhibits the CYP17A1 enzyme. Enzalutamide, as another example, is an androgen receptor antagonist. As yet another example, galeterone is a selective CYP17 inhibitor and an androgen receptor antagonist. Methods that may be used to identify potent antiandrogens are described, for example, by Tran C et al., Science, 2009 324(5928):787-90, incorporated by reference herein. Any of the antiandrogens described herein may be used in any of the methods provided herein.

The compound that increases the cell surface expression of PSMA can also be an mTOR inhibitor. Examples of mTOR inhibitors for use as provided herein include, without limitation, rapamycin, everolimus (AFINITOR®, ZORTRESS®) and temsirolimus (TORISEL®). The term "mTOR inhibitor" includes mTOR inhibitors, mTOR inhibitor analogs, and mTOR inhibitor derivatives.

Also provided herein are compositions, for example, pharmaceutical compositions, which comprise a compound that increases cell surface expression of PSMA, a PSMA ligand conjugate, or a combination of the two. In some embodiments, a composition is administered to a subject (*e.g*., a mammal, such as a human). In some embodiments, a composition comprising a PSMA ligand conjugate is administered to a subject and another composition comprising a compound that increases cell surface expression of PSMA is separately administered to the subject, either sequentially or concurrently. Alternatively, in some embodiments, a composition comprising both a PSMA ligand conjugate and a compound that increases cell surface expression of PSMA is administered to a subject.

In some embodiments, a composition comprising a compound that increases cell surface expression of PSMA is first administered to a subject, and then a composition comprising a PSMA ligand conjugate is administered to the subject immediately after, within an hour, within a couple of hours, within a day, within 2 days, within 3 days, within 4 days, with 5 days, within 6 days, or within a week or more. Either composition may be administered once or more than once (*e.g*., twice, thrice, etc.)

As used herein, "a compound that increases cell surface expression of PSMA," refers to a compound that increases the cell surface expression of PSMA by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 125%, at least 150%, at least 175%, at least 200% or more, relative to normal cell surface expression of a PSMA-expressing cell or relative to cell surface expression in the absence of such compound. For example, an antiandrogen may, in some embodiments, increase the cell surface expression of PSMA on cancer cells by at least 20%, or more.

A composition, in some embodiments, includes a physiologically or pharmaceutically acceptable carrier, excipient, or stabilizer combined with a compound that increases cell surface expression of PSMA and/or aPSMA ligand conjugate. As used herein, "pharmaceutically acceptable carrier" or "physiologically acceptable carrier" includes any and all salts, solvents, dispersion media, coatings, antibacterial and antifungal agents, isotonic and absorption delaying agents, and the like that are physiologically compatible. A "pharmaceutically-acceptable carrier," as used herein, refers to one or more compatible solid or liquid fillers, diluents or encapsulating substances that are suitable for administration into a human. The term "carrier" denotes an organic or inorganic ingredient, natural or synthetic, with which the active ingredient is combined to facilitate the application. A carrier may be suitable for intravenous, intramuscular, subcutaneous, parenteral, spinal or epidermal administration (*e.g*., by injection or infusion).

In some embodiments, a composition may be administered to a subject in pharmaceutically-acceptable amounts and in pharmaceutically-acceptable compositions. The term "pharmaceutically acceptable" means a non-toxic material that does not interfere with the effectiveness of the biological activity of the active ingredients (*e.g*., PSMA ligands, anticancer agents, cytotoxic agents, antiandrogens, mTOR inhibitors). Such compositions may contain salts, buffering agents, preservatives, compatible carriers, and optionally other therapeutic agents, such as supplementary immune potentiating agents including adjuvants, chemokines and cytokines. When used in medicine, the salts should be pharmaceutically acceptable, but non-pharmaceutically acceptable salts may conveniently be used to prepare pharmaceutically-acceptable salts thereof and are not excluded.

A salt retains the desired biological activity of the parent compound and does not impart any undesired toxicological effects (*see e.g.,* Berge, S. M., et al. (1977) J. Pharm. Sci. 66: 1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.
The pharmaceutical compositions may contain suitable buffering agents, including: acetic acid in a salt; citric acid in a salt; boric acid in a salt; and phosphoric acid in a salt.

In some embodiments, a composition may contain suitable preservatives, such as: benzalkonium chloride; chlorobutanol; parabens and/or thimerosal.

In some embodiments, a composition may conveniently be presented in unit dosage form and may be prepared by any of the methods well-known in the art of pharmacy. All methods include the step of bringing the active compound(s) (*e.g*., PSMA ligand, anticancer agent, cytotoxic agent and/or compound that upregulates cell surface expression of PSMA) into association with a carrier that constitutes one or more accessory ingredients. In some embodiments, compositions are prepared by uniformly and intimately bringing the active compound into association with a liquid carrier, a finely divided solid carrier, or both, and then, if necessary, shaping the product.

Compositions suitable for parenteral administration conveniently comprise a sterile aqueous or non-aqueous preparation of PSMA ligand conjugates and/or compounds that increase cell surface expression of PSMA (*e.g*., antiandrogens, mTOR inhibitors), which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid may be used in the preparation of injectables. Carrier formulations suitable for oral, subcutaneous, intravenous, intramuscular, etc. administration can be found in Remington's Pharmaceutical Sciences, Mack Publishing Co., Easton, Pa. Any of the compositions provided herein may be sterile.

Active compounds (*e.g.*, PSMA ligand, anticancer agent, cytotoxic agent and/or compound that upregulates cell surface expression of PSMA) can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. *See, e.g.,* Sustained and Controlled Release Drug Delivery Systems, J. R. Robinson, ed., Marcel Dekker, Inc., New York, 1978.

A composition can be administered by any conventional route, including injection or by gradual infusion over time. The administration may, for example, be oral, intravenous, intraperitoneal, intramuscular, intracavity, intratumor, or transdermal. When compositions are used therapeutically, preferred routes of administration include intravenous and by pulmonary aerosol. Techniques for preparing aerosol delivery systems containing antibodies are well known to those of skill in the art. Generally, such systems should utilize components that will not significantly impair the biological properties of the antibodies, such as the paratope binding capacity (see, for example, Sciarra and Cutie, "Aerosols," in Remington's Pharmaceutical Sciences, 18th edition, 1990, pp. 1694-1712; incorporated by reference). Those of skill in the art can readily determine the various parameters and conditions for producing antibody aerosols without resorting to undue experimentation.

Compositions as provided herein, in some embodiments, may be administered in effective amounts. An "effective amount" is that amount of an active compound (*e.g*., PSMA ligand conjugate and/or compound that increases cell surface expression of PSMA) that alone, or together with further doses, produces the desired response, *e.g*., inhibits cell proliferation of PSMA-expressing cells and/or kills PSMA-expressing cells. For cancer, this may involve only slowing the progression of a cancer, for example, temporarily, although more preferably, it involves halting the progression of the cancer permanently. This can be monitored by routine methods. The desired response to treatment of cancer or other disease or condition also can be delaying the onset or even preventing the onset of the cancer or other disease or condition.

Such effective amounts will depend, of course, on the particular condition being treated (*e.g.*, PSMA-expressing cancer), the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient/subject may insist upon a lower dose or tolerable dose for medical reasons, psychological reasons or for virtually any other reason.

Compositions as provided herein, in some embodiments, are sterile and contain an effective amount of PSMA ligand conjugates and/or compound that increases cell surface expression of PSMA, etc. for producing the desired response in a unit of weight or volume suitable for administration to a patient/subject. The response can, for example, be measured by determining the physiological effects of the composition, such as regression of a tumor or decrease of disease symptoms. Other assays will be known to one of ordinary skill in the art and can be employed for measuring the level of the response.

The doses of compositions administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

The synergistic effect provided by various combinations of PSMA ligand conjugates with compounds provided herein, such as antiandrogens or mTOR inhibitors, may result in efficacious doses which are lower than the doses required for efficacy when either component of the combination is used singly. The added advantage of a dose lowering effect may have the added benefit of a wider safety profile, *i.e.,* fewer adverse side effects.

For example, doses of PSMA ligand conjugate and/or compound that increases cell surface expression of PSMA can range from about 10 µg/kg to about 100,000 µg/kg. Based on the composition, the dose can be delivered continuously, such as by continuous pump, or at periodic intervals. Desired time intervals of multiple doses of a particular composition can be determined without undue experimentation by one skilled in the art. Other protocols for the administration of compositions will be known to one of ordinary skill in the art, in which the dose amount, schedule of administration, sites of administration, mode of administration and the like vary from the foregoing.

In some embodiments, the dose of PSMA ligand conjugate is administered intravenously. In such embodiments, the dose of PSMA ligand conjugate may be about 1.0 mg/kg to 2.5 mg/kg. For example, in some embodiments, the dose of PSMA ligand conjugate administered intravenously is 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, or 2.5 mg/kg. In some embodiments, the dose of PSMA ligand administered intravenously conjugate is about 1.0 mg/kg to 2.3 mg/kg. In an embodiment, the PSMA ligand conjugated is a PSMA ADC, and the PSMA ADC is provided in a dose of about 1.8 mg/kg to 2.3 mg/kg.

The length of time during which a PSMA ligand conjugate is administered administered intravenously may vary. In some embodiments, a PSMA ligand conjugate may be administered intravenously for 30 minutes, 35 minutes, 40 minutes, 45 minutes, 50 minutes, 55 minutes, 60 minutes, 65 minutes, 70 minutes, 75 minutes, 80 minutes, 85 minutes or 90 minutes.

In some embodiments, a PSMA ligand conjugate is administered intravenously at repeated intervals such as, for example, once a week, once every two weeks, or once every three weeks for up to a total of four, six or eight doses. In some embodiments, the PSMA ligand conjugate is administered intravenously twice a week, or more.

In some embodiments, a PSMA ligand conjugate may be administered intravenously for about 60 minutes once every three weeks at a dose of about 1.0 mg/kg to 2.3 mg/kg for up to a total of eight doses.

In some embodiments, a compound as provided herein, such as an antiandrogen, is administered in the form of oral capsules. In such embodiments, the dose of such compound may be about 40 mg to about 160 mg (*e.g.,* 20 mg or 40 mg per capsule). For example, in some embodiments, the dose of such compound administered orally is 40 mg, 60 mg, 80 mg, 100 mg, 120 mg, 140 mg or 160 mg. In some embodiments, the oral dose of such compound is administered once daily, or twice daily, for about 21 to about 28 days. In some embodiments, the dose of such compound is 80 mg (*e.g.,* 2 oral capsules, 40 mg each) to 160 mg (*e.g.,* 4 oral capsules, 40 mg each) and is administered once daily (OD) for 28 days. In some embodiments, such compound is enzulatamide, at a dose of 80 mg (*e.g.,* 2 oral capsules, 40 mg each) to 160 mg (*e.g.,* 4 oral capsules, 40 mg each) and is administered once daily (OD) for 28 days.

In some embodiments, the oral dose of a compound as provided herein, such as an antiandrogen, may be about 500 mg to about 1000 mg. For example, in some embodiments, the dose of such compound administered orally is 500 mg, 550 mg, 600 mg, 650 mg, 70 mg 0, 750 mg, 800 mg, 850 mg, 900 mg, 950 mg or 1000 mg. In some embodiments, the oral dose of such compound is administered once daily, or twice daily, for about 21 to about 28 days. In some embodiments, the dose of such compound is 500 mg to 1000 and is administered once daily (OD) for 28 days. In some embodiments, such compound is abiraterone or abiraterone acetate, at a dose of 500 mg to 1000 mg, and is administered once daily (OD) for 28 days.

In some embodiments, an mTOR inhibitor is administered in an I.V. dose of about 5 to 25 mg at weekly intervals. In another embodiment, the mTOR inhibitor is administered in an I.V. dose of 5 to 10 mg at weekly inervals. In one embodiment, the mTOR inhibitor is temsirolimus (Toresal®) dosed 25 mg I.V. over 30 to 60 minutes at weekly intervals.

In general, doses of radionuclides delivered by a PSMA ligand conjugate provided herein can range from about 0.01 mCi/Kg to about 10 mCi/kg. In some embodiments, the dose of radionuclide ranges from about 0.1 mCi/Kg to about 1.0 mCi/kg. In one embodiment, the PSMA ligand conjugate is ¹³¹I-MIP-1095 provided in an I.V. dose of about 1 to 10 GBq. In another embodiment, ¹³¹I-MIP-1095 is provided in a dose range of 2 to 8 GBq. In yet another embodiment, the mean dose is about 5 GBq. The optimal dose of a given isotope can be determined empirically by simple routine titration experiments well known to one of ordinary skill in the art.

Administration of compositions provided herein to mammals other than humans, *e.g*. for testing purposes or veterinary therapeutic purposes, is carried out under substantially the same conditions as described above.

Compositions (*e.g*., that comprise PSMA ligand conjugate and/or compound that increases cell surface expression of PSMA) as provided herein have *in vitro* and *in vivo* diagnostic and therapeutic utilities. For example, these compounds can be administered to cells in culture, *e.g., in vitro* or *ex vivo,* or in a subject, *e.g., in vivo,* to treat, prevent or diagnose cancer or other disease or condition. As used herein, the term "subject" is intended to include humans and non-human animals. Preferred subjects include a human patient having a disorder characterized by expression, typically aberrant expression (*e.g*., overexpression) of PSMA.

The compositions provided herein, in some embodiments, may be used in conjunction with other therapeutic treatment modalities. Such other treatments include surgery, radiation, cryosurgery, thermotherapy, hormone treatment, chemotherapy, vaccines, and other immunotherapies.

Subjects with prostate cancer, in some embodiments, have undergone, are undergoing, or will undergo hormone therapy. Thus, in some embodiments, the compositions provided herein may be administered to a subject subsequent to, together with, or prior to hormone therapy, such as for prostate cancer. Examples of hormone therapies for prostate cancer include, without limitation: luteinizing hormone-releasing hormone agonists (*e.g*., leuprolide, goserelin, and buserelin), which can stop the testicles from making testosterone; antiandrogens (*e.g*., flutamide, bicalutamide, enzalutamide and nilutamide), as discussed elsewhere herein, which can block the action of androgens such as testosterone; drugs that can prevent the adrenal glands from making androgens (*e.g*., ketoconazole and aminoglutethimide); orchiectomy, which is a surgical procedure to remove one or both testicles, the main source of male hormones such as testosterone, to decrease the amount of hormone being produced; and estrogens, which can prevent the testicles from making testosterone.

A myriad of subjects may benefit from the methods and compositions provided herein. In some embodiments, such a subject has progressive metastatic castration-resistant prostate cancer despite a castrate level of serum testosterone (*e.g*., < 50 mg/dL) and having had prior chemotherapy with docetaxel. In other embodiments, the subject has metastatic castration resistant prostate cancer, has had prior treatment with taxane chemotherapy and has received and progressed on abiraterone and/or enzalutamide. In yet other embodiments, the subject has progressive metastatic castration-resistant prostate cancer despite a castrate level of serum testosterone, has had prior treatment with abiraterone and/or enzalutamide, and has had no prior treatment with cytotoxic chemotherapy. In still other embodiments, the subject has progressive metastatic castration-resistant prostate cancer despite a castrate level of serum testosterone and has had one prior treatment with abiraterone and/or enzalutamide. In further embodiments, the subject has progressive metastatic castration-resistant prostate cancer despite a castrate level of serum testosterone and has had no prior treatment with abiraterone and or enzalutamide. In additional embodiments, the subject has asymptomatic or minimally symptomatic metastatic castration-resistant prostate cancer despite a castrate level of serum testosterone and has had no prior treatment with abiraterone and/or enzalutamide. In some embodiments, the subject has stable metastatic castration-resistant prostate cancer and is receiving treatment with abiraterone and/or enzalutamide. In other embodiments, the subject has biochemically recurrent prostate cancer and has previously undergone a primary therapy (*e.g.,* radical prostatectomy (*e.g.,* open, laparoscopic, or robot-assisted) or radiation therapy (e.g., dose-escalated three-dimensional conformal RT, intensity-modulated RT, brachytherapy, or a combination thereof)). In yet other embodiments, the subject has localized high-risk prostate cancer (*e.g*., prostate specific antigen (PSA) greater than 10 nanogram per milliliter (ng/ml); PSA velocity greater than 2 ng/ml per /year (defined as a rise in PSA of greater than 2 ng/ml in the preceding 12 month period); Gleason score greater than or equal to 7 (4+3); or Gleason score 6 if either PSA greater than or equal to 10 ng/ml or PSA velocity greater than or equal to 2 ng/ml/year) and is a candidate for prostatectomy.

Also provided herein are kits comprising the composition(s). In some embodiments, the kits comprise a container containing a compound that increases cell surface expression of PSMA, and a container containing a PSMA ligand conjugate (or the components thereof). The kits can further contain at least one additional reagent as provided herein. In some embodiments, a kit may comprise a carrier being compartmentalized to receive in close confinement therein one or more containers or series of containers such as test tubes, vials, flasks, bottles, syringes, or the like. One container or series of containers may contain one or more compound that increases cell surface expression of PSMA. Another container, or series of containers in some embodiments, may contain a PSMA ligand conjugate (or the components thereof). The components of the kits can be packaged either in aqueous medium or in lyophilized form. The components of the conjugates can be supplied either in fully conjugated form, in the form of intermediates or as separate moieties to be conjugated by the user of the kit.

Kits may, in some embodiments, also comprise a diluent and/or instructions for reconstituting lyophilized forms of the PSMA ligand conjugates and/or compounds for increasing cell surface expression of PSMA, or instructions for diluting aqueous components of the kits. Kits may also comprise instructions for combining a compound that increases cell surface expression of PSMA and/or a PSMA ligand conjugate.

The invention is further exemplified in the following clauses:
1. A method of inhibiting proliferation of prostate-specific membrane antigen (PSMA)-expressing cancer cells, the method comprising:
   contacting PSMA-expressing cancer cells with an antiandrogen; and
   contacting the PSMA-expressing cancer cells with a PSMA ligand-anticancer agent conjugate.
2. The method of clause 1, wherein the PSMA ligand of the conjugate comprises an antibody, or antigen-binding fragment thereof, that binds specifically PSMA.
3. The method of clause 2, wherein the antibody or antigen-binding fragment thereof is an antibody selected from the group consisting of: PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1 and Abgenix 4.152.1, or an antigen-binding fragment thereof.
4. The method of clause 2, wherein the antibody or antigen-binding fragment thereof comprises (i) the three complementarity determining regions of a heavy chain variable region comprising an amino acid sequence set forth as SEQ ID NO:15 and (ii) the three complementarity determining regions of a light chain variable region comprising an amino acid sequence set forth as SEQ ID NO: 17.
5. The method of clause 2, wherein the antibody or antigen-binding fragment thereof comprises (i) the three complementarity determining regions of a heavy chain variable region comprising an amino acid sequence set forth as SEQ ID NO:19 and (ii) the three complementarity determining regions of a light chain variable region comprising an amino acid sequence set forth as SEQ ID NO: 21.
6. The method of clause 2, wherein the antibody or antigen-binding fragment thereof comprises (i) the three complementarity determining regions of a heavy chain variable region comprising an amino acid sequence set forth as SEQ ID NO:23 and (ii) the three complementarity determining regions of a light chain variable region comprising an amino acid sequence set forth as SEQ ID NO: 25.
7. The method of clause 2, wherein the antibody or antigen-binding fragment thereof comprises (i) the three complementarity determining regions of a heavy chain variable region comprising an amino acid sequence set forth as SEQ ID NO:27 and (ii) the three complementarity determining regions of a light chain variable region comprising an amino acid sequence set forth as SEQ ID NO: 29.
8. The method of clause 2, wherein the antibody or antigen-binding fragment thereof comprises (i) the three complementarity determining regions of a heavy chain variable region comprising an amino acid sequence set forth as SEQ ID NO: 31 and (ii) the three complementarity determining regions of a light chain variable region comprising an amino acid sequence set forth as SEQ ID NO: 33.
9. The method of clause 2, wherein the antibody or antigen-binding fragment thereof is antibody PSMA 10.3, AB-PG1-XG1-006, AB-PG1-XG1-026, AB-PG1-XG1-051, AB-PG1-XG1-069, AB-PG1-XG1-077, or an antigen-binding fragment thereof.
10. The method of clause 2, wherein the antibody or antigen-binding fragment thereof is antibody E99, J415, J533 or J591 or an antibody produced by a hybridoma having ATCC Accession Number HB-12101, HB-12109, HB-12127or HB-12126, or an antigen -binding fragment thereof.
11. The method of clause 2, wherein the antibody or antigen-binding fragment thereof is an antibody produced by a hybidoma having ATCC Accession Number HB12060 (3F5.4G6), HB12309 (3D7-1.1), HB12310 (4E10-1.14), HB12489 (1G3), HB12495 (1G9), HB12490 (2C7), HB12494 (3C4), HB12491 (3C6), HB12484 (3C9), HB12486 (3E6), HB12488 (3E11), HB12485 (3G6), HB12493 (4D4), HB12487 (4D8), HB12492 (4C8B9), HB12664 (3F6), HB12678 (2E4), HB12665 (3C2), HB12672 (2D4), HB12660 (4C8G8), HB12675 (2C4), HB12663 (4C11), HB12661 (1D11), HB12667 (4E8), HB12674 (2G5), HB12620 (4E6), HB12677 (1F4), HB12666 (2E3), HB12662 (3D8), HB12668 (4F8), HB12673 (3D2), HB12676 (1G7), HB12669 (3D4), HB12679 (5G10) or HB12671 (5E9), or an antigen-binding fragment thereof.
12. The method of clause 1, wherein the PSMA ligand of the conjugate comprises a small molecule ligand that binds specifically PSMA.
13. The method of clause 12, wherein the small molecule ligand binds an enzymatic site on PSMA.
14. The method of clause 12, wherein the small molecule ligand comprises MIP-1095, MIP-1072, GL2 or DUPA.
15. The method of clause 14, wherein the PSMA ligand-anticancer agent conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.
16. The method of any one of clauses 1-15, wherein the anticancer agent comprises an auristatin, tubulysin,a pyrrolobenzodiazepine dimer, calicheamicin, colchicine, ispinesib, combrestatin A4, maytansinoid DM1, maytansinoid DM4, doxorubicin, or a cytotoxic radionuclide.
17. The method of clause 16, wherein the auristatin comprises monomethylauristatin norephedrine or monomethylauristatin phenylalanine.
18. The method of clause 16, wherein the PSMA ligand-anticancer agent conjugate comprises EC1069 or EC1719.
19. The method of clause 1, wherein the PSMA ligand-anticancer agent conjugate comprises BIND-014.
20. The method of any one of clauses 1-19, wherein the compound that increases cell surface expression of PSMA is an antiandrogen.
21. The method of clause 20, wherein the antiandrogen blocks enzyme cytochrome CYP17.
22. The method of clause 20, wherein the antiandrogen blocks enzyme cytochrome CYP17A1.
23. The method of clause 20, wherein the antiandrogen is an androgen receptor antagonist.
24. The method of any one of clauses 1-20, wherein the antiandrogen is abiraterone, enzalutamide, nilutamide, flutamide, bicalutamide, ARN 509, galeterone or orteronel.
25. The method of clause 24, wherein the antiandrogen is enzalutamide, abiraterone or ARN 509.
26. The method of any one of clauses 1-25, wherein the antiandrogen is in an amount effective to upregulate PSMA expression.
27. The method of any one of clauses 1-26, wherein the step of contacting the PSMA-expressing cancer cells with the antiandrogen and the step of contacting the PSMA-expressing cancer cells with the PSMA ligand-anticancer agent conjugate are concurrent.
28. The method of any one of clauses 1-26, wherein the step of contacting the PSMA-expressing cancer cells with the antiandrogen and the step of contacting the PSMA-expressing cancer cells with the PSMA ligand-anticancer agent conjugate are sequential.
29. The method of clause 28, wherein the step of contacting the PSMA-expressing cancer cells with the antiandrogen is prior to the step of contacting the PSMA-expressing cancer cells with the PSMA ligand-anticancer agent conjugate.
30. The method of clause 29, wherein the step of contacting the PSMA-expressing cancer cells with the PSMA ligand-anticancer agent conjugate occurs within one week of the step of contacting the PSMA-expressing cancer cells with the antiandrogen.
31. The method of any one of clauses 1-30, wherein the PSMA-expressing cancer cells are in contact with the antiandrogen for at least 3 days.
32. The method of clause 31, wherein the PSMA-expressing cancer cells are in contact with the antiandrogen for at least 7 days.
33. The method of clause 32, wherein the PSMA-expressing cancer cells are in contact with the antiandrogen for at least 14 days.
34. The method of clause 33, wherein the PSMA-expressing cancer cells are in contact with the antiandrogen for at least 21 days or at least 28 days.
35. The method of any one of clauses 1-34, wherein the PSMA-expressing cancer cells comprise androgen-independent PSMA-expressing cancer cells.
36. The method of any one of clauses 1-35, wherein the PSMA-expressing cancer cells comprise PSMA-expressing cancer cells insensitive to antiandrogen therapy.
37. The method of clause 36, wherein the PSMA-expressing cancer cells insensitive to antiandrogen therapy are re-sensitized to antiandrogen therapy after the step of contacting the PSMA-expressing cancer cells with the antiandrogen.
38. The method of any one of clauses 1-37, wherein the PSMA-expressing cancer cells are of a tumor.
39. The method of any one of clauses 1-38, wherein the PSMA-expressing cancer cells are PSMA-expressing prostate cancer cells.
40. The method of any one of clauses 1-38, wherein the PSMA-expressing cancer cells are PSMA-expressing non-prostate cancer cells.
41. The method of clause 40, wherein the PSMA-expressing cancer cells are of the neovasculature of non-prostate cancer cells.
42. The method of any one of clauses 1-41, wherein the PSMA-expressing cancer cells are of a subject.
43. The method of clause 42, wherein the subject has progressive metastatic castration-resistant prostate cancer.
44. The method of clauses 42 or 43, wherein the subject has had prior chemotherapy with at least one taxane.
45. The method of any one of clauses 42-44, wherein the subject has had prior treatment with one or more antiandrogens.
46. The method of any one of clauses 42-45, wherein the subject has prostate cancer that has progressed despite prior treatment.
47. The method of clause 42 or 43, wherein the subject has not had prior cytotoxic chemotherapy.
48. The method of clause 1, wherein the antiandrogen is enzalutamide, abiraterone or ARN 509, and the PSMA ligand-anticancer agent conjugate is a PSMA ADC.
49. A method of inhibiting proliferation of prostate-specific membrane antigen (PSMA)-expressing cancer cells, the method comprising:
   contacting PSMA-expressing cancer cells with an mTOR inhibitor; and
   contacting the PSMA-expressing cancer cells with a PSMA ligand-anticancer agent conjugate.
50. The method of clause 49, wherein the PSMA ligand of the conjugate comprises an antibody, or antigen-binding fragment thereof, that binds specifically PSMA.
51. The method of clause 50, wherein the antibody or antigen-binding fragment thereof is an antibody selected from the group consisting of: PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1 and Abgenix 4.152.1, or an antigen-binding fragment thereof.
52. The method of clause 50, wherein the antibody or antigen-binding fragment thereof comprises (i) the three complementarity determining regions of a heavy chain variable region comprising an amino acid sequence set forth as SEQ ID NO:15 and (ii) the three complementarity determining regions of a light chain variable region comprising an amino acid sequence set forth as SEQ ID NO: 17.
53. The method of clause 50, wherein the antibody or antigen-binding fragment thereof comprises (i) the three complementarity determining regions of a heavy chain variable region comprising an amino acid sequence set forth as SEQ ID NO:19 and (ii) the three complementarity determining regions of a light chain variable region comprising an amino acid sequence set forth as SEQ ID NO: 21.
54. The method of clause 50, wherein the antibody or antigen-binding fragment thereof comprises (i) the three complementarity determining regions of a heavy chain variable region comprising an amino acid sequence set forth as SEQ ID NO:23 and (ii) the three complementarity determining regions of a light chain variable region comprising an amino acid sequence set forth as SEQ ID NO: 25.
55. The method of clause 50, wherein the antibody or antigen-binding fragment thereof comprises (i) the three complementarity determining regions of a heavy chain variable region comprising an amino acid sequence set forth as SEQ ID NO:27 and (ii) the three complementarity determining regions of a light chain variable region comprising an amino acid sequence set forth as SEQ ID NO: 29.
56. The method of clause 50, wherein the antibody or antigen-binding fragment thereof comprises (i) the three complementarity determining regions of a heavy chain variable region comprising an amino acid sequence set forth as SEQ ID NO: 31 and (ii) the three complementarity determining regions of a light chain variable region comprising an amino acid sequence set forth as SEQ ID NO: 33.
57. The method of clause 50, wherein the antibody or antigen-binding fragment thereof is antibody PSMA 10.3, AB-PG1-XG1-006, AB-PG1-XG1-026, AB-PG1-XG1-051, AB-PG1-XG1-069, AB-PG1-XG1-077, or an antigen-binding fragment thereof.
58. The method of clause 50, wherein the antibody or antigen-binding fragment thereof is antibody E99, J415, J533 or J591 or an antibody produced by a hybridoma having ATCC Accession Number HB-12101, HB-12109, HB-12127or HB-12126, or an antigen -binding fragment thereof.
59. The method of clause 50, wherein the antibody or antigen-binding fragment thereof is an antibody produced by a hybridoma having ATCC Accession Number HB12060 (3F5.4G6), HB12309 (3D7-1.1), HB12310 (4E10-1.14), HB12489 (1G3), HB12495 (1G9), HB12490 (2C7), HB12494 (3C4), HB12491 (3C6), HB12484 (3C9), HB12486 (3E6), HB12488 (3E11), HB12485 (3G6), HB12493 (4D4), HB12487 (4D8), HB12492 (4C8B9), HB12664 (3F6), HB12678 (2E4), HB12665 (3C2), HB12672 (2D4), HB12660 (4C8G8), HB12675 (2C4), HB12663 (4C11), HB12661 (1D11), HB12667 (4E8), HB12674 (2G5), HB12620 (4E6), HB 12677 (1F4), HB 12666 (2E3), HB 12662 (3D8), HB 12668 (4F8), HB 12673 (3D2), HB12676 (1G7), HB12669 (3D4), HB12679 (5G10) or HB12671 (5E9), or an antigen-binding fragment thereof.
60. The method of clause 49, wherein the PSMA ligand of the conjugate comprises a small molecule ligand that binds specifically PSMA.
61. The method of clause 60, wherein the small molecule ligand binds an enzymatic site on PSMA.
62. The method of clause 60, wherein the small molecule ligand comprises MIP-1095, MIP-1072, GL2 or DUPA.
63. The method of clause 62, wherein the PSMA ligand-anticancer agent conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.
64. The method of any one of clauses 49-63, wherein the anticancer agent comprises an auristatin, tubulysin,a pyrrolobenzodiazepine dimer, calicheamicin, colchicine, ispinesib, combrestatin A4, maytansinoid DM1, maytansinoid DM4, doxorubicin, or a cytotoxic radionuclide.
65. The method of clause 64, wherein the auristatin comprises monomethylauristatin norephedrine or monomethylauristatin phenylalanine.
66. The method of clause 64, wherein the PSMA ligand-anticancer agent conjugate comprises EC1069 or EC1719.
67. The method of clause 49, wherein the PSMA ligand-anticancer agent conjugate comprises BIND-014.
68. The method of any one of clauses 49-67, wherein the mTOR inhibitor is rapamycin.
69. The method of any one of clauses 49-68, wherein the mTOR inhibitor is in an amount effective to upregulate PSMA expression.
70. The method of any one of clauses 49-69, wherein the step of contacting the PSMA-expressing cancer cells with the mTOR inhibitor and the step of contacting the PSMA-expressing cancer cells with the PSMA ligand-anticancer agent conjugate are concurrent.
71. The method of any one of clauses 49-69, wherein the step of contacting the PSMA-expressing cancer cells with the mTOR inhibitor and the step of contacting the PSMA-expressing cancer cells with the PSMA ligand-anticancer agent conjugate are sequential.
72. The method of clause 71, wherein the step of contacting the PSMA-expressing cancer cells with the mTOR inhibitor is prior to the step of contacting the PSMA-expressing cancer cells with the PSMA ligand-anticancer agent conjugate.
73. The method of clause 72, wherein the PSMA-expressing cancer cells are in contact with the mTOR inhibitor for at least 7 days.
74. The method of any one of clauses 49-73, wherein the PSMA-expressing cancer cells comprise androgen-independent PSMA-expressing cancer cells.
75. The method of any one of clauses 49-74, wherein the PSMA-expressing cancer cells comprise PSMA-expressing cancer cells insensitive to antiandrogen therapy.
76. The method of clause 75, wherein the PSMA-expressing cancer cells insensitive to antiandrogen therapy are re-sensitized to antiandrogen therapy after the step of contacting the PSMA-expressing cancer cells with the mTOR inhibitor.
77. The method of any one of clauses 49-76, wherein the PSMA-expressing cancer cells are of a tumor.
78. The method of any one of clauses 49-77, wherein the PSMA-expressing cancer cells are PSMA-expressing prostate cancer cells.
79. The method of any one of clauses 49-77, wherein the PSMA-expressing cancer cells are PSMA-expressing non-prostate cancer cells.
80. The method of clause 79, wherein the PSMA-expressing cancer cells are of the neovasculature of non-prostate cancer cells.
81. The method of any one of clauses 49-80, wherein the PSMA-expressing cancer cells are of a subject.
82. The method of clause 81, wherein the subject has progressive metastatic castration-resistant prostate cancer.
83. The method of clauses 81 or 82, wherein the subject has had prior chemotherapy with at least one taxane.
84. The method of any one of clauses 81-83, wherein the subject has had prior treatment with one or more antiandrogens.
85. The method of any one of clauses 81-84, wherein the subject has prostate cancer that has progressed despite prior treatment.
86. The method of clause 81 or 82, wherein the subject has not had prior cytotoxic chemotherapy.
87. The method of clause 49, wherein the mTOR inhibitor is rapamycin, and the PSMA ligand-anticancer agent conjugate is a PSMA ADC.
88. A method of specific delivery of a cytotoxic agent to PSMA-expressing cells in a subject, comprising:
   administering to a subject a compound that increases cell surface expression of PSMA; and
   administering to the subject a PSMA ligand-cytotoxic agent conjugate.
89. The method of clause 88, wherein the PSMA ligand of the conjugate comprises an antibody, or antigen-binding fragment thereof, that binds specifically PSMA.
90. The method of clause 89, wherein the antibody or antigen-binding fragment thereof is an antibody selected from the group consisting of: PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1 and Abgenix 4.152.1, or an antigen-binding fragment thereof.
91. The method of clause 89, wherein the antibody or antigen-binding fragment thereof comprises (i) the three complementarity determining regions of a heavy chain variable region comprising an amino acid sequence set forth as SEQ ID NO:15 and (ii) the three complementarity determining regions of a light chain variable region comprising an amino acid sequence set forth as SEQ ID NO: 17.
92. The method of clause 89, wherein the antibody or antigen-binding fragment thereof comprises (i) the three complementarity determining regions of a heavy chain variable region comprising an amino acid sequence set forth as SEQ ID NO:19 and (ii) the three complementarity determining regions of a light chain variable region comprising an amino acid sequence set forth as SEQ ID NO: 21.
93. The method of clause 89, wherein the antibody or antigen-binding fragment thereof comprises (i) the three complementarity determining regions of a heavy chain variable region comprising an amino acid sequence set forth as SEQ ID NO:23 and (ii) the three complementarity determining regions of a light chain variable region comprising an amino acid sequence set forth as SEQ ID NO: 25.
94. The method of clause 89, wherein the antibody or antigen-binding fragment thereof comprises (i) the three complementarity determining regions of a heavy chain variable region comprising an amino acid sequence set forth as SEQ ID NO:27 and (ii) the three complementarity determining regions of a light chain variable region comprising an amino acid sequence set forth as SEQ ID NO: 29.
95. The method of clause 89, wherein the antibody or antigen-binding fragment thereof comprises (i) the three complementarity determining regions of a heavy chain variable region comprising an amino acid sequence set forth as SEQ ID NO: 31 and (ii) the three complementarity determining regions of a light chain variable region comprising an amino acid sequence set forth as SEQ ID NO: 33.
96. The method of clause 89, wherein the antibody or antigen-binding fragment thereof is antibody PSMA 10.3, AB-PG1-XG1-006, AB-PG1-XG1-026, AB-PG1-XG1-051, AB-PG1-XG1-069, AB-PG1-XG1-077, or an antigen-binding fragment thereof.
97. The method of clause 89, wherein the antibody or antigen-binding fragment thereof is antibody E99, J415, J533 or J591 or an antibody produced by a hybridoma having ATCC Accession Number HB-12101, HB-12109, HB-12127or HB-12126, or an antigen -binding fragment thereof.
98. The method of clause 89, wherein the antibody or antigen-binding fragment thereof is an antibody produced by a hybidoma having ATCC Accession Number HB12060 (3F5.4G6), HB12309 (3D7-1.1), HB12310 (4E10-1.14), HB12489 (1G3), HB12495 (1G9), HB12490 (2C7), HB12494 (3C4), HB12491 (3C6), HB12484 (3C9), HB12486 (3E6), HB12488 (3E11), HB12485 (3G6), HB12493 (4D4), HB12487 (4D8), HB12492 (4C8B9), HB12664 (3F6), HB12678 (2E4), HB12665 (3C2), HB12672 (2D4), HB12660 (4C8G8), HB12675 (2C4), HB12663 (4C11), HB12661 (1D11), HB12667 (4E8), HB12674 (2G5), HB12620 (4E6), HB 12677 (1F4), HB 12666 (2E3), HB 12662 (3D8), HB 12668 (4F8), HB 12673 (3D2), HB12676 (1G7), HB12669 (3D4), HB12679 (5G10) or HB12671 (5E9), or an antigen-binding fragment thereof.
99. The method of clause 88, wherein the PSMA ligand of the conjugate comprises a small molecule ligand that binds specifically PSMA.
100. The method of clause 99, wherein the small molecule ligand binds an enzymatic site on PSMA.
101. The method of clause 99, wherein the small molecule ligand comprises MIP-1095, MIP-1072, GL2 or DUPA.
102. The method of clause 101, wherein the PSMA ligand-cytotoxic agent conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.
103. The method of any one of clauses 88-102, wherein the cytotoxic agent comprises an auristatin, tubulysin,a pyrrolobenzodiazepine dimer, calicheamicin, colchicine, ispinesib, combrestatin A4, maytansinoid DM1, maytansinoid DM4, doxorubicin, or a cytotoxic radionuclide.
104. The method of clause 103, wherein the auristatin comprises monomethylauristatin norephedrine or monomethylauristatin phenylalanine.
105. The method of clause 103, wherein the PSMA ligand-cytotoxic agent conjugate comprises EC1069 or EC1719.
106. The method of clause 88, wherein the PSMA ligand-cytotoxic agent conjugate comprises BIND-014.
107. The method of any one of clauses 88-106, wherein the compound that increases cell surface expression of PSMA is an antiandrogen.
108. The method of clause 107, wherein the antiandrogen blocks enzyme cytochrome CYP17.
109. The method of clause 107, wherein the antiandrogen blocks enzyme cytochrome CYP17A1.
110. The method of clause 107, wherein the antiandrogen is an androgen receptor antagonist.
111. The method of clause 107, wherein the antiandrogen is abiraterone, enzalutamide, nilutamide, flutamide, bicalutamide, ARN 509, galeterone or orteronel.
112. The method of clause 111, wherein the antiandrogen is enzalutamide, abiraterone or ARN 509.
113. The method of any one of clauses 88-106, wherein the compound that increases cell surface expression of PSMA is an mTOR inhibitor.
114. The method of clause 113, wherein the mTOR inhibitor is rapamycin.
115. The method of any one of clauses 88-114, wherein the step of administering the compound and the step of administering the PSMA ligand-cytotoxic agent conjugate are concurrent.
116. The method of any one of clauses 88-114, wherein the step of administering the compound and the step of administering the PSMA ligand-cytotoxic agent conjugate are sequential.
117. The method of clause 116, wherein the step of administering the compound is prior to the step of administering the PSMA ligand-cytotoxic agent conjugate.
118. The method of clause 117, wherein the step of administering the PSMA ligand-cytotoxic agent conjugate occurs within one week of the step of administering the compound.
119. The method of any one of clauses 88-118, wherein the PSMA-expressing cells are in contact with the compound for at least 3 days.
120. The method of clause 119, wherein the PSMA-expressing cells are in contact with the compound for at least 7 days.
121. The method of clause 120, wherein the PSMA-expressing cells are in contact with the compound for at least 14 days.
122. The method of clause 121, wherein the PSMA-expressing cells are in contact with the compound for at least 21 days or at least 28 days.
123. The method of any one of clauses 88-122, wherein the PSMA-expressing cells comprise androgen-independent PSMA-expressing cancer cells.
124. The method of any one of clauses 88-123, wherein the PSMA-expressing cells comprise PSMA-expressing cancer cells insensitive to antiandrogen therapy.
125. The method of clause 124, wherein the PSMA-expressing cells insensitive to antiandrogen therapy are re-sensitized to antiandrogen therapy after the step of administering the compound.
126. The method of any one of clauses 88-125, wherein the PSMA-expressing cells are of a tumor.
127. The method of any one of clauses 88-126, wherein the PSMA-expressing cells are PSMA-expressing prostate cancer cells.
128. The method of any one of clauses 88-126, wherein the PSMA-expressing cells are PSMA-expressing non-prostate cancer cells.
129. The method of clause 128, wherein the PSMA-expressing cells are of the neovasculature of non-prostate cancer cells.
130. The method of any one of clauses 88-129, wherein the PSMA-expressing cells are of a subj ect.
131. The method of clause 130, wherein the subject has progressive metastatic castration-resistant prostate cancer.
132. The method of clauses 130 or 131, wherein the subject has had prior chemotherapy with at least one taxane.
133. The method of any one of clauses 130-132, wherein the subject has had prior treatment with one or more antiandrogens.
134. The method of any one of clauses 130-133, wherein the subject has prostate cancer that has progressed despite prior treatment.
135. The method of clause 130 or 131, wherein the subject has not had prior cytotoxic chemotherapy.
136. The method of clause 88, wherein the compound that increases cell surface expression of PSMA is enzalutamide, abiraterone, ARN 509 or rapamycin, and the PSMA ligand-cytotoxic agent conjugate is a PSMA ADC.
137. A composition comprising a compound that increases cell surface expression of PSMA and a PSMA ligand-anticancer agent conjugate or PSMA ligand-cytotoxic agent conjugate.
138. The composition of clause 137, wherein the PSMA ligand of the conjugate comprises an antibody, or antigen-binding fragment thereof, that binds specifically PSMA.
139. The composition of clause 138, wherein the antibody or antigen-binding fragment thereof is an antibody selected from the group consisting of: PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1 and Abgenix 4.152.1, or an antigen-binding fragment thereof.
140. The composition of clause 138, wherein the antibody or antigen-binding fragment thereof comprises (i) the three complementarity determining regions of a heavy chain variable region comprising an amino acid sequence set forth as SEQ ID NO:15 and (ii) the three complementarity determining regions of a light chain variable region comprising an amino acid sequence set forth as SEQ ID NO: 17.
141. The composition of clause 138, wherein the antibody or antigen-binding fragment thereof comprises (i) the three complementarity determining regions of a heavy chain variable region comprising an amino acid sequence set forth as SEQ ID NO:19 and (ii) the three complementarity determining regions of a light chain variable region comprising an amino acid sequence set forth as SEQ ID NO: 21.
142. The composition of clause 138, wherein the antibody or antigen-binding fragment thereof comprises (i) the three complementarity determining regions of a heavy chain variable region comprising an amino acid sequence set forth as SEQ ID NO:23 and (ii) the three complementarity determining regions of a light chain variable region comprising an amino acid sequence set forth as SEQ ID NO: 25.
143. The composition of clause 138, wherein the antibody or antigen-binding fragment thereof comprises (i) the three complementarity determining regions of a heavy chain variable region comprising an amino acid sequence set forth as SEQ ID NO:27 and (ii) the three complementarity determining regions of a light chain variable region comprising an amino acid sequence set forth as SEQ ID NO: 29.
144. The composition of clause 138, wherein the antibody or antigen-binding fragment thereof comprises (i) the three complementarity determining regions of a heavy chain variable region comprising an amino acid sequence set forth as SEQ ID NO: 31 and (ii) the three complementarity determining regions of a light chain variable region comprising an amino acid sequence set forth as SEQ ID NO: 33.
145. The composition of clause 138, wherein the antibody or antigen-binding fragment thereof is antibody PSMA 10.3, AB-PG1-XG1-006, AB-PG1-XG1-026, AB-PG1-XG1-051, AB-PG1-XG1-069, AB-PG1-XG1-077, or an antigen-binding fragment thereof.
146. The composition of clause 138, wherein the antibody or antigen-binding fragment thereof is antibody E99, J415, J533 or J591 or an antibody produced by a hybridoma having ATCC Accession Number HB-12101, HB-12109, HB-12127or HB-12126, or an antigen - binding fragment thereof.
147. The composition of clause 138, wherein the antibody or antigen-binding fragment thereof is an antibody produced by a hybidoma having ATCC Accession Number HB12060 (3F5.4G6), HB12309 (3D7-1.1), HB12310 (4E10-1.14), HB12489 (1G3), HB12495 (1G9), HB12490 (2C7), HB12494 (3C4), HB12491 (3C6), HB12484 (3C9), HB12486 (3E6), HB 12488 (3E11), HB 12485 (3G6), HB 12493 (4D4), HB 12487 (4D8), HB 12492 (4C8B9), HB12664 (3F6), HB12678 (2E4), HB12665 (3C2), HB12672 (2D4), HB12660 (4C8G8), HB12675 (2C4), HB12663 (4C11), HB12661 (1D11), HB12667 (4E8), HB12674 (2G5), HB 12620 (4E6), HB 12677 (1F4), HB 12666 (2E3), HB 12662 (3D8), HB 12668 (4F8), HB12673 (3D2), HB12676 (1G7), HB12669 (3D4), HB12679 (5G10) or HB12671 (5E9), or an antigen-binding fragment thereof.
148. The composition of clause 137, wherein the PSMA ligand of the conjugate comprises a small molecule ligand that binds specifically PSMA.
149. The composition of clause 148, wherein the small molecule ligand binds an enzymatic site on PSMA.
150. The composition of clause 148, wherein the small molecule ligand comprises MIP-1095, MIP-1072, GL2 or DUPA.
151. The composition of clause 150, wherein the PSMA ligand conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.
152. The composition of any one of clauses 137-151, wherein the anticancer agent or cytotoxic agent comprises an auristatin, tubulysin,a pyrrolobenzodiazepine dimer, calicheamicin, colchicine, ispinesib, combrestatin A4, maytansinoid DM1, maytansinoid DM4, doxorubicin, or a cytotoxic radionuclide.
153. The composition of clause 152, wherein the auristatin comprises monomethylauristatin norephedrine or monomethylauristatin phenylalanine.
154. The composition of clause 152, wherein the PSMA ligand conjugate comprises EC1069 or EC1719.
155. The composition of clause 137, wherein the PSMA ligand conjugate comprises BIND-014.
156. The composition of any one of clauses 137-155, wherein the compound that increases cell surface expression of PSMA is an antiandrogen.
157. The composition of clause 156, wherein the antiandrogen blocks enzyme cytochrome CYP17.
158. The composition of clause 156, wherein the antiandrogen blocks enzyme cytochrome CYP17A1.
159. The composition of clause 156, wherein the antiandrogen is an androgen receptor antagonist.
160. The composition of clause 156, wherein the antiandrogen is abiraterone, enzalutamide, nilutamide, flutamide, bicalutamide, ARN 509, galeterone or orteronel.
161. The composition of clause 160, wherein the antiandrogen is enzalutamide, abiraterone or ARN 509.
162. The composition of any one of clauses 137-155, wherein the compound that increases cell surface expression of PSMA is an mTOR inhibitor.
163. The composition of clause 162, wherein the mTOR inhibitor is rapamycin.
164. The composition of any one of clauses 137-163, further comprising a pharmaceutically acceptable carrier and/or excipient.
165. The composition of clause 137, wherein the compound that increases cell surface expression of PSMA is enzalutamide, abiraterone, ARN 509 or rapamycin, and the PSMA ligand conjugate is a PSMA ADC.
166. A kit comprising:
   a container containing a compound that increases cell surface expression of PSMA of a PSMA-expressing cell; and
   a container containing a PSMA ligand-anticancer agent conjugate or PSMA ligand-cytotoxic agent conjugate.
167. The kit of clause 166, wherein the PSMA ligand of the conjugate comprises an antibody, or antigen-binding fragment thereof, that binds specifically PSMA.
168. The kit of clause 167, wherein the antibody or antigen-binding fragment thereof is an antibody selected from the group consisting of: PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1 and Abgenix 4.152.1, or an antigen-binding fragment thereof.
169. The kit of clause 167, wherein the antibody or antigen-binding fragment thereof comprises (i) the three complementarity determining regions of a heavy chain variable region comprising an amino acid sequence set forth as SEQ ID NO:15 and (ii) the three complementarity determining regions of a light chain variable region comprising an amino acid sequence set forth as SEQ ID NO: 17.
170. The kit of clause 167, wherein the antibody or antigen-binding fragment thereof comprises (i) the three complementarity determining regions of a heavy chain variable region comprising an amino acid sequence set forth as SEQ ID NO:19 and (ii) the three complementarity determining regions of a light chain variable region comprising an amino acid sequence set forth as SEQ ID NO: 21.
171. The kit of clause 167, wherein the antibody or antigen-binding fragment thereof comprises (i) the three complementarity determining regions of a heavy chain variable region comprising an amino acid sequence set forth as SEQ ID NO:23 and (ii) the three complementarity determining regions of a light chain variable region comprising an amino acid sequence set forth as SEQ ID NO: 25.
172. The kit of clause 167, wherein the antibody or antigen-binding fragment thereof comprises (i) the three complementarity determining regions of a heavy chain variable region comprising an amino acid sequence set forth as SEQ ID NO:27 and (ii) the three complementarity determining regions of a light chain variable region comprising an amino acid sequence set forth as SEQ ID NO: 29.
173. The kit of clause 167, wherein the antibody or antigen-binding fragment thereof comprises (i) the three complementarity determining regions of a heavy chain variable region comprising an amino acid sequence set forth as SEQ ID NO: 31 and (ii) the three complementarity determining regions of a light chain variable region comprising an amino acid sequence set forth as SEQ ID NO: 33.
174. The kit of clause 167, wherein the antibody or antigen-binding fragment thereof is antibody PSMA 10.3, AB-PG1-XG1-006, AB-PG1-XG1-026, AB-PG1-XG1-051, AB-PG1-XG1-069, AB-PG1-XG1-077, or an antigen-binding fragment thereof.
175. The kit of clause 167, wherein the antibody or antigen-binding fragment thereof is antibody E99, J415, J533 or J591 or an antibody produced by a hybridoma having ATCC Accession Number HB-12101, HB-12109, HB-12127or HB-12126, or an antigen -binding fragment thereof.
176. The kit of clause 167, wherein the antibody or antigen-binding fragment thereof is an antibody produced by a hybidoma having ATCC Accession Number HB12060 (3F5.4G6), HB12309 (3D7-1.1), HB12310 (4E10-1.14), HB12489 (1G3), HB12495 (1G9), HB12490 (2C7), HB12494 (3C4), HB12491 (3C6), HB12484 (3C9), HB12486 (3E6), HB12488 (3E11), HB12485 (3G6), HB12493 (4D4), HB12487 (4D8), HB12492 (4C8B9), HB12664 (3F6), HB12678 (2E4), HB12665 (3C2), HB12672 (2D4), HB12660 (4C8G8), HB12675 (2C4), HB12663 (4C11), HB12661 (1D11), HB12667 (4E8), HB12674 (2G5), HB12620 (4E6), HB 12677 (1F4), HB 12666 (2E3), HB 12662 (3D8), HB 12668 (4F8), HB 12673 (3D2), HB12676 (1G7), HB12669 (3D4), HB12679 (5G10) or HB12671 (5E9), or an antigen-binding fragment thereof.
177. The kit of clause 166, wherein the PSMA ligand of the conjugate comprises a small molecule ligand that binds specifically PSMA.
178. The kit of clause 177, wherein the small molecule ligand binds an enzymatic site on PSMA.
179. The kit of clause 177, wherein the small molecule ligand comprises MIP-1095, MIP-1072, GL2 or DUPA.
180. The kit of clause 179, wherein the PSMA ligand conjugate comprises MIP-1095 or MIP-1072 conjugated to a cytotoxic radionuclide selected from the group consisting of I¹²³, I¹²⁵, I¹³¹, I¹²⁴ Br⁷⁵ Br⁷⁷ and F¹⁸.
181. The kit of any one of clauses 166-180, wherein the anticancer agent or cytotoxic agent comprises an auristatin, tubulysin,a pyrrolobenzodiazepine dimer, calicheamicin, colchicine, ispinesib, combrestatin A4, maytansinoid DM1, maytansinoid DM4, doxorubicin, or a cytotoxic radionuclide.
182. The kit of clause 181, wherein the auristatin comprises monomethylauristatin norephedrine or monomethylauristatin phenylalanine.
183. The kit of clause 181, wherein the PSMA ligand conjugate comprises EC1069 or EC1719.
184. The kit of clause 166, wherein the PSMA ligand conjugate comprises BIND-014.
185. The kit of any one of clauses 166-184, wherein the compound that increases cell surface expression of PSMA is an antiandrogen.
186. The kit of clause 185, wherein the antiandrogen blocks enzyme cytochrome CYP17.
187. The kit of clause 185, wherein the antiandrogen blocks enzyme cytochrome CYP17A1.
188. The kit of clause 185, wherein the antiandrogen is an androgen receptor antagonist.
189. The kit of clause 185, wherein the antiandrogen is abiraterone, enzalutamide, nilutamide, flutamide, bicalutamide, ARN 509, galeterone or orteronel.
190. The kit of clause 189, wherein the antiandrogen is enzalutamide, abiraterone or ARN 509.
191. The kit of any one of clauses 166-184, wherein the compound that increases cell surface expression of PSMA is an mTOR inhibitor.
192. The kit of clause 191, wherein the mTOR inhibitor is rapamycin.
193. The kit of any one of clauses 166-192, further comprising a pharmaceutically acceptable carrier and/or excipient.
194. The kit of any one of clauses 166-193, wherein the compound and/or the PSMA ligand conjugate is in aqueous medium.
195. The kit of any one of clauses 166-193, wherein the compound and/or the PSMA ligand conjugate is lyophilized.
196. The kit of any of clauses 166-195, further comprising a diluent.
197. The kit of clause 195 or 196, further comprising instructions for reconstituting the compound and/or the PSMA ligand conjugate.
198. The kit of any one of clauses 166-197, further comprising instructions for combining the compound and/or the PSMA ligand conjugate.
199. The kit of clause 166, wherein the compound that increases cell surface expression of PSMA is enzalutamide, abiraterone, ARN 509 or rapamycin, and the PSMA ligand conjugate is a PSMA ADC.
200. A method of inhibiting proliferation of prostate-specific membrane antigen (PSMA)-expressing cancer cells, the method comprising:
   contacting PSMA-expressing cancer cells with prednisone; and
   contacting the PSMA-expressing cancer cells with a PSMA ligand-anticancer agent or PSMA ligand-cytotoxic agent conjugate.
201. The method of clause 200, wherein the PSMA ligand of the conjugate comprises an antibody, or antigen-binding fragment thereof, that binds specifically PSMA.
202. The method of clause 200, wherein the PSMA ligand of the conjugate comprises a small molecule ligand that binds specifically PSMA.
203. The method of clause 202, wherein the PSMA ligand conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.
204. The method of any one of clauses 200-203, wherein the anticancer agent comprises an auristatin, tubulysin,a pyrrolobenzodiazepine dimer, calicheamicin, colchicine, ispinesib, combrestatin A4, maytansinoid DM1, maytansinoid DM4, doxorubicin, or a cytotoxic radionuclide.
205. The method of clause 204, wherein the auristatin comprises monomethylauristatin norephedrine or monomethylauristatin phenylalanine.
206. The method of any one of clauses 200-205, wherein the method further comprises contacting the PSMA expressing cells with a compound that increases cell surface expression of PSMA.
207. The method of clause 206, wherein the compound is an antiandrogen or an mTOR inhibitor.
208. The method of clause 207, wherein the antiandrogen is abiraterone, enzalutamide, nilutamide, flutamide, bicalutamide, ARN 509, galeterone or orteronel.
209. The method of clause 208, wherein the antiandrogen is enzalutamide, abiraterone or ARN 509.
210. The method of any one of clauses 200-209, wherein the steps of contacting are concurrent.
211. The method of any one of clauses 200-209, wherein the steps of contacting are sequential.
212. The method of clause 211, wherein the step of contacting the PSMA-expressing cancer cells with the prednisone and/or compound is prior to the step of contacting the PSMA-expressing cancer cells with the PSMA ligand conjugate.
213. The method of any one of clauses 200-212, wherein the PSMA-expressing cancer cells comprise androgen-independent PSMA-expressing cancer cells.
214. The method of any one of clauses 200-213, wherein the PSMA-expressing cancer cells comprise PSMA-expressing cancer cells insensitive to antiandrogen therapy.
215. The method of any one of clauses 200-214, wherein the PSMA-expressing cancer cells are of a subject.
216. The method of clause 209, wherein the antiandrogen is enzalutamide, abiraterone or ARN 509, and the PSMA ligand-anticancer agent conjugate is a PSMA ADC.
217. A composition comprising prednisone and a PSMA ligand conjugate.
218. The composition of clause 217, wherein the PSMA ligand of the conjugate comprises an antibody, or antigen-binding fragment thereof, that binds specifically PSMA.
219. The composition of clause 217, wherein the PSMA ligand of the conjugate comprises a small molecule ligand that binds specifically PSMA.
220. The composition of clause 219, wherein the PSMA ligand conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.
221. The composition of any one of clauses 217-220, wherein the anticancer agent or cytotoxic agent comprises an auristatin, tubulysin,a pyrrolobenzodiazepine dimer, calicheamicin, colchicine, ispinesib, combrestatin A4, maytansinoid DM1, maytansinoid DM4, doxorubicin, or a cytotoxic radionuclide.
222. The composition of clause 221, wherein the auristatin comprises monomethylauristatin norephedrine or monomethylauristatin phenylalanine.
223. The composition of any one of clauses 217-222, wherein the composition further comprises a compound that increases cell surface expression of PSMA.
224. The composition of clause 223, wherein the compound is an antiandrogen or an mTOR inhibitor.
225. The composition of clause 224, wherein the antiandrogen is abiraterone, enzalutamide, nilutamide, flutamide, bicalutamide, ARN 509, galeterone or orteronel.
226. The composition of clause 225, wherein the antiandrogen is enzalutamide, abiraterone or ARN 509.
227. The composition of any one of clauses 217-226, further comprising a pharmaceutically acceptable carrier and/or excipient.
228. The composition of clause 224, wherein the compound that increases cell surface expression of PSMA is enzalutamide, abiraterone, ARN 509 or rapamycin, and the PSMA ligand conjugate is a PSMA ADC.
229. A kit comprising:
   a container containing prednisone; and
   a container containing a PSMA ligand conjugate.
230. The kit of clause 229, wherein the PSMA ligand of the conjugate comprises an antibody, or antigen-binding fragment thereof, that binds specifically PSMA.
231. The kit of clause 229, wherein the PSMA ligand of the conjugate comprises a small molecule ligand that binds specifically PSMA.
232. The kit of clause 231, wherein the PSMA ligand conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.
233. The kit of any one of clauses 229-232, wherein the PSMA ligand conjugate comprises an auristatin, tubulysin,a pyrrolobenzodiazepine dimer, calicheamicin, colchicine, ispinesib, combrestatin A4, maytansinoid DM1, maytansinoid DM4, doxorubicin, or a cytotoxic radionuclide.
234. The kit of clause 233, wherein the auristatin comprises monomethylauristatin norephedrine or monomethylauristatin phenylalanine.
235. The kit of any one of clauses 229-234, wherein the kit further comprises a compound that increases cell surface expression of PSMA.
236. The kit of clause 235, wherein the compound is an antiandrogen or an mTOR inhibitor.
237. The kit of clause 236, wherein the antiandrogen is abiraterone, enzalutamide, nilutamide, flutamide, bicalutamide, ARN 509, galeterone or orteronel.
238. The kit of clause 237, wherein the antiandrogen is enzalutamide, abiraterone or ARN 509.
239. The kit of any one of clauses 229-238, further comprising a pharmaceutically acceptable carrier and/or excipient.
240. The kit of any one of clauses 229-239, wherein any or all of the components of the kit are in an aqueous medium.
241. The kit of any one of clauses 229-239, wherein any or all of the components of the kit are lyophilized.
242. The kit of any of clauses 229-241, further comprising a diluent.
243. The kit of clause 241 or 242, further comprising instructions for reconstituting the any or all of the components of the kit.
244. The kit of any one of clauses 229-243, further comprising instructions for combining any or all of the components of the kit.
245. The kit of clause 236, wherein the compound that increases cell surface expression of PSMA is enzalutamide, abiraterone, ARN 509 or rapamycin, and the PSMA ligand conjugate is a PSMA ADC.

The present invention is further illustrated by the following **Examples,** which in no way should be construed as further limiting. The entire contents of all of the references (including literature references, issued patents, published patent applications, and co-pending patent applications) cited throughout this application are hereby expressly incorporated by reference. However, the citation of any reference is not intended to be admission that said reference is prior art.

### EXAMPLES

### Example 1: PSMA ADC synergizes with antiandrogens and rapamycin.

Inhibitors were tested for anti-proliferative activity individually and in combination across a range of concentrations in a matrix fashion. Inhibition data were compared with Bliss predictions, and the differences between the experimental and predicted results were calculated for each point in the matrix. Positive differences indicate supra-additive or synergistic effects. Negative differences indicate antagonism. Results were assessed for statistical significance, as described below.

Mean inhibition data and Bliss differences are shown in **FIG. 2** for PSMA antibody-drug conjugate (PSMA ADC) combined with enzalutamide, abiraterone or rapamycin. Bliss differences are depicted via heat maps of values that are positive (medium gray), negative (dark gray), or near zero (light gray). Boxes are used to indicate statistically significant Bliss differences and the corresponding percent inhibitions. PSMA ADC exhibited similar single-agent activity against LNCaP and C4-2 cells, with IC₅₀ values of approximately 200 pM in each case. These results are in line with prior reports (20, 21). Enzalutamide, abiraterone, and rapamycin each inhibited proliferation of LNCaP cells (**FIG. 2A**) but had minimal antiproliferative effects on C4-2 cells when used as single agents (**FIG. 2B**).

Despite lack of direct antiproliferative activity in C4-2 cells, enzalutamide, abiraterone and rapamycin all significantly enhanced the activity of PSMA ADC. Bliss differences ranged to nearly 40% and were statistically significant across a range of inhibitor concentrations and levels of inhibition (**FIG. 2B**). No significant antagonism was observed for these combinations under any condition. Statistically significant synergy also was observed for PSMA ADC/enzalutamide and PSMA ADC/abiraterone combinations in LNCaP cells (**FIG. 2A**); however, the breadth and magnitude were more limited as compared to C4-2 cells. Similarly, Bliss differences were generally positive across the matrix of concentrations for the PSMA ADC/rapamycin combination in LNCaP cells; however, no value reached statistical significance.

Mean inhibition data and Bliss differences are shown in **FIG. 13** for PSMA ADC combined with the antiandrogens, ARN-509 or TOK-001. Bliss differences are depicted via heat maps of values that are positive (medium gray), negative (dark gray), or near zero (light gray). ARN-509 and TOK-001 each inhibited proliferation of LNCaP cells but had minimal antiproliferative effects on C4-2 cells when used as single agents. Despite lack of direct antiproliferative activity in C4-2 cells, ARN-509 and TOK-001 enhanced the activity of PSMA ADC.

To determine if the observed synergies were unique to PSMA ADC/antiandrogen combinations, a small molecule ligand was tested in combination with antiandrogens. Mean inhibition data and Bliss differences are shown in **FIG. 10** for the small molecule PSMA ligand-anticancer agent conjugate, EC1069, combined with enzalutamide. Heat maps illustrate Bliss differences that are positive for synergy (medium gray), negative (dark gray), or near zero (light gray).

As an initial step towards dissecting mechanisms of synergy, the antiandrogens and rapamycin were combined with free MMAE and with unmodified PSMA mAb. Free MMAE showed subnanomolar potency (IC₅₀ ∼ 0.5 nM) against both LNCaP and C4-2 cells, as expected (21). When paired with enzalutamide or abiraterone, free MMAE exhibited additive activity in LNCaP cells and additive to weakly synergistic activity on C4-2 (**FIG. 3**). Similar results were obtained when docetaxel, another microtubule inhibitor, was combined with enzalutamide. There were only sporadic instances of statistically significant synergy for the docetaxel/enzalutamide combination (**FIG. 3**).

The rapamycin/MMAE combination showed additive effects in LNCaP and moderate synergy in C4-2 cells (**FIG. 4**). Thus, rapamycin synergized less potently with MMAE as compared with PSMA ADC on both cell lines. Nonetheless, the rapamycin/MMAE combination appeared to be as or more potent than the enzalutamide/MMAE or abiraterone/MMAE combination in C4-2 cells. Weak to moderate synergy was observed between rapamycin and enzalutamide in both cell lines. In C4-2 cells, non-inhibitory concentrations of enzalutamide enhanced the weak antiproliferative activity of rapamycin (**FIG. 4**).

Unmodified PSMA mAb was inactive both alone and in combination with enzalutamide, abiraterone or rapamycin (**FIG. 5**). Due to the very limited activity of the individual agents or combinations, assays were performed only twice in some cases. Thus, the robust synergies observed upon combining PSMA ADC with antiandrogens are specific to the conjugate and are not reproduced with either of its individual components.

As a control, a mock combination was prepared and assessed for synergy by adding PSMA ADC to the assay plate via two separate additions. As expected, the mock combination did not show statistically significant synergy or antagonism in either cell line.

### Example 2: Antiandrogens reversibly increase PSMA expression in a time- and dose-dependent manner.

Additional studies examined treatment-induced changes in PSMA expression as a potential correlate of synergy. Flow cytometry and Western blotting were used to assess cell-surface and total PSMA, respectively.

Enzalutamide and abiraterone increased cell-surface levels of PSMA in a dose-dependent manner in both cell lines **(****FIG. 6A-D**). PSMA expression was approximately doubled at antiandrogens concentrations above 1 mM at 7 days post-treatment. In contrast, rapamycin increased PSMA expression in C4-2 cells only (**FIG. 6E-F**). Low nanomolar concentrations of rapamycin were sufficient to induce a >2-fold increase in PSMA expression in C4-2 cells.

To probe the dynamics of expression, C4-2 cells were cultured in enzalutamide (1 mM) for 3 weeks prior to continued culture in the absence of drug for an additional 8 days. Cell-surface PSMA was measured by flow cytometry. PSMA expression increased steadily over time in the presence of enzalutamide. After 21 days, PSMA expression on treated cells was nearly 4-fold greater than expression on untreated cells passaged in parallel. PSMA expression returned to baseline levels within 7 days of culture in the absence of enzalutamide (**FIG. 6G**).

Treatment-induced increases in PSMA were also apparent by Western blotting (**FIG. 7**). The magnitude of increase was approximately 5-fold for enzalutamide-treated LNCaP cells as determined by serial dilution of lysates and semi-quantitative analysis of the Western blots (data not shown). Enzalutamide, abiraterone and rapamycin upregulated PSMA to similar extents in C4-2 cells. The magnitude of PSMA expression in the presence of the antiandrogens approached but seldom exceeded the expression seen in cells cultured in charcoal-stripped serum. Overall, the flow cytometry and Western blot data showed consistent patterns of treatment-induced changes in PSMA expression.

### Example 3: Treatment-induced changes in AR, PSA and PI3K pathway components.

Levels of AR, PSA, total Akt, phospho-Akt (S473), total S6, and phospho-S6 were also evaluated pre- and post-treatment by Western blotting. Actin was probed as a measure of total cell loading. As expected, antiandrogen-induced upregulation of PSMA was accompanied by downregulation of PSA (**FIG. 7**). In contrast, rapamycin increased PSA expression. This result is consistent with prior observations for rapamycin or its analogs (28, 29). The co-upregulation of PSMA and PSA by rapamycin contrasts with the opposing effects on PSMA and PSA exerted by antiandrogens. AR protein levels were affected modestly by treatment (**FIG. 7**).

Akt activation was a common response to antiandrogen or rapamycin treatment of LNCaP cells. However, treatment had limited effects on Akt activation in C4-2 cells. Similarly, the antiandrogens increased levels of phospho-S6 in LNCaP but not C4-2 cells. These findings are consistent with an mTOR-mediated adaptation to antiandrogen treatment in LNCaP cells. Rapamycin-mediated activation of Akt is known to occur via disruption of a negative feedback loop involving insulin receptor substrate 1 (30-33). As expected, rapamycin effectively ablated S6 phosphorylation in both LNCaP and C4-2, indicating that rapamycin is pharmacologically active in both cell lines (**FIG. 7**).

### Example 5: Additive to weakly synergistic effects for other drug combinations.

In exploratory studies, an additional group of compounds was screened for activity alone and in combination with PSMA ADC. These agents include the Akt inhibitor MK-2206 (34); the PI3K inhibitor GDC-0941 (35); prednisone; and SB-743921 (36), an inhibitor of the kinesin spindle protein (ksp) Eg5. MK-2206 and GDC-0941 are selective inhibitors of upstream components of the PI3K/mTOR pathway. Prednisone is a component of abiraterone- and docetaxel-based treatment regimens in prostate cancer. SB-743931 was included in our study based on gene expression profiling within the cBio cancer genomics database (37), which revealed coordinated expression (P=0.000004) of the genes for PSMA and Eg5 within primary and metastatic prostate cancer tumors. Each of these compounds showed additive to weakly synergistic activity when combined with PSMA ADC (**FIG. 8**). That is, Bliss differences trended towards synergy for most conditions and reached statistical significance in a few isolated cases. No significant antagonism was observed for any combination. Similar results were obtained when prednisone was replaced with its active metabolite, prednisolone.

**Table 2** provides an overview of the synergies observed in this study. For each drug:drug combination, the breadth of synergy is represented as the percentage of evaluable conditions that resulted in statistically significant synergy across the matrix of drug concentrations. Combinations are sorted, highest to lowest, according to the breadth of synergy observed in LNCaP cells. The PSMA ADC/enzalutamide and PSMA ADC/abiraterone combinations showed the broadest synergy in LNCaP cells. Next were PSMA ADC combinations with prednisone or SB-743921. For the PSMA ADC/SB-743921 combination, the data should be interpreted with caution, since several drug-drug combinations resulted in >100% inhibition and thus were not evaluable for Bliss differences. Further testing of this combination over a more focused range of concentrations is warranted. Most combinations did not show significant synergy in LNCaP cells.

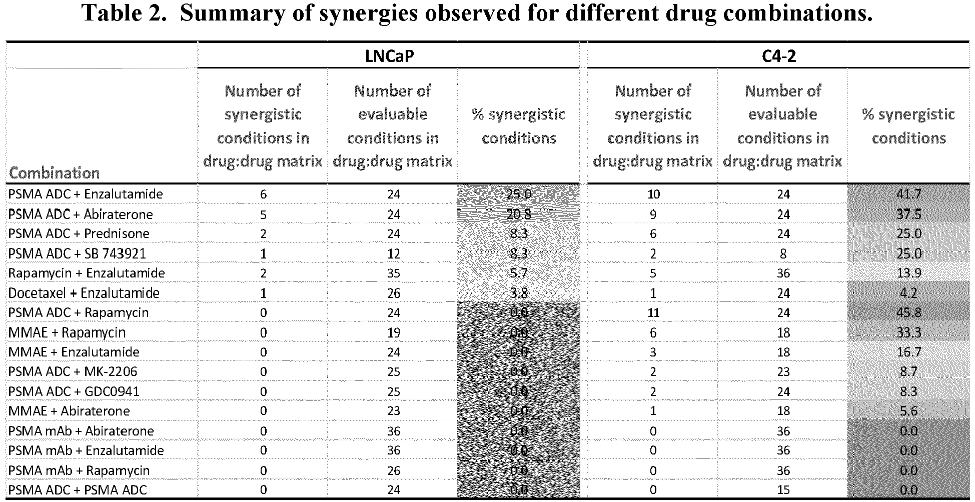

Synergy was more widespread in C4-2 cells. The PSMA ADC/enzalutamide and PSMA ADC/abiraterone combinations exhibited a high frequency of synergistic conditions in C4-2 cells, as did rapamycin in combinations with PSMA ADC or MMAE. Other combinations (e.g., PSMA ADC in combination with MK-2206 or GDC0941) also exhibited significant synergy in C4-2 but not LNCaP cells (**Table 2**). Combinations that did not show significant synergy in either LNCaP or C4-2 cells were those that involved PSMA mAb and the PSMA ADC mock combination.

### Materials and Methods

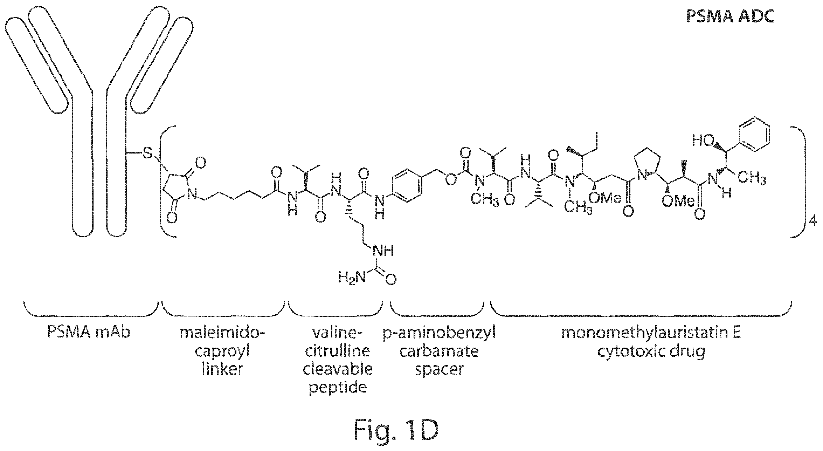

**Materials** LNCaP cells were obtained from American Type Culture Collection (ATCC). C4-2 is an androgen-independent subclone of LNCaP (22). LNCaP and C4-2 are characterized by a T877A mutation in AR and loss of PTEN expression (23, 24). The cell lines allow investigations of androgen dependence in an isogenic background. Both cell lines were passaged in RPMI1640 (Mediatech Inc.) supplemented with 2 mM L-glutamine, 10 mM HEPES, 1 mM sodium pyruvate, 100 µM non-essential amino acids, 1% penicillin/streptomycin and 10% fetal bovine serum (FBS, Life Technologies). Cells were drawn from a common bank and then used within 10 passages. PSMA ADC was prepared as described (20), and enzalutamide and abiraterone (parent drug) were obtained from MedKoo Biosciences (**FIG. 1**). Prednisone and prednisolone were purchased from Sigma; rapamycin was from EMD Millipore. GDC0941, MK-226 and SB-743921 were from Selleck Chemicals. The following antibodies were obtained from Santa Cruz Biotechnology: Anti-AR (sc-7305, 441) and anti-PSA (sc-7638, C-19). Two murine anti-PSMA mAbs were used, MAB544 (Maine Biotechnology) for Westerns and 3.9 (Progenies) for flow cytometry. The following antibodies were obtained from Cell Signaling: Total Akt (#2920S), phospho-Akt (S473, #4051L), total-S6 (#2317S), and phospho-S6 (#2211L). Anti-actin antibody (MAB1501) was from Millipore. Isotype-specific secondary IRDye antibodies were from LI-COR Biosciences.

**Cell viability assay** Cells were plated at a density of 1x10³ cells in 25 µL in 384-well white-colored microplates (Perkin Elmer) and cultured overnight. The next day, cells were treated with 1 or 2 drugs in a total volume of 50 µL. Cultures were incubated at 37°C for 7 days. Viability was assessed on days 0 and 7 using CellTiter-Glo (Promega) and an Analyst GT luminescence reader at 560nm emission (Molecular Devices). The percent inhibition of proliferation was calculated as (Vᵤ - Vₜ)/(Vᵤ - V₀) x 100, where Vᵤ, Vₜ and V₀ represent the viability values for untreated cells on day 7, treated cells on day 7, and cells prior to treatment on day 0, respectively. A value of 100% reflects complete inhibition of proliferation (Vt = V₀). Values of >100% reflect cytotoxic compounds or combinations, where Vₜ < V₀.

**Flow cytometry** For short-term treatments (≤7 days), cells were plated on 24-well plates (BD Falcon) overnight, at a density of 200,000 cells per 500 µL. The next day, wells were exchanged with 500 µL of fresh media that included inhibitor, and incubated at 37°C until the indicated time point. For the long-term treatments (>7 days), cells were plated on T-25 flasks (BD Falcon) overnight, at a density of 500,000 cells per flask in 5 mL of media. 5 mL of fresh media with or without treatment was exchanged the next day. On weekly splits thereafter, cells were detached with Cell Dissociation solution (Sigma), washed with fresh media, counted using Vi-CELL XR (Beckman Coulter), and split into two T-25 flasks for continued culture with or without inhibitor. Prior to analysis, cells were detached, counted and suspended in PBS (PBS(-), without Ca/Mg, Life Technologies) containing 0.3% bovine serum albumin (BSA, Sigma) and 0.1% sodium azide (VWR). Cells (100,000 in 100 µL) were placed in round-bottom 96-well plates (Falcon) and incubated for 30 minutes at room temperature with 1 µg/mL of anti-PSMA mAb 3.9 conjugated to phycoerythrin (PE). Cells then were washed twice with 200 µL PBS/BSA/azide buffer and read using a FACSCalibur instrument (BD Biosciences). A mouse IgG2b-PE conjugate of irrelevant specificity (Abcam) was included as an isotype control.

**Western blotting** Cells (300,000) were incubated overnight in 6-well plates (BD Falcon) in 4 mL of 10% FBS RPMI1640 media prior to addition of inhibitors. Plates were incubated at 37°C for an additional 7 days, washed once with 5 mL of cold PBS(-) and placed on ice. Cells were lysed with 130 µL of RIPA Lysis Buffer (Santa Cruz Biotechnology) and then centrifuged at 14,000 x g for 10 minutes at 4°C. The supernatant was quantitated for protein concentration using a BCA kit (Pierce/Thermo), resolved under reducing conditions using NuPAGE Novex 4-12% Bis-Tris gels (Life Technologies), and transferred to nitrocellulose using the iBlot 7-Minute Blotting System (Life Technologies). Membranes were blocked using Odyssey Blocking Reagent (LI-COR Biosciences), incubated with primary and secondary antibodies, and visualized using an Odyssey infrared imager (LI-COR Biosciences).

**Synergy calculations and statistical methods** Drug combinations were tested in 3 to 7 independent repeat cell viability assays unless otherwise indicated. Inhibition data were fit to a four-parameter logistic equation using GraphPad Prism. Potential non-additive effects were evaluated by the Bliss independence method (25-27). Briefly, the predicted Bliss value for the combination (F_{c}) was calculated as F_{c} = Fₐ + F_{b} - (Fₐ x F_{b}), where Fₐ and F_{b} represent the observed fractional growth inhibitions caused by compounds A and B used alone. Bliss differences were calculated by subtracting the predicted value (F_{c}) from the experimentally observed inhibition. Mean Bliss differences were calculated for each point in a matrix of drug-drug combinations, converted to percentages, and assessed for statistical significance from the null value of zero using two-sided t-tests with a significance level of 0.05. Statistical evaluations were not performed in cases where the fractional growth inhibition exceeded unity, since such values fall outside the Bliss framework. In addition, Bliss differences of less than 5% were considered to be of limited biological relevance and were excluded from synergy considerations.

### References

1. MacVicar GR, Hussain MH. Emerging therapies in metastatic castration-sensitive and castration-resistant prostate cancer. Curr Opin Oncol 2013;25:252-60.
2. Rathkopf D, Scher HI. Androgen receptor antagonists in castration-resistant prostate cancer. Cancer J 2013;19:43-9.
3. Ferraldeschi R, de Bono J. Agents that target androgen synthesis in castration-resistant prostate cancer. Cancer J 2013;19:34-42.
4. Richards J, Lim AC, Hay CW, Taylor AE, Wingate A, Nowakowska K, et al. Interactions of abiraterone, eplerenone, and prednisolone with wild-type and mutant androgen receptor: a rationale for increasing abiraterone exposure or combining with MDV3100. Cancer Res 2012;72:2176-82.
5. Soifer HS, Souleimanian N, Wu S, Voskresenskiy AM, Collak FK, Cinar B, et al. Direct regulation of androgen receptor activity by potent CYP17 inhibitors in prostate cancer cells. J Biol Chem 2012;287:3777-87.
6. Carver BS, Chapinski C, Wongvipat J, Hieronymus H, Chen Y, Chandarlapaty S, et al. Reciprocal feedback regulation of PI3K and androgen receptor signaling in PTEN-deficient prostate cancer. Cancer Cell 2011;19:575-86.
7. Thomas C, Lamoureux F, Crafter C, Davies BR, Beralidi E, Fazli L, et al. Synergistic targeting of PI3K/AKT-pathway and androgen-receptor axis significantly delays castration-resistant prostate cancer progression in vivo. Mol Cancer Ther 2013;Epub ahead of print.
8. Wright GL, Jr., Grob BM, Haley C, Grossman K, Newhall K, Petrylak D, et al. Upregulation of prostate-specific membrane antigen after androgen-deprivation therapy. Urology 1996;48:326-34.
9. Mostaghel EA, Page ST, Lin DW, Fazli L, Coleman IM, True LD, et al. Intraprostatic androgens and androgen-regulated gene expression persist after testosterone suppression: therapeutic implications for castration-resistant prostate cancer. Cancer Res 2007;67:5033-41.
10. Evans MJ, Smith-Jones PM, Wongvipat J, Navarro V, Kim S, Bander NH, et al. Noninvasive measurement of androgen receptor signaling with a positron-emitting radiopharmaceutical that targets prostate-specific membrane antigen. Proc Natl Acad Sci U S A 2011;108:9578-82.
11. Miyamoto DT, Lee RJ, Stott SL, Ting DT, Wittner BS, Ulman M, et al. Androgen receptor signaling in circulating tumor cells as a marker of hormonally responsive prostate cancer. Cancer Discov 2012;2:995-1003.
12. Foss CA, Mease RC, Cho SY, Kim HJ, Pomper MG. GCPII imaging and cancer. Curr Med Chem 2012;19:1346-59.
13. Olson WC, Heston WDW, Rajasekaran AK. Clinical trials of cancer therapies targeting prostate-specific membrane antigen. Reviews on Recent Clinical Trials 2007;2:182-90.
14. Perner S, Hofer MD, Kim R, Shah RB, Li H, Moller P, et al. Prostate-specific membrane antigen expression as a predictor of prostate cancer progression. Hum Pathol 2007;38:696-701.
15. Ross JS, Sheehan CE, Fisher HA, Kaufman RP, Jr., Kaur P, Gray K, et al. Correlation of primary tumor prostate-specific membrane antigen expression with disease recurrence in prostate cancer. Clin Cancer Res 2003;9:6357-62.
16. Silver DA, Pellicer I, Fair WR, Heston WD, Cordon-Cardo C. Prostate-specific membrane antigen expression in normal and malignant human tissues. Clin Cancer Res 1997;3:81-5.
17. Chang SS, Reuter VE, Heston WD, Bander NH, Grauer LS, Gaudin PB. Five different anti-prostate-specific membrane antigen (PSMA) antibodies confirm PSMA expression in tumor-associated neovasculature. Cancer Res 1999;59:3192-8.
18. Younes A, Bartlett NL, Leonard JP, Kennedy DA, Lynch CM, Sievers EL, et al. Brentuximab vedotin (SGN-35) for relapsed CD30-positive lymphomas. N Engl J Med 2010;363:1812-21.
19. Verma S, Miles D, Gianni L, Krop IE, Welslau M, Baselga J, et al. Trastuzumab emtansine for HER2-positive advanced breast cancer. N Engl J Med 2012;367:1783-91.
20. Ma D, Hopf C, Malewicz AD, Donovan GD, Senter PD, Goeckeler WF, et al. Potent antitumor activity of an auristatin-conjugated, fully human monoclonal antibody to prostate-specific membrane antigen. Clin Cancer Res 2006;12:2591-6.
21. Wang X, Ma D, Olson WC, Heston WD. In vitro and in vivo responses of advanced prostate tumors to PSMA ADC, an auristatin-conjugated antibody to prostate-specific membrane antigen. Mol Cancer Ther 2011;10:1728-39.
22. Wu HC, Hsieh JT, Gleave ME, Brown NM, Pathak S, Chung LW. Derivation of androgen-independent human LNCaP prostatic cancer cell sublines: role of bone stromal cells. Int J Cancer 1994;57:406-12.
23. Veldscholte J, Berrevoets CA, Ris-Stalpers C, Kuiper GG, Jenster G, Trapman J, et al. The androgen receptor in LNCaP cells contains a mutation in the ligand binding domain which affects steroid binding characteristics and response to antiandrogens. J Steroid Biochem Mol Biol 1992;41:665-9.
24. McMenamin ME, Soung P, Perera S, Kaplan I, Loda M, Sellers WR. Loss of PTEN expression in paraffin-embedded primary prostate cancer correlates with high Gleason score and advanced stage. Cancer Res 1999;59:4291-6.
25. Bliss CI. The toxicity of poisons applied jointly. Ann Appl Biol 1939;26:585-615.
26. Wallin JJ, Guan J, Prior WW, Lee LB, Berry L, Belmont LD, et al. GDC-0941, a novel class I selective PI3K inhibitor, enhances the efficacy of docetaxel in human breast cancer models by increasing cell death in vitro and in vivo. Clin Cancer Res 2012;18:3901-11.
27. Wong M, Tan N, Zha J, Peale FV, Yue P, Fairbrother WJ, et al. Navitoclax (ABT-263) reduces Bcl-x(L)-mediated chemoresistance in ovarian cancer models. Mol Cancer Ther 2012;11:1026-35.
28. Squillace RM, Miller D, Wardwell SD, Wang F, Clackson T, Rivera VM. Synergistic activity of the mTOR inhibitor ridaforolimus and the antiandrogen bicalutamide in prostate cancer models. Int J Oncol 2012;41:425-32.
29. Wang Y, Mikhailova M, Bose S, Pan CX, deVere White RW, Ghosh PM. Regulation of androgen receptor transcriptional activity by rapamycin in prostate cancer cell proliferation and survival. Oncogene 2008;27:7106-17.
30. Carracedo A, Ma L, Teruya-Feldstein J, Rojo F, Salmena L, Alimonti A, et al. Inhibition of mTORC1 leads to MAPK pathway activation through a PI3K-dependent feedback loop in human cancer. J Clin Invest 2008;118:3065-74.
31. O'Reilly KE, Rojo F, She QB, Solit D, Mills GB, Smith D, et al. mTOR inhibition induces upstream receptor tyrosine kinase signaling and activates Akt. Cancer Res 2006;66: 1500-8.
32. Sarbassov DD, Guertin DA, Ali SM, Sabatini DM. Phosphorylation and regulation of Akt/PKB by the rictor-mTOR complex. Science 2005;307:1098-101.
33. Easton JB, Kurmasheva RT, Houghton PJ. IRS-1: auditing the effectiveness of mTOR inhibitors. Cancer Cell 2006;9:153-5.
34. Hirai H, Sootome H, Nakatsuru Y, Miyama K, Taguchi S, Tsujioka K, et al. MK-2206, an allosteric Akt inhibitor, enhances antitumor efficacy by standard chemotherapeutic agents or molecular targeted drugs in vitro and in vivo. Mol Cancer Ther 2010;9:1956-67.
35. Folkes AJ, Ahmadi K, Alderton WK, Alix S, Baker SJ, Box G, et al. The identification of 2-(1H-indazol-4-yl)-6-(4-methanesulfonyl-piperazin-1-ylmethyl)-4-morpholin-4-yl-t hieno[3,2-d]pyrimidine (GDC-0941) as a potent, selective, orally bioavailable inhibitor of class I PI3 kinase for the treatment of cancer. J Med Chem 2008;51:5522-32.
36. Good JA, Wang F, Rath O, Kaan HY, Talapatra SK, Podgorski D, et al. Optimized S-trityl-L-cysteine-based inhibitors of kinesin spindle protein with potent in vivo antitumor activity in lung cancer xenograft models. J Med Chem 2013;56:1878-93.
37. Cerami E, Gao J, Dogrusoz U, Gross BE, Sumer SO, Aksoy BA, et al. The cBio cancer genomics portal: an open platform for exploring multidimensional cancer genomics data. Cancer Discov 2012;2:401-4.
38. Zhu ML, Horbinski CM, Garzotto M, Qian DZ, Beer TM, Kyprianou N. Tubulintargeting chemotherapy impairs androgen receptor activity in prostate cancer. Cancer Res 2010;70:7992-8002.
39. Darshan MS, Loftus MS, Thadani-Mulero M, Levy BP, Escuin D, Zhou XK, et al. Taxane-induced blockade to nuclear accumulation of the androgen receptor predicts clinical responses in metastatic prostate cancer. Cancer Res 2011;71:6019-29.
40. Dang ZC, Lowik CW. Removal of serum factors by charcoal treatment promotes adipogenesis via a MAPK-dependent pathway. Mol Cell Biochem 2005;268:159-67.
41. Tran C, Ouk S, Clegg NJ, Chen Y, Watson PA, Arora V, et al. Development of a second-generation antiandrogen for treatment of advanced prostate cancer. Science 2009;324:787-90.
42. Clegg NJ, Wongvipat J, Joseph JD, Tran C, Ouk S, Dilhas A, et al. ARN-509: a novel antiandrogen for prostate cancer treatment. Cancer Res 2012;72:1494-503.
43. Joseph JD, Lu N, Qian J, Sensintaffar J, Shao G, Brigham D, et al. A clinically relevant androgen receptor mutation confers resistance to second-generation antiandrogens enzalutamide and ARN-509. Cancer Discov 2013;3:1020-9.
44. Chen CD, Welsbie DS, Tran C, Baek SH, Chen R, Vessella R, et al. Molecular determinants of resistance to antiandrogen therapy. Nat Med 2004;10:33-9.
45. Pienta KJ, Abate-Shen C, Agus DB, Attar RM, Chung LW, Greenberg NM, et al. The current state of preclinical prostate cancer animal models. Prostate 2008;68:629-39.
46. Garcia JA, Danielpour D. Mammalian target of rapamycin inhibition as a therapeutic strategy in the management of urologic malignancies. Mol Cancer Ther 2008;7:1347-54.
47. Sarker D, Reid AH, Yap TA, de Bono JS. Targeting the PI3K/AKT pathway for the treatment of prostate cancer. Clin Cancer Res 2009;15:4799-805.
48. Bitting RL, Armstrong AJ. Targeting the PI3K/Akt/mTOR pathway in castration-resistant prostate cancer. Endocr Relat Cancer 2013;20:R83-R99.
49. Zhao L, Au JL, Wientjes MG. Comparison of methods for evaluating drug-drug interaction. Front Biosci (Elite Ed) 2010;2:241-9.

## Claims

1. An *in vitro* method of inhibiting proliferation of prostate-specific membrane antigen (PSMA)-expressing cancer cells, the method comprising:
contacting PSMA-expressing cancer cells with an antiandrogen, wherein the antiandrogen is an androgen receptor antagonist; and
contacting the PSMA-expressing cancer cells with a PSMA ligand-anticancer agent conjugate or a PSMA ligand-cytotoxic agent conjugate.

2. An antiandrogen, wherein the antiandrogen is an androgen receptor antagonist, for use in an *in vivo* method of inhibiting proliferation of prostate-specific membrane antigen (PSMA)-expressing cancer cells in a subject, the method comprising:
contacting PSMA-expressing cancer cells with the antiandrogen; and
contacting the PSMA-expressing cancer cells with a PSMA ligand-anticancer agent conjugate or a PSMA ligand-cytotoxic agent conjugate.

3. A PSMA ligand-anticancer agent conjugate or a PSMA ligand-cytotoxic agent conjugate for use in an *in vivo* method of inhibiting proliferation of prostate-specific membrane antigen (PSMA)-expressing cancer cells in a subject, the method comprising:
contacting PSMA-expressing cancer cells with an antiandrogen, wherein the antiandrogen is an androgen receptor antagonist; and
contacting the PSMA-expressing cancer cells with the PSMA ligand-anticancer agent conjugate or the PSMA ligand-cytotoxic agent conjugate.

4. A composition comprising an antiandrogen and a PSMA ligand-anticancer agent conjugate or PSMA ligand-cytotoxic agent conjugate, for use in an *in vivo* method of inhibiting proliferation of prostate-specific membrane antigen (PSMA)-expressing cancer cells, wherein the antiandrogen is an androgen receptor antagonist.

5. A kit comprising:
a container containing antiandrogen, wherein the antiandrogen is an androgen receptor antagonist; and
a container containing a PSMA ligand-anticancer agent conjugate or PSMA ligand-cytotoxic agent conjugate;
optionally wherein the antiandrogen and/or the PSMA ligand-anticancer agent conjugate or PSMA ligand-cytotoxic agent conjugate is in aqueous medium or is lyophilized; and
optionally further comprising a pharmaceutically acceptable carrier and/or an excipient and/or a diluent.

6. The method of claim 1, the compounds for use of claims 2 or 3, the composition for use of claim 4, or the kit of claim 5, wherein the PSMA ligand of the conjugate comprises an antibody, or antigen-binding fragment thereof, that binds specifically PSMA.

7. The method, compounds for use, composition for use or kit of claim 6, wherein:
a) the antibody or antigen-binding fragment thereof is an antibody selected from the group consisting of: PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1 and Abgenix 4.152.1, or an antigen-binding fragment thereof;
b) the antibody or antigen-binding fragment thereof comprises (i) the three complementarity determining regions of a heavy chain variable region comprising an amino acid sequence set forth as SEQ ID NO:15 and (ii) the three complementarity determining regions of a light chain variable region comprising an amino acid sequence set forth as SEQ ID NO: 17;
c) the antibody or antigen-binding fragment thereof comprises (i) the three complementarity determining regions of a heavy chain variable region comprising an amino acid sequence set forth as SEQ ID NO:19 and (ii) the three complementarity determining regions of a light chain variable region comprising an amino acid sequence set forth as SEQ ID NO: 21;
d) the antibody or antigen-binding fragment thereof comprises (i) the three complementarity determining regions of a heavy chain variable region comprising an amino acid sequence set forth as SEQ ID NO:23 and (ii) the three complementarity determining regions of a light chain variable region comprising an amino acid sequence set forth as SEQ ID NO: 25;
e) the antibody or antigen-binding fragment thereof comprises (i) the three complementarity determining regions of a heavy chain variable region comprising an amino acid sequence set forth as SEQ ID NO:27 and (ii) the three complementarity determining regions of a light chain variable region comprising an amino acid sequence set forth as SEQ ID NO: 29;
f) the antibody or antigen-binding fragment thereof comprises (i) the three complementarity determining regions of a heavy chain variable region comprising an amino acid sequence set forth as SEQ ID NO: 31 and (ii) the three complementarity determining regions of a light chain variable region comprising an amino acid sequence set forth as SEQ ID NO: 33;
g) the antibody or antigen-binding fragment thereof is antibody PSMA 10.3, AB-PG1-XG1-006, AB-PG1-XG1-026, AB-PG1-XG1-051, AB-PG1-XG1-069, AB-PG1-XG1-077, or an antigen-binding fragment thereof;
h) the antibody or antigen-binding fragment thereof is antibody E99, J415, J533 or J591 or an antibody produced by a hybridoma having ATCC Accession Number HB-12101, HB-12109, HB-12127or HB-12126, or an antigen -binding fragment thereof; or
i) the antibody or antigen-binding fragment thereof is an antibody produced by a hybidoma having ATCC Accession Number HB12060 (3F5.4G6), HB12309 (3D7-1.1), HB12310 (4E10-1.14), HB12489 (1G3), HB12495 (1G9), HB12490 (2C7), HB12494 (3C4), HB12491 (3C6), HB12484 (3C9), HB12486 (3E6), HB12488 (3E11), HB12485 (3G6), HB 12493 (4D4), HB 12487 (4D8), HB 12492 (4C8B9), HB 12664 (3F6), HB 12678 (2E4), HB12665 (3C2), HB12672 (2D4), HB12660 (4C8G8), HB12675 (2C4), HB12663 (4C11), HB12661 (1D11), HB12667 (4E8), HB12674 (2G5), HB12620 (4E6), HB12677 (1F4), HB12666 (2E3), HB12662 (3D8), HB12668 (4F8), HB12673 (3D2), HB12676 (1G7), HB12669 (3D4), HB12679 (5G10) or HB12671 (5E9), or an antigen-binding fragment thereof.

8. The method of claim 1, the compounds for use of claims 2 or 3, the composition for use of claim 4, or the kit of claim 5, wherein the PSMA ligand of the conjugate comprises a small molecule ligand that binds specifically PSMA, optionally wherein the small molecule ligand binds an enzymatic site on PSMA.

9. The method, compounds for use, composition for use or kit of claim 8, wherein:
i) the small molecule ligand comprises MIP-1095, MIP-1072, GL2 or DUPA; or
ii) the PSMA ligand-anticancer agent conjugate comprises MIP-1095 or MIP-1072 conjugated to a cytotoxic radionuclide selected from the group consisting of I¹²³, I¹²⁵, I¹³¹, I¹²⁴ Br⁷⁵, Br⁷⁷ and F¹⁸.

10. The method of any one of claims 1 or 6 to 9, the compounds for use of claims 2, 3 or 6 to 9, the composition for use of claims 4 or 6 to 9, or the kit of claims 5 to 9, wherein the anticancer agent comprises an auristatin, tubulysin, a pyrrolobenzodiazepine dimer, calicheamicin, colchicine, ispinesib, combrestatin A4, maytansinoid DM1, maytansinoid DM4, doxorubicin, or a cytotoxic radionuclide, optionally wherein the auristatin comprises monomethylauristatin norephedrine or monomethylauristatin phenylalanine, optionally wherein the PSMA ligand-anticancer agent conjugate comprises EC1069 or EC1719.

11. The method of claim 1, compounds for use of claims 2 or 3, composition for use of claim 4 or kit of claim 5, wherein the PSMA ligand-anticancer agent conjugate comprises BIND-014.

12. The method of any one of claims 1 or 6 to 11, the compounds for use of claims 2, 3 or 6 to 11, the composition for use of claims 4 or 6 to 11, or the kit of claims 5 to 11, wherein the androgen receptor antagonist increases cell surface expression of PSMA in PSMA-expressing cells.

13. The method of any one of claims 1 or 6 to 12, the compounds for use of any one of claims 2, 3 or 6 to 12, the composition for use of any one of claims 4 or 6 to 12, or the kit of claims 5 to 12, wherein the androgen receptor antagonist is abiraterone, enzalutamide, nilutamide, flutamide, bicalutamide, ARN 509, galeterone or orteronel.

14. The method of any one of claims 1 or 5 to 13, or the compounds for use of any one of claims 2, 3 or 5 to 13, wherein the step of contacting the PSMA-expressing cancer cells with the antiandrogen and the step of contacting the PSMA-expressing cancer cells with the PSMA ligand-anticancer agent conjugate or the PSMA ligand-cytotoxic agent conjugate are concurrent, or wherein the step of contacting the PSMA-expressing cancer cells with the antiandrogen and the step of contacting the PSMA-expressing cancer cells with the PSMA ligand-anticancer agent conjugate or the PSMA ligand-cytotoxic agent conjugate are sequential.

15. The method of any one of claims 1 or 6 to 14, the compounds for use of any one of claims 2, 3 or 6 to 14, or the composition for use of any one of claims 4 or 6 to 13, wherein the PSMA-expressing cancer cells:
a) comprise androgen-independent PSMA-expressing cancer cells;
b) comprise PSMA-expressing cancer cells insensitive to antiandrogen therapy;
c) are of a tumor;
d) are PSMA-expressing prostate cancer cells; and/or
e) are PSMA-expressing non-prostate cancer cells.

16. The method of claim 1 or 6 to 15, the compounds for use of any one of claims 2, 3 or 6 to 15, the composition for use of any one of claims 4 or 6 to 13 or 15, or the kit of claims 5 to 13, wherein the androgen receptor antagonist is enzalutamide, abiraterone or ARN 509, and the PSMA ligand-anticancer agent conjugate is a PSMA ADC.

17. The method, the compounds for use, the composition for use or the kit of claim 8, wherein the small molecule ligand comprises a glutamate moiety, a urea moiety and an amino acid moiety, preferably wherein the amino acid moiety is a lysine or a glutamate.

18. The method of claim 1, the compounds for use of claims 2 or 3, the composition for use of claim 4, or the kit of claim 5, wherein the PSMA ligand-anticancer agent conjugate or the PSMA ligand-cytotoxic agent conjugate comprises a cytotoxic radionuclide.

19. The method, the compounds for use, the composition for use, or the kit of claim 18, wherein:
i) the cytotoxic radionuclide emits Auger and/or low energy electrons;
ii) the cytotoxic radionuclide emits Auger and/or low energy electrons and the cytotoxic radionuclide comprises ¹²³I, ¹²⁴I, ¹²⁵I, ¹³¹I, ⁷⁵Br, ⁷⁷Br and ¹⁸F;
iii) the cytotoxic radionuclide comprises an alpha-emitting isotope;
iv) the cytotoxic radionuclide comprises an alpha-emitting isotope and the cytotoxic radionuclide comprises ²²⁵Ac, ²¹¹At, ²¹²Bi, ²¹³Bi, ²¹²Pb, ²²⁴Ra, ²²³Ra;
v) the cytotoxic radionuclide comprises a beta-emitting isotope; or
vi) the cytotoxic radionuclide comprises a beta-emitting isotope and the cytotoxic radionuclide comprises ¹⁸⁶Rh, ¹⁸⁸Rh, ¹⁷⁷Lu, ⁹⁰Y, ¹³¹I, ⁶⁷Cu, ⁶⁴Cu, ¹⁵³Sm, ¹⁶⁶Ho.
